# EUROPEAN PATENT SPECIFICATION

(11) **EP 3 897 848 B1**
(45) Date of publication and mention of the grant of the patent: **11.10.2023**
(21) Application number: 19831816.4
(22) Date of filing: 19.12.2019
(51) Int. Cl.: A61K 31/496, A61P 35/00, A61K 31/416, A61K 31/437, C12Q 1/48

(54) **CANCER TREATMENTS**
KREBSBEHANDLUNGEN
TRAITEMENTS DU CANCER

(30) Priority: 19.12.2018 GB 201820660
(43) Date of publication of application: 27.10.2021
(73) Proprietor: Imperial College Innovations Limited, London SW7 2AZ (GB)
(72) Inventor: TATE, Edward William, London W12 0BZ (GB); FARONATO, Monica, London W12 0BZ (GB); CALADO, Dinis, London NW1 1AT (GB); LUEG, Gregor, London NW1 1AT (GB)
(74) Representative: Sagittarius IP
(86) International application number: PCT/GB2019/053615
(87) International publication number: WO 2020/128475

(56) References cited:
- WO-A1-2014/067002
- WO-A1-2017/011907
- M. GABAY ET AL: "MYC Activation Is a Hallmark of Cancer Initiation and Maintenance", COLD SPRING HARBOR PERSPECTIVES IN MEDICINE, vol. 4, no. 6, 1 June 2014 (2014-06-01), pages a014241-a014241, XP55669248, DOI: 10.1101/cshperspect.a014241

## Description

### INTRODUCTION

The present invention relates to compounds which display activity as inhibitors of the human *N*-myristoyl transferases (NMT) for use in the treatment of diffuse large B-cell lymphoma in a subject who has been identified as benefiting from being administered a NMT inhibitor. The present invention also relates to compounds for use as aforesaid in combination with one or more other therapeutic agents.

### BACKGROUND OF THE INVENTION

Hyperproliferative disorders, such as cancer, typically evolve though a multistage process, in which the growth and proliferation of the cancerous cell is driven by several progressive genetic mutations and epigenetic abnormalities (Weinstein et al., Cancer Res., 2008, 68(9), 3077- 3080). This multifaceted mode of action and, in particular, the simultaneous activation of oncogenes and deactivation of tumour surpressor genes makes the task of developing effective cancer treatments extremely difficult (Sharma et al., Genes and Development, 2007, 21, 3214-3231). In fact, cancer is often described as a "moving target" due to its progressive nature.

Many forms of cancer, such as, for example, myelomas, lymphomas, leukeamias, neuroblastomas and certain solid tumours (e.g. breast cancers), can be extremely complex and challenging to treat. Remissions and relapses as a result of resistance to chemotherapy in subjects can be fatal, and survival times for subjects who develop a resistance to common forms of chemotherapy are typically very short. There therefore remains a need for new and improved methods for treating cancers, together with improved methods for enhancing the efficacy of current chemotherapies, particularly in those subjects resistant, refractory, or otherwise not responsive to treatment with such chemotherapies.

"Oncogene addiction", first coined by Bernard Weinstein in 2008, is a term used to describe the absolute dependence of some cancers on one or only a few genes for maintaining a malignant phenotype, and this is an area of research currently being explored for its prospects in providing new and effective cancer treatments (Weinstein et al., Cancer Res., 2008, 68(9), 3077- 3080). Within this particular field of research, the identification of "transcriptionally addicted" cancers, which are cancers having an absolute reliance on certain transcriptional factors, has recently yielded some promising leads for new treatments (Bradner et al., Cell, 2017, 168, 629-643). One family of transcriptional factors of particular interest is that of the *MYC* regulator genes.

*MYC* regulator genes encode a family of transcription factors involved in cell proliferation, growth, differentiation and apoptosis. Members of the *MYC* transcription factor family include *c-MYC, MYCN* and *MYCL,* sometimes referred to as *c-Myc, N-Myc* and *L-Myc,* (Kalkat, et al., 2013, Regulation and Function of the MYC Oncogene, John Wiley & Sons Ltd). Activation of normal *MYC* genes affects numerous cellular processes, including cell cycle progression, cell growth and division, metabolism, telomerase activity, adhesion and motility, angiogenesis and differentiation. *MYC,* has further been identified as a strong proto-oncogene and its mutated versions are often found to be upregulated and/or constitutively expressed in certain types of cancers, such as haematological cancers and solid tumour malignancies (Miller et al., Clin. Cancer Res., 2012, 18(20), 5546-5553). As an example, *c-MYC* overexpression has been observed in up to 30% of cases of diffuse large B-cell lymphoma (DLBCL), the most common type of aggressive lymphoma (Chisholm et al., Am. J. Surg. Pathol., 2015, 39(3), 294-303). Furthermore, around 15% of breast tumours have been shown to be overexpressant in *MYC* (Xu et al., Genes and Cancer, 2010, 1(6), 629-640), and, further still, around 20% of human renal cell adenocarcinomas (RCC) have been idenitifed as overexpressing in *MYC* (Shroff et al., Cell, 2015, 112(21), 6539-6544). Moreover, mutations and/or copy number gains of the *MYC* gene are observed in roughly 28% of all cancers listed in "The Cancer Genone Atlas" (TCGA) database (Schaub et al., Cell Systems, 2018, 6, 282-300).

WO2014/067002 (Pacylex Pharmaceuticals Inc.) discloses a method of treating a subject having a cancer deficient in NMT2, comprising: administering to said subject an NMT inhibitor.

However, despite the attractiveness of targeting the *MYC* oncogene and exploiting the vulnerability of *MYC* addicition shown by specific cancers, the development of effective treatments for *MYC* addicted cancers has been slow, and currently no drugs or methods of treatment that directly target *MYC* overexpressed cancers have been approved for use.

Thus, there remains a need for new strategies and treatments which are able to treat patients with transcriptionally addicted cancers and, in particular, *MYC* addicted cancers.

The present invention was devised with the foregoing in mind.

### SUMMARY OF THE INVENTION

According to a first aspect of the present invention, there is provided a NMT inhibitor for use in the treatment of diffuse large B-cell lymphoma in a subject who has been identified as benefiting from being administered a NMT inhibitor by a method which comprises administering a therapeutically effective amount of a NMT inhibitor, or a pharmaceutically acceptable salt, solvate or hydrate thereof to the subject, wherein said subject has been identified as benefiting from being administered a NMT inhibitor as determined by a method comprising the steps of:
i) taking a sample of diffuse large B-cell lymphoma cells taken from said subject;
ii) analysing the cells of step i) to check for the presence of one or more structural alterations in the *MYC* locus (e.g. chromosomal rearrangements, copy number gains and/or mutations of the *MYC* oncogene);
iii) determining whether one or more structural alterations (e.g. chromosomal rearrangements, copy number gains and/or mutations) are present in the *MYC* locus of the sample of diffuse large B-cell lymphoma cells when compared to a control; and
iv) determining whether the subject will benefit from being administered a NMT inhibitor in order to treat said diffuse large B-cell lymphoma, wherein if the sample of diffuse large B-cell lymphoma cells contain one or more structural alterations (e.g. chromosomal rearrangements, copy number gains and/or mutations) in the *MYC* locus, then the subject will benefit from being administered a NMT inhibitor, and if the sample of diffuse large B-cell lymphoma cells do not contain one or more structural alterations (e.g. chromosomal rearrangements, copy number gains and/or mutations) in the *MYC* locus, then the subject will not benefit from being administered a NMT inhibitor.

There is also provided a NMT inhibitor or a pharmaceutically acceptable salt, solvate or hydrate thereof, for use as described above wherein the one or more structural alterations are selected from mutations, copy-number gains and/or chromosomal rearrangements.

According to a further aspect of the present invention, there is provided a NMT inhibitor, or a pharmaceutically acceptable salt, solvate or hydrate thereof, for use as described above, wherein the one or more structural alterations are mutations.

According to a further aspect of the present invention, there is provided a NMT inhibitor for use in the treatment of diffuse large B-cell lymphoma in a subject who has been identified as benefiting from being administered a NMT inhibitor by a method which comprises administering a therapeutically effective amount of a NMT inhibitor, or a pharmaceutically acceptable salt, solvate or hydrate thereof to the subject, wherein said subject has been identified as benefiting from being administered a NMT inhibitor as determined by a method comprising the steps of:
i) measuring the level of MYC expression in a sample of diffuse large B-cell lymphoma cells taken from said subject;
ii) comparing the level of MYC expression from step i) with a control;
iii) determining whether the MYC expression in the sample of diffuse large B-cell lymphoma cells is increased compared to the control; and
iv) determining whether the subject will benefit from being administered a NMT inhibitor in order to treat said diffuse large B-cell lymphoma, wherein if the MYC expression in the sample of diffuse large B-cell lymphoma cells is higher than in the control, then the subject will benefit from being administered a NMT inhibitor, and if the MYC expression in the sample of diffuse large B-cell lymphoma cells is not higher than in the control, then the subject will not benefit from being administered a NMT inhibitor.

According to a further aspect of the present invention, there is provided a NMT inhibitor which is a compound of Formula I shown below, or a pharmaceutically acceptable salt, hydrate or solvate thereof: 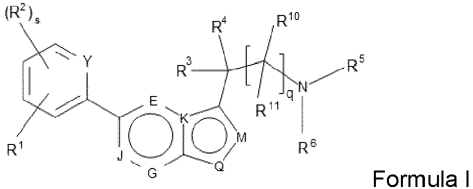 wherein all variables shown in Formula I are as defined elsewhere herein.

According to a further aspect of the present invention, there is provided a NMT inhibitor which is a compound of Formula (IA^^) shown below, or a pharmaceutically acceptable salt, hydrate or solvate thereof: 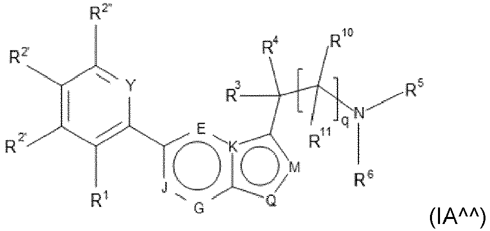 wherein all variables shown in Formula (IA^^) are as defined elsewhere herein.

According to a further aspect of the present invention, there is provided a NMT inhibitor which is a compound of Formula (II) shown below, or a pharmaceutically acceptable salt, hydrate or solvate thereof: 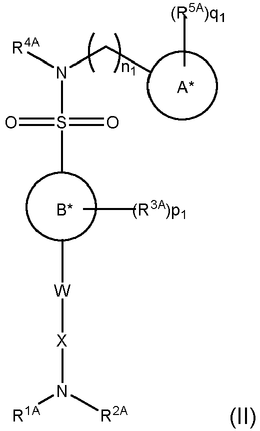 wherein all variables shown in Formula (II) are as defined elsewhere herein.

According to a further aspect of the present invention, there is provided a NMT inhibitor which is a compound of Formula (III) shown below, or a pharmaceutically acceptable salt, hydrate or solvate thereof: 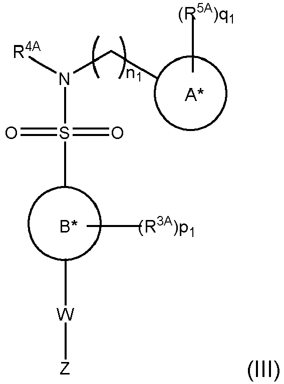 wherein all variables shown in Formula (III) are as defined elsewhere herein.

According to a further aspect of the present invention, there is provided a NMT inhibitor, or a pharmaceutically acceptable salt, solvate or hydrate thereof, for use as described above in combination with one or more other therapeutic agents.

### BRIEF DESCRIPTION OF THE DRAWINGS

Figure 1 shows the representative flow cytometry analysis for the expression of c-MYC and the cell size in the P-493-6 cell line.
Figure 2 shows the representative flow cytometry analysis of EdU incorporation, clicked to an AlexaFluor 488 fluorochrom.
Figure 3 shows the quantification of the relative proportion of cells that are EdU positive and the cell number in a 100 uL of cell suspension.
Figure 4 shows metabolic viability of the P-493-6 cell line treated with Compound 2 at the shown concentrations for 48 hours and 72 hours, with different expression levels of c-MYC induced.
Figure 5 shows the metabolic viability of the P-493-6 cell line treated with Compound 6, Compound 5 and Compound 1 at the shown concentrations for 72 hours, with different expression levels of c-MYC induced.
Figure 6 shows the cell quantification data for the P-493-6 cell line with high c-MYC expression.
Figure 7 shows the cell quantification data for the P-493-6 cell line with medium c-MYC expression.
Figure 8 shows the cell quantification data for the P-493-6 cell line with low c-MYC expression.

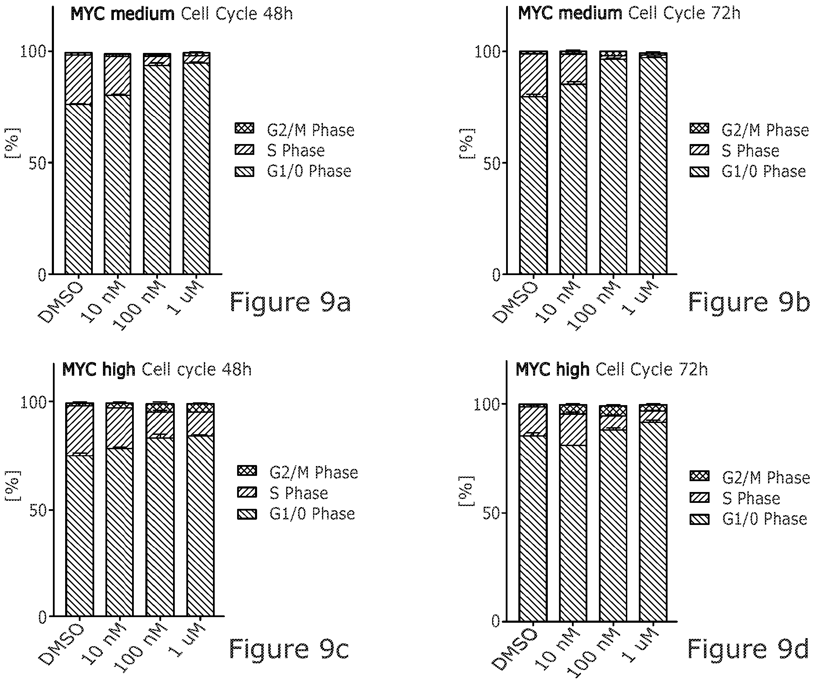

Figure 9 shows the cell cycle quantification for the P-493-6 cell line at 48 hours and 72 hours with medium and high c-MYC expression.
Figure 10 shows the representative western blot showing MYCN protein expression following time course treatment with 100 nM of Tamoxifen in the SKNAS-ER-MYCN and Shep-ER-MYCN cell lines.
Figure 11 shows the representative flow cytometric analysis of the cell cycle profile for the Shep-ER-MYCN cell line, with and without induction of *MYCN.*
Figure 12 shows the representative flow cytometric analysis of the cell cycle profile for the SKNAS-ER-MYCN cell line, with and without induction of *MYCN.*
Figure 13 shows the representative flow cytometric analysis of the protein synthesis, via incorporation of OPP, of the Shep-ER-MYCN and SKNAS-ER-MYCN cell lines with or without induction of *MYCN.*

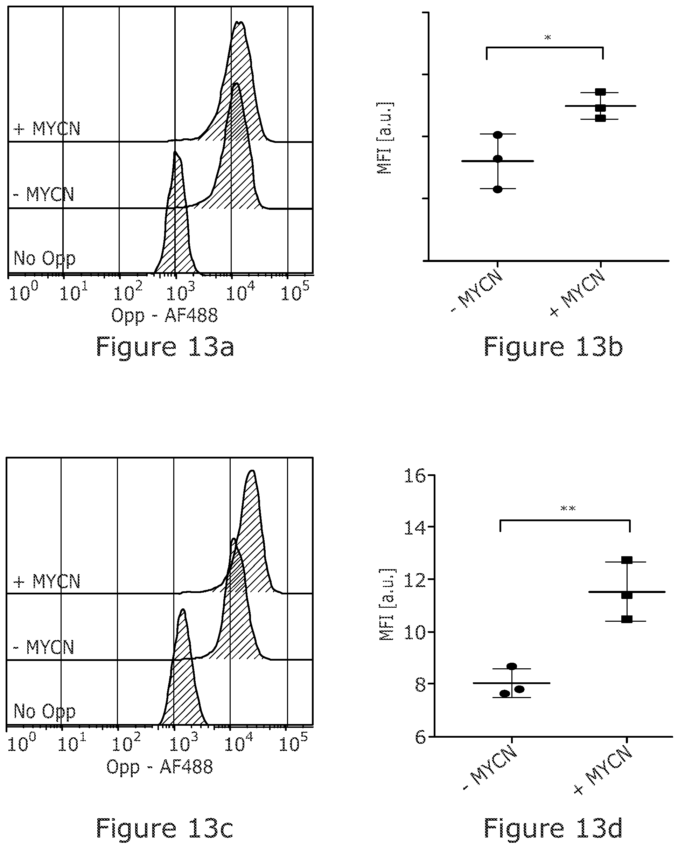

Figure 13 shows the metabolic viability of the Shep-ER-MYCN and SKNAS-ER-MYCN cell lines treated with Compound 2 at the shown concentrations for 48 hours and 72 hours, with and without induction of *MYCN.*

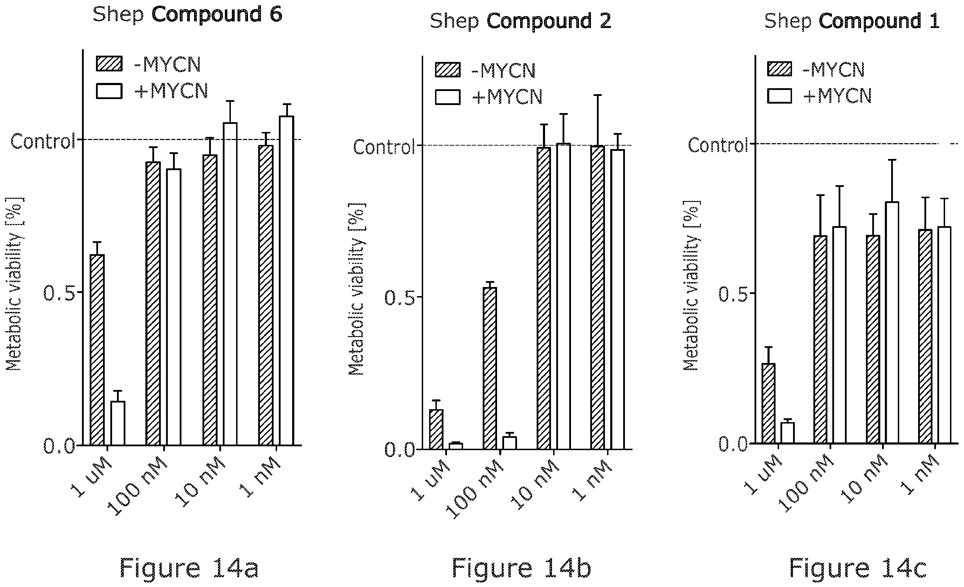

Figure 14 shows the metabolic viability of the Shep-ER-MYCN cell line treated with Compounds 1, 2 and 6 at the shown concentrations for 72 hours, with and without induction of *MYCN.*

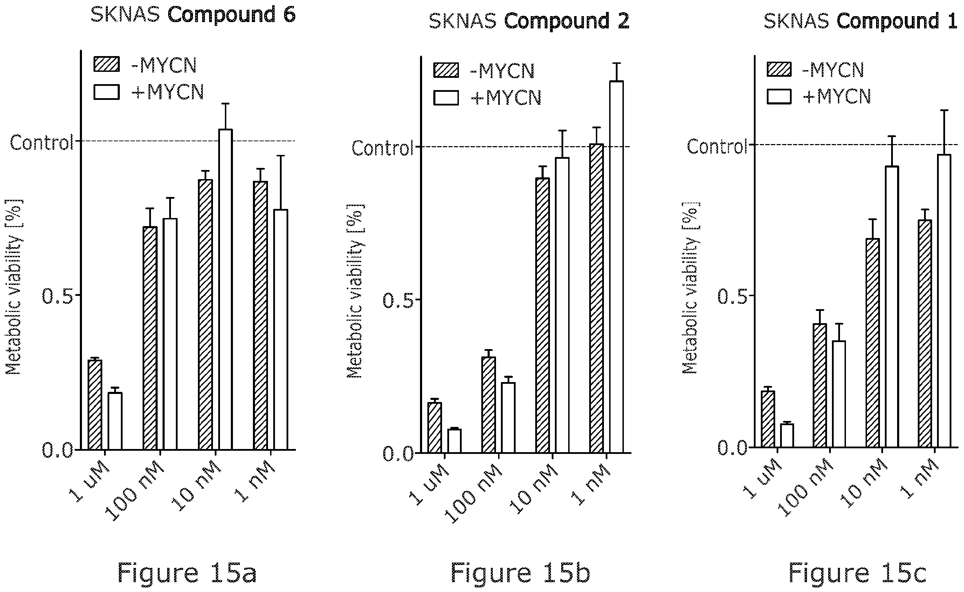

Figure 15 shows the metabolic viability of the SKNAS-ER-MYCN cell line treated with Compounds 1, 2 and 6 at the shown concentrations for 92 hours, with and without induction of *MYCN.*

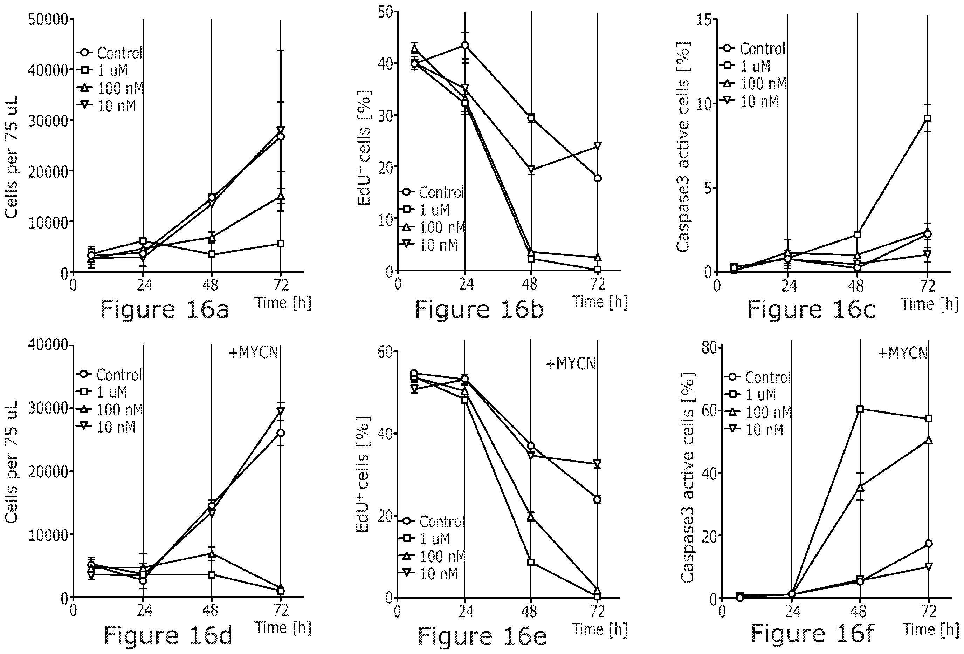

Figure 16 shows the cell quantification data for the Shep-ER-MYCN cell line without and with *MYCN* induction upon administration of Compound 2.

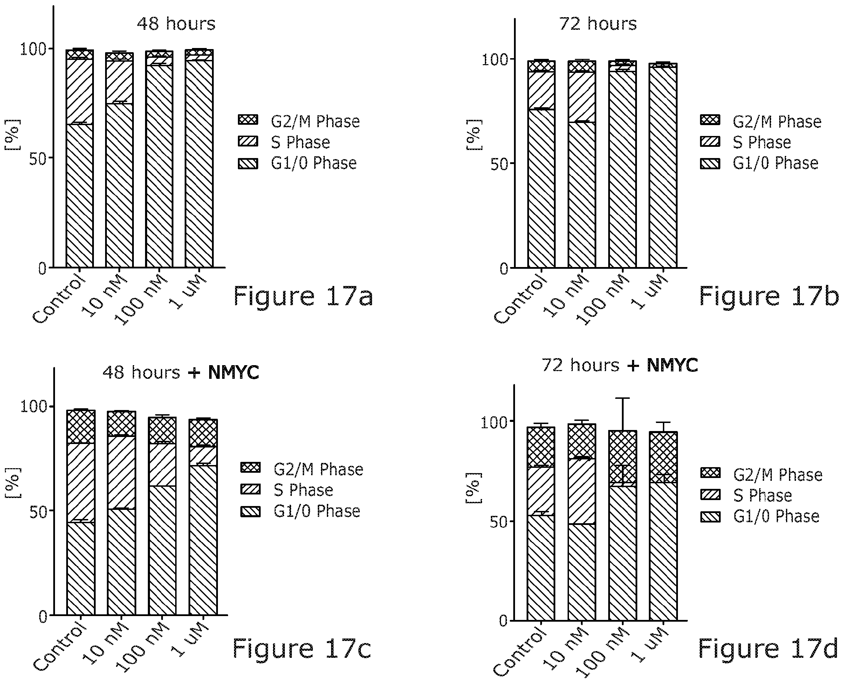

Figure 17 shows the quantification of the cell cycle profile of the Shep-ER-MYCN cell line with and without induction of *MYCN* upon administration of Compound 2 at 48 and 72 hours.

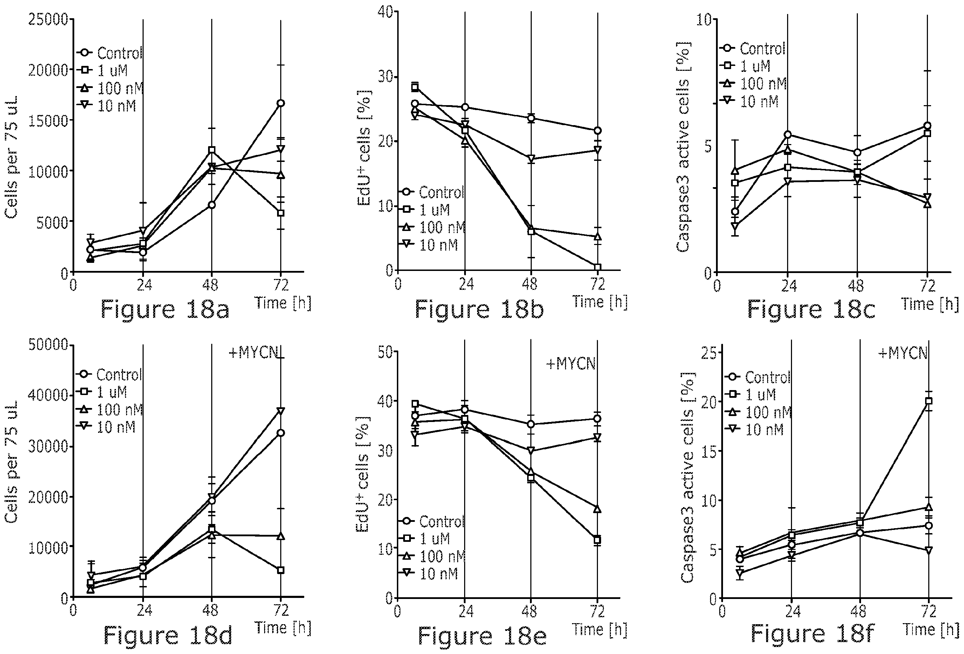

Figure 18 shows the cell quantification data for the SKNAS-ER-MYCN cell line without and with *MYCN* induction upon administration of Compound 2.

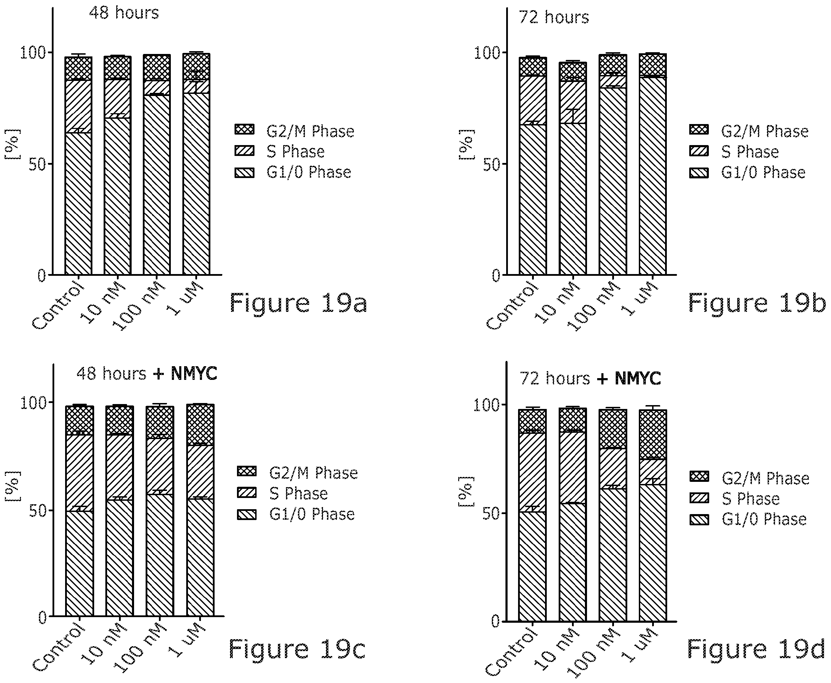

Figure 19 shows the quantification of the cell cycle profile of the SKNAS-ER-MYCN cell line without and with *MYCN* induction upon administration of Compound 2.

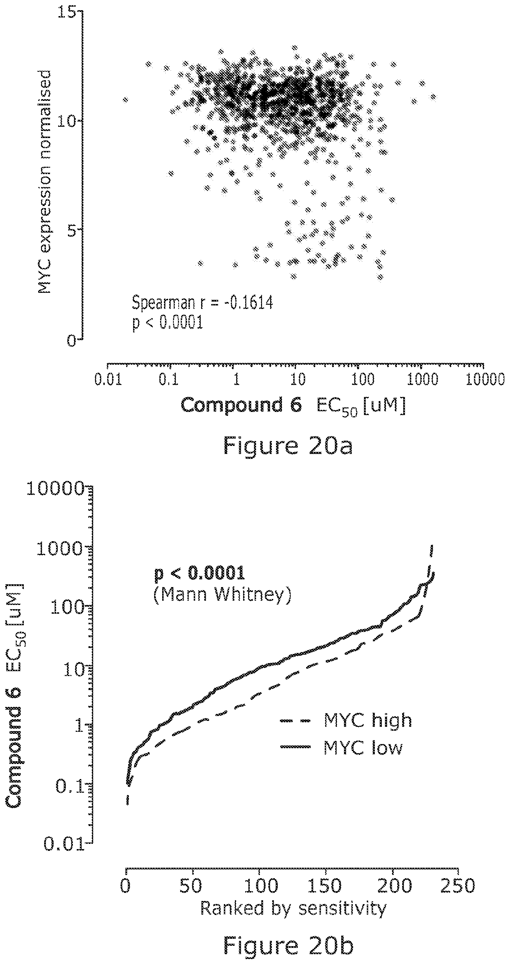

Figure 20 shows the global comparison of the correlation between sensitivity to the NMT inhibitor Compound 6, measured as EC50, and normalised c-MYC expression.

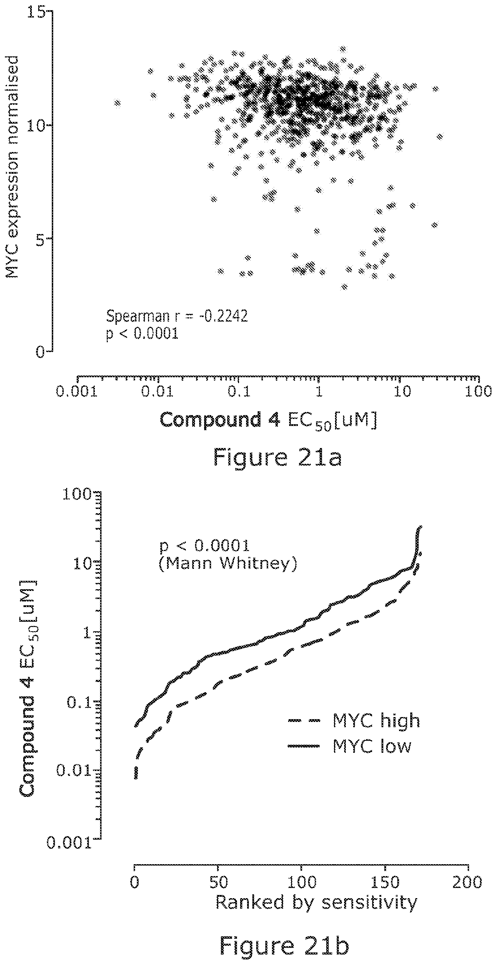

Figure 21 shows the global comparison of the correlation between sensitivity to the NMT inhibitor Compound 4, measured as EC50, and normalised c-MYC expression.

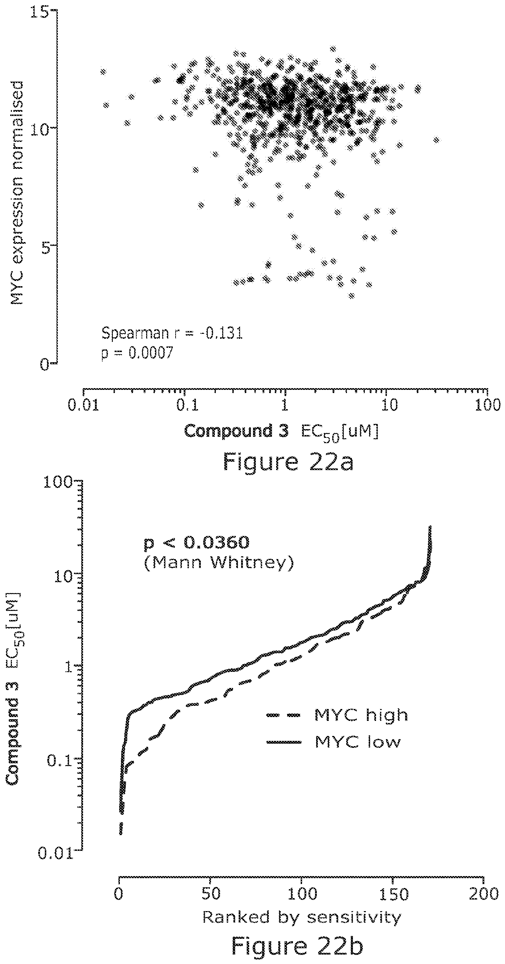

Figure 22 shows the global comparison of the correlation between sensitivity to the NMT inhibitor Compound 3, measured as EC50, and normalised c-MYC expression.

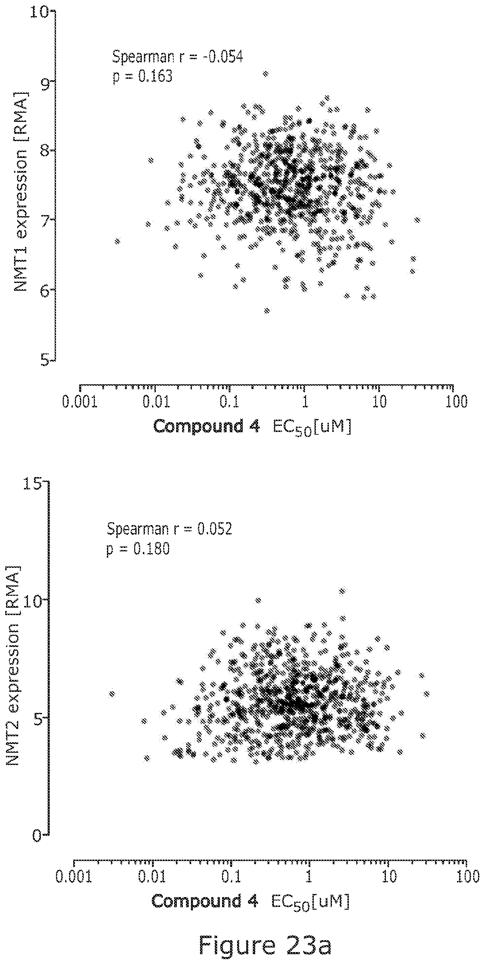

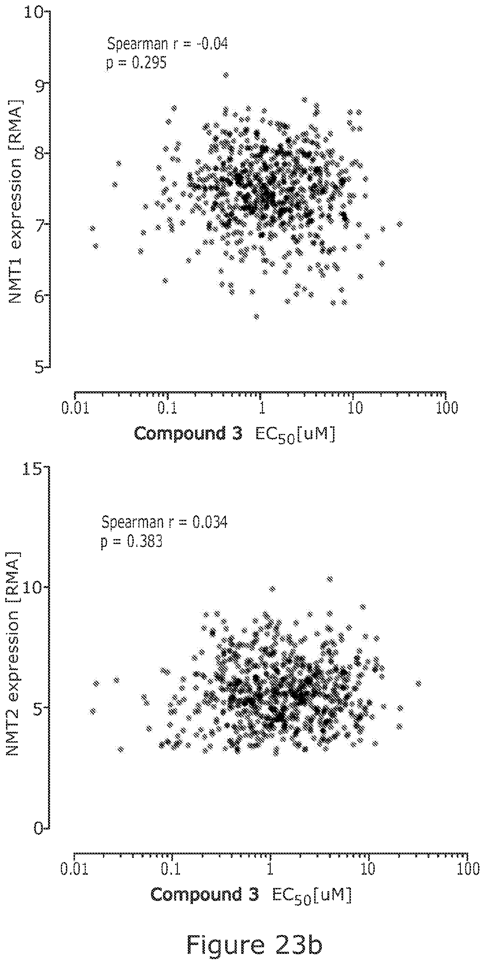

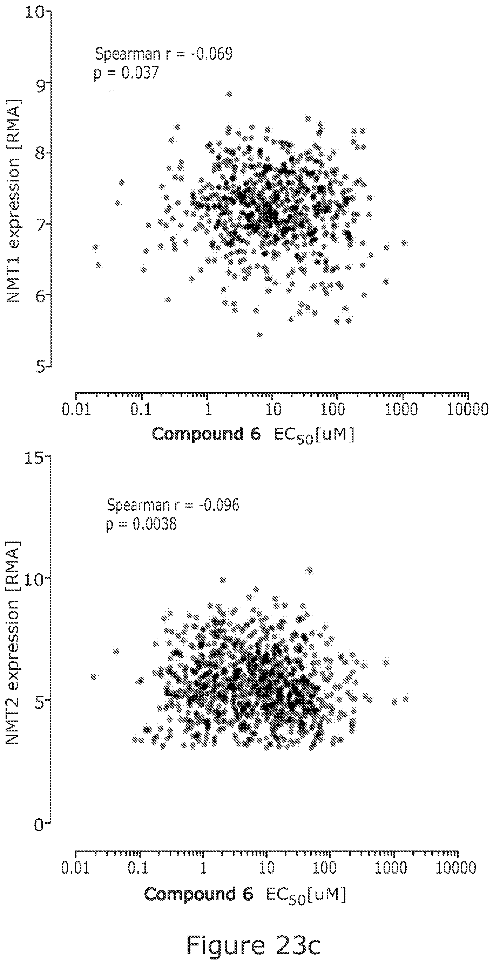

Figure 23 shows the global comparison of the correlation between sensitivity to the NMT inhibitors Compound 6, Compund 4 and Compound 3, measured as EC50 and normalised NMT1 and NMT2 expression.

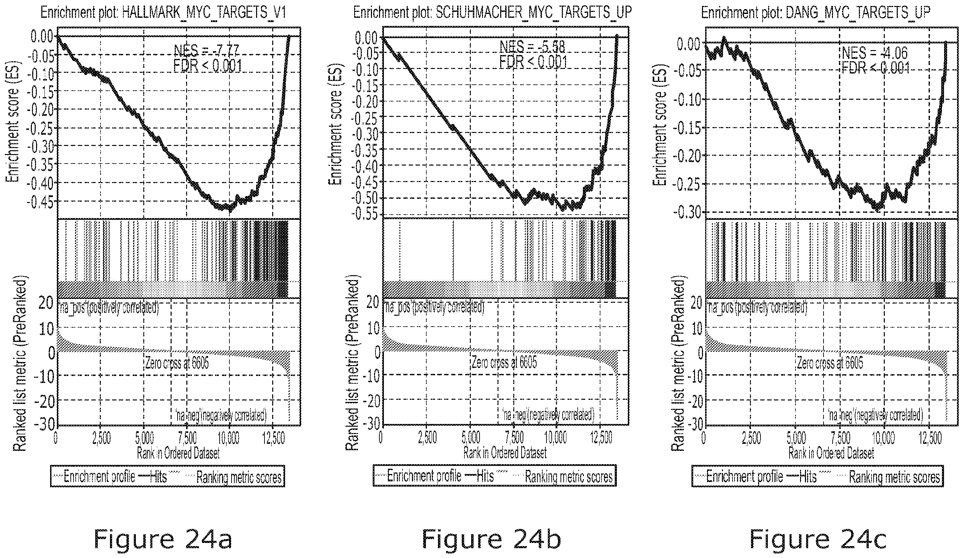

Figure 24 shows the effect of 100 nM Compound 2 for 24 hours on different *MYC* gene sets, measured via RNAseq in BL41 cells.

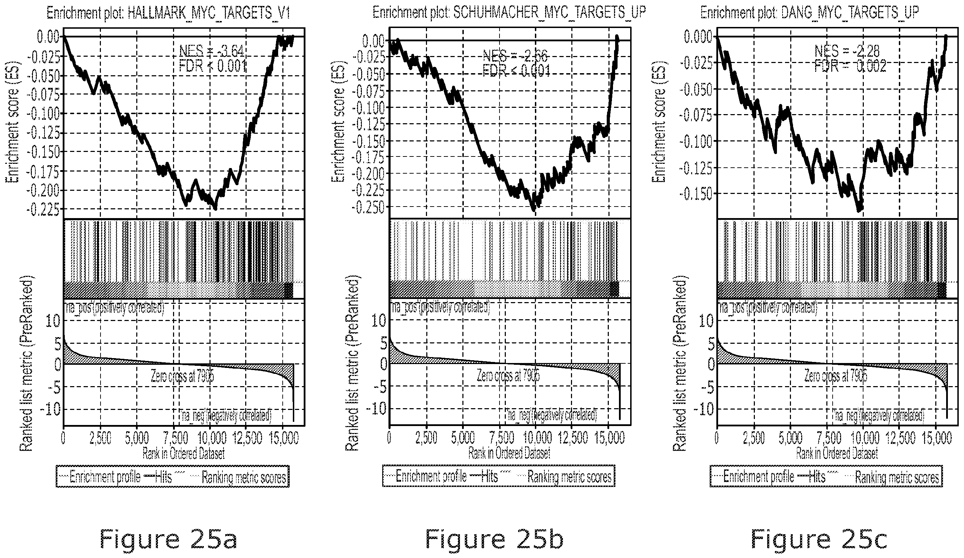

Figure 25 shows the effect of 100 nM Compound 2 for 24 hours on different *MYC* gene sets, measured via RNAseq in HeLa cells.

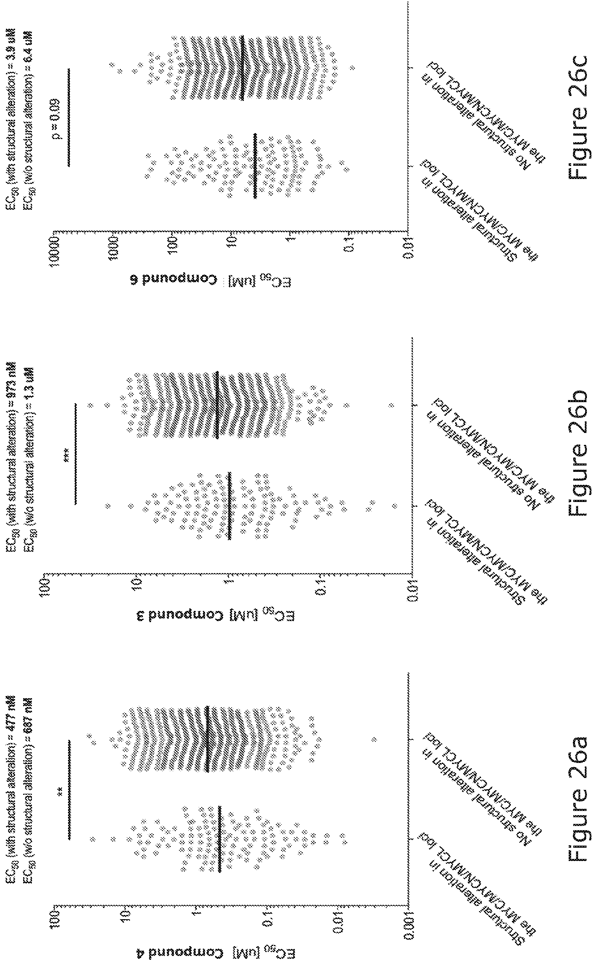

Figure 26 shows the effect on the sensitivity, measured as EC50, of the presence of structural alterations in the genomic loci of *c-MYC, MYCN* and *MYCL* for Compound 4, Compound 3 and Compound 6.

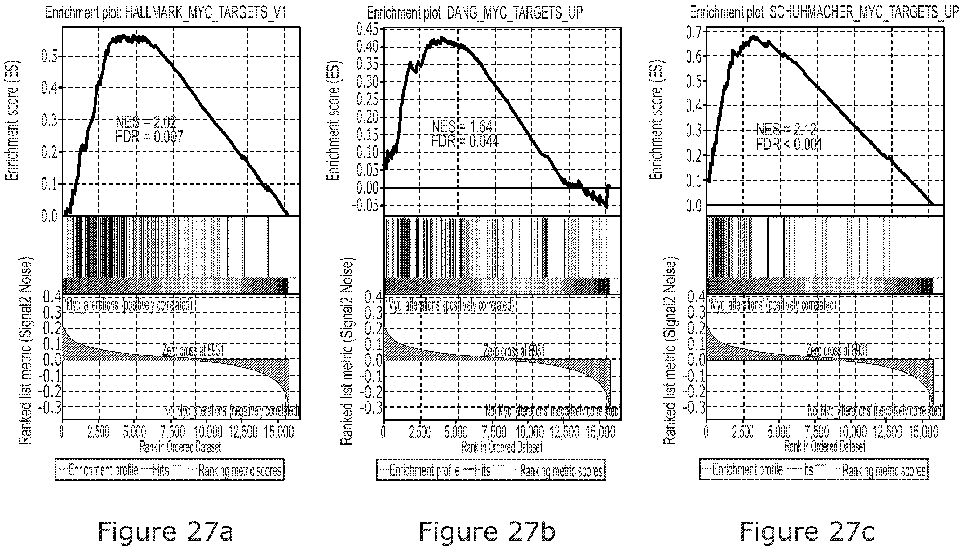

Figure 27 shows the activation of different *MYC* gene sets if structural alterations in the genomic loci of *c-MYC, MYCN* and *MYCL* are present.

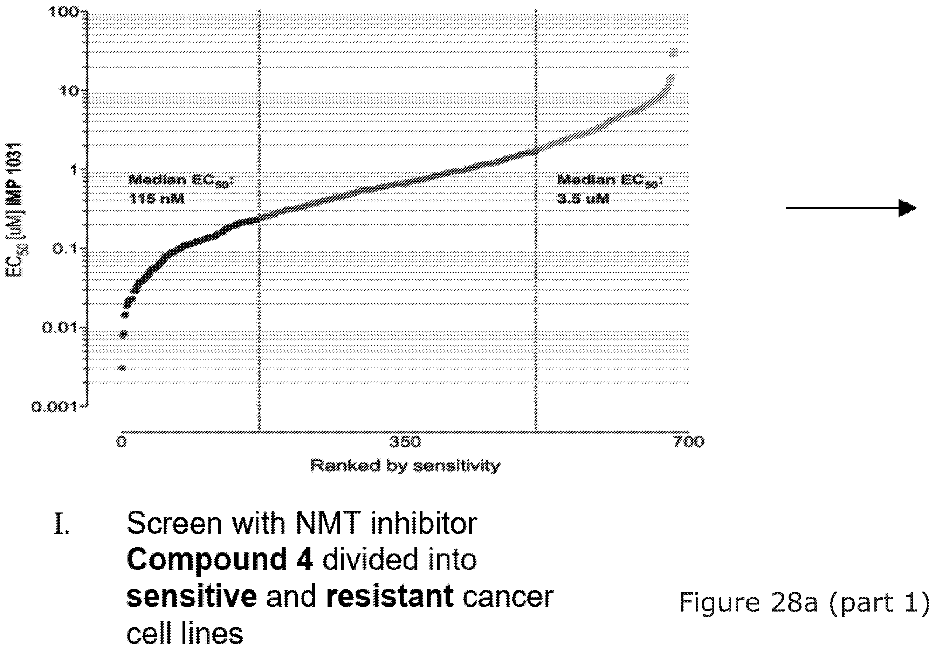

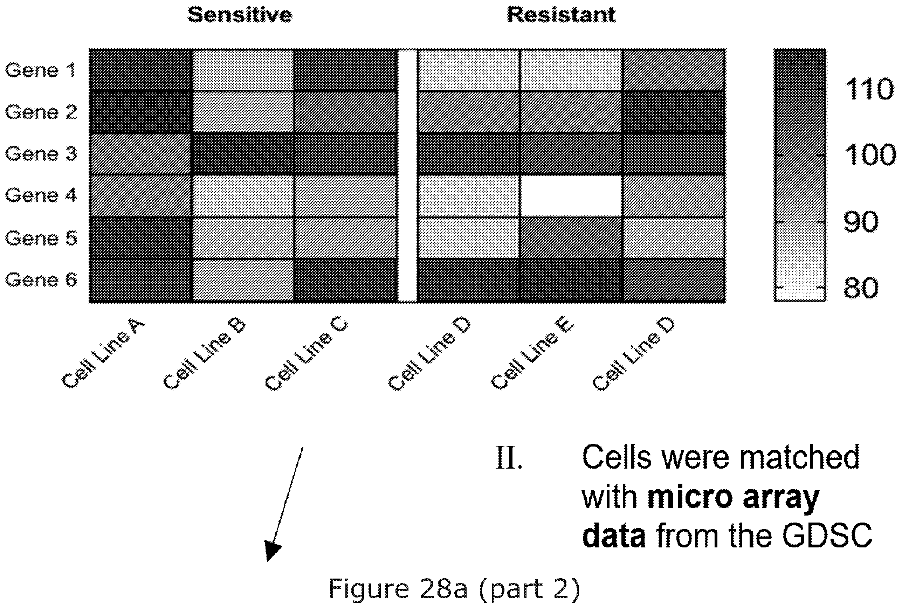

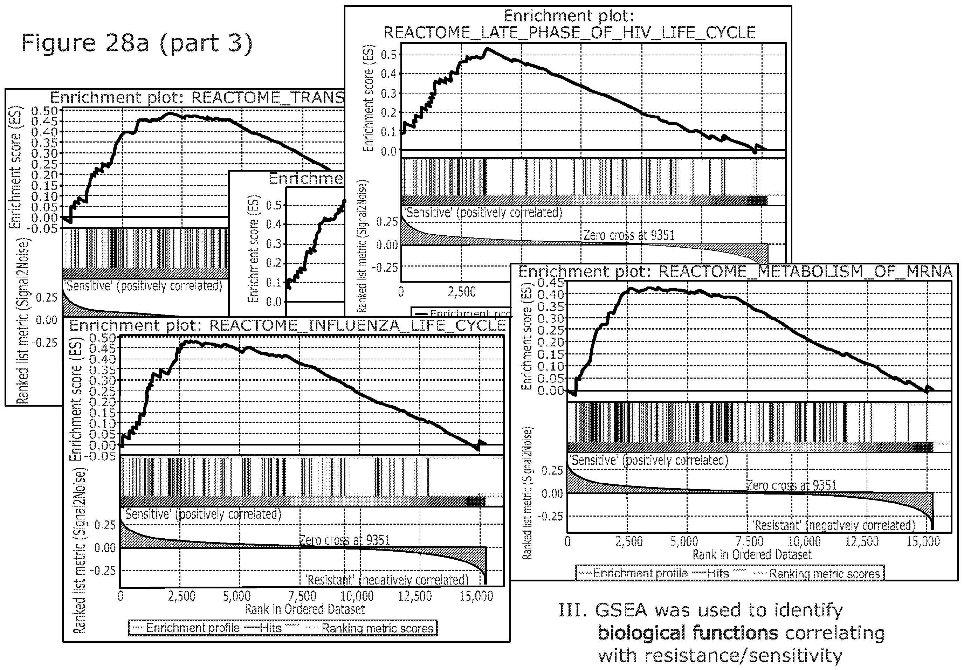

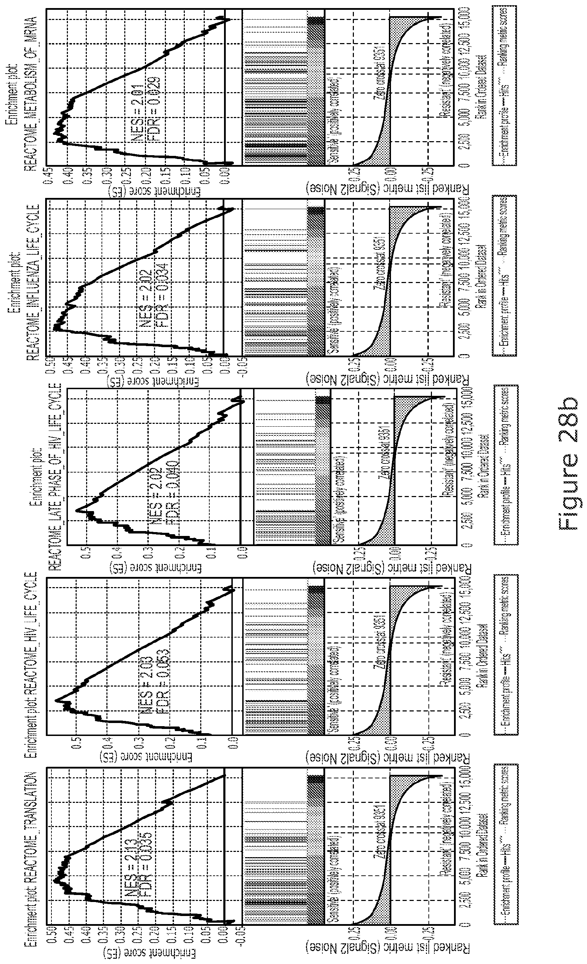

Figure 28 shows the GSEA analysis on the cancer cell line screen with Compound 4

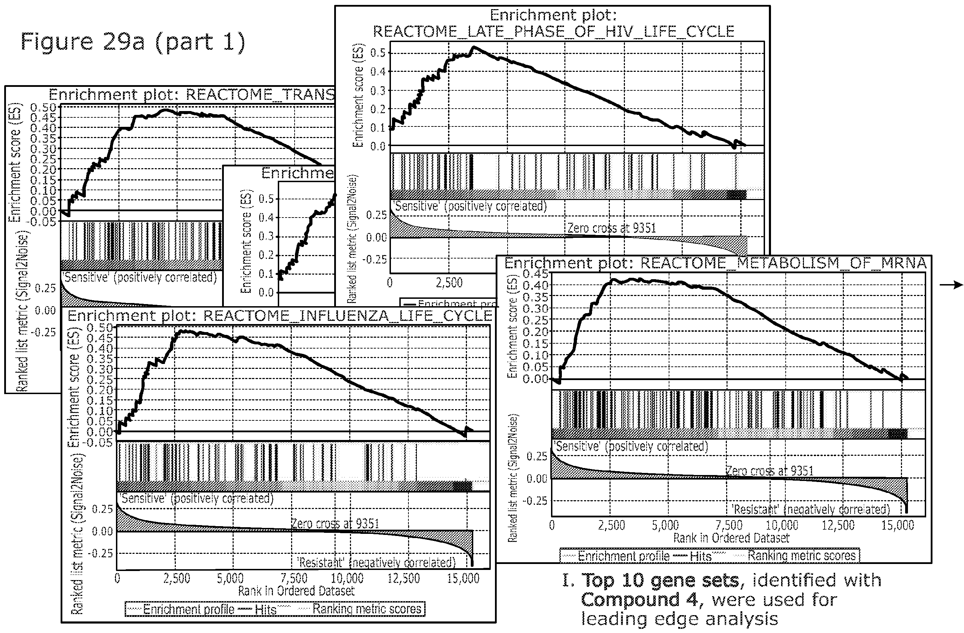

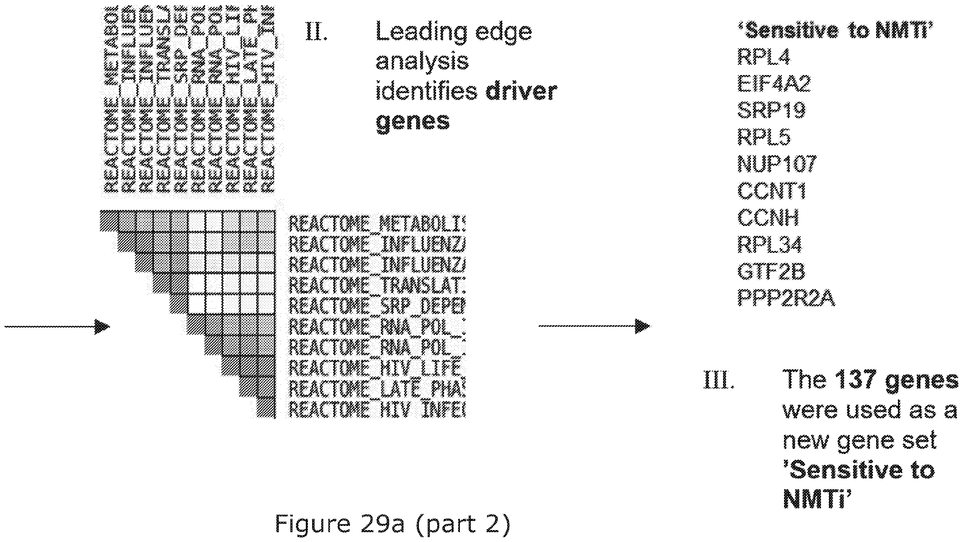

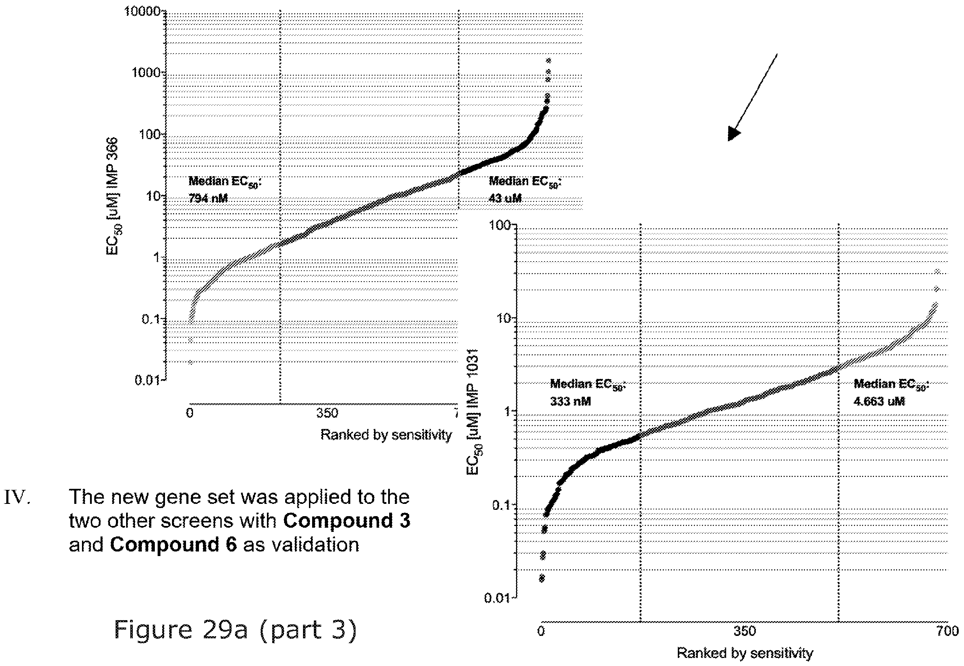

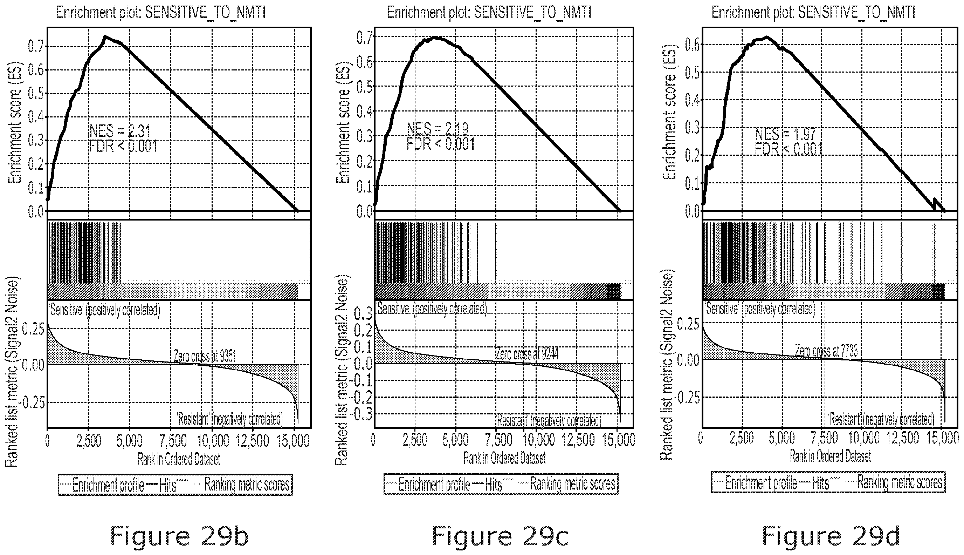

Figure 29 shows the leading-edge analysis on the cancer cell line screen with Compound 4, and the generation of a gene set 'Sensitive to NMTi'.

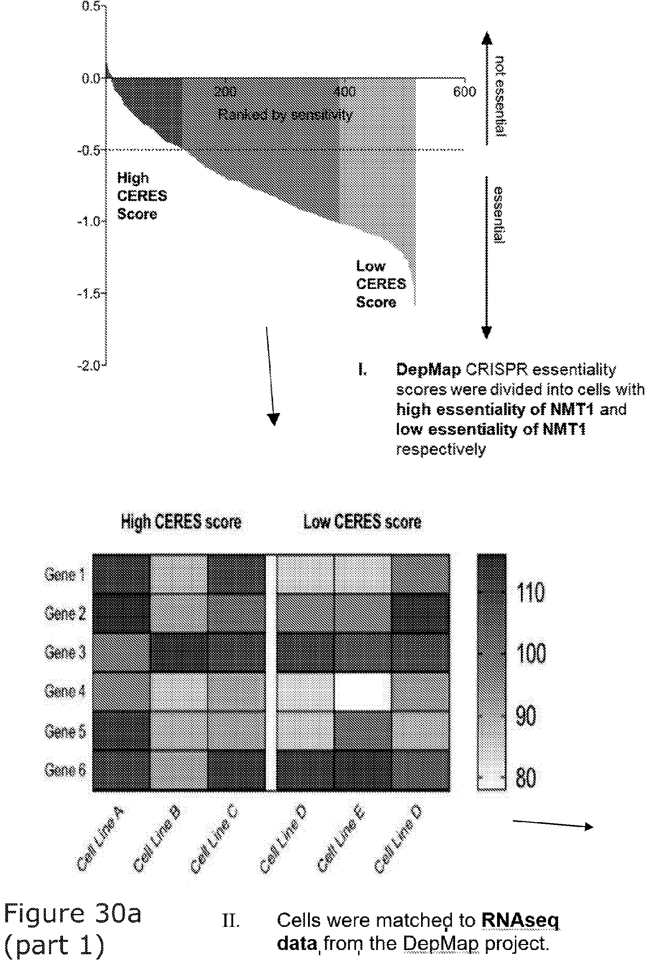

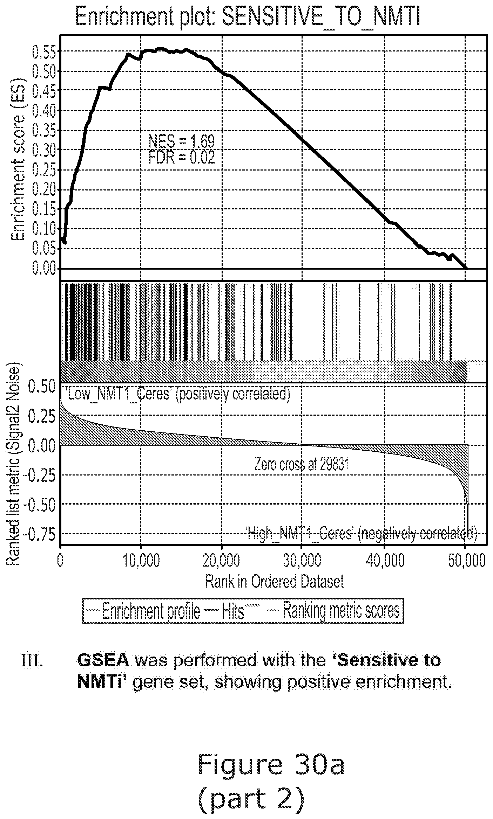

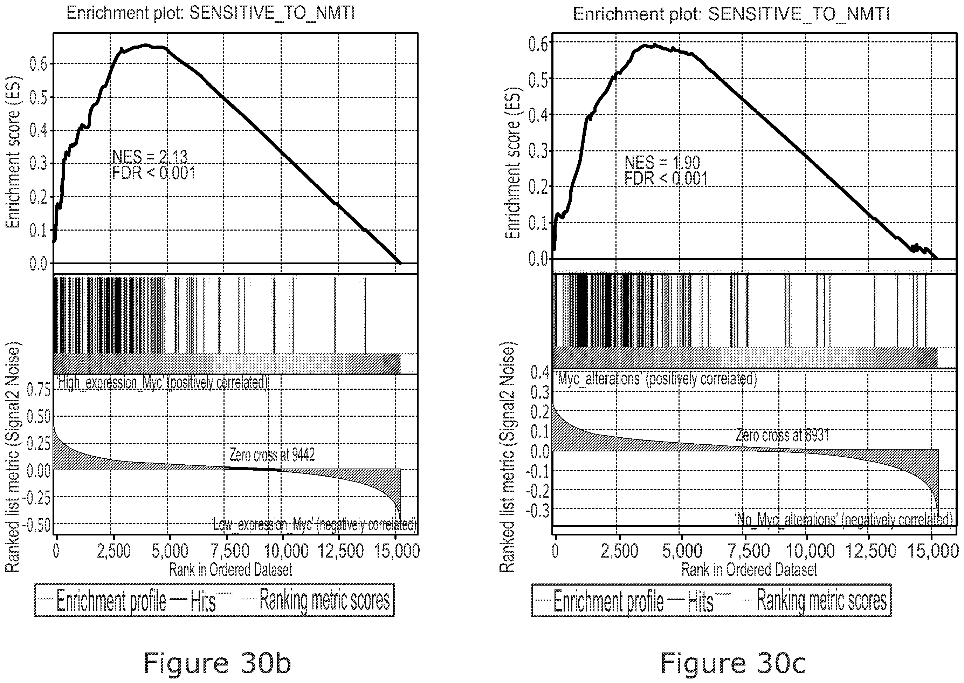

Figure 30 shows the application of the gene set 'Sensitive to NMTi' on a gene knock out essentiality screen and its upregulation in cancers with high c-Myc expression and/or structural alterations in the loci of *c-MYCIMYCNIMYCL.*

### DETAILED DESCRIPTION OF THE INVENTION

### Definitions

Unless otherwise stated, the following terms used in the specification and claims have the following meanings set out below.

It is to be appreciated that references to "treating" or "treatment" include prophylaxis as well as the alleviation of established symptoms of a condition. "Treating" or "treatment" of a state, disorder or condition therefore includes: (1) preventing or delaying the appearance of clinical symptoms of the state, disorder or condition developing in a human that may be afflicted with or predisposed to the state, disorder or condition but does not yet experience or display clinical or subclinical symptoms of the state, disorder or condition, (2) inhibiting the state, disorder or condition, i.e., arresting, reducing or delaying the development of the disease or a relapse thereof (in case of maintenance treatment) or at least one clinical or subclinical symptom thereof, or (3) relieving or attenuating the disease, i.e., causing regression of the state, disorder or condition or at least one of its clinical or subclinical symptoms.

A "therapeutically effective amount" means the amount of a compound that, when administered to a mammal for treating a disease, is sufficient to effect such treatment for the disease. The "therapeutically effective amount" will vary depending on the compound, the disease and its severity and the age, weight, etc., of the mammal to be treated.

The term "cancer" used herein will be understood to mean any neoplastic growth in a subject, including an initial tumour and any metastases. The cancer can be of the liquid or solid tumour type. Liquid tumours include, for example, tumours of haematological origin (haematological cancer), including, e.g., myelomas (e.g., multiple myeloma), leukeamias (e.g., Waldenstrom's syndrome, chronic lymphocytic leukemia, other leukeamias), and lymphomas (e.g., B-cell lymphomas). Solid tumours can originate in organs, and include cancers such as lung, breast, prostate, ovary, colon, kidney, and liver.

The term "cancerous cell" or "cancer cell" used herein will be understood to mean a cell that shows aberrant cell growth, such as increased cell growth. A cancerous cell may be a hyperplastic cell, a cell that shows a lack of contact inhibition of growth *in vitro,* a tumour cell that is incapable of metastasis *in vivo,* or a metastatic cell that is capable of metastasis *in vivo.* Cancer cells include, but are not limited to, carcinomas, such as myelomas, leukeamias (e.g., chronic lymphocytic leukaemia, myeloid leukaemia and B-acute lymphocytic leukaemia), lymphomas (e.g., follicular lymphoma, mantle cell lymphoma, diffuse large B-cell lymphoma or Hodgkins disease) and blastomas (e.g. retinoblastoma or glioblastoma).

The term *"MYC"* used herein will be understood to mean the whole family of regulator genes (transcriptional factors) that fall within the *MYC* gene family. Members of the *MYC* transcription factor family include c*-MYC, MYCN* and *MYCL,* and thus reference to *"MYC"* herein will be understood to covers all of such family members. The non-italisised terms "MYC", "c-MYC", "MYCN" or "MYCL" will, unless stated otherwise, be understood to mean the proteins derived from the corresponding *MYC* gene. For example, a reference to "MYCN" will be understood to mean the protein derived from the *"MYCN"* gene. Such nomenclature is consistent with the nomenclature used in the art. In certain instances, the terms *"MYC",* "c-*MYC", "MYCN"* and *"MYCL"* may be used synonymously with the respective uncapitalised terms *"Myc", "c-Myc", "N-Myc" and "L-Myc".*

The terms "overexpressed", "overexpression" and "overexpressing" used herein may be taken to mean the expression (i.e. level/amount) of mRNA and/or protein found in a particular cell (i.e. cancer cell) is elevated compared to the expression (i.e. level/amount) of mRNA and/or protein found in a normal, healthy cell (i.e. a cancer-free cell). Thus, for example, a *"MYC* overexpressing cancer" will be understood to be a cancer which expresses elevated RNA transcript and/or protein levels of *MYC* compared to the RNA transcript and/or protein levels of *MYC* found in normal, healthy cells. Suitably, the *MYC* overexpressing cancer is a cancer wherein the levels of RNA transcript and/or protein of *MYC* are at least 25% greater than the RNA transcript and/or protein levels of *MYC* in a normal, healthy cell. More suitably, the *MYC* overexpressing cancer is a cancer wherein the levels of RNA transcript and/or protein of *MYC* are at least 50% greater than the RNA transcript and/or protein levels of *MYC* in a normal, healthy cell. Even more suitably, the *MYC* overexpressing cancer is a cancer wherein the levels of RNA transcript and/or protein of *MYC* are at least 100% greater than the RNA transcript and/or protein levels of *MYC* in a normal, healthy cell. Yet more suitably, the *MYC* overexpressing cancer is a cancer wherein the levels of RNA transcript and/or protein of *MYC* are at least 200% greater than the RNA transcript and/or protein levels of *MYC* in a normal, healthy cell. Most suitably, *the MYC* overexpressing cancer is a cancer wherein the levels of RNA transcript and/or protein of *MYC* are at least 400% greater than the RNA transcript and/or protein levels of *MYC* in a normal, healthy cell. The level of expression may be determined by any suitable means known in the art. For example, the level of expression of *MYC* may be determined by measuring MYC protein levels. The MYC protein levels may be measured using any suitable technique known in the art, such as, for example, SDS-PAGE followed by Western blot using suitable antibodies raised against the target protein. In addition, or alternatively, the level of expression of *MYC* may be determined by measuring the level of mRNA. The level of mRNA may be measured using any suitable technique known in the art, such as, for example, northern blot, quantitative RT-PCR (qRT-PCR) or immunohistochemical (IHC), see for example Kluk et al., PLos One, 2012, 7(4), 1-9.

The terms "transcriptional addiction" and "transcriptionally addicted" used herein are terms of the art and may be understood to refer to cancers, and more specifically cancer cells, that exhibit a dependence on the transcription of a particular oncogene for continued growth and proliferation. In such "transcriptionally addicted" cancers, reducing the transcription of the oncogene, or inhibiting the oncogene which the cancer cells are transcriptionally addicted to, results in significant apoptosis and/or differentiation of said cancer cells. Thus, it will be understood that the terms *"MYC* addiction" and *"MYC* addicted" refer to cancers, and more specifically cancer cells, that exhibit a dependence on the *MYC* oncogene for continued growth and proliferation.

The term *"MYC* dysregulated cancer" used herein will be understood to mean a cancer in which one or more of the regulation mechanisms of the *MYC* oncogene are altered (e.g. mutated) or stabilised. The term "regulation mechanisms" will further be understood to mean any normal process of the *MYC* gene such as, for example, transcription or translation. Thus, it will be understood that the term *"MYC* dysregulated cancer" suitably covers both: i) mutations to the *MYC* oncogene which result in, for example, overexpression of the protein/mRNA of MYC; and ii) mutations to the *MYC* oncogene which result in, for example, a stabilisation of the protein/mRNA of MYC.

The term "locus" used herein will be understood to mean the location of a gene on a chromosome. Thus, the term *"MYC* locus" will be understood to mean the position on a chromosome corresponding to the *MYC* gene. Hence, the term "one or more structural alterations of the *MYC* locus" will be readily understood to mean that one or more alterations (e.g. mutations, amplifications and/or chromosomal rearrangements) are present at a location of the chromosome which corresponds to the *MYC* gene.

The term "hydrocarbyl" used herein will be understood to mean any compound straight or branched chain saturated, unsaturated or partially unsaturated hydrocarbon groups. Suitable examples of "hydrocarbyl" groups may include, for example, "alkyl", "alkenyl", "alkynyl" and/or "haloalkyl" groups, each of which are as defined hereinbelow.

The term "alkyl" used herein will be understood to mean straight and branched chain saturated hydrocarbon groups. Examples of "alkyl" groups include methyl, ethyl, n-propyl, iso-propyl, n-butyl, t-butyl, i-butyl, sec-butyl, pentyl and hexyl groups. Among unbranched alkyl groups, there are preferred methyl, ethyl, n-propyl, iso-propyl, n-butyl groups. Among branched alkyl groups, there may be mentioned t-butyl, i-butyl, 1-ethylpropyl and 1-ethylbutyl groups.

The term "Cₘ₋ₙ" or "(m-nC) group" used alone or as a prefix, refers to any group having m to n carbon atoms.

As used herein, the term "alkenyl" means both straight and branched chain unsaturated hydrocarbon groups with at least one carbon carbon double bond. Examples of alkenyl groups include ethenyl, propenyl, butenyl, pentenyl and hexenyl. Preferred alkenyl groups include ethenyl, 1-propenyl, 2-propenyl and but-2-enyl.

As used herein, the term "alkynyl" means both straight and branched chain unsaturated hydrocarbon groups with at least one carbon carbon triple bond. Examples of alkynyl groups include ethynyl, propynyl, butynyl, pentynyl and hexynyl. Preferred alkynyl groups include ethynyl, 1-propynyl and 2-propynyl.

As used herein, the term " carbocyclyl" (or "carbocycle") is intended to mean any 3- to 13-membered carbon ring system, which may be saturated, partially unsaturated, or aromatic. The carbon ring system may be monocyclic or contain more than one ring (e.g. the ring system may be bicyclic). Examples of monocyclic saturated carbocycles include cyclopropyl, cyclobutyl, cyclopentyl, cyclohexyl, cycloheptyl, cyclooctyl. Examples of bicyclic saturated carbocycles include bicyclooctane, bicyclononane, bicyclodecane (decalin) and bicyclooctane. A further example of a saturated carbocycle is adamantane. Examples of monocyclic non- saturated carbocycles include cyclobutene, cyclopentene, cyclopentadiene, cyclohexene. Examples of aromatic carbocycles include phenyl and naphthyl. Further examples of aromatic carbocycles include tetrahydronaphthyl (tetralin) and indane.

As used herein, the term "cycloalkyl" means a saturated group in a ring system. A cycloalkyl group can be monocyclic or bicyclic. A bicyclic group may, for example, be fused or bridged. Examples of monocyclic cycloalkyl groups include cyclopropyl, cyclobutyl and cyclopentyl. Other examples of monocyclic cycloalkyl groups are cyclohexyl, cycloheptyl and cyclooctyl. Examples of bicyclic cycloalkyl groups include bicyclo [2. 2.1]hept-2-yl. Preferably, the cycloalkyl group is monocyclic.

As used herein, the term "halogen" or "halo" means fluorine, chlorine, bromine or iodine. Fluorine, chlorine and bromine are particularly preferred.

As used herein, the term "haloalkyl" means an alkyl group having a halogen substituent, the terms "alkyl" and "halogen" being understood to have the meanings outlined above. Similarly, the term "dihaloalkyl" means an alkyl group having two halogen substituents and the term "trihaloalkyl" means an alkyl group having three halogen substituents. Examples of haloalkyl groups include fluoromethyl, chloromethyl, bromomethyl, fluoromethyl, fluoropropyl and fluorobutyl groups; examples of dihaloalkyl groups include difluoromethyl and difluoroethyl groups; examples of trihaloalkyl groups include trifluoromethyl and trifluoroethyl groups.

As used herein, the term "heterocyclyl" (or heterocycle) means an aromatic or a non-aromatic cyclic group of carbon atoms wherein from one to four of the carbon atoms is/are replaced by one or more heteroatoms independently selected from nitrogen, oxygen or sulfur. A heterocyclyl (or heterocycle) group may, for example, be monocyclic or bicyclic. In a bicyclic heterocyclyl (or heterocycle) group there may be one or more heteroatoms in each ring, or only in one of the rings. A heteroatom may be S, O or N, and is preferably O or N.

Examples of monocyclic non-aromatic heterocyclyl (or heterocycle) include aziridinyl, azetidinyl, pyrrolidinyl, imidazolidinyl, pyrazolidinyl, piperidinyl, piperazinyl, tetrahydrofuranyl, tetrahydropyranyl, morpholinyl, thiomorpholinyl and azepanyl.

Examples of monocyclic aromatic heterocyclyl (or heterocycle) groups include furanyl, thienyl, pyrrolyl, oxazolyl, thiazolyl, imidazolyl, oxadiazolyl, thiadiazolyl, pyridyl, triazolyl, triazinyl, tetrazolyl, pyridazyl, isothiazolyl, isoxazolyl, pyrazinyl, pyrazolyl and pyrimidinyl.

Examples of bicyclic aromatic heterocyclyl groups (or heterocycle) include quinoxalinyl, quinazolinyl, pyridopyrazinyl, benzoxazolyl, benzothiophenyl, benzimidazolyl, naphthyridinyl, quinolinyl, benzofuranyl, indolyl, benzothiazolyl, oxazolyl[4,5-b]pyridiyl, pyridopyrimidinyl, isoquinolinyl and benzodroxazole. Further examples of bicyclic aromatic heterocyclyl groups include those in which one of the rings is aromatic and the other is non-aromatic, such as dihydrobenzofuranyl, indanyl, indolinyl, isoindolinyl, tetrahydroisoquinolinyl, tetrahydroquinolyl and benzoazepanyl.

The term "optionally substituted" refers to either groups, structures, or molecules that are substituted and those that are not substituted. The term "wherein a/any CH, CH₂, CH₃ group or heteroatom (i.e. NH) within a R¹ group is optionally substituted" suitably means that (any) one of the hydrogen radicals of the R¹ group is substituted by a relevant stipulated group.

Where optional substituents are chosen from "one or more" groups, it is to be understood that this definition includes all substituents being chosen from one of the specified groups or the substituents being chosen from two or more of the specified groups.

The phrase "NMT inhibitor of the invention" means those compounds which display activity as inhibitors of the human *N*-myristoyl transferases (NMT) which are disclosed herein, both generically and specifically.

### Therapeutic Uses and Applications

To their surprise, the inventors of the present invention have found that upon administering a NMT inhibitor to a cancer with overexpressed levels of the *MYC* oncogene, and/or to a cancer with one or more structural alterations in the *MYC* locus (e.g. one or more chromosomal rearrangements or mutations), a significant improvement in both the kill rate and time to kill of the cancer, together with a lowering of the concentration of NMT inhibitor needed to adversely effect the cell is achieved compared to administration of a NMT inhibitor to a cancer without overexpressed levels of the *MYC* oncogene.

Following much investigation, the inventors discovered there to be a significant correlation between the mRNA expression levels of, for instance, *c-MYC* and the responsiveness to an NMT inhibitor in three pharmacogenomics screens (see, for example, Figures 20 to 22). This correlation was observed using two different types of analysis (Spearman correlation between c-Myc expression and EC50, and dividing cells by high and low expression of c-Myc and comparing both groups) and was found to be observed for a selection of structurally diverse NMT inhibitors in different types of cancer cell lines (e.g. 676 to 913 cancer cell lines).

Furthermore, the inventors also observed that one or more structural alterations in the *MYC* locus (e.g. *MYCN* and *MYCL*) correlated significantly with an increased sensitivity to an NMT inhibitor. These observations were again consistent across three pharmacogenomics screens, and upon using a selection of structurally diverse NMT inhibitors and different cancer cell lines (676 cancer cell lines). See, for example, Figure 26.

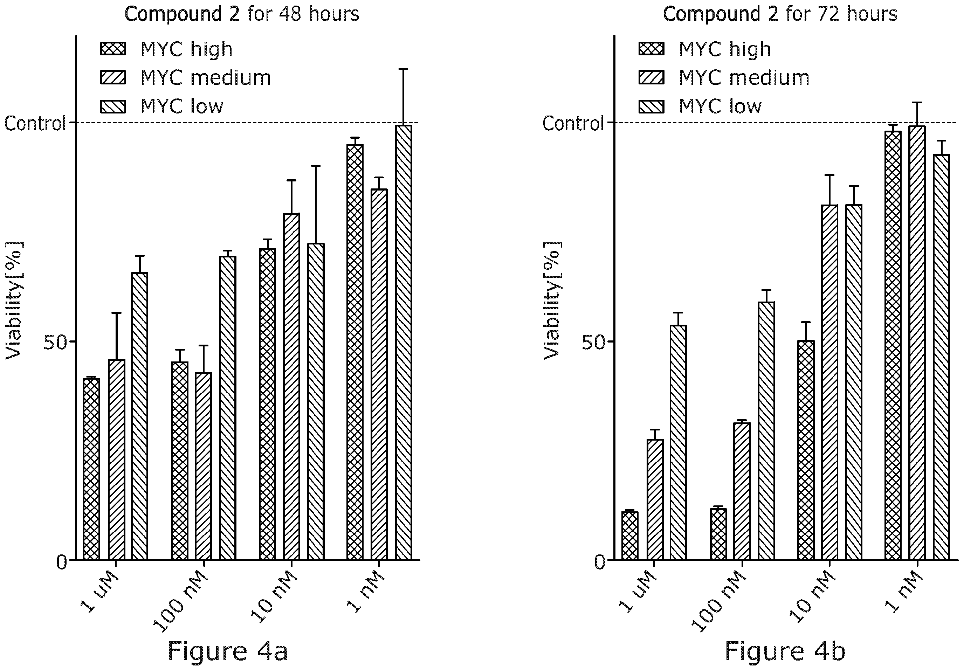

In addition to these strong correlations, the inventors also discovered that when the expression of *c-MYC* or *MYCN* was enforced in three different genetically modified cell lines, an increase in the responsivess to an NMT inhibitor was observed, as evidenced by the lower effective concentrations of NMT inhibitor needed to impart cell death in cell lines with high *MYC* expression compared to those with low *MYC* expression, and also the shorter timeframes for cell death - see, for instance, Figures 4, 5, 14 and 15.

The oncogenes *c-MYC* and *MYCN* are common diagnostic markers for particularly aggressive types of malignancies and there are recent findings demonstrating that *c-MYC* and *MYCN* overexpression and/or mutation correlate strongly with some of the worst clinical outcomes (Jung et al., Tumor and Stem Cell Biology, 2016, 65(16), 7065-7070, Habermann et al., Blood, 2016, 128(22), 155, Xu et al., Genes Cancer, 2010, 1(6), 629-640). There therefore remains a need for improved treatments that are able to target cancers where one or more structural alterations of the *MYC* oncogene exist.

In line with the advantageous properties described hereinabove, the inventors have therefore discovered that the class of NMT inhibitor compounds, particularly the NMT inhibitor compounds defined herein, are especially well-suited for application in the treatment of cancers which: i) are addicted to the *MYC* oncogene; and/or ii) have one or more structural alterations in the *MYC* oncogene locus.

Thus, the present invention provides a NMT inhibitor for use in the treatment of diffuse large B-cell lymphoma in a subject who has been identified as benefiting from being administered a NMT inhibitor by a method which comprises administering a therapeutically effective amount of a NMT inhibitor, or a pharmaceutically acceptable salt, solvate or hydrate thereof to the subject, wherein said subject has been identified as benefiting from being administered a NMT inhibitor as determined by a method comprising the steps of:
i) taking a sample of diffuse large B-cell lymphoma cells taken from said subject;
ii) analysing the cells of step i) to check for the presence of one or more structural alterations in the MYC locus (e.g. chromosomal rearrangements, copy number gains and/or mutations of the MYC oncogene);
iii) determining whether one or more structural alterations (e.g. chromosomal rearrangements, copy number gains and/or mutations) are present in the MYC locus of the sample of diffuse large B-cell lymphoma cells when compared to a control; and
iv) determining whether the subject will benefit from being administered a NMT inhibitor in order to treat said diffuse large B-cell lymphoma, wherein if the sample of diffuse large B-cell lymphoma cells contain one or more structural alterations (e.g. chromosomal rearrangements, copy number gains and/or mutations) in the MYC locus, then the subject will benefit from being administered a NMT inhibitor, and if the sample of diffuse large B-cell lymphoma cells do not contain one or more structural alterations (e.g. chromosomal rearrangements, copy number gains and/or mutations) in the MYC locus, then the subject will not benefit from being administered a NMT inhibitor.

The present invention further provides a NMT inhibitor, or a pharmaceutically acceptable salt, solvate or hydrate thereof, for use in the treatment of diffuse large B-cell lymphoma, wherein said diffuse large B-cell lymphoma comprises one or more structural alterations of the *MYC* locus. Non-limiting examples of "structural alterations" include, for example, mutations, copy-number gains and/or chromosomal rearrangements. In one embodiment, there is provided a NMT inhibitor, or a pharmaceutically acceptable salt, solvate or hydrate thereof, for use in the treatment of diffuse large B-cell lymphoma, wherein said diffuse large B-cell lymphoma comprises one or more mutations of the *MYC* locus. Suitably, the present invention provides a NMT inhibitor, or a pharmaceutically acceptable salt, solvate or hydrate thereof, for use in the treatment of diffuse large B-cell lymphoma, wherein said diffuse large B-cell lymphoma comprises one or more mutations of the *MYC* locus which impart overexpression of *MYC.* More suitably, the present invention provides a NMT inhibitor, or a pharmaceutically acceptable salt, solvate or hydrate thereof, for use in the treatment of diffuse large B-cell lymphoma, wherein said diffuse large B-cell lymphoma comprises one or more mutations of the *MYC* locus which impart overexpression of *c-MYC* or *MYCN.* In one particular embodiment, the present invention provides a NMT inhibitor, or a pharmaceutically acceptable salt, solvate or hydrate thereof, for use in the treatment of diffuse large B-cell lymphoma, wherein said diffuse large B-cell lymphoma comprises one or more mutations of *c-MYC.* In another particular embodiment, the invention provides a NMT inhibitor, or a pharmaceutically acceptable salt, solvate or hydrate thereof, for use in the treatment of diffuse large B-cell lymphoma, wherein said diffuse large B-cell lymphoma comprises one or more mutations of *MYCN.*

In a particular embodiment, there is provided a NMT inhibitor, or a pharmaceutically acceptable salt, solvate or hydrate thereof, for use in the treatment of diffuse large B-cell lymphoma, wherein said diffuse large B-cell lymphoma comprises one or more mutations of the *MYC* locus which impart stabiliation of *MYC.*

In an embodiment, the one or more structural alterations are chromosomal rearrangements. Thus, suitably, step ii) of the above method involves analysing the diffuse large B-cell lymphoma cells of step i) to check for the presence of one or more chromosomal rearrangements in the *MYC* locus. The person skilled in the art will be able to readily determine suitable techniques for checking for the presence of one or more chromosomal rearrangements in the *MYC* locus. One, non-limiting, example of a suitable technique for checking for the presence of one or more chromosomal rearrangements in the *MYC* locus is fluorescence in-situ hybridisation (FISH).

In another embodiment, the one or more structural alterations are mutations. Thus, suitably, step ii) of the above method involves analysing the diffuse large B-cell lymphoma cells of step i) to check for the presence of one or more mutations in the *MYC* locus. The person skilled in the art will be able to readily determine suitable techniques for checking for the presence of one or more mutations in the *MYC* locus. One, non-limiting, example of a suitable technique for checking for the presence of one or more mutations in the *MYC* locus is gene sequencing.

Suitably, the control of step iii) is the structural arrangement of the *MYC* locus found in a normal, healthy cell, specifically, the control of step iii) is the chromosomal arrangement and/or genetic sequence found in the *MYC* locus of a normal, healthy cell.

It will be understood that the sample of diffuse large B-cell lymphoma cells taken from said subject may be obtained by any suitable method known in the art. For instance, the sample of diffuse large B-cell lymphoma cells taken from said subject may be those taken from a biopsy or may be a sample of circulating tumour cells (CTCs) taken from the subject.

According to a further aspect of the present invention, there is provided a NMT inhibitor for use in the treatment of diffuse large B-cell lymphoma in a subject who has been identified as benefiting from being administered a NMT inhibitor by a method which comprises administering a therapeutically effective amount of a NMT inhibitor, or a pharmaceutically acceptable salt, solvate or hydrate thereof to the subject, wherein said subject has been identified as benefiting from being administered a NMT inhibitor as determined by a method comprising the steps of:
i) measuring the level of MYC expression in a sample of diffuse large B-cell lymphoma cells taken from said subject;
ii) comparing the level of MYC expression from step i) with a control;
iii) determining whether the MYC expression in the sample of diffuse large B-cell lymphoma cells is increased compared to the control; and
iv) determining whether the subject will benefit from being administered a NMT inhibitor in order to treat said diffuse large B-cell lymphoma, wherein if the MYC expression in the sample of diffuse large B-cell lymphoma cells is higher than in the control, then the subject will benefit from being administered a NMT inhibitor, and if the MYC expression in the sample of diffuse large B-cell lymphoma cells is not higher than in the control, then the subject will not benefit from being administered a NMT inhibitor.

It will be appreciated that the level of *MYC* expression in the sample of diffuse large B-cell lymphoma cells may be determined by any suitable means known in the art. For example, the level of expression of *MYC* may be determined by measuring MYC protein levels. The MYC protein levels may be measured using any suitable technique known in the art, such as, for example, SDS-PAGE followed by Western blot using suitable antibodies raised against the target protein. In addition, or alternatively, the level of expression of *MYC* may be determined by measuring the level of mRNA. The level of mRNA may be measured using any suitable technique known in the art, such as, for example, northern blot or quantitative RT-PCR (qRT-PCR).

Suitably, the control of step ii) above is the *MYC* expression level found in a normal, healthy cell, for example, a normal, healthy cell of the same type as the cell being investigated. It will be understood that the *MYC* expression level found in a normal, healthy cell may also be determined using any of the techniques described in paragraph [0059] above.

Suitably, the NMT inhibitor may be any of the NMT inhibitors described hereinbelow.

### Routes of Administration

The NMT inhibitors for use in the present invention may be administered to a subject by any convenient route of administration, whether systemically/ peripherally or topically (i.e., at the site of desired action).

Routes of administration include, but are not limited to, oral (e.g, by ingestion); buccal; sublingual; transdermal (including, e.g., by a patch, plaster, etc.); transmucosal (including, e.g., by a patch, plaster, etc.); intranasal (e.g., by nasal spray); ocular (e.g., by eye drops); pulmonary (e.g., by inhalation or insufflation therapy using, e.g., via an aerosol, e.g., through the mouth or nose); rectal (e.g., by suppository or enema); vaginal (e.g., by pessary); parenteral, for example, by injection, including subcutaneous, intradermal, intramuscular, intravenous, intra-arterial, intracardiac, intrathecal, intraspinal, intracapsular, subcapsular, intraorbital, intraperitoneal, intratracheal, subcuticular, intraarticular, subarachnoid, and intrasternal; by implant of a depot or reservoir, for example, subcutaneously or intramuscularly. In certain embodiments, the NMT inhibitors for use in the present invention are administered to a subject by oral administration (e.g, by ingestion).

The amount of NMT inhibitor which is required to achieve a therapeutic effect will, of course, vary with the particular compound, the route of administration, the subject under treatment, including the type, species, age, weight, sex, and medical condition of the subject and the renal and hepatic function of the subject, and the particular disorder or disease being treated, as well as its severity. An ordinarily skilled physician, veterinarian or clinician can readily determine and prescribe the effective amount of the drug required to prevent, counter or arrest the progress of the condition.

An effective amount of the NMT inhibitor for use in the treatment of diffuse large B-cell lymphoma as described herein is an amount sufficient to treat or prevent diffuse large B-cell lymphoma mentioned herein, slow its progression and/or reduce the symptoms associated with diffuse large B-cell lymphoma.

When orally administered to humans, the NMT inhibitor for use in the present invention will generally be administered at an amount of between about 0.01 mg per kg of body weight per day (mg/kg/day) to about 100 mg/kg/day, preferably 0.01 mg per kg of body weight per day (mg/kg/day) to 10 mg/kg/day, and most preferably 0.1 to 5.0 mg/kg/day, for adult humans.

The size of the dose for therapeutic or prophylactic purposes of a compound of the formula I will naturally vary according to the nature and severity of the conditions, the age and sex of the animal or patient and the route of administration, according to well-known principles of medicine.

In using a NMT inhibitor for the treatment of diffuse large B-cell lymphoma it will generally be administered so that a daily dose in the range, for example, 0.1 mg/kg to 75 mg/kg body weight is received, given if required in divided doses. In general, lower doses will be administered when a parenteral route is employed. Thus, for example, for intravenous or intraperitoneal administration, a dose in the range, for example, 0.1 mg/kg to 30 mg/kg body weight will generally be used. Similarly, for administration by inhalation, a dose in the range, for example, 0.05 mg/kg to 25 mg/kg body weight will be used. Oral administration may also be suitable, particularly in tablet form. Typically, unit dosage forms will contain about 0.5 mg to 0.5 g of a NMT inhibitor for use in this invention.

### Combination Therapies

The NMT inhibitor defined hereinbefore may be applied as a sole therapy or be administered in combination with one or more other therapeutic agents, or may be administered in combination with conventional surgery or radiotherapy.

Such conjoint treatment may be achieved by way of the simultaneous, sequential or separate dosing of the NMT inhibitor and the one or more other therapeutic agents of the treatment. Such combination products may employ the NMT inhibitors of this invention within any suitable dosage range, such as, for example, the dosage range described hereinabove, and the other pharmaceutically-active agent may be within its approved dosage range.

Thus, the present invention further provides a NMT inhibitor, or a pharmaceutically acceptable salt, solvate or hydrate thereof, for use in combination with one or more other therapeutic agents.

Suitable, but non-limiting, examples of other therapeutic agents which may be administered in combination with the NMT inhibitor include one or more other chemotherapeutic agents.

For example, the NMT inhibitor may be administered in addition to any currently recommended treatment (also known as the "standard of care") for cancer such as diffuse large B-cell lymphoma.

### N-myristoyl transferase (NMT) inhibitors

*N*-myristoyl transferase (NMT) is a monomeric enzyme, which is ubiquitous in eukaryotes. NMT catalyses an irreversible co-translational transfer of myristic acid (a saturated 14-carbon fatty acid) from myristoyl-Coenzyme A (myr-CoA) to a protein substrate containing an *N*-terminal glycine with formation of an amide bond (Farazi, T.A., G. Waksman, and J.I. Gordon, J. Biol. Chem., 2001. 276(43): p. 39501-39504).

There are two types of human NMT, human NMT1 (HsNMT1) and human NMT2 (HsNMT2). Inhibition of human NMT has been suggested as a target for treating or preventing various diseases or disorders, for example hyperproliferative disorders (cancers, e.g. human colorectal cancer, gallbladder carcinoma, brain tumours, and lymphomas such as B-cell lymphoma) (Resh MD. 1993. Biochern. Biophys.Acta 1115, 307-22; Berthiaume LG, Beuachamp E, WO2017/011907). As NMT plays a key role in protein trafficking, mediation of protein-protein interactions, stabilization of protein structures and signal transduction in living systems, inhibition of the NMT enzyme has the potential to disrupt multi-protein pathways. This is an attractive characteristic to reduce the risk of the development of resistance in, for example, treatment or prevention of hyperproliferative disorders.

Compounds active as inhibitors of NMT have previously been disclosed, see for example WO00/37464 (Roche), WO2010/026365 (University of Dundee), WO2013/083991 (Imperial Innovations Limited) and WO2017/001812 (Imperial Innovations Limited), and their use in the treatment of cancer has been described.

Thus, the NMT inhibitors for use in the present invention are species (e.g. compounds) which display activity as inhibitors of N-myristoyl transferase (NMT). Furthermore, the term "NMT inhibitor" as used herein will be understood to cover any species which binds to NMT and inhibits its activity. The inhibitors may act as competitive inhibitors, or partial competitive inhibitors. The inhibitor may bind to NMT at the myr-CoA binding pocket or at the peptide binding pocket (or inhibit NMT through another mechanism). The NMT inhibitor of the present invention preferably bind and inhibit NMT through the peptide binding pocket.

Suitably, the NMT inhibitors for use in the present invention are compounds which display activity as inhibitors of N-myristoyl transferase (NMT). Suitable compounds which display activity as inhibitors of NMT are known in the art. Non-limiting examples of suitable NMT inhibitors are described in, for example, WO00/37464 (Roche), WO2010/026365 (University of Dundee), WO2013/083991 (Imperial Innovations Limited) and WO2017/001812 (Imperial Innovations Limited).

In a particular embodiment, the NMT inhibitor for use in the present invention is a compound of Fomula I, Formula II or Formula III, as defined herein, or a pharmaceutically acceptable salt, solvate or hydrate thereof. For the avoidance of doubt, this embodiment of the present invention also encompasses all sub-formulae of Formula I, Formula II and Formula III described herein, such as for example, compounds of Formula (IA^^) and/or Formula Ila.

In certain embodiments, the NMT inhibitor for use in the present invention is a compound of Fomula I or Formula II, or a pharmaceutically acceptable salt, solvate or hydrate thereof. In other embodiments, the NMT inhibitor for use in the present invention is a compound of Fomula I or Formula III, or a pharmaceutically acceptable salt, solvate or hydrate thereof. In yet further embodiments, the NMT inhibitor for use in the present invention is a compound of Fomula II or Formula III, or a pharmaceutically acceptable salt, solvate or hydrate thereof.

In particular embodiments, the NMT inhibitor for use in the present invention is a compound of Fomula I or a pharmaceutically acceptable salt, solvate or hydrate thereof. Suitably, the NMT inhibitor for use in the present invention is a compound of Fomula (IA^^) or a pharmaceutically acceptable salt, solvate or hydrate thereof.

In other embodiments, the NMT inhibitor for use in the present invention is a compound of Fomula II, or a pharmaceutically acceptable salt, solvate or hydrate thereof. Suitably, the NMT inhibitor of the present invention is a compound of Fomula IIa, or a pharmaceutically acceptable salt, solvate or hydrate thereof.

### NMT inhibitors of Formula (I)

As outlined above, in certain embodiments, the NMT inhibitor is a compound of Formula (I) shown below, or a pharmaceutically acceptable salt, hydrate or solvate thereof: 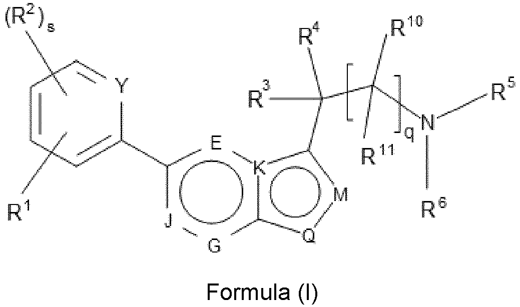 wherein:
Y is selected from the group consisting of -CH-, -C(R²)- and -N-;
R¹ is a group of formula -X-L-A;
wherein:
   X is selected from the group consisting of -O-, -N(H)- and -S-, or is absent;
   L is selected from the group consisting of -(CHR¹²)ₘ- and -(CHR¹²)ₘO-, or is absent;
   m is 1, 2 or 3; and
   A is a 6-10-membered aromatic carbocycle or a 5-10-membered aromatic heterocycle, said aromatic carbocycle or heterocycle being optionally substituted with 1, 2, or 3 substituents each independently selected from the group consisting of -F, -Cl, -Br, -OCH₃, -OCF₃, -CN,-C₁₋₆alkyl optionally substituted by up to 3 halogen, hydroxyl, or -OC₁₋₄alkyl groups, -S(O)C₁₋₄alkyl, -S(O)₂C₁₋₄alkyl, -C(O)N(R⁹)₂, - C(O)N(R¹³)C₁₋₄alkylOC₁₋₄alkyl , -C(O)N(C₁₋₄alkylOC₁₋₄alkyl)₂, -CH-₂C(O)N(R⁹)₂, -CH₂C(O)N(R¹³)C₁₋₄alkylOC₁₋₄alkyl , -CH₂C(O)N(C₁₋₄alkylOC₁₋₄alkyl)₂, -S(O)₂NHC₁₋₄alkyl, -S(O)₂N(C₁₋₄alkyl)₂, -NHC₁₋₄alkyl, -N(C₁₋₄alkyl)₂, -NHC(O)C₁₋₄alkyl,-NHC(O)CF₃,-NHS(O)₂C₁₋₄alkyl, CH₂N(R¹³)₂, CH₂N(R¹³)C(O)C₁₋₄alkyl, CH₂N(R¹³)S(O)₂C₁₋₄alkyl, - CH₂S(O)₂C₁₋₄alkyl, and CO₂H;

s is 0, 1, 2, or 3;
each R² is independently selected from the group consisting of -F, -Cl, -Br, - OCH₃, -OCF₃, -CN, -C₁₋₄alkyl optionally substituted by up to 3 halogen or hydroxyl groups, -S(O)C₁₋₄alkyl, -S(O)₂C₁₋₄alkyl, -S(O)₂NHC₁₋₄alkyl, - S(O)₂N(C₁₋₄alkyl)₂, -NHC₁₋₄alkyl, -N(C₁₋₄alkyl)₂, -NHC(O)C₁₋₄alkyl, -NHC(O)CF₃, and -NHS(O)₂C₁₋₄alkyl;
E, J and G are each independently nitrogen or C(R⁷);
K is carbon or nitrogen;
and wherein:
   i) when K is carbon, either Q is N(R⁸) and M is nitrogen or C(R⁷), or Q is nitrogen and M is N(R⁸); or
   ii) when K is nitrogen, Q is nitrogen or C(R⁷) and M is nitrogen or C(R⁷); and further wherein at least 2 of E, J, G, K, Q and M are selected from the group consisting of carbon and C(R⁷);
q is 0 or 1;
R³ is hydrogen or methyl; R⁴ is hydrogen or methyl;
R⁵ is hydrogen or C₁₋₆alkyl optionally substituted by up to 3 -F, -Cl, -Br, -OH, - OCH₃, -OCF₃ or -CN groups;
R⁶ is hydrogen or C₁₋₆alkyl optionally substituted by up to 3 -F, -Cl, -Br, -OH, - OCH₃, -OCF₃ or -CN groups;
or the R⁵ and R⁶ groups and the N they are bonded to form a 4 to 7 membered non-aromatic heterocycle, the heterocycle optionally comprising 1 or 2 further heteroatoms selected from N, O and S, optionally substituted by up to 3 -F, - Cl, -Br, -OH, -OCH₃, -OCF₃ or -CN groups;
when present R¹⁰ is hydrogen or methyl;
when present R¹¹ is hydrogen or methyl;
or the R³ group and the R⁵ group and the intervening atoms form a 3 to 7 membered non-aromatic heterocycle composed of the intervening atoms and bond, or the intervening atoms and -(CHR^{a})ᵣ-;
or the R¹⁰ group and the R⁵ group and the intervening atoms form a 3 to 7 membered non-aromatic heterocycle composed of the intervening atoms and - (CHR^{a})ᵣ-;
r is 1, 2, 3, 4 or 5; R^{a} is hydrogen or methyl;
each R⁷ is independently selected from the group consisting of hydrogen, halogen, C₁₋₄alkoxy, and C₁₋₄alkyl optionally substituted with 1, 2 or 3 halogens; and
R⁸ is selected from the group selected from hydrogen and C₁₋₄alkyl;
each R⁹ is independently selected from the group consisting of hydrogen and C₁₋₄alkyl, or two R⁹ groups and the N they are bonded to form a 4 to 7 membered non-aromatic heterocycle, the heterocycle optionally comprising 1 or 2 further heteroatoms selected from N, O and S;
each R¹² is independently selected from the group consisting of hydrogen, C₁₋₆alkyl optionally substituted by up to 3 -F, -Cl, -Br, I, -OH, -OCH₃, -OCF₃ or -CN groups, C₁₋₆alkenyl optionally substituted by up to 3 -F, -Cl, -Br, I, -OH, -OCH₃, -OCF₃ or -CN groups, and C₁₋₆alkynyl optionally substituted by up to 3 -F, -Cl, -Br, I, -OH, -OCH₃, -OCF₃ or -CN groups; and each R¹³ is independently selected from the group consisting of hydrogen and C₁₋₄alkyl

NMT inhibitors of Formula (I) are further described in WO2017/001812. It will be understood that suitable and preferred NMT inhibitors of Formula (I) of the present invention may include any of the compounds (generic or specific) disclosed in WO2017/001812.

In one embodiment, X is selected from the group consisting of -O-, -N(H)- and -S-. In another embodiment, L is selected from the group consisting of -(CHR¹²)ₘ- and -(CHR¹²)ₘO-. Suitably, X is selected from the group consisting of -O-, -N(H)- and -S-, and L is selected from the group consisting of -(CHR¹²)ₘ- and -(CHR¹²)ₘO-.

In certain embodiments, E, J, G and M are each C(R⁷), and K and Q are each nitrogen. In other embodiments, q is 1, R¹⁰ is hydrogen and R¹¹ is hydrogen. Suitably, q is 1, R¹⁰ is hydrogen, R¹¹ is hydrogen, E, J, G and M are each C(R⁷), and K and Q are each nitrogen.

In another embodiment, A is a 6-10-membered aromatic carbocycle or a 5-10-membered aromatic heterocycle, said aromatic carbocycle or heterocycle being optionally substituted with 1, 2, or 3 substituents each independently selected from the group consisting of -F, -Cl, -Br, -OCH₃, -OCF₃, -CN, -C₁₋₆alkyl optionally substituted by up to 3 halogen, hydroxyl, or -OC₁₋₄alkyl groups, -S(O)C₁₋₄alkyl, -S(O)₂C₁₋₄alkyl, -C(O)N(R⁹)₂, -CH₂C(O)N(R⁹)₂, -S(O)₂NHC₁₋₄alkyl, -S(O)₂N(C₁₋₄alkyl)₂, -NHC₁₋₄alkyl, -N(C₁₋₄alkyl)₂, -NHC(O)C₁₋₄alkyl, - CH₂NHC(O)C₁₋₄alkyl,-NHC(O)CF₃ and -NHS(O)₂C₁₋₄alkyl.

In yet another embodiment, A is a 6-10-membered aromatic carbocycle or a 5-10-membered aromatic heterocycle, said aromatic carbocycle or heterocycle being optionally substituted with 1, 2, or 3 substituents each independently selected from the group consisting of -F, -Cl, -Br, -OCH₃, -OCF₃, -CN, -C₁₋₆alkyl optionally substituted by up to 3 halogen, hydroxyl, or -OC₁₋₄alkyl groups, -S(O)C₁₋₄alkyl, -S(O)₂C₁₋₄alkyl, -C(O)N(R⁹)₂, -CH₂C(O)N(R⁹)₂, -S(O)₂NHC₁₋₄alkyl, -S(O)₂N(C₁₋₄alkyl)₂, -NHC₁₋₄alkyl, -N(C₁₋₄alkyl)₂, -NHC(O)C₁₋₄alkyl,-CH₂NHC(O)C₁₋₄alkyl,-NHC(O)CF₃, -NHS(O)₂C₁₋₄alkyl, CH₂NH₂, CH₂NHC₁₋₄alkyl, CH₂NC₁₋₄alkylC(O)C₁₋₄alkyl, CH₂NHS(O)₂C₁₋₄alkyl, -CH₂S(O)₂C₁₋₄alkyl, CH₂NC₁₋₄alkylS(O)₂C₁₋₄alkyl.

In certain embodiments, the NMT inhibitor has the formula (IA), such as, for example, Formula (Iα), or the NMT inhibitor has the formula IB, such as, for example, Formula Iβ, shown below: 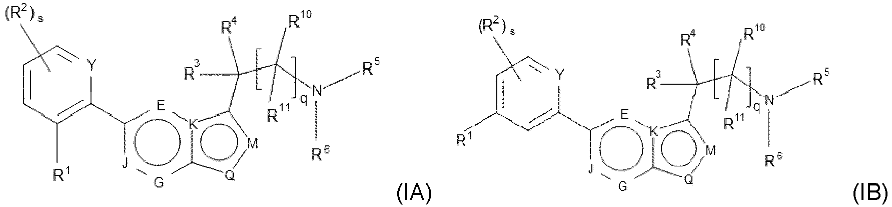 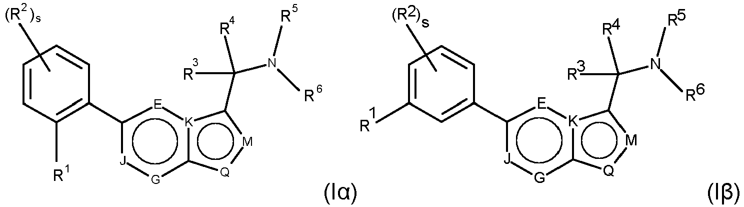 wherein each of R¹, R², s, q, E, J G, K, Q, M, q, R³, R⁴, R⁵ and R⁶, R⁷, R⁸, R⁹, R¹⁰ and R¹¹ are as defined in Formula (I).

In one particular embodiment, the NMT inhibitor has the formula (IA*) shown below: 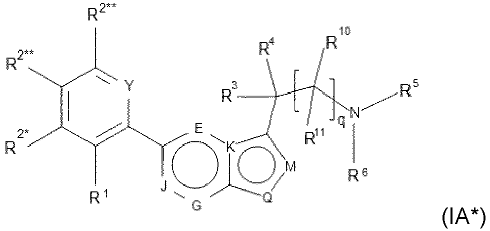 wherein:
each R^{2*} is independently selected from the group consisting of -F, -Cl, -Br, -OCH₃, - OCF₃, -CN, -C₁₋₄alkyl optionally substituted by up to 3 halogen or hydroxyl groups, - S(O)C₁₋₄alkyl, -S(O)₂C₁₋₄alkyl, -S(O)₂NHC₁₋₄alkyl, -S(O)₂N(C₁₋₄alkyl)₂, -NHC₁₋₄alkyl, - N(C₁₋₄alkyl)₂, -NHC(O)C₁₋₄alkyl, -NHC(O)CF₃, and -NHS(O)₂C₁₋₄alkyl (preferably selected from the group consisting of -F, -Cl, -Br, -OCH₃, -OCF₃, -CN, -C₁₋₄alkyl optionally substituted by up to 3 halogen or hydroxyl groups; more preferably selected from the group consisting of -F and -Cl; most preferably -F);
R^{2**} is selected from the group consisting of -H, -F, -Cl, -Br, -OCH₃, -OCF₃, -CN, -C₁₋₄alkyl optionally substituted by up to 3 halogen or hydroxyl groups, -S(O)C₁₋₄alkyl,-S(O)₂C₁₋₄alkyl, -S(O)₂NHC₁₋₄alkyl, -S(O)₂N(C₁₋₄alkyl)₂, -NHC₁₋₄alkyl, -N(C₁₋₄alkyl)₂,-NHC(O)C₁₋₄alkyl, -NHC(O)CF₃, and -NHS(O)₂C₁₋₄alkyl (preferably selected from the group consisting of -H, -F, -Cl, -Br, -OCH₃, -OCF₃, -CN, -C₁₋₄alkyl optionally substituted by up to 3 halogen or hydroxyl groups; more preferably selected from the group consisting of -H, -F and-CI); and
wherein each of R¹, q, E, J, G, K, Q, M, R³, R⁴, R⁵, R⁶, R⁷, R⁸, R⁹, R¹⁰ and R¹¹ are as defined in Formula (I).

Suitably, the NMT inhibitor is a compound of formula (IA***) shown below: 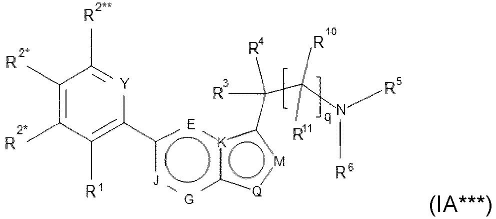 wherein each of R¹, R^{2*}, R^{2**}, q, E, J, G, K, Q, M, R³, R⁴, R⁵, R⁶, R⁷, R⁸, R⁹, R¹⁰ and R¹¹ are as defined hereinabove.

In a particular embodiment, the NMT inhibitor of the invention is a compound of Formula (IA**) or (IB) shown below: 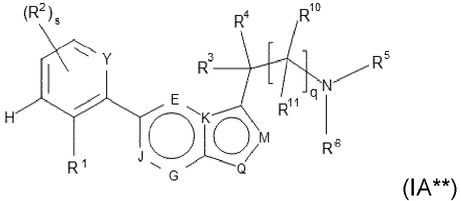 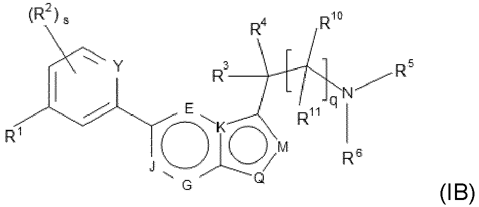 wherein s is 0, 1 or 2 for Formula (IA**); and s is 0, 1, 2 or 3 for Formula (IB); and wherein R¹, R², q, E, J, G, K, Q, M, R³, R⁴, R⁵, R⁶, R⁷, R⁸, R⁹, R¹⁰ and R¹¹ are each as defined in Formula (I).

Suitably, at least two of the variable ring atoms in the core bicyclic moiety of Formula (I), or any one of sub-formulae IA, Iα, IB, Iβ, IA*, IA** and IA***, are carbon. In other words, at least two of E, J, G, K, Q and M are selected from the group consisting of C(R⁷) and carbon (with it being possible for E, J, G, Q and M to be C(R⁷), and it being possible for K to be carbon).

Suitably, at least one of the variable ring atoms in the core bicyclic moiety of Formula (I), or any one of sub-formulae IA, Iα, IB, Iβ, IA*, IA** and IA***, is nitrogen. In other words at least one of E, J, G, K, Q and M is selected from the group consisting of nitrogen and N(R⁸). By way of explanation, K is either carbon or nitrogen and, where K is carbon, either either Q is N(R⁸) and M is nitrogen or C(R⁷), or Q is nitrogen and M is N(R⁸).

In one preferred embodiment of the NMT inhibitor of the invention, E, J and G are each C(R⁷), K is carbon, Q is N(R⁸), and M is nitrogen.

In one preferred embodiment of the NMT inhibitor of the invention, E, J and G are each C(R⁷), and K, Q and M are each nitrogen.

In one preferred embodiment of the NMT inhibitor of the invention, E and G are each C(R⁷), and J, K, Q and M are each nitrogen.

In one preferred embodiment of the NMT inhibitor of the invention, J and G are each C(R⁷), and E, K, Q and M are each nitrogen.

In one preferred embodiment of the NMT inhibitor of the invention, E, J, G and M are each C(R⁷), and K and Q are each nitrogen.

More preferably, E, J and G are each C(R⁷), K is carbon, Q is N(R⁸), and M is nitrogen; E, J and G are each C(R⁷), and K, Q and M are each nitrogen; or E, J, G and M are each C(R⁷), and K and Q are each nitrogen. Most preferably E, J and G are each C(R⁷), K is carbon, Q is N(R⁸), and M is nitrogen; or E, J and G are each C(R⁷), and K, Q and M are each nitrogen.

In certain embodiments Y is -CH- or -C(R^{2'})-; preferably Y is -CH-. In another embodiment Y is -N-.

In one preferred embodiment, s is 0, 1 or 2, and, where present, each R² is independently selected from the group consisting of -F, -Cl, -Br, -OCH₃, -OCF₃, -CN, and -C₁₋₄alkyl optionally substituted by up to 3 halogen or hydroxyl groups. More preferably R² is F or Cl.

In one preferred embodiment, A is an aromatic carbocycle or heterocyclel selected from the group consisting of phenyl, pyridinyl, quinolinyl, imidazolyl, benzimidazolyl, pyrazolyl, thiazolyl, 1,2,3-triazolyl and 1,2,4-triazolyl, said aromatic carbocycle or heterocycle being optionally substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups; C(O)N(R⁹)₂ (for example -C(O)N(H)C₁₋₄alkyl or - C(O)N(R⁹)₂ wherein the two R⁹ groups and the N they are bonded to form a 4 to 7 membered non-aromatic heterocycle, the heterocycle optionally comprising 1 or 2 further heteroatoms selected from N, O and S (for example wherein the two R⁹ and the N they are bonded to form a morpholine or pyrrolidine ring)),-CH₂C(O)N(R⁹)₂ (for example -CH₂C(O)N(H)C₁₋₄alkyl), -CH-₂C(O)N(R⁹)₂ (for example -CH₂C(O)N(H)C₁₋₄alkyl), -C(O)N(R¹³)C₁₋₄alkylOC₁₋₄alkyl (for example -C(O)N(H)C₁₋₄alkylOC₁₋₄alkyl), -N(R⁹)C(O)C₁₋₄alkyl, -CH₂N(R¹³)₂, CH₂N(R⁹)C(O)C₁₋₄alkyl or CH₂N(R¹³)S(O)₂C₁₋₄alkyl; and more preferably C(O)N(R⁹)₂ (for example -C(O)N(H)C₁₋₄alkyl),or -CH₂C(O)N(R⁹)₂ (for example -CH₂C(O)N(H)C₁₋₄alkyl). Preferably A is selected from the group consisting optionally substituted pyrazolyl and thiazolyl.

In one preferred embodiment, A is optionally substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups (preferably one -OC₁₋₄alkyl group; more preferably one -OCH₃ group); C(O)N(R⁹)₂ (for example -C(O)N(H)C₁₋₄alkyl or -C(O)N(R⁹)₂ wherein the two R⁹ groups and the N they are bonded to form a 4 to 7 membered non-aromatic heterocycle, the heterocycle optionally comprising 1 or 2 further heteroatoms selected from N, O and S (for example wherein the two R⁹ and the N they are bonded to form a morpholine or pyrrolidine ring)),-CH₂C(O)N(R⁹)₂ (for example -CH₂C(O)N(H)C₁₋₄alkyl), -CH₂C(O)N(R⁹)₂ (for example -CH₂C(O)N(H)C₁₋₄alkyl),-C(O)N(R¹³)C₁₋₄alkylOC₁₋₄alkyl (for example -C(O)N(H)C₁₋₄alkylOC₁₋₄alkyl), -N(R⁹)C(O)C₁₋₄alkyl, -CH₂N(R¹³)₂, CH₂N(R⁹)C(O)C₁₋₄alky or CH₂N(R¹³)S(O)₂C₁₋₄alkyl I; preferably C(O)N(R⁹)₂ (for example -C(O)N(H)C₁₋₄alkyl),-CH₂C(O)N(R⁹)₂ (for example -CH₂C(O)N(H)C₁₋₄alkyl). Preferably A is selected from the group consisting optionally substituted pyrazolyl and thiazolyl.

In certain preferred embodiments, A is optionally substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups (preferably one -OC₁₋₄alkyl group; more preferably one-OCH₃ group); -C(O)N(H)C₁₋₄alkyl (for example -C(O)N(H)CH₃) or -C(O)N(R⁹)₂ wherein the two R⁹ groups and the N they are bonded to form a 4 to 7 membered non-aromatic heterocycle, the heterocycle optionally comprising 1 or 2 further heteroatoms selected from N, O and S (for example wherein the two R⁹ and the N they are bonded to form a morpholine or pyrrolidine ring), -CH₂C(O)N(H)C₁₋₄alkyl, -CH-₂C(O)N(H)C₁₋₄alkyl, -C(O)N(H)C₁₋₄alkylOC₁₋₄alkyl, -N(R⁹)C(O)C₁₋₄alky, -CH₂N(R¹³)₂, CH₂N(R⁹)C(O)C₁₋₄alkyl or CH₂N(R¹³)S(O)₂C₁₋₄alkyl. Preferably A is selected from the group consisting optionally substituted pyrazolyl and thiazolyl.

In even more preferred embodiments, A is optionally substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups (preferably one -OC₁₋₄alkyl group; more preferably one-OCH₃ group); -C(O)N(H)C₁₋₄alkyl (for example -C(O)N(H)CH₃) or -C(O)N(R⁹)₂ wherein the two R⁹ and the N they are bonded to form a morpholine or pyrrolidine ring (preferably a morpholine ring), -CH₂C(O)N(H)C₁₋₄alkyl, -CH-₂C(O)N(H)C₁₋₄alkyl, and -C(O)N(H)C₁₋₄alkylOC₁₋₄alkyl; and more preferably substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups; -C(O)N(H)C₁₋₄alkyl (for example -C(O)N(H)CH₃) or -C(O)N(R⁹)₂ wherein the two R⁹ and the N they are bonded to form a morpholine or pyrrolidine ring (preferably a morpholine ring), -C(O)N(H)C₁₋₄alkylOC₁₋₄alkyl and CH₂N(R¹³)S(O)₂C₁₋₄alkyl. Preferably A is selected from the group consisting optionally substituted pyrazolyl and thiazolyl.

In one preferred embodiment, A is optionally substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups preferably one -OC₁₋₄alkyl group; more preferably one -OCH₃ group); C(O)N(R⁹)₂ (for example -C(O)N(H)C₁₋₄alkyl or -C(O)N(R⁹)₂ wherein the two R⁹ groups and the N they are bonded to form a 4 to 7 membered non-aromatic heterocycle, the heterocycle optionally comprising 1 or 2 further heteroatoms selected from N, O and S (for example wherein the two R⁹ and the N they are bonded to form a morpholine or pyrrolidine ring)),-CH₂C(O)N(R⁹)₂ (for example -CH-₂C(O)N(H)C₁₋₄alkyl), -CH₂C(O)N(R⁹)₂ (for example -CH₂C(O)N(H)C₁₋₄alkyl), -C(O)N(R¹³)C₁₋₄alkylOC₁₋₄alkyl (for example -C(O)N(H)C₁₋₄alkylOC₁₋₄alkyl), -N(R⁹)C(O)C₁₋₄alkyl, CH₂N(R⁹)C(O)C₁₋₄alkyl and CH₂N(R¹³)S(O)₂C₁₋₄alkyl. Preferably A is selected from the group consisting optionally substituted pyrazolyl and thiazolyl.

In one preferred embodiment, A is substituted with 1, 2, or 3 groups, and at least one of the substituents is -C₁₋₄alkyl (for example methyl), wherein each -C₁₋₄alkyl is optionally substituted by up to 3 halogen, hydroxyl or -OC₁₋₄alkyl groups (preferably one -OC₁₋₄alkyl group; more preferably one -OCH₃ group); C(O)N(R⁹)₂ (for example -C(O)N(H)C₁₋₄alkyl or - C(O)N(R⁹)₂ wherein the two R⁹ groups and the N they are bonded to form a 4 to 7 membered non-aromatic heterocycle, the heterocycle optionally comprising 1 or 2 further heteroatoms selected from N, O and S (for example wherein the two R⁹ and the N they are bonded to form a morpholine or pyrrolidine ring)),-CH₂C(O)N(R⁹)₂ (for example -CH₂C(O)N(H)C₁₋₄alkyl), -CH-₂C(O)N(R⁹)₂ (for example -CH₂C(O)N(H)C₁₋₄alkyl), -C(O)N(R¹³)C₁₋₄alkylOC₁₋₄alkyl (for example -C(O)N(H)C₁₋₄alkylOC₁₋₄alkyl), -N(R⁹)C(O)C₁₋₄alkyl, -CH₂N(R¹³)₂, CH₂N(R⁹)C(O)C₁₋₄alkyl or CH₂N(H)S(O)₂C₁₋₄alkyl. Preferably A is selected from the group consisting optionally substituted pyrazolyl and thiazolyl.

In one preferred embodiment, A is substituted with 1, 2, or 3 groups, and at least one of the substituents is -CH₂N(R¹³)₂ or C₁₋₄alkyl (for example methyl), wherein each -C₁₋₄alkyl is optionally substituted by up to 3 halogen, hydroxyl or -OC₁₋₄alkyl groups (preferably one -OC₁₋₄alkyl group; more preferably one -OCH₃ group).

In another preferred embodiment, A is substituted with 1, 2, or 3 groups, and at least one of the substituents is C(O)N(R⁹)₂ (for example -C(O)N(H)C₁₋₄alkyl or -C(O)N(R⁹)₂ wherein the two R⁹ groups and the N they are bonded to form a 4 to 7 membered non-aromatic heterocycle, the heterocycle optionally comprising 1 or 2 further heteroatoms selected from N, O and S (for example wherein the two R⁹ and the N they are bonded to form a morpholine or pyrrolidine ring)), CH₂N(R⁹)C(O)C₁₋₄alkyl or CH₂N(R¹³)S(O)₂C₁₋₄alkyl (for example CH₂N(H)S(O)₂C₁₋₄alkyl).

It has been surprisingly found that where A is substituted with one carboxamide containing group, stability of the NMT inhibitor is improved. In one preferred embodiment A is substituted with 1, 2, or 3 groups, and at least one of the substituents is -C(O)N(R⁹)₂, - C(O)N(R¹³)C₁₋₄alkylOC₁₋₄alkyl , -C(O)N(C₁₋₄alkylOC₁₋₄alkyl)₂, -CH₂C(O)N(R⁹)₂, -CH-₂C(O)N(R¹³)C₁₋₄alkylOC₁₋₄alkyl , -CH₂C(O)N(C₁₋₄alkylOC₁₋₄alkyl)₂, -NHC(O)C₁₋₄alkyl,-NHC(O)CF₃, CH₂N(R¹³)C(O)C₁₋₄alkyl. In another preferred embodiment, A is substituted with 1, 2, or 3 groups, and at least one of the substituents is -C(O)N(R⁹)₂, -C(O)N(R¹³)C₁₋₄alkylOC₁₋₄alkyl , -C(O)N(C₁₋₄alkylOC₁₋₄alkyl)₂, -CH₂C(O)N(R⁹)₂, -CH₂C(O)N(R¹³)C₁₋₄alkylOC₁₋₄alkyl ,-CH₂C(O)N(C₁₋₄alkylOC₁₋₄alkyl)₂, -NHC(O)C₁₋₄alkyl,-NHC(O)CF₃, CH₂N(R¹³)C(O)C₁₋₄alkyl, CO₂H, and CH₂N(H)S(O)₂C₁₋₄alkyl.

More preferably, at least one of the substituents is C(O)N(R⁹)₂ (for example-C(O)N(H)C₁₋₄alkyl or -C(O)N(R⁹)₂ wherein the two R⁹ groups and the N they are bonded to form a 4 to 7 membered non-aromatic heterocycle, the heterocycle optionally comprising 1 or 2 further heteroatoms selected from N, O and S (for example wherein the two R⁹ and the N they are bonded to form a morpholine or pyrrolidine ring)),-CH₂C(O)N(R⁹)₂ (for example -CH-₂C(O)N(H)C₁₋₄alkyl), -CH₂C(O)N(R⁹)₂ (for example -CH₂C(O)N(H)C₁₋₄alkyl), -C(O)N(R¹³)C₁₋₄alkylOC₁₋₄alkyl (for example -C(O)N(H)C₁₋₄alkylOC₁₋₄alkyl), -N(R⁹)C(O)C₁₋₄alkyl, CH₂N(R⁹)C(O)C₁₋₄alkyl or CH₂N(R¹³)S(O)₂C₁₋₄alkyl (for example CH₂N(H)S(O)₂C₁₋₄alkyl).

Even more preferably, at least one of the substituents is C(O)N(R⁹)₂ (for example-C(O)N(H)C₁₋₄alkyl) or -CH₂C(O)N(R⁹)₂ (for example -CH₂C(O)N(H)C₁₋₄alkyl). In such embodiments, preferably A is substituted pyrazolyl (such as a 4-pyrazolyl) or thiazolyl (such as a 5-thiazolyl).

In one preferred embodiment, X is O. In another preferred embodiment X is absent.

In one preferred embodiment, L is -(CH₂)ₘ- or -(CH₂)ₘO-; more preferably L is - (CH₂)ₘ-. In one preferred embodiment m is 1 or 2; preferably 2. In one preferred embodiment X is -O-; L is -(CH₂)ₘ and m is 1 or 2.

In one preferred embodiment where, for example, the NMT inhibitor is used as a diagnostic agent for the diagnosis of a disease or disorder in which inhibition of NMT provides a therapeutic or prophylactic effect, or as reference compound in a method of discovering other inhibitors of NMT, L is -(CHR¹²)ₘ- or -(CHR¹²)ₘO-; and one R¹² is a terminal C₁₋₆alkynyl optionally substituted by up to 3 -F, -Cl, -Br, I, -OH, -OCH₃, -OCF₃ or -CN groups, and more preferably one R¹² is a terminal unsubstituted C₁₋₆alkynyl. Preferably, when present, all other R¹² groups are hydrogen.

In one preferred embodiment R⁷ is hydrogen or methyl, and/or R⁸ is hydrogen or methyl.

For the avoidance of doubt, when X is absent and L is present, R¹ is a group of formula -L-A, in which group L is directly bonded to group A and to the phenyl ring shown in formula (I). When X is present and L is absent, R¹ is a group of formula -X-A, in which group X is directly bonded to group A and to the phenyl ring shown in formula (I). When X and L are both absent, R¹ is a group of formula -A, in which group A is directly bonded to the phenyl ring shown in formula (I).

In one preferred embodiment, X is O, L is -(CH₂)ₘ-, m is 1 or 2, and A is an aromatic carbocycle or heterocycle selected from the group consisting of phenyl, pyridinyl, quinolinyl, imidazolyl, benzimidazolyl, pyrazolyl, thiazolyl, 1,2,3-triazolyl and 1,2,4-triazolyl, said aromatic carbocycle or heterocycle being optionally substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups; -C(O)N(R⁹)₂ (for example -C(O)N(H)C₁₋₄alkyl); and -CH₂C(O)N(R⁹)₂ (for example -CH₂C(O)N(H)C₁₋₄alkyl). More preferably A is selected from the group consisting optionally substituted pyrazolyl and thiazolyl.

In one preferred embodiment, X is absent, L is -(CH₂)ₘ-, m is 3, and A is an aromatic carbocycle or heterocycle selected from the group consisting of phenyl, pyridinyl, quinolinyl, imidazolyl, benzimidazolyl, pyrazolyl, thiazolyl, 1,2,3-triazolyl and 1,2,4-triazolyl, said aromatic carbocycle or heterocycle being optionally substituted with 1, 2, or 3 groups independently selected from the group consisting of -C₁₋₄alkyl (for example methyl), wherein each -C₁₋₄alkyl is optionally substituted by up to 3 halogen, hydroxyl or -OC₁₋₄alkyl groups; -C(O)N(R⁹)₂ (for example -C(O)N(H)C₁₋₄alkyl); and -CH₂C(O)N(R⁹)₂ (for example -CH₂C(O)N(H)C₁₋₄alkyl). Preferably A is selected from the group consisting optionally substituted pyrazolyl and thiazolyl.

In one preferred embodiment, R³ and R⁴ are both hydrogen. In another preferred embodiment, R³ is hydrogen and R⁴ is methyl.

In one preferred embodiment, R⁵ and R⁶ are both methyl. In another preferred embodiment, R⁵ and R⁶ are both hydrogen. In another preferred embodiment, R⁵ is hydrogen and R⁶ is methyl.

In another embodiment, q is 0 or 1 and the R³ group and the R⁵ group and the intervening atoms form a 3 to 7 membered non-aromatic heterocycle composed of the intervening atoms and bond, or the intervening atoms and -(CHR^{a})ᵣ-. Preferably a 4 to 6 membered non-aromatic heterocycle is formed; and more preferably a 4 membered non-aromatic heterocycle. In embodiments where a 4 membered non-aromatic heterocycle is formed, preferably q is 1.

In another embodiment, q is 1 and the R¹⁰ group and the R⁵ group and the intervening atoms form a 3 to 7 membered non-aromatic heterocycle composed of the intervening atoms and -(CHR^{a})ᵣ-. Preferably a 4 to 6 membered non-aromatic heterocycle is formed.

It has been surprisingly found that stability, and in particular t_{½}, of the NMT inhibitors according to the invention can be improved when q is 1, and when q is 1 and the R³ group and the R⁵ group and the intervening atoms form a non-aromatic heterocycle composed of the intervening atoms and -(CHR^{a})ᵣ-, for example when a 4 membered ring is formed wherein r is 1. Therefore, in one preferred embodiment q is 1 and the R³ group and the R⁵ group and the intervening atoms form a 4 membered non-aromatic heterocycle composed of the intervening atoms and -(CHR^{a})ᵣ-, wherein r is 1.

It has also been surprisingly found that rapid metabolisim can be achieved when q is 0. Compounds having a short t_{½} can be advantageous to reduce side effects and/or for administration methods in which rapid metabolism is advantageous, for example delivery by inhalation. Therefore, in another preferred embodiment q is 0.

In one preferred embodiment, A is phenyl, X is -O-; L is -(CH₂)ₘ-; m is 1 or 2; s is 0; E, J and G are each C(R⁷); K is carbon; Q is N(R⁸); M is nitrogen; R³ and R⁴ are each hydrogen; R⁵ and R⁶ are each methyl; and R⁸ is hydrogen.

In one preferred embodiment, A is 3-pyridinyl.

In one preferred embodiment, the NMT inhibitor is a compound of Formula (IA') or (IB') shown below: 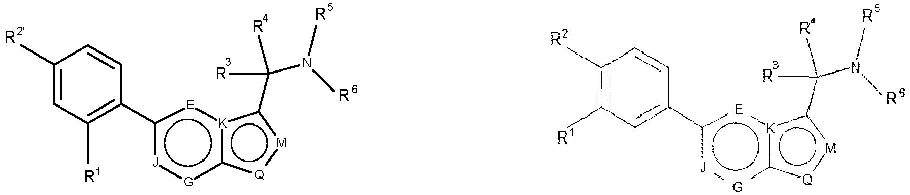 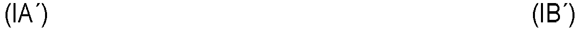 wherein R¹ is a group of formula -X-L-A; A is 3-pyridinyl; X is -O-; L is -(CH₂)ₘ-; m is 1 or 2; R^{2'} is hydrogen or fluorine; Q is N(R⁸); M is nitrogen; E, J and G are each C(R⁷); K is carbon; R³ and R⁴ are each hydrogen; R⁵ and R⁶ are each methyl; and R⁸ is hydrogen.

In one preferred embodiment, A is 4-quinolinyl. In one preferred embodiment, A is 4-quinolinyl; X is -O-; L is -(CH₂)ₘ-; m is 1 or 2; s is 0; E, J and G are each C(R⁷); K is carbon; Q is N(R⁸); M is nitrogen; R³ and R⁴ are each hydrogen; R⁵ and R⁶ are each methyl; and R⁸ is hydrogen.

In one preferred embodiment, A is 1-imidazolyl, said imidazolyl being optionally substituted by 1 or 2 methyl groups.

In one preferred embodiment, the NMT inhibitor is a compound of Formula (IA') or (IB') shown below: 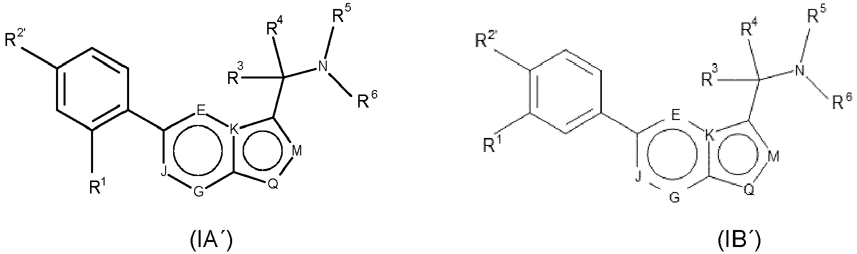 wherein R¹ is a group of formula -X-L-A; A is 1-imidazolyl, said imidazolyl being optionally substituted by 1 or 2 methyl groups; X is -O-; L is -(CH₂)ₘ-; m is 1 or 2; R^{2'} is hydrogen or fluorine; E, J and G are each C(R⁷); K is carbon; Q is N(R⁸); M is nitrogen; R³ and R⁴ are each hydrogen; R⁵ and R⁶ are each methyl; and R⁸ is hydrogen or methyl.

In one preferred embodiment, A is 1-benzimidazolyl.

In one preferred embodiment, the NMT inhibitor is a compound of Formula (IA') shown below: 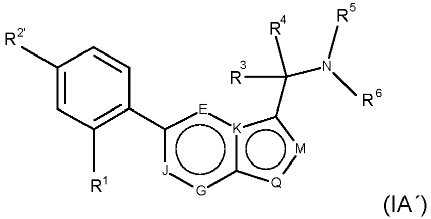 wherein R¹ is a group of formula -X-L-A; A is 1-benzimidazolyl; X is -O-; L is -(CH₂)ₘ-; m is 1 or 2; R^{2'} is fluorine or hydrogen; E, J and G are each C(R⁷); K is carbon; Q is N(R⁸); M is nitrogen; R³ and R⁴ are each hydrogen; R⁵ and R⁶ are each methyl; and R⁸ is hydrogen.

In one preferred embodiment, A is an optionally substituted 4-pyrazolyl, such as a 4-pyrazolyl optionally substituted by up to 3 substituents independently selected from the group consisting of -C₁₋₄alkyl, wherein each -C₁₋₄alkyl is optionally substituted by up to 3 halogen, hydroxyl or -OC₁₋₄alkyl groups; -C(O)N(R⁹)₂ (for example -C(O)N(H)C₁₋₄alkyl), and -CH-₂C(O)N(R⁹)₂ (for example -CH₂C(O)N(H)C₁₋₄alkyl); and R⁹ where present is each selected from the group consisting of hydrogen and -C₁₋₄alkyl, or two R⁹ groups and the N they are bonded to from a 4 to 7 membered non-aromatic heterocycle, the heterocycle optionally comprising 1 or 2 further heteroatoms selected from N, O and S. Preferably A is 4-pyrazolyl optionally substituted by up to 3 -C₁₋₄alkyl; -CH₂OC₁₋₄alkyl, CF₂H, CF₃, C(O)N(Me)₂, -C(O)-1-pyrazole; or -C(O)-4-morpholine groups. More preferably A is 4-pyrazolyl substituted with one or two methyl groups and one -C₁₋₄alkyl; -CH₂OC₁₋₄alkyl, CF₂H, CF₃, C(O)N(Me)₂, -C(O)-1-pyrazole; or -C(O)-4-morpholine group.

In one preferred embodiment, the NMT inhibitor is a compound of Formula (IA"), such as, for example Formula (Iα"), shown below: 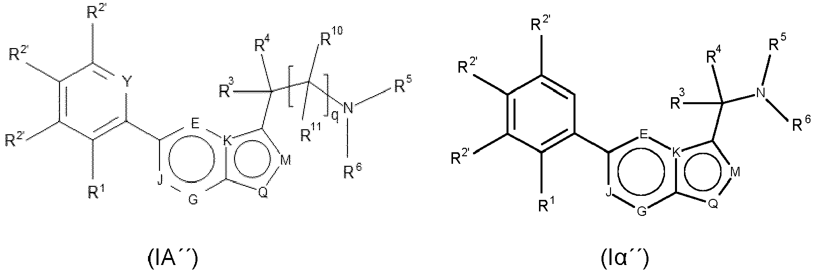
wherein R¹ is a group of formula -X-L-A; A is 4-pyrazolyl, said pyrazolyl being optionally substituted with up to 3 methyl groups; X is -O- or absent (preferably -O-); L is -(CH₂)ₘ- or - (CH₂)ₘ-O- (preferably -(CH₂)ₘ-); m is 1, 2 or 3 (preferably 1 or 2); each R² is independently selected from the group consisting of hydrogen, fluorine, chlorine, -CN and methyl; R³ and R⁴ are each hydrogen; R³ is hydrogen or methyl; R⁴ is hydrogen or methyl; R⁵ is hydrogen or methyl; R⁶ is hydrogen or methyl; when present R¹⁰ is hydrogen or methyl; when present R¹¹ is hydrogen or methyl;
or the R³ group and the R⁵ group and the intervening atoms form a 3 to 7 membered non-aromatic heterocycle composed of the intervening atoms and bond, or the intervening atoms and -(CHR^{a})ᵣ-; or the R¹⁰ group and the R⁵ group and the intervening atoms form a 3 to 7 membered non-aromatic heterocycle composed of the intervening atoms and -(CHR^{a})ᵣ-; (more preferably at least one of R⁵ and R⁶ is methyl, most preferably R⁵ and R⁶ are both methyl);
r is 1, 2, 3, 4 or 5; R^{a} is hydrogen or methyl;
R⁷ where present is hydrogen or methyl; R⁸ where present is hydrogen or methyl; and E, J, G, K, Q and M are as defined in formula (I).

Within the above embodiment, preferably:
i) E, J and G are each C(R⁷), K is carbon, Q is N(R⁸), M is nitrogen; and R⁸ is hydrogen or methyl;
ii) E, J and G are each C(R⁷), and K, Q and M are each nitrogen;
iii) E and G are each C(R⁷), and J, K, Q and M are each nitrogen;
iv) J and G are each C(R⁷), and E, K, Q and M are each nitrogen; or
v) E, J, G and M are each C(R⁷), and K and Q are each nitrogen; and more preferably E, J and G are each C(R⁷), and K, Q and M are each nitrogen; E, J and G are each C(R⁷), K is carbon, Q is N(R⁸), M is nitrogen, and R⁸ is hydrogen or methyl; or E, J, G and M are each C(R⁷), and K and Q are each nitrogen.

Also within that embodiment, preferably, Y is -CH- or -C(R²)-.

Also within that embodiment, the NMT inhibitor may:
1) have formula (IA^) shown below: 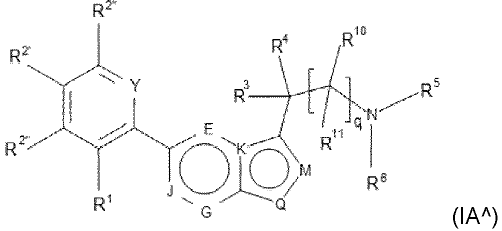 and wherein R^{2'} is selected from the group consisting of fluorine, chlorine, -CN and methyl (preferably fluorine); and R^{2"} is selected from the group consisting of hydrogen, fluorine, chlorine, -CN and methyl; or
2) have formula (IA^^) shown below: 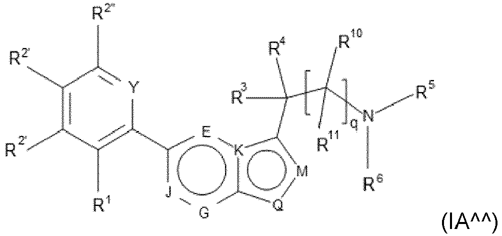 wherein R^{2'} is selected from the group consisting of fluorine, chlorine, -CN and methyl (preferably fluorine); and R^{2"} is selected from the group consisting of hydrogen, fluorine, chlorine, -CN and methyl; or
3) have the formula (IA^^^) shown below: 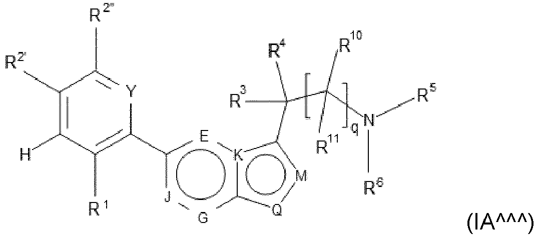 wherein R^{2'} is selected from the group consisting of fluorine, chlorine, -CN and methyl (preferably fluorine); and R^{2"} is selected from the group consisting of hydrogen, fluorine, chlorine, -CN and methyl.

In certain embodiments within the above-mentioned embodiment, R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2; R^{2'} is selected from the group consisting of fluorine, chlorine -CN and methyl (preferably fluorine); q is 0; R³ is hydrogen or methyl; R⁴ is hydrogen or methyl; R⁵ is hydrogen or methyl; R⁶ is hydrogen or methyl; or the R³ group and the R⁵ group and the intervening atoms form a 3 to 7 membered non-aromatic heterocycle composed of the intervening atoms and bond, or the intervening atoms and -(CHR^{a})ᵣ-; (more preferably R⁵ and R⁶ are both methyl); R⁷ where present is hydrogen or methyl; R⁸ where present is hydrogen or methyl; and E, J, G, K, Q and M are as defined in formula (I); and, preferably
i) E, J and G are each C(R⁷), K is carbon, Q is N(R⁸), M is nitrogen; and R⁸ is hydrogen or methyl;
ii) E, J and G are each C(R⁷), and K, Q and M are each nitrogen;
iii) E and G are each C(R⁷), and J, K, Q and M are each nitrogen;
iv) J and G are each C(R⁷), and E, K, Q and M are each nitrogen; or
v) E, J, G and M are each C(R⁷), and K and Q are each nitrogen.

In certain embodiments of the above-mentioned embodiment, R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2; R^{2'} is fluorine; R³ and R⁴ are each hydrogen; q is 0, R⁵ and R⁶ are each independently hydrogen or methyl (more preferably R⁵ and R⁶ are both methyl); or the R³ group and the R⁵ group and the intervening atoms form a 3 to 7 membered non-aromatic heterocycle composed of the intervening atoms and bond, or the intervening atoms and -(CHR^{a})ᵣ-; R⁷ where present is hydrogen or methyl; R⁸ is methyl; K is carbon; Q is N(R⁸); M is nitrogen; and E, J and G are as defined in formula (I); or

R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2; R² is fluorine; R³ and R⁴ are each hydrogen; R⁵ and R⁶ are each independently hydrogen or methyl (more preferably R⁵ and R⁶ are both methyl); R⁷ where present is hydrogen or methyl; Q, M and K are each nitrogen; and E, J and G are each C(R⁷).

In one preferred embodiment, the NMT inhibitor is a compound of Formula (IA') shown below: 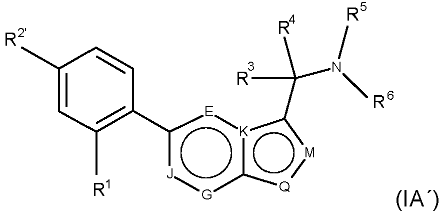
wherein R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2;
R^{2'} is selected from the group consisting of fluorine, chlorine -CN and methyl (preferably fluorine); R³ is hydrogen or methyl; R⁴ is hydrogen or methyl; R⁵ is hydrogen or methyl; R⁶ is hydrogen or methyl; or
the R³ group and the R⁵ group and the intervening atoms form a 3 to 7 membered non-aromatic heterocycle composed of the intervening atoms and bond, or the intervening atoms and -(CHR^{a})ᵣ-; (more preferably R⁵ and R⁶ are both methyl); R⁷ where present is hydrogen or methyl; R⁸ where present is hydrogen or methyl; and E, J, G, K, Q and M are as defined in formula (I).

Within that embodiment, preferably:
i) E, J and G are each C(R⁷), K is carbon, Q is N(R⁸), M is nitrogen; and R⁸ is hydrogen or methyl;
ii) E, J and G are each C(R⁷), and K, Q and M are each nitrogen;
iii) E and G are each C(R⁷), and J, K, Q and M are each nitrogen;
iv) J and G are each C(R⁷), and E, K, Q and M are each nitrogen; or
v) E, J, G and M are each C(R⁷), and K and Q are each nitrogen.

In one preferred embodiment, the NMT inhibitor is a compound of Formula (IA') shown below: 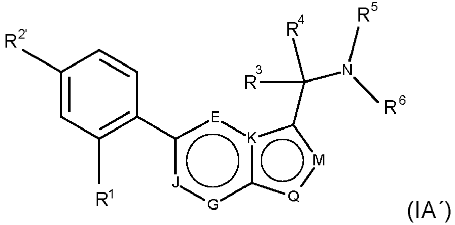 wherein R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2; R^{2'} is fluorine; R³ and R⁴ are each hydrogen; R⁵ and R⁶ are each independently hydrogen or methyl (more preferably R⁵ and R⁶ are both methyl); R⁷ where present is hydrogen or methyl; R⁸ is methyl; K is carbon; Q is N(R⁸); M is nitrogen; and E, J and G are as defined in formula (I).

In one preferred embodiment, the NMT inhibitor is a compound of Formula (IA') shown below: 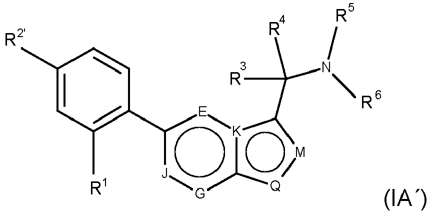
wherein R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2;
R^{2'} is fluorine; R³ and R⁴ are each hydrogen; R⁵ and R⁶ are each independently hydrogen or methyl (more preferably R⁵ and R⁶ are both methyl); R⁷ where present is hydrogen or methyl; Q, M and K are each nitrogen; and E, J and G are each C(R⁷).

In one preferred embodiment, the NMT inhibitor is a compound Formula (IAʺʺ) shown below: 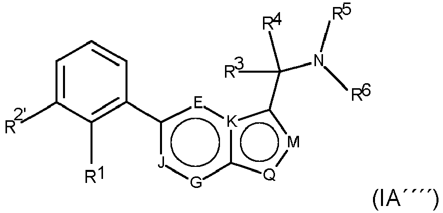 wherein R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2; R^{2'} is selected from the group consisting of fluorine, -CN and methyl; R³ and R⁴ are each hydrogen; R⁵ and R⁶ are each independently hydrogen or methyl (more preferably R⁵ and R⁶ are both methyl); R⁷ where present is hydrogen or methyl; R⁸ where present is hydrogen or methyl; and E, J, G, K, Q and M are as defined in formula (I).

Within that embodiment, preferably:
i) E, J and G are each C(R⁷), K is carbon, Q is N(R⁸), M is nitrogen; and R⁸ is hydrogen or methyl;
ii) E, J and G are each C(R⁷), and K, Q and M are each nitrogen;
iii) E and G are each C(R⁷), and J, K, Q and M are each nitrogen;
iv) J and G are each C(R⁷), and E, K, Q and M are each nitrogen; or
v) E, J, G and M are each C(R⁷), and K and Q are each nitrogen.

In one preferred embodiment, the compound has the formula (IAʺʺ) 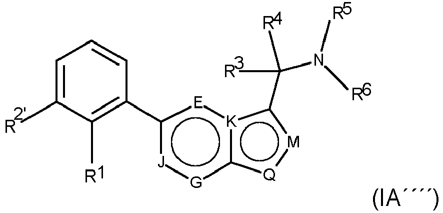 wherein R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2; R^{2'} is fluorine; R³ and R⁴ are each hydrogen; R⁵ and R⁶ are each independently hydrogen or methyl (more preferably R⁵ and R⁶ are both methyl); R⁷ where present is hydrogen or methyl; R⁸ is methyl; K is carbon; Q is N(R⁸); M is nitrogen; and E, J and G are as defined in formula (I).

In one preferred embodiment, the NMT inhibitor is a compound of Formula (IAʺʺ) shown below: 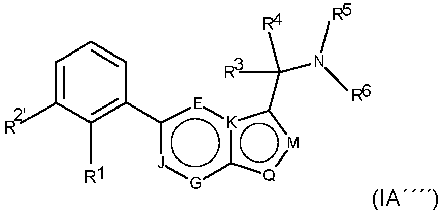 wherein R¹ is a group of formula -X-L-A; A is 4-pyrazolyl, said pyrazolyl being optionally substituted with up to 3 methyl groups; X is -O- or absent; L is -(CH₂)ₘ- or -(CH₂)ₘ-O-; m is 2; R^{2'} is fluorine; R³ and R⁴ are each hydrogen; R⁵ and R⁶ are each independently hydrogen or methyl (more preferably R⁵ and R⁶ are both methyl); R⁷ where present is hydrogen or methyl; Q, M and K are each nitrogen; and E, J and G are each C(R⁷).

In one preferred embodiment, A is an optionally substituted 5-thiazolyl, such as a 5-thiazolyl optionally substituted by 1 or 2 methyl groups.

In one preferred embodiment, the NMT inhibitor is a compound of Formula (IA"), such as, for example, a compound of Formula (Iα"), shown below: 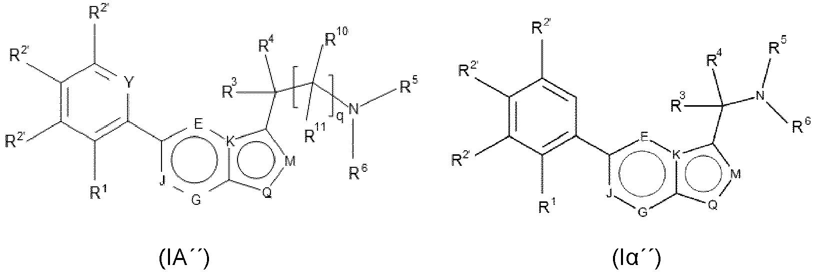
or the NMT inhibitor is a compound of Formula (IA') shown below: 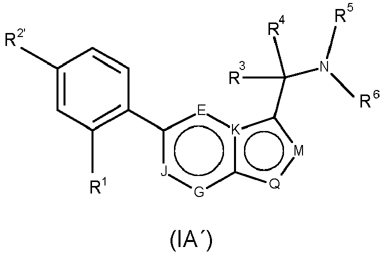
wherein R¹ is a group of formula -X-L-A; A is 5-thiazolyl optionally substituted with 1 or 2 methyl groups (more preferably A is 5-thiazolyl substituted with one methyl group at the 4-position; or substituted with two methyl groups, one at the 2-position and one at the 4-position); X is -O-; L is -(CH₂)ₘ-; m is 1, 2 or 3 (preferably 1 or 2); R^{2'} is hydrogen, chlorine or fluorine (preferably fluorine); R³ is hydrogen or methyl; R⁴ is hydrogen or methyl; R⁵ is hydrogen or methyl; R⁶ is hydrogen or methyl; when present R¹⁰ is hydrogen or methyl; when present R¹¹ is hydrogen or methyl;
or the R³ group and the R⁵ group and the intervening atoms form a 3 to 7 membered non-aromatic heterocycle composed of the intervening atoms and bond, or the intervening atoms and -(CHR^{a})ᵣ-; or the R¹⁰ group and the R⁵ group and the intervening atoms form a 3 to 7 membered non-aromatic heterocycle composed of the intervening atoms and -(CHR^{a})ᵣ-; r is 1, 2, 3, 4 or 5; R^{a} is hydrogen or methyl (preferably R³ and R⁴ are each independently hydrogen or methyl; and R⁵ and R⁶ are each independently hydrogen or methyl;) K is carbon; Q is N(R⁸); M is nitrogen; R⁸ is methyl; and E, J and G are as defined in formula (I).

Also within that embodiment, preferably, Y is -CH- or -C(R^{2'})-.

Also within that embodiment, the NMT inhibitor may:
1) have formula (IA^) shown below: 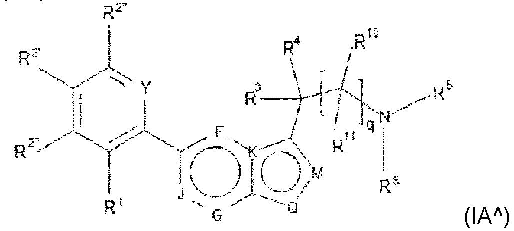 wherein R^{2'} is selected from the group consisting of fluorine and chlorine (preferably fluorine); and R^{2"} is selected from the group consisting of hydrogen, fluorine and chlorine;
2) have formula (IA^^) shown below: 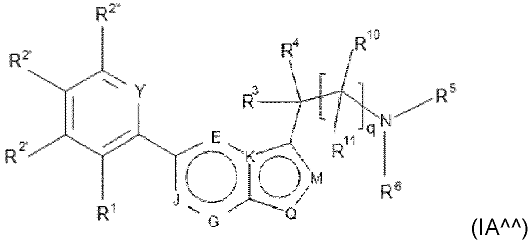 wherein R^{2'} is selected from the group consisting of fluorine and chlorine (preferably fluorine); and R^{2"} is selected from the group consisting of hydrogen, fluorine and chlorine; or
3) have formula (IA^^^) shown below: 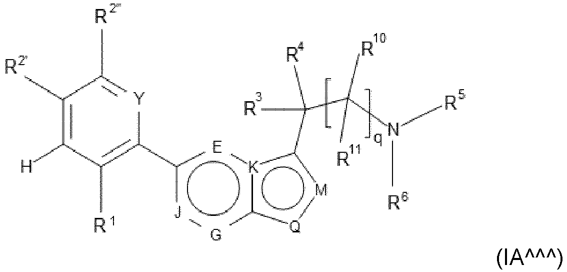 wherein R^{2'} is selected from the group consisting of fluorine and chlorine (preferably fluorine); and R^{2"} is selected from the group consisting of hydrogen, fluorine and chlorine.

In one preferred embodiment, the NMT inhibitor is a compound of Formula (IA') shown below: 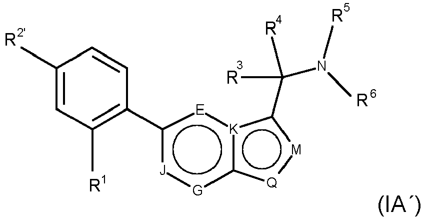 wherein R¹ is a group of formula -X-L-A; A is 5-thiazolyl optionally substituted with 1 or 2 methyl groups (more preferably A is 5-thiazolyl substituted with one methyl group at the 4-position; or substituted with two methyl groups, one at the 2-position and one at the 4-position); X is -O-; L is -(CH₂)ₘ-; m is 1, 2 or 3 (preferably 3); R^{2'} is hydrogen, chlorine or fluorine (preferably fluorine); R³ is hydrogen or methyl; R⁴ is hydrogen or methyl; R⁵ is hydrogen or methyl; R⁶ is hydrogen or methyl; when present R¹⁰ is hydrogen or methyl; when present R¹¹ is hydrogen or methyl; or the R³ group and the R⁵ group and the intervening atoms form a 3 to 7 membered non-aromatic heterocycle composed of the intervening atoms and bond, or the intervening atoms and -(CHR^{a})ᵣ-; or the R¹⁰ group and the R⁵ group and the intervening atoms form a 3 to 7 membered non-aromatic heterocycle composed of the intervening atoms and -(CHR^{a})ᵣ-; r is 1, 2, 3, 4 or 5; R^{a} is hydrogen or methyl (preferably R³ and R⁴ are each independently hydrogen or methyl; and R⁵ and R⁶ are each independently hydrogen or methyl); K, Q and M are each nitrogen; and E, J and G are as defined in formula (I).

In one preferred embodiment, A is selected from the group consisting of optionally substituted 1,2,4-triazol-1-yl, optionally substituted 1,2,4-triazol-4-yl, optionally substituted 1,2,4-triazol-3-yl and optionally substituted 1,2,3-triazol-4-yl. Within that embodiment, preferably X is absent and L is -(CH₂)₃-. A is preferably optionally substituted 1,2,4-triazol-1-yl.

In one preferred embodiment, the NMT inhibitor is a compound of Formula (IA‴) shown below: 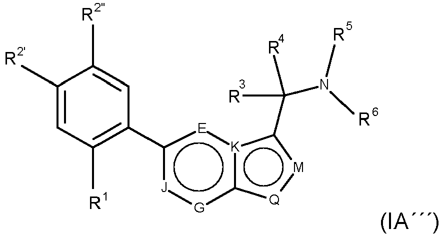 wherein R¹ is a group of formula -X-L-A; A is selected from the group consisting of optionally substituted 1,2,4-triazol-1-yl, optionally substituted 1,2,4-triazol-4-yl, optionally substituted 1,2,4-triazol-3-yl and optionally substituted 1,2,3-triazol-4-yl; X is -O- or absent; L is -(CH₂)ₘ-; m is 2 or 3; R^{2'} is hydrogen or fluorine (more preferably R^{2'} is fluorine); R^{2"} is hydrogen or - OCH₃; R³ and R⁴ are each hydrogen; R⁵ and R⁶ are each methyl; K is carbon; Q is N(R⁸); M is nitrogen; R⁸ is methyl or hydrogen; and E, J and G are as defined in formula (I). Within that embodiment, preferably X is absent and L is -(CH₂)₃-.

In one preferred embodiment, the NMT inhibitor is a compound of Formula (IA‴) shown below: 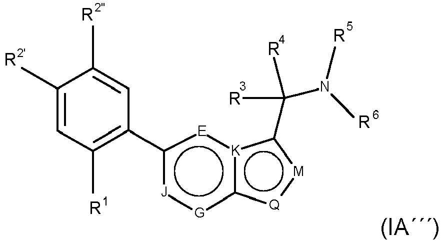 wherein R¹ is a group of formula -X-L-A; A is selected from the group consisting of optionally substituted 1,2,4-triazol-1-yl, optionally substituted 1,2,4-triazol-4-yl, optionally substituted 1,2,4-triazol-3-yl and optionally substituted 1,2,3-triazol-4-yl; X is -O- or absent; L is -(CH₂)ₘ-; m is 2 or 3; R^{2'} is hydrogen or fluorine (more preferably R^{2'} is fluorine); R^{2"} is hydrogen or - OCH₃; R³ and R⁴ are each hydrogen; R⁵ and R⁶ are each methyl; K, Q and M are each nitrogen; and E, J and G are as defined in formula (I). Within that embodiment, preferably X is absent and L is -(CH₂)₃-.

In one preferred embodiment, the NMT inhibitor is a compound of Formula (IA‴) shown below: 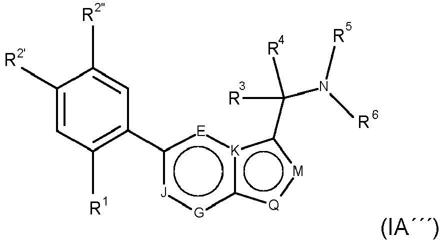 wherein R¹ is a group of formula -X-L-A; A is selected from the group consisting of optionally substituted 1,2,4-triazol-1-yl, optionally substituted 1,2,4-triazol-4-yl, optionally substituted 1,2,4-triazol-3-yl and optionally substituted 1,2,3-triazol-4-yl; X is absent; L is -(CH₂)₃-; R^{2'} is fluorine; R^{2"} is hydrogen or -OCH₃; R³ and R⁴ are each hydrogen; R⁵ and R⁶ are each methyl; K, Q and M are each nitrogen; and E, J and G are as defined in formula (I).

In one embodiment, the NMT inhibitor is a compound of Formula (IB) shown below: 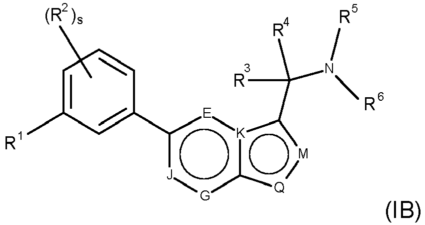 wherein R¹ is a group of formula -X-L-A; X is -O-; L is -(CH₂)ₘ-; m is 1 or 2; A is selected from the group consisting of optionally substituted 3-pyridinyl, 4-pyridinyl and 1-imidazolyl; s is 0; R³ and R⁴ are each hydrogen; R⁵ and R⁶ are each methyl; K is carbon; Q is N(R⁸); M is nitrogen; and R⁸ is hydrogen; and E, J, and G are as defined in formula (I).

In one embodiment, the NMT inhibitor is a compound of Formula (IB), such as, for example, a compound of Formula (Iβ), shown below: 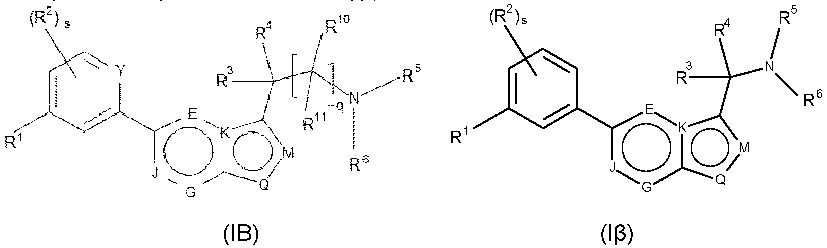 wherein R¹ is a group of formula -X-L-A; X is -O-; L is -(CH₂)ₘ-; m is 1 or 2; A is selected from the group consisting of optionally substituted 3-pyridinyl, 4-pyridinyl and 1-imidazolyl; s is 0 or 1; R² is fluorine; q is 0 or 1 (preferably q is 0); R³ and R⁴ are each hydrogen; R⁵ and R⁶ are each methyl; R¹⁰ and R¹¹ are each hydrogen or methyl; K, Q and M are each nitrogen; and E, J, and G are as defined in formula (I).

In one embodiment, the NMT inhibitor is any one of the following compounds: 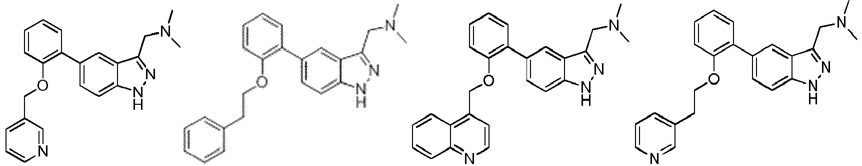 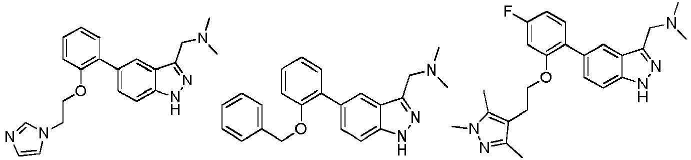 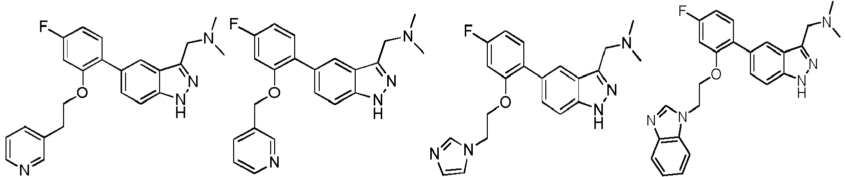 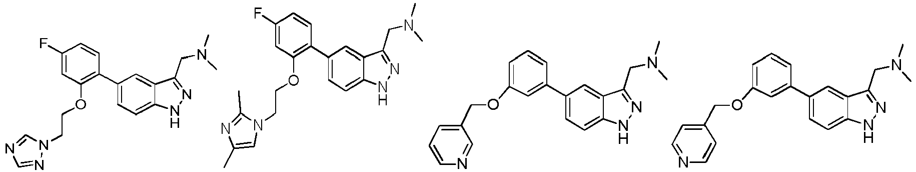 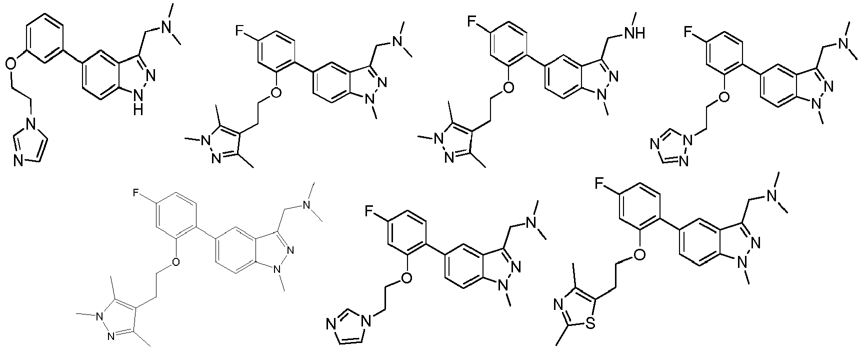 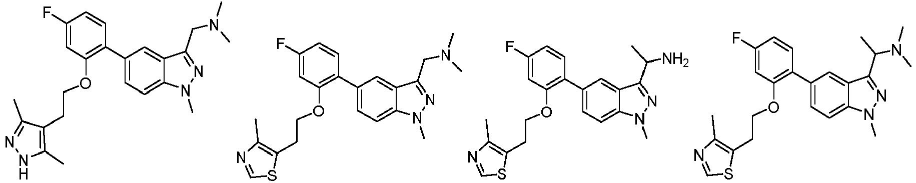 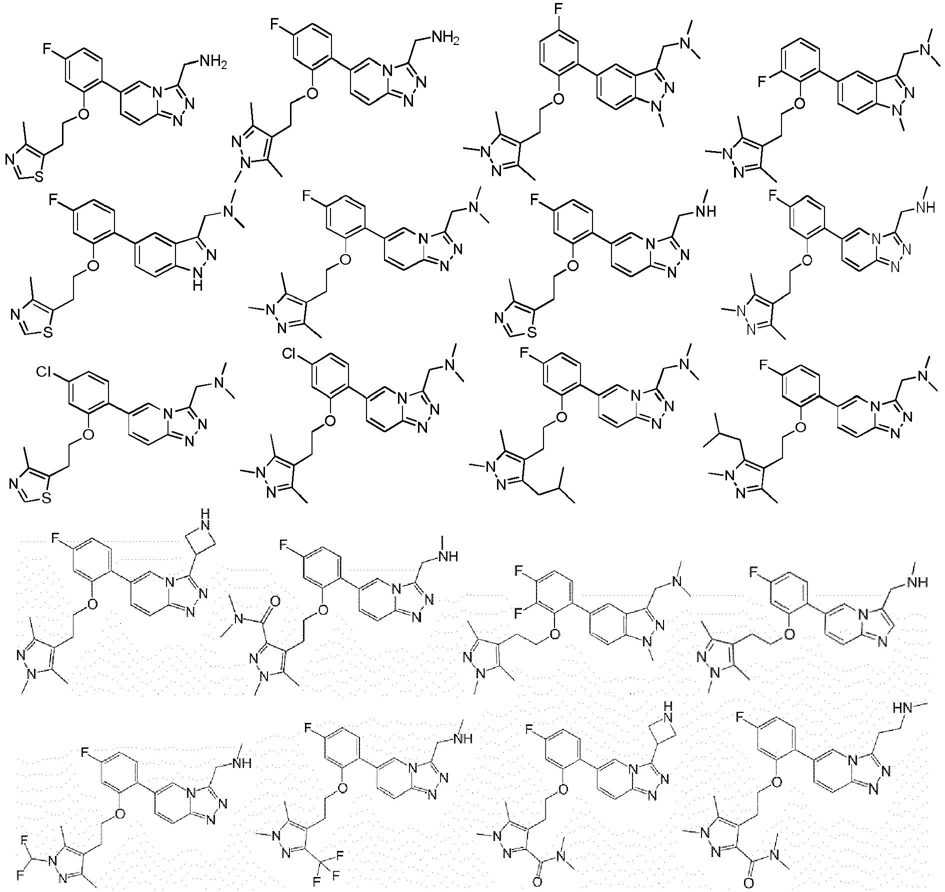 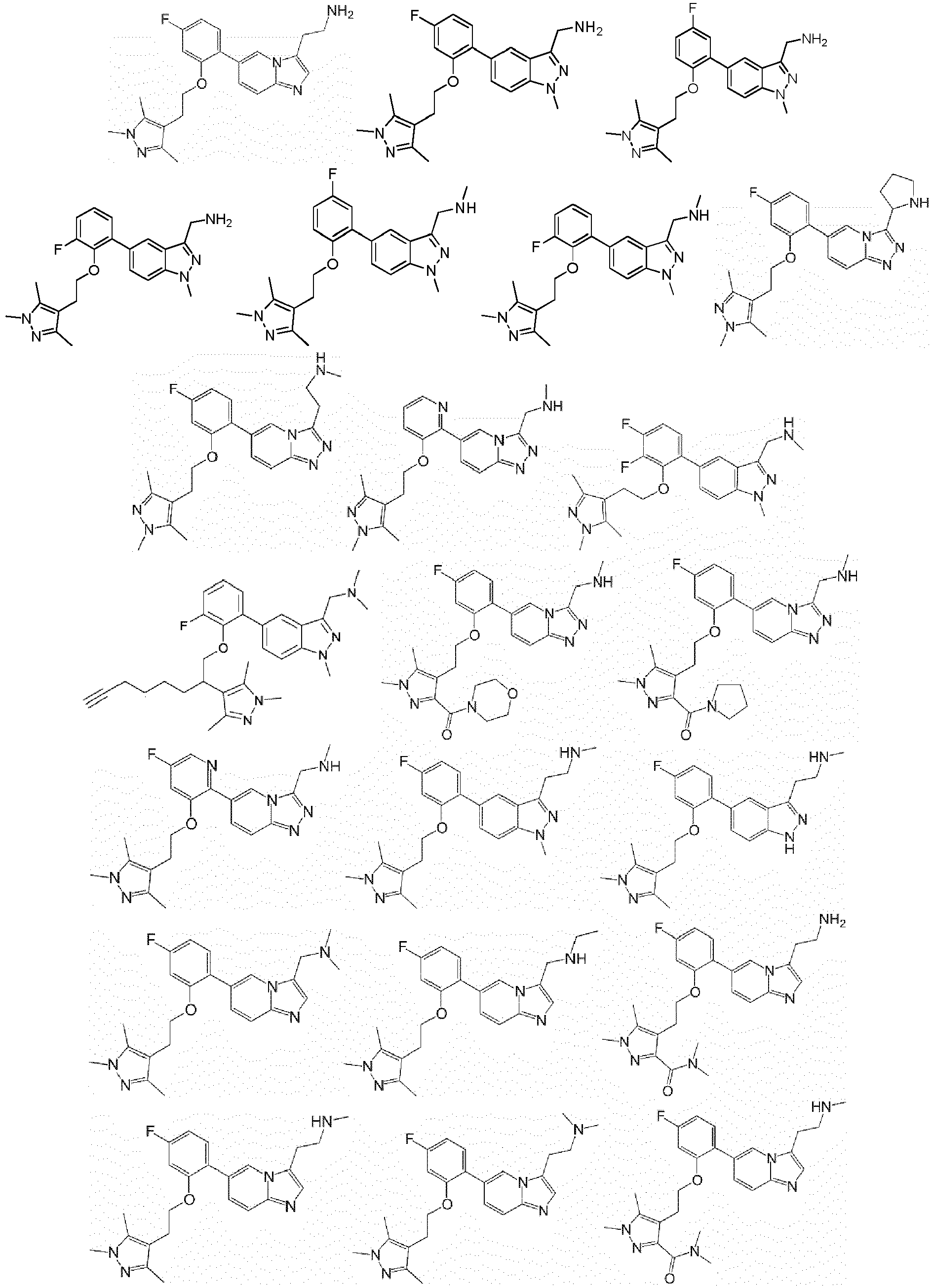 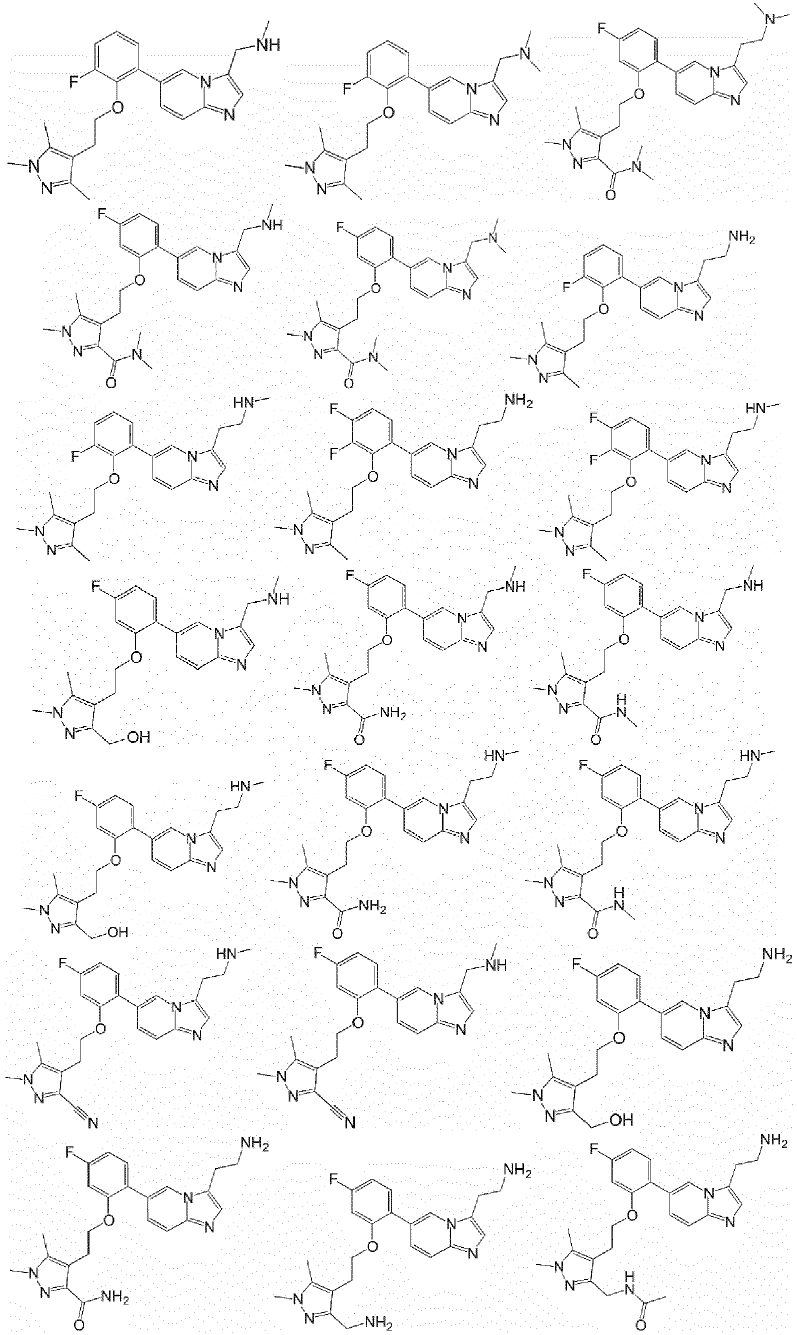 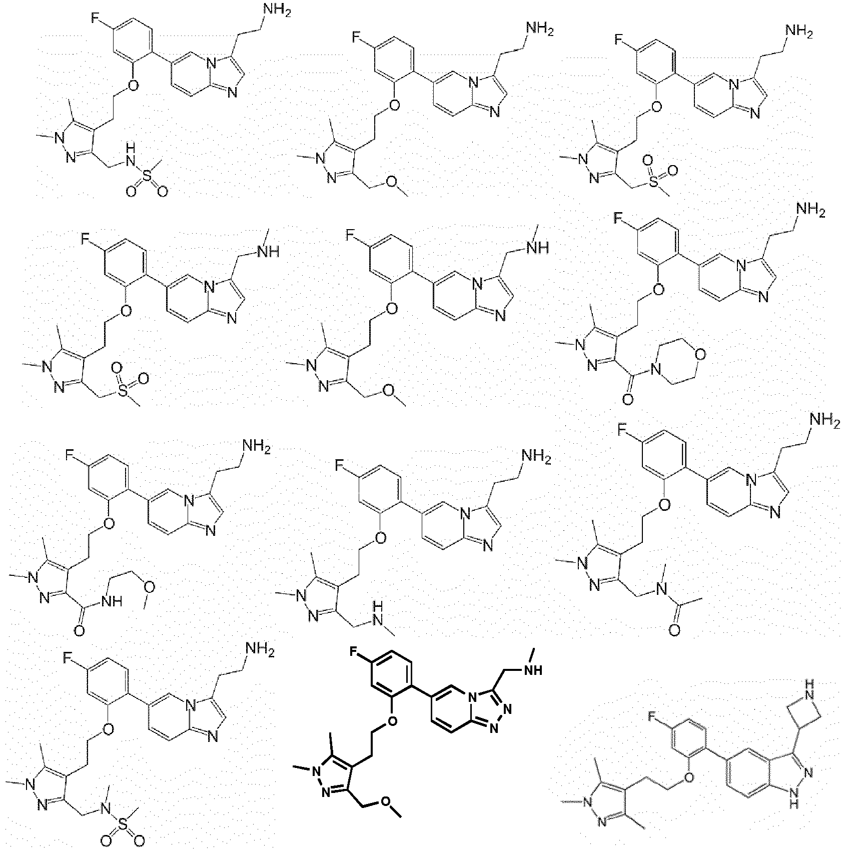 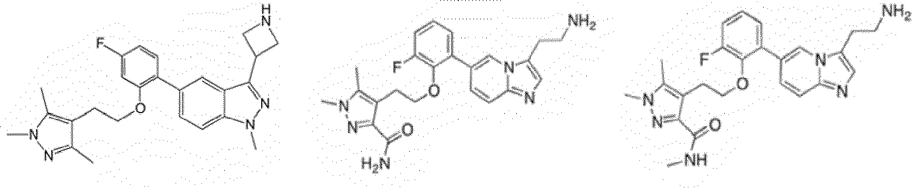 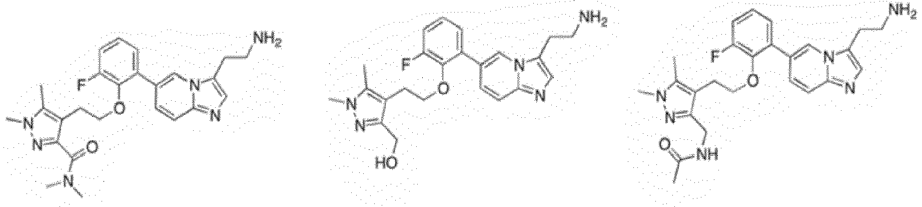 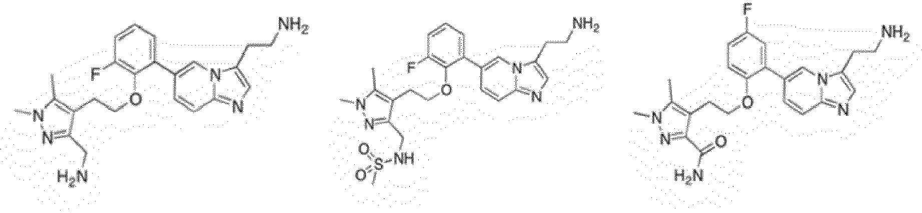 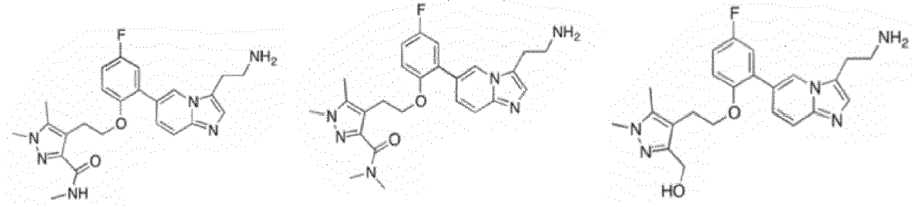 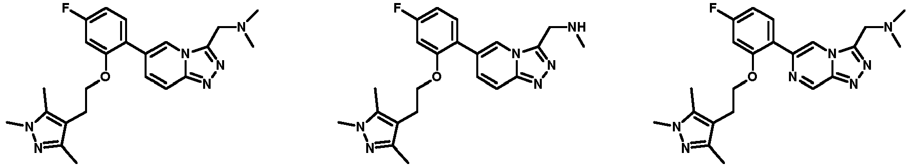 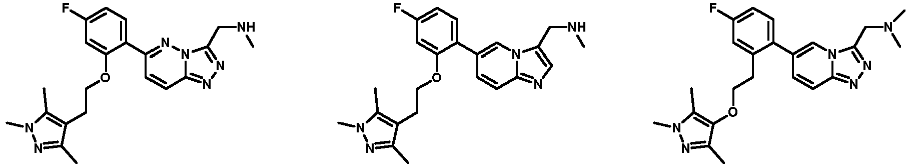 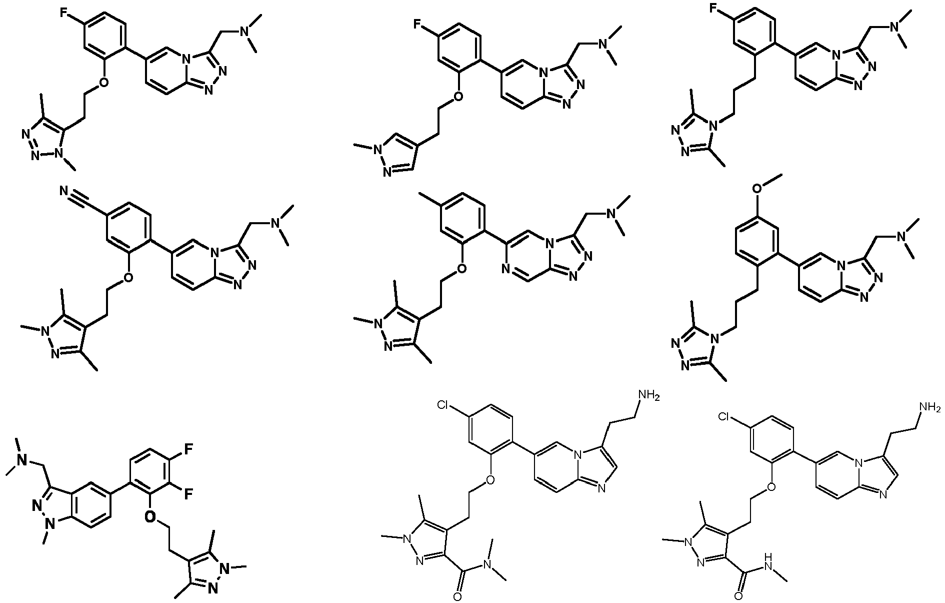 or a pharmaceutically acceptable salt, hydrate or solvate thereof.

In another embodiment, the NMT inhibitor is any one of the following compounds: 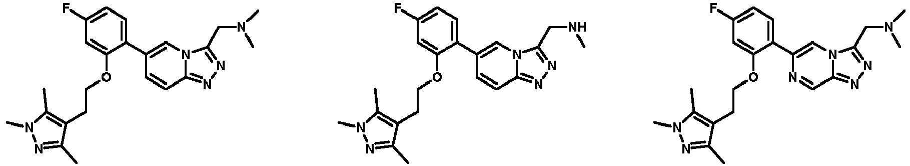 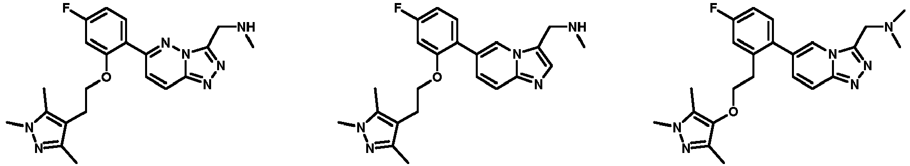 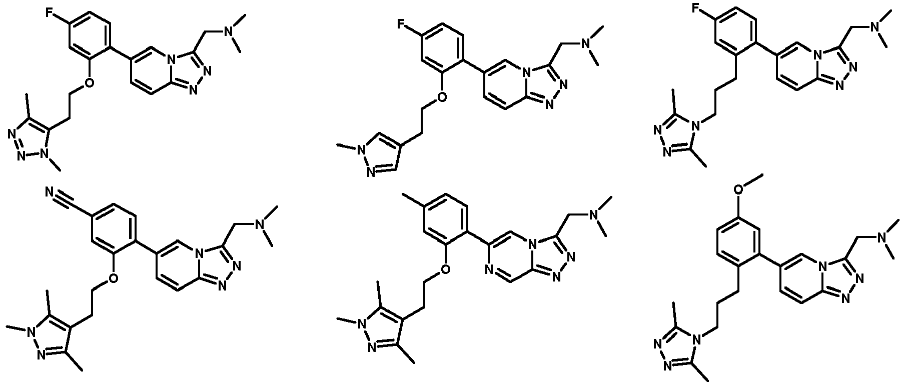 or a pharmaceutically acceptable salt, hydrate or solvate thereof.

In another embodiment, the NMT inhibitor is any one of the following compounds: 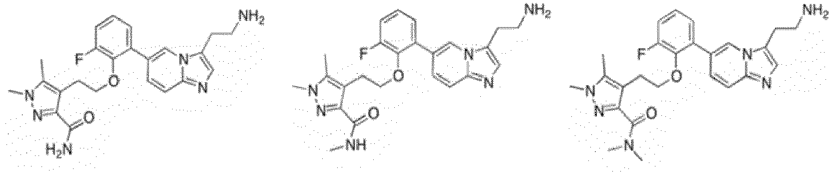 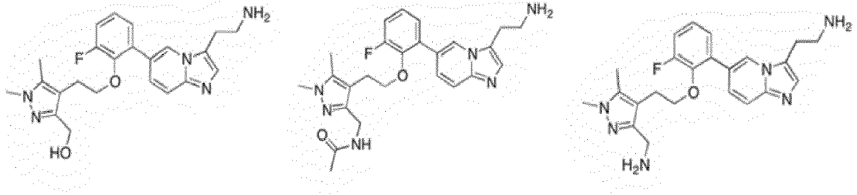 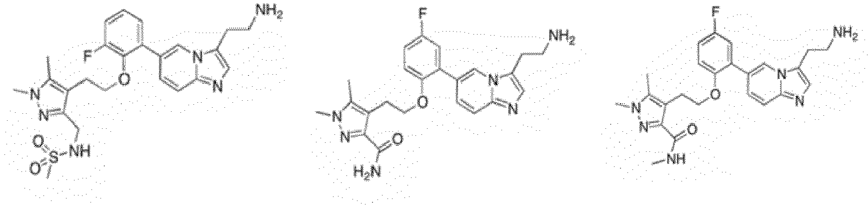 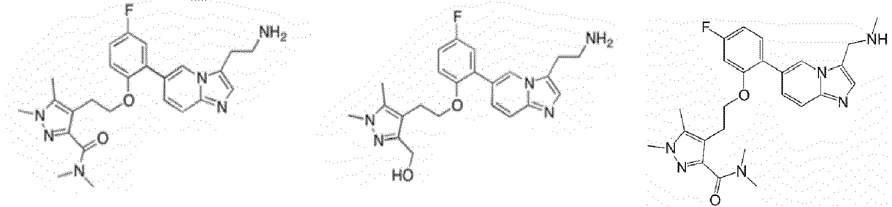 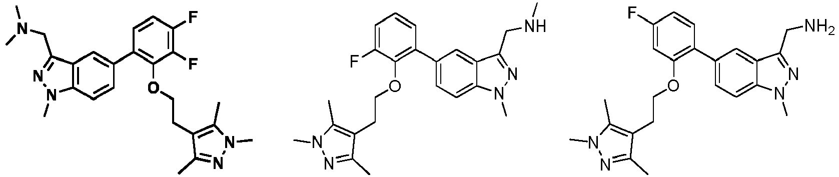 or a pharmaceutically acceptable salt, hydrate or solvate thereof.

In another embodiment, the NMT inhibitor is any one of the following compounds: 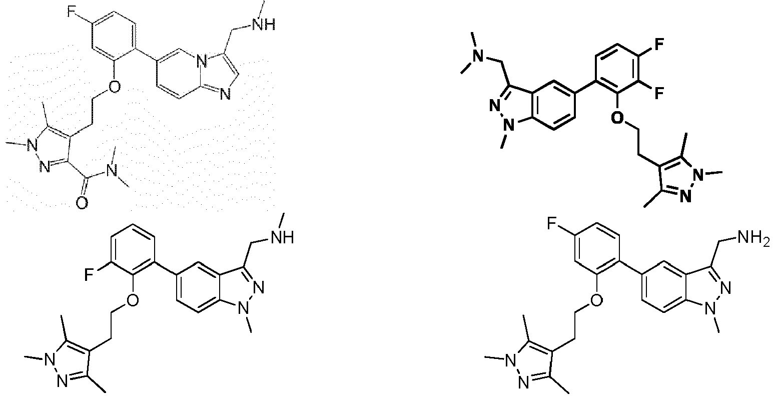 or a pharmaceutically acceptable salt, hydrate or solvate thereof.

In another embodiment, the NMT inhibitor is any one of the following compounds: 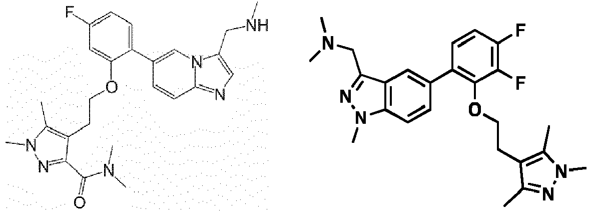 or a pharmaceutically acceptable salt, hydrate or solvate thereof.

In another particular embodiment, the NMT inhibitor is the following compound: 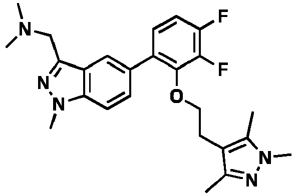 or a pharmaceutically acceptable salt, hydrate or solvate thereof.

In another particular embodiment, the NMT inhibitor is a compound of Formula (Id), shown below, or a pharmaceutically acceptable salt, hydrate or solvate thereof: 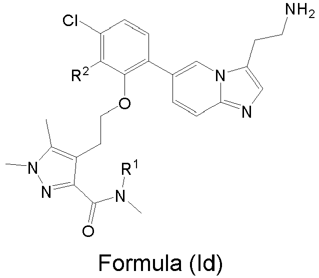 wherein:
R¹ is H or -CH₃; and
R² is H or F.

In another particular embodiment, the NMT inhibitor is selected from any one of the following compounds: 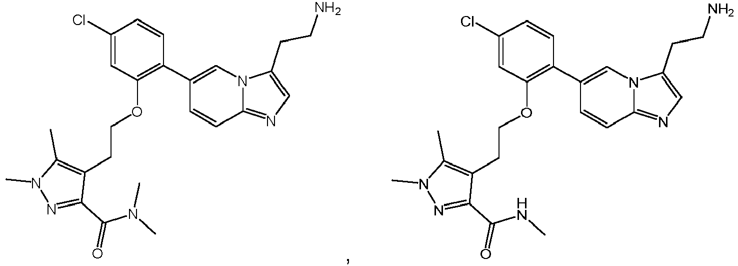 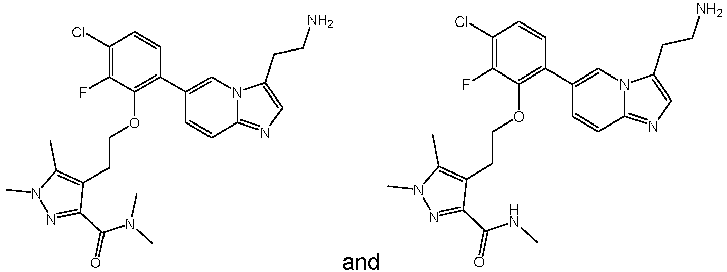 (i.e. 4-(2-{2-[3-(2-aminoethyl)imidazo[1,2-a]pyridin-6-yl]-5-chlorophenoxy}ethyl)-N,N,1,5-tetramethyl-1H-pyrazole-3-carboxamide, 4-(2-{2-[3-(2-aminoethyl)imidazo[1,2-a]pyridin-6-yl]-5-chlorophenoxy}ethyl)-N,1,5-trimethyl-1H-pyrazole-3-carboxamide, 4-(2-{6-[3-(2-aminoethyl)imidazo[1,2-a]pyridin-6-yl]-3-chloro-2-fluorophenoxy}ethyl)-N, N,1,5-tetramethyl-1H-pyrazole-3-carboxamide and 4-(2-{6-[3-(2-aminoethyl)imidazo[1,2-a]pyridin-6-yl]-3-chloro-4-fluorophenoxy}ethyl)-N,1,5-trimethyl-1H-pyrazole-3-carboxamide respectively) or a pharmaceutically acceptable salt, hydrate or solvate thereof.

In another particular embodiment, the NMT inhibitor is selected from any one of the following compounds: 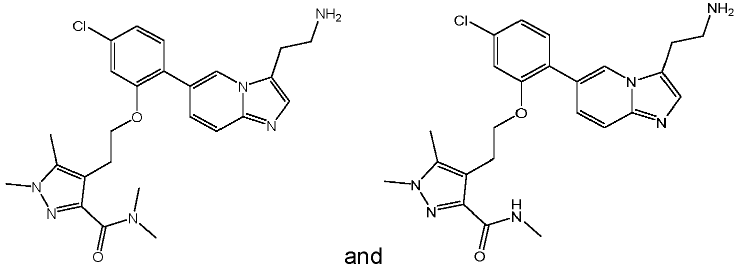 (i.e. 4-(2-{2-[3-(2-aminoethyl)imidazo[1,2-a]pyridin-6-yl]-5-chlorophenoxy}ethyl)-N,N,1,5-tetramethyl-1H-pyrazole-3-carboxamide and 4-(2-{2-[3-(2-aminoethyl)imidazo[1,2-a]pyridin-6-yl]-5-chlorophenoxy}ethyl)-N,1,5-trimethyl-1H-pyrazole-3-carboxamide) or a pharmaceutically acceptable salt, hydrate or solvate thereof.

In another particular embodiment, the NMT inhibitor is the following compound: 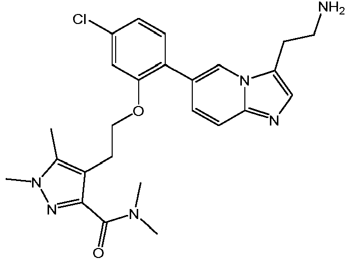 (i.e. 4-(2-{2-[3-(2-aminoethyl)imidazo[1,2-a]pyridin-6-yl]-5-chlorophenoxy}ethyl)-N,N,1,5-tetramethyl-1H-pyrazole-3-carboxamide) or a pharmaceutically acceptable salt, hydrate or solvate thereof.

### NMT inhibitors of Formula (II) and Formula (III)

In certain embodiments, the NMT inhibitor is a compound of Formula (II) or Formula (III) shown below, or a pharmaceutically acceptable salt, hydrate or solvate thereof: 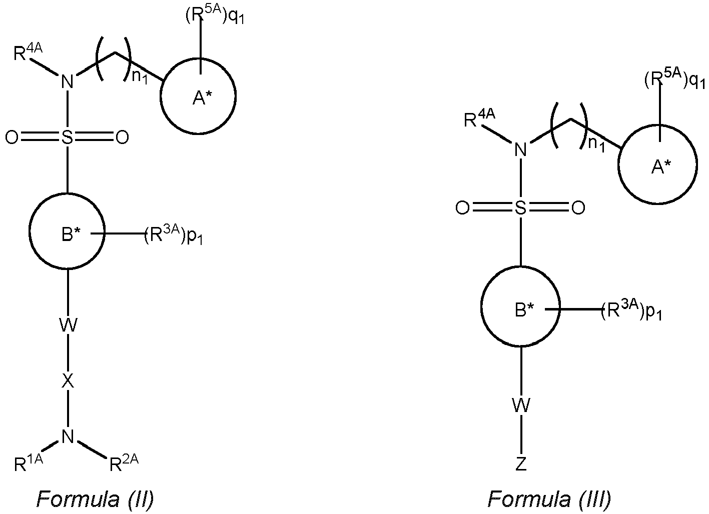 wherein:
n₁ is 0, 1, 2, 3, 4, 5 or 6;
Ring A*, is an optionally substituted nitrogen containing aryl group wherein each substitutable carbon or nitrogen in Ring A* is optionally and independently substituted by one or more R^{5A} and wherein if Ring A* contains an -NH- moiety that nitrogen may be optionally substituted by C₁₋₆alkyl (e.g. methyl); and wherein R^{4A} and Ring A* together with the atoms to which they are attached may form a cyclic group,
Ring B* is an optionally substituted aryl or heteroaryl group wherein each substitutable carbon or heteroatom in Ring B* is optionally and independently substituted by one or more R^{3A};
W and X, one of which may be absent, are independently selected from R^{11A}, hydrocarbyl (e.g. C₁₋₈ alkyl, alkenyl, alkynyl, or haloalkyl) optionally substituted with R^{11A}, and -(CH₂)ₖ₁-heterocyclyl optionally substituted with R^{12A}; k₁ is 0, 1, 2, 3, 4, 5 or 6;
R^{1A}, R^{2A}, R^{3A}, R^{4A} and R^{5A} are independently selected from hydrogen, R^{12A}, hydrocarbyl (e.g. C₁₋₆ alkyl, alkenyl, alkynyl, or haloalkyl) optionally substituted with R^{12A}, and a-(CH₂)_{L1}- heterocyclyl optionally substituted with one or more R^{12A}; wherein R^{1A} and R^{2A} taken together with the atoms to which they are attached may form a heterocycle, optionaly substituted with one or more R^{12A}, wherein R^{1A} and/or R^{2A} taken together with W or X may form a heterocycle optionally substituted with one or more R^{12A}; and
wherein one or more of R^{3A} and R^{5A} taken together with the atoms to which they are attached may form a carbocycle, for example heterocyclyl, optionally substituted with R^{12A}; L₁ is 0, 1, 2, 3, 4, 5 or 6;
wherein:
   each R^{11A} and R^{12A} is independently selected from halogen, trifluoromethyl, cyano, thio, nitro, oxo, =NR^{13A}, -OR^{13A}, -SR^{13A}, -C(O)R^{13A}, -C(O)OR^{13A}, -OC(O)R^{13A}, -NR^{13A}COR^{14A}, -NR^{13A}CON(R^{14A})₂, -NR^{13A}COR^{14A},-NR^{13A}CO₂R^{14A}, -S(O)R^{13A}, -S(O)₂R^{13A}, -SON(R^{13A})₂, -NR^{13A}S(O)₂R^{14A};-CSR^{13A}, -N(R^{13A})R^{14A}, -C(O)N(R^{13A})R^{14A}, -SO₂N(R^{13A})R^{14A} and R^{15A};
   R^{13A} and R^{14A} are each independently selected from hydrogen or R^{15A};
   R^{15A} is selected from hydrocarbyl (e.g. C₁₋₆alkyl, alkenyl, alkynyl, or haloalkyl), carbocyclyl and -(CH₂)ₘ₁-heterocyclyl, and each R^{15A} is optionally and independently substituted with one or more of halogen, cyano, amino, hydroxy, C₁₋₆alkyl or cycloalkyl and C₁₋₆alkoxy;
   m₁ is 0, 1, 2, 3, 4, 5 or 6;
p₁ is 0,1, 2, 3 or 4; the values of R^{4A} may be the same or different; and
q, is 0, 1, 2, 3 or 4; wherein the values of R^{5A} may be the same or different;
Y and Z, one or both of which may be absent, are independently selected from hydrogen, R^{16A}, hydrocarbyl (e.g.C₁₋₆alkyl, alkenyl, alkynyl.or haloalkyl) optionally substituted with R^{16A}, and -(CH₂)ᵣ₁-heterocyclyl optionally substituted with R^{16A}, wherein each R^{16A} is independently selected from halogen, trifluoromethyl, cyano, thio, nitro, oxo, =NR^{17A}, -OR^{17A}, -SR^{17A}, -C(O)R^{17A}, -C(O)OR^{17A}, -OC(O)R^{17A}, - NR^{17A}COR^{18A},-NR^{17A}CON(R^{18A})₂, -NR^{17A}COR^{18A}, -NR^{17A}CO₂R^{18A}, -S(O)R^{17A}, -S(O)₂R^{17A},-SON(R^{17A})₂, -NR^{17A}S(O)₂R^{18A}; -CSR^{17A}, -N(R^{17A})R^{18A}, -C(O)N(R^{17A})R^{18A},-SO₂N(R^{17A})R^{18A} and R^{19A}; r₁ is 0, 1,2, 3, 4, 5 or 6;
wherein:
   R^{17A} and R^{18A} are each independently selected from hydrogen or R^{19A};
   R^{19A} is selected from hydrocarbyl (e.g. C₁₋₆alkyl, alkenyl, alkynyl.or haloalkyl), carbocyclyl and -(CH2)ₛ₁-heterocyclyl, and each R^{19A} is optionally and independently substituted with one or more of halogen, cyano, amino, hydroxy, C₁₋₆alkyl and C₁₋₆alkoxy; and
   s₁ is 0, 1, 2, 3, 4, 5 or 6.

NMT inhibitors of Formula (II) are further described in WO2010/026365. It will be understood that suitable and preferred NMT inhibitors of Formula (II) of the present invention may therefore include any of the compounds disclosed in WO2010/026365.

Suitably, the NMT inhibitor is a compound of Formula (IIa) shown below, or a pharmaceutically acceptable salt, solvate or hydrate thereof: 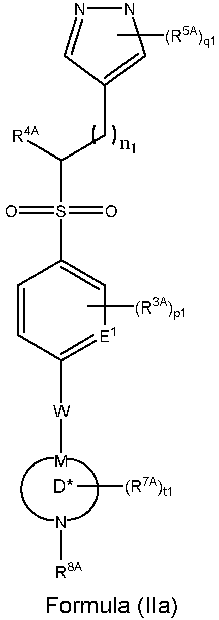 wherein:
n₁ is 0, 1, 2, 3, 4, 5 or 6;
E¹ is independently selected from C and N;
W is selected from a hydrocarbyl optionally substituted with R^{11A}, an optionally substituted aryl or heteroaryl group and a carbocyclyl optionally substituted with one or more of halogen, cyano, amino, hydroxy, C₁₋₆alkyl and C₁₋₆alkoxy groups;
M is selected from C and N;
R^{3A}, R^{4A} and R^{5A} are independently selected from hydrogen, R^{12A}, hydrocarbyl optionally substituted with R^{12A}, and -(CH₂)_{L1}-heterocyclyl optionally substituted with R^{12A};
L₁ is 0, 1, 2, 3, 4, 5 or 6;
wherein each R^{11A} and R^{12A} is independently selected from halogen, trifluoromethyl, cyano, thio, nitro, oxo, =NR^{13A}, -OR^{13A}, -SR^{13A}, -C(O)R^{13A}, -OC(O)R^{13A}, -NR^{13A}COR^{14A}, -NR^{13A}CON(R^{13A})₂,-NR^{13A}COR^{14A}, -NR^{13A}CO₂R^{14A}, -S(O)R^{13A}, -S(O)₂R^{13A}, -SON(R^{13A})₂,-NR^{13A}S(O)₂R^{14A}; -CSR^{13A}, -N(R^{13A})R^{14A}, -C(O)N(R^{13A})R^{14A},-SO₂N(R^{13A})R^{14A} and R^{15A};
R^{13A} and R^{14A} are each independently selected from hydrogen or R^{15A};
wherein R^{15A} is selected from hydrocarbyl, carbocyclyl and -(CH₂)ₘ₁-heterocyclyl, and each R^{15A} is optionally and independently substituted with one or more of halogen, cyano, amino, hydroxy, C₁₋₆alkyl and C₁₋₆alkoxy;
Ring D* is an optionally substituted nitrogen containing 6 or 7 membered heterocycle, wherein each substitutable carbon or nitrogen in Ring D* is optionally and independently substituted by one or more R^{7A};
R^{7A} is independently selected from hydrogen, R^{20A}, hydrocarbyl optionally substituted with R^{20A}, and -(CH₂)_{V1}- heterocyclyl optionally substituted with R^{20A};
v₁ is 0, 1, 2, 3, 4, 5 or 6;
wherein each R^{20A} is independently selected from halogen, trifluoromethyl, cyano, thio, nitro, oxo, =NR^{21A}, -OR^{21A}, -SR^{21A}, -C(O)R^{21A}, -C(O)OR^{21A}, -OC(O) R^{21A}, -NR^{21A}COR^{22A}, -NR^{21A}CON(R^{22A})₂, -NR^{21A}COR^{22A},-NR^{21A}CO₂R^{22A}, -S(O)R^{21A}, -S(O)₂R^{21A}, -SON(R^{21A})₂, -NR^{21A}S(O)₂R^{22A};-CSR^{21A}, N(R^{21A}) R^{22A}, -C(O)N(R^{21A})R^{22A}, -SO₂N(R^{21A})R^{22A} and R^{23A};
wherein R^{21A} and R^{22A} are each independently selected from hydrogen or R^{23A};
wherein R^{23A} is selected from hydrocarbyl, carbocyclyl and -(CH₂)_{w1}-heterocyclyl, and each R^{23A} is optionally and independently substituted with one or more of halogen, cyano, amino, hydroxy, C₁₋₆alkyl and C₁₋₆alkoxy;
w₁ is 0, 1, 2, 3, 4, 5 or 6;
R^{8A} is selected from the list of optional substituents represented by the group R 4A ;
m₁ is 0, 1, 2, 3, 4, 5 or 6;
p¹ is 0, 1, 2, 3 or 4, wherein the values of R^{4A} may be the same or different;
q, is 0, 1, 2, 3 or 4, wherein the values of R^{5A} may be the same or different; and
t₁ is 0, 1,2, 3, 4, 5 or 6, wherein the values of R^{7A} may be the same or different.

More suitably, the NMT inhibitor is a compound of Formula (Ila) shown below, or a pharmaceutically acceptable salt, solvate or hydrate thereof: 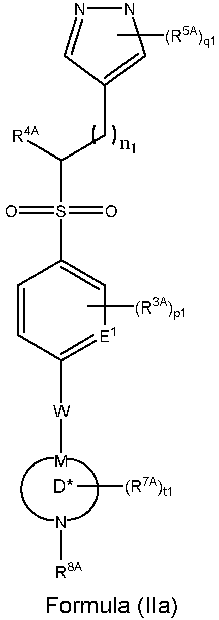 wherein:
n₁ is 0 or 1;
E¹ is C;
W is a (1-4C)hydrocarbyl, an aryl (e.g. phenyl) or heteroaryl group (e.g. pyridinyl);
M is selected from C and N;
R^{3A}, R^{4A} and R^{5A} are independently selected from hydrogen, R^{12A}, and (1-3C)hydrocarbyl optionally substituted with R^{12A};
R^{12A} is independently selected from halogen, trifluoromethyl, cyano, thio, nitro, oxo, -OR^{13A}, -SR^{13A}, -C(O)R^{13A}, C(O)OR^{13A}, -OC(O)R^{13A},-NR^{13A}COR^{14A} and R^{15A};
R^{13A} and R^{14A} are each independently selected from hydrogen or a (1-4C)hydrocarbyl (e.g. methyl);
Ring D* is an optionally substituted nitrogen containing 6 or 7 membered heterocycle, wherein each substitutable carbon or nitrogen in Ring D* is optionally and independently substituted by one or more R^{7A};
R^{7A} is independently selected from hydrogen, (1-4C)hydrocarbyl, halogen, trifluoromethyl, cyano, thio, nitro or oxo;
R^{8A} is a hydrogen or a (1-4C)hydrocarbyl (e.g. methyl);
p₁ is 0, 1 or 2, wherein the values of R^{4A} may be the same or different;
q, is 3, wherein the values of R^{5A} may be the same or different; and
t₁ is 0, 1 or 2, wherein the values of R^{7A} may be the same or different.

In a particular embodiment, the NMT inhibitor is any one of the compounds shown in Table 1 of WO2010/026365.

In a further embodiment, the NMT inhibitor is a compound selected from: 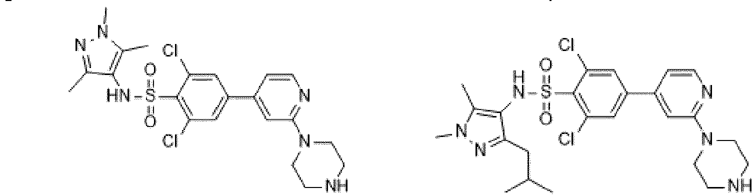 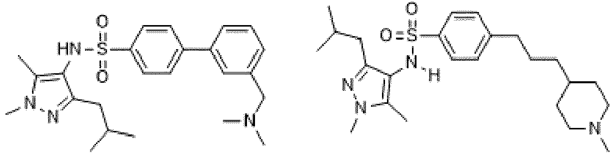 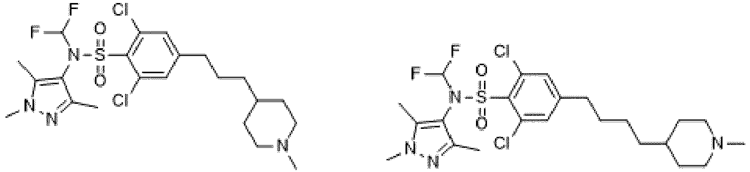 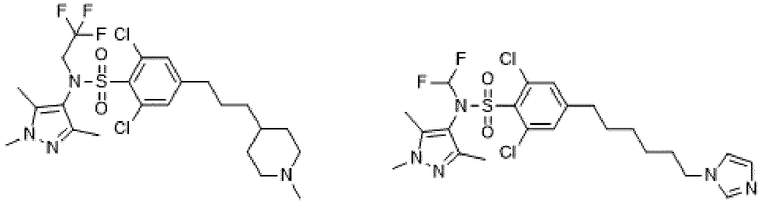 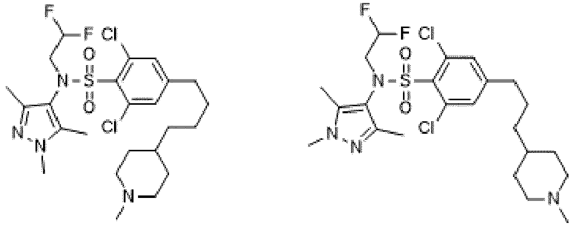 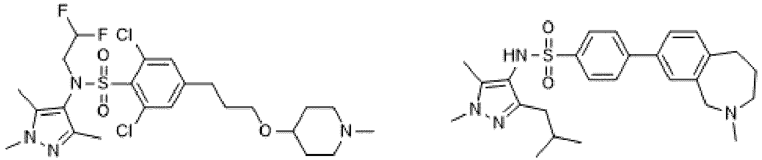 or a pharmaceutically acceptable salt, solvate or hydrate thereof.

In a further embodiment, the NMT inhibitor is a compound selected from: 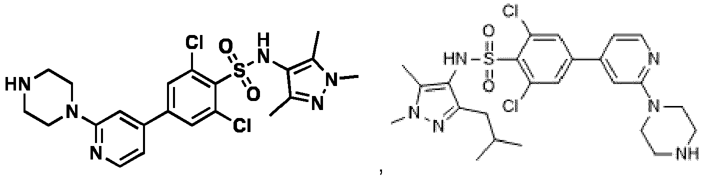 or a pharmaceutically acceptable salt, solvate or hydrate thereof.

In yet a further embodiment, the NMT inhibitor is: 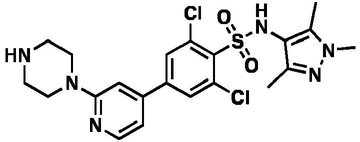 or a pharmaceutically acceptable salt, solvate or hydrate thereof.

### Other possible NMT inhibitors

Other suitable NMT inhibitors may include, for example, any one of the compounds displaying activity as an inhibitor of N-myristoyl transferase (NMT) described in WO00/37464 (Roche) or WO2013/083991 (Imperial Innovations Limited).

In certain embodiments, the NMT inhibitor is any one of the compounds displaying activity as an inhibitor of N-myristoyl transferase (NMT) described in WO2013/083991. It will be appreciated that this embodiment encompasses both generic and specific compounds described in WO2013/083991.

In a particular embodiment, the NMT inhibitor of the present invention is any one of compounds 1-140 disclosed in WO2013/083991.

Salts of the NMT inhibitors for use in the invention are those wherein a counter-ion is pharmaceutically acceptable. Suitable pharmaceutically acceptable salts according to the invention include those formed with organic or inorganic acids or bases. In particular, suitable salts formed with acids according to the invention include those formed with mineral acids, strong organic carboxylic acids, such as alkanecarboxylic acids of 1 to 4 carbon atoms which are unsubstituted or substituted, for example, by halogen, such as saturated or unsaturated dicarboxylic acids, such as hydroxycarboxylic acids, such as amino acids, or with organic sulfonic acids, such as (C₁-C₄)-alkyl- or aryl-sulfonic acids which are unsubstituted or substituted, for example by halogen. Pharmaceutically acceptable acid addition salts include those formed from hydrochloric, hydrobromic, sulphuric, nitric, citric, tartaric, acetic, phosphoric, lactic, pyruvic, acetic, trifluoroacetic, succinic, perchloric, fumaric, maleic, glycolic, lactic, salicylic, oxaloacetic, methanesulfonic, ethanesulfonic, p-toluenesulfonic, formic, benzoic, malonic, naphthalene-2-sulfonic, benzenesulfonic, isethionic, ascorbic, malic, phthalic, aspartic, and glutamic acids, lysine and arginine. For example, it may be the hydrochloric (HCl) salt.

Compounds that have the same molecular formula but differ in the nature or sequence of bonding of their atoms or the arrangement of their atoms in space are termed "isomers". Isomers that differ in the arrangement of their atoms in space are termed "stereoisomers". Stereoisomers that are not mirror images of one another are termed "diastereomers" and those that are non-superimposable mirror images of each other are termed "enantiomers". When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (i.e., as (+) or (-)-isomers respectively). A chiral compound can exist as either individual enantiomer or as a mixture thereof. A mixture containing equal proportions of the enantiomers is called a "racemic mixture".

The NMT inhibitor may possess one or more asymmetric centers; such compounds can therefore be produced as individual (R)- or (S)-stereoisomers or as mixtures thereof. It is to be understood that the NMT inhibitors for use in the present invention encompasses all optical, diastereoisomers and geometric isomers and mixtures thereof that possess NMT inhibition activity.

The present invention also encompasses NMT inhibitors as defined herein which comprise one or more isotopic substitutions. For example, H may be in any isotopic form, including 1H, 2H(D), and 3H (T); C may be in any isotopic form, including 12C, 13C, and 14C; and O may be in any isotopic form, including 160 and 180; and the like.

Those skilled in the art of organic chemistry will appreciate that many organic compounds can form complexes with solvents in which they are reacted or from which they are precipitated or crystallized. These complexes are known as "solvates". For example, a complex with water is known as a "hydrate". Solvates, such as hydrates, exist when the drug substance incorporates solvent, such as water, in the crystal lattice in either stoichiometric or non-stoichiometric amounts. Drug substances are routinely screened for the existence of hydrates since these may be encountered at any stage of the drug manufacturing process or upon storage of the drug substance or dosage form. Solvates are described in S. Byrn et al., Pharmaceutical Research, 1995. 12(7): p. 954-954, and Water-Insoluble Drug Formulation, 2nd ed. R. Liu, CRC Press, page 553. Accordingly, it will be understood by the skilled person that the NMT inhibitors for use in the present invention may be present in the form of solvates, wherein the associated solvent is a pharmaceutically acceptable solvent. For example, a hydrate is an example of a pharmaceutically acceptable solvate.

It is also to be understood that the NMT inhibitors may exhibit polymorphism, and that the invention encompasses all such forms that possess the herein described activity.

The NMT inhibitors for use in the present invention may also exist in a number of different tautomeric forms and references to NMT inhibitors for use in the present invention include all such forms. For the avoidance of doubt, where a compound can exist in one of several tautomeric forms, and only one is specifically described or shown, all others are nevertheless embraced. Examples of tautomeric forms include keto-, enol-, and enolate-forms, as in, for example, the following tautomeric pairs: keto/enol (illustrated below), imine/enamine, amide/imino alcohol, amidine/amidine, nitroso/oxime, thioketone/enethiol, and nitro/aci-nitro.

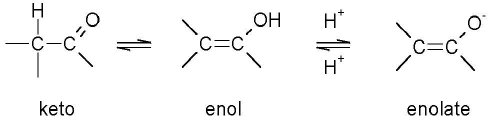

The NMT inhibitor for use in the present invention may be administered in the form of a pro-drug which is broken down in the human or animal body to release the NMT inhibitor for use in the present invention. A pro-drug may be used to alter the physical properties and/or the pharmacokinetic properties of the NMT inhibitor. A pro-drug can be formed when the NMT inhibitor contains a suitable group or substituent to which a property-modifying group can be attached. Examples of pro-drugs include *in vivo* cleavable ester derivatives that may be formed at a carboxy group or a hydroxy group in an NMT inhibitor for use in the present invention, and *in-vivo* cleavable amide derivatives that may be formed at a carboxy group or an amino group in an NMT inhibitor for use in the present invention.

For example, the NMT inhibitors for use in the present invention may have an appropriate group which may be converted to an amide or a carbamate. Typical amide and carbamate groups formed from a basic nitogen in the NMT inhibitors of the present invention, for example, the compounds of Formula (I), include >NR^{G}C(O)R^{G}, >NR^{G}CO₂R^{G}, and >NR^{G}SO₂R^{G}, where R^{G} is selected from the group consisting of C₁₋₈alkyl, C₂₋₈alkenyl, C₂₋₈alkynyl, C₃₋₈cycloalkyl and C₃₋₈cycloalklC₁₋₈alkyl, haloC₁₋₈alkyl, dihaloC₁₋₈alkyl, trihaloC₁₋₈alkyl, phenyl and phenylC₁₋₄alkyl; more preferably R^{G} is selected from the group consisting of C₁₋₈alkyl, C₂₋₆alkenyl, C₂₋₆alkynyl, C₃₋₈cycloalkyl and C₃₋₈cycloalkylC₁₋₈alkyl

### Particularly preferred embodiments

Particularly preferred embodiments of the present invention are set out hereinbelow in numbered paragraphs 1.1 to 1.12.

### Treatment of diffuse large B-cell lymphoma comprising one or more structural alterations of the MYC locus

1.1 A NMT inhibitor, or a pharmaceutically acceptable salt, solvate or hydrate thereof, for use in the treatment of diffuse large B-cell lymphoma comprising one or more structural alterations of the *MYC* locus, wherein:
   i) the one or more structural alterations are selected from mutations, copy-number gains and/or chromosomal rearrangements; and
   ii) the NMT inhibitor is a compound of Formula I (e.g. Formula IA^^) or Formula II (e.g. Formula Ila) described herein.
1.2 A NMT inhibitor, or a pharmaceutically acceptable salt, solvate or hydrate thereof, for use in the treatment of diffuse large B-cell lymphoma comprising one or more structural alterations of the *MYC* locus,
   wherein:
   i) the one or more structural alterations are mutations; and
   ii) the NMT inhibitor is a compound of Formula I, preferably Formula (IA^^), (e.g. Compound 1, Compound 2, Compound 3 or Compound 4) described herein.
1.3 A NMT inhibitor, or a pharmaceutically acceptable salt, solvate or hydrate thereof, for use in the treatment of diffuse large B-cell lymphoma comprising one or more structural alterations of the *MYC* locus (e.g. one or more structural alterations of the *c-MYC* and/or *MYCN* locus),
   wherein:
   i) the one or more structural alterations are mutations; and
   ii) the NMT inhibitor is a compound of Formula (IA^^) (e.g. Compound 1, Compound 2, Compound 3 or Compound 4) shown below, or a pharmaceutically acceptable salt, solvate or hydrate thereof: 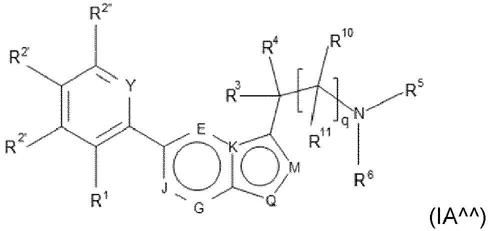 wherein:
      R¹ is a group of formula -X-L-A;
      A is 4-pyrazolyl, said pyrazolyl being optionally substituted with up to 3 methyl groups;
      X is -O- or absent;
      L is -(CH₂)ₘ- or -(CH₂)ₘ-O-;
      m is 2;
      R^{2'} is selected from the group consisting of fluorine, chlorine -CN and
      methyl (preferably fluorine);
      R^{2"} is selected from the group consisting of hydrogen, fluorine, chlorine, -CN and methyl;
      q is 0 or 1;
      R³ is hydrogen or methyl;
      R⁴ is hydrogen or methyl;
      R⁵ is hydrogen or methyl;
      R⁶ is hydrogen or methyl; or
      the R³ group and the R⁵ group and the intervening atoms form a 3 to 7 membered non-aromatic heterocycle composed of the intervening atoms and bonds, (more preferably R⁵ and R⁶ are both methyl);
      R⁷ where present is hydrogen or methyl;
      R⁸ where present is hydrogen or methyl;
      R⁹ and R¹⁰ where present are hydrogen or methyl; and
      E, J, G, K, Q and M are:
         i) E, J and G are each C(R⁷), K is carbon, Q is N(R⁸), M is nitrogen; and R⁸ is hydrogen or methyl;
         ii) E, J and G are each C(R⁷), and K, Q and M are each nitrogen;
         iii) E and G are each C(R⁷), and J, K, Q and M are each nitrogen;
         iv) J and G are each C(R⁷), and E, K, Q and M are each nitrogen; or
         v) E, J, G and M are each C(R⁷), and K and Q are each nitrogen.
1.4 A NMT inhibitor, or a pharmaceutically acceptable salt, solvate or hydrate thereof, for use in the treatment of diffuse large B-cell lymphoma comprising one or more structural alterations of the *MYC* locus (e.g. one or more structural alterations of the *c-MYC* and/or *MYCN* locus),
   wherein:
   i) the one or more structural alterations are mutations which impart overexpression of *MYC* (e.g. *c-MYC* or *MYCN*); and
   ii) the NMT inhibitor is a compound of Formula (IA^^) (e.g. Compound 1, Compound 2, Compound 3 or Compound 4) shown below, or a pharmaceutically acceptable salt, solvate or hydrate thereof: 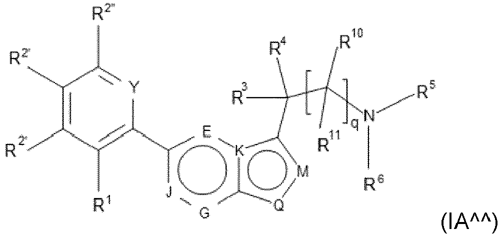 wherein:
      R¹ is a group of formula -X-L-A;
      A is 4-pyrazolyl, said pyrazolyl being optionally substituted with up to 3 substituent groups selected from C₁₋₄alkyl (e.g. methyl) and -C(O)N(R⁹)₂ (e.g. -C(O)N(CH₃)₂);
      X is -O- or absent;
      L is -(CH₂)ₘ- or -(CH₂)ₘ-O-;
      m is 2;
      R^{2'} is selected from the group consisting of fluorine or chlorine (preferably fluorine);
      R^{2"} is selected from the group consisting of hydrogen, fluorine or chlorine;
      q is 0 or 1;
      R³ is hydrogen or methyl;
      R⁴ is hydrogen or methyl;
      R⁵ is hydrogen or methyl;
      R⁶ is hydrogen or methyl; or
      the R³ group and the R⁵ group and the intervening atoms form a 3 to 7 membered non-aromatic heterocycle composed of the intervening atoms and bonds, (more preferably R⁵ and R⁶ are both methyl);
      R⁷ where present is hydrogen or methyl;
      R⁸ where present is hydrogen or methyl;
      R⁹ and R¹⁰ where present are hydrogen or methyl; and
      E, J, G, K, Q and M are:
         i) E, J and G are each C(R⁷), K is carbon, Q is N(R⁸), M is nitrogen; and R⁸ is hydrogen or methyl; or
         ii) E, J, G and M are each C(R⁷), and K and Q are each nitrogen;
   with the proviso that A is substituted with no more than one -C(O)N(R⁹)₂ group.
1.5 A NMT inhibitor, or a pharmaceutically acceptable salt, solvate or hydrate thereof, for use in the treatment of diffuse large B-cell lymphoma comprising one or more structural alterations of the *MYC* locus (e.g. one or more structural alterations of the *c-MYC* and/or *MYCN* locus),
   wherein:
   i) the one or more structural alterations are mutations which impart overexpression of *MYC* (e.g. *c-MYC* or *MYCN*); and
   ii) the NMT inhibitor is a compound of Formula (IA^^) (e.g. Compound 1 or Compound 2) shown below, or a pharmaceutically acceptable salt, solvate or hydrate thereof: 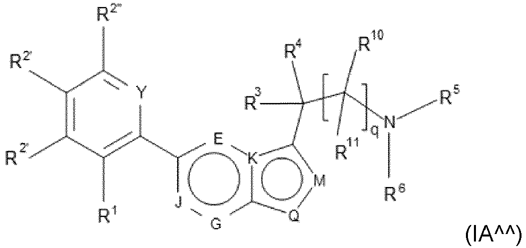
   wherein:
   R¹ is a group of formula -X-L-A;
   A is 4-pyrazolyl, said pyrazolyl being optionally substituted with up to 3 substituent groups selected from methyl and -C(O)N(CH₃)₂;
   X is -O- or absent;
   L is -(CH₂)ₘ- or -(CH₂)ₘ-O-;
   m is 2;
   R^{2'} is selected from the group consisting of fluorine or chlorine (preferably fluorine);
   R^{2"} is selected from the group consisting of hydrogen, fluorine or chlorine;
   q is 0;
   R³ is hydrogen or methyl;
   R⁴ is hydrogen or methyl;
   R⁵ is hydrogen or methyl;
   R⁶ is hydrogen or methyl; or
   the R³ group and the R⁵ group and the intervening atoms form a 3 to 7 membered non-aromatic heterocycle composed of the intervening atoms and bonds, (more preferably R⁵ and R⁶ are both methyl);
   E, J, G, K, Q and M are:
      i) E, J and G are each CH, K is carbon, Q is N(R⁸), M is nitrogen; and R⁸ is hydrogen or methyl; or
      ii) E, J, G and M are each CH, and K and Q are each nitrogen;
   with the proviso that A is substituted with no more than one -C(O)N(CH₃)₂ group.
1.6 A NMT inhibitor, or a pharmaceutically acceptable salt, solvate or hydrate thereof, for use in the treatment of diffuse large B-cell lymphoma comprising one or more structural alterations of the *MYC* locus (e.g. one or more structural alterations of the *c-MYC* and/or *MYCN* locus),
   wherein:
   i) the one or more structural alterations are mutations which impart overexpression of *MYC* (e.g. *c-MYC* or *MYCN*), preferably, such that the levels of RNA transcript and/or protein of *MYC* (e.g. *c-MYC* or *MYCN*) are at least 25% greater than the RNA transcript and/or protein levels of *MYC* (e.g. *c-MYC* or *MYCN*) found in a normal, healthy cell; and
   ii) the NMT inhibitor is a compound of Formula (IA^^) (e.g. Compound 1 or Compound 2) shown below, or a pharmaceutically acceptable salt, solvate or hydrate thereof: 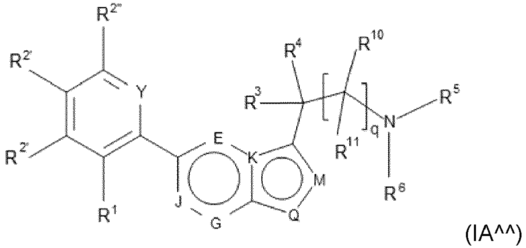 wherein:
      R¹ is a group of formula -X-L-A;
      A is 4-pyrazolyl, said pyrazolyl being optionally substituted with up to 3 substituent groups selected from methyl and -C(O)N(CH₃)₂;
      X is -O- or absent;
      L is -(CH₂)ₘ- or -(CH₂)ₘ-O-;
      m is 2;
      R^{2'} is selected from the group consisting of fluorine or chlorine (preferably fluorine);
      R^{2"} is selected from the group consisting of hydrogen, fluorine or chlorine;
      q is 0;
      R³ is hydrogen or methyl;
      R⁴ is hydrogen or methyl;
      R⁵ is hydrogen or methyl;
      R⁶ is hydrogen or methyl; or
      the R³ group and the R⁵ group and the intervening atoms form a 3 to 7 membered non-aromatic heterocycle composed of the intervening atoms and bonds, (more preferably R⁵ and R⁶ are both methyl);
      E, J, G, K, Q and M are:
         i) E, J and G are each CH, K is carbon, Q is N(R⁸), M is nitrogen; and R⁸ is hydrogen or methyl; or
         ii) E, J, G and M are each CH, and K and Q are each nitrogen;
   with the proviso that A is substituted with no more than one -C(O)N(CH₃)₂ group.

### Treatment of diffuse large B-cell lymphoma

1.7 A NMT inhibitor, or a pharmaceutically acceptable salt, solvate or hydrate thereof, for use in the treatment of diffuse large B-cell lymphoma, wherein:
   i) *MYC* is overexpressed in said diffuse large B-cell lymphoma; and
   ii) the NMT inhibitor is a compound of Formula I (e.g. Formula IA^^) or Formula II (e.g. Formula Ila) described herein.
1.8 A NMT inhibitor, or a pharmaceutically acceptable salt, solvate or hydrate thereof, for use in the treatment of diffuse large B-cell lymphoma:
   wherein:
   i) *MYC* is overexpressed in said diffuse large B-cell lymphoma; and
   ii) the NMT inhibitor is a compound of Formula I, preferably Formula (IA^^), (e.g.
Compound 1, Compound 2, Compound 3 or Compound 4) described herein. 1.9 A NMT inhibitor, or a pharmaceutically acceptable salt, solvate or hydrate thereof, for use in the treatment of diffuse large B-cell lymphoma:
   wherein:
   i) *MYC* is overexpressed in said diffuse large B-cell lymphoma, preferably, such that the levels of RNA transcript and/or protein of *MYC* in the diffuse large B-cell lymphoma are at least 25% greater than the RNA transcript and/or protein levels of *MYC* found in a normal, healthy cell; and
   ii) the NMT inhibitor is a compound of Formula I, preferably Formula (IA^^), (e.g. Compound 1, Compound 2, Compound 3 or Compound 4) described herein.
1.10 A NMT inhibitor, or a pharmaceutically acceptable salt, solvate or hydrate thereof, for use in the treatment of diffuse large B-cell lymphoma;
   wherein:
   i) *MYC* is overexpressed in said diffuse large B-cell lymphoma, preferably, such that the levels of RNA transcript and/or protein of *MYC* in the diffuse large B-cell lymphoma are at least 25% greater than the RNA transcript and/or protein levels of *MYC* found in a normal, healthy cell; and
   ii) the NMT inhibitor is a compound of Formula (IA^^) (e.g. Compound 1, Compound 2, Compound 3 or Compound 4) shown below, or a pharmaceutically acceptable salt, solvate or hydrate thereof: 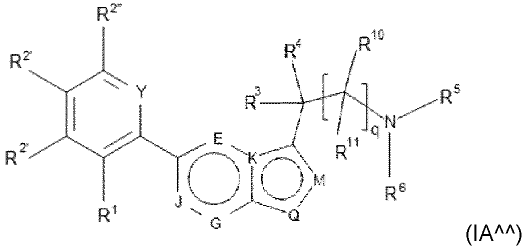 wherein:
      R¹ is a group of formula -X-L-A;
      A is 4-pyrazolyl, said pyrazolyl being optionally substituted with up to 3 substituent groups selected from C₁₋₄alkyl, wherein each -C₁₋₄alkyl is optionally substituted by up to 3 halogen, hydroxyl or -OC₁₋₄alkyl groups; -C(O)N(R⁹)₂ (for example -C(O)N(H)C₁₋₄alkyl), and -CH-₂C(O)N(R⁹)₂ (for example -CH₂C(O)N(H)C₁₋₄alkyl);
      X is -O- or absent;
      L is -(CH₂)ₘ- or -(CH₂)ₘ-O-;
      m is 2;
      R^{2'} is selected from the group consisting of fluorine, chlorine -CN and
      methyl (preferably fluorine);
      R^{2"} is selected from the group consisting of hydrogen, fluorine, chlorine, -CN and methyl;
      q is 0 or 1;
      R³ is hydrogen or methyl;
      R⁴ is hydrogen or methyl;
      R⁵ is hydrogen or methyl;
      R⁶ is hydrogen or methyl; or
      the R³ group and the R⁵ group and the intervening atoms form a 3 to 7 membered non-aromatic heterocycle composed of the intervening atoms and bonds, (more preferably R⁵ and R⁶ are both methyl);
      R⁷ where present is hydrogen or methyl;
      R⁸ where present is hydrogen or methyl;
      R⁹ and R¹⁰ where present are hydrogen or methyl; and
      E, J, G, K, Q and M are:
         i) E, J and G are each C(R⁷), K is carbon, Q is N(R⁸), M is nitrogen; and R⁸ is hydrogen or methyl;
         ii) E, J and G are each C(R⁷), and K, Q and M are each nitrogen;
         iii) E and G are each C(R⁷), and J, K, Q and M are each nitrogen;
         iv) J and G are each C(R⁷), and E, K, Q and M are each nitrogen; or
         v) E, J, G and M are each C(R⁷), and K and Q are each nitrogen.
1.11 A NMT inhibitor, or a pharmaceutically acceptable salt, solvate or hydrate thereof, for use in the treatment of diffuse large B-cell lymphoma;
   wherein:
   i) *MYC* is overexpressed in said diffuse large B-cell lymphoma, preferably, such that the levels of RNA transcript and/or protein of *MYC* in diffuse large B-cell lymphoma are at least 25% greater than the RNA transcript and/or protein levels of *MYC* found in a normal, healthy cell; and
   ii) the NMT inhibitor is a compound of Formula (IA^^) (e.g. Compound 1, Compound 2, Compound 3 or Compound 4) shown below, or a pharmaceutically acceptable salt, solvate or hydrate thereof: 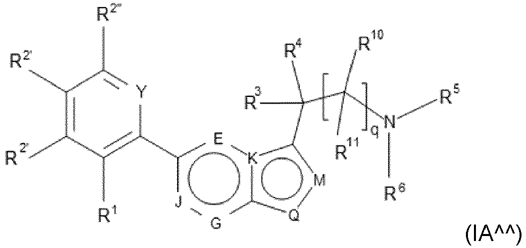 wherein:
      R¹ is a group of formula -X-L-A;
      A is 4-pyrazolyl, said pyrazolyl being optionally substituted with up to 3 substituent groups selected from C₁₋₄alkyl (e.g. methyl) and -C(O)N(R⁹)₂ (e.g. -C(O)N(CH₃)₂);
      X is -O- or absent;
      L is -(CH₂)ₘ- or -(CH₂)ₘ-O-;
      m is 2;
      R^{2'} is selected from the group consisting of fluorine or chlorine (preferably fluorine);
      R^{2"} is selected from the group consisting of hydrogen, fluorine or chlorine;
      q is 0 or 1;
      R³ is hydrogen or methyl;
      R⁴ is hydrogen or methyl;
      R⁵ is hydrogen or methyl;
      R⁶ is hydrogen or methyl; or the R³ group and the R⁵ group and the intervening atoms form a 3 to 7 membered non-aromatic heterocycle composed of the intervening atoms and bonds, (more preferably R⁵ and R⁶ are both methyl);
      R⁷ where present is hydrogen or methyl;
      R⁸ where present is hydrogen or methyl;
      R⁹ and R¹⁰ where present are hydrogen or methyl; and
      E, J, G, K, Q and M are:
         i) E, J and G are each C(R⁷), K is carbon, Q is N(R⁸), M is nitrogen; and R⁸ is hydrogen or methyl; or
         ii) E, J, G and M are each C(R⁷), and K and Q are each nitrogen;
   with the proviso that A is substituted with no more than one -C(O)N(R⁹)₂ group.
1.12 A NMT inhibitor, or a pharmaceutically acceptable salt, solvate or hydrate thereof, for use in the treatment of diffuse large B-cell lymphoma;
   wherein:
   i) *MYC* is overexpressed in said diffuse large B-cell lymphoma, preferably, such that the levels of RNA transcript and/or protein of *MYC* in the diffuse large B-cell lymphoma are at least 25% greater than the RNA transcript and/or protein levels of *MYC* found in a normal, healthy cell; and
   ii) the NMT inhibitor is a compound of Formula (IA^^) (e.g. Compound 1 or Compound 2) shown below, or a pharmaceutically acceptable salt, solvate or hydrate thereof: 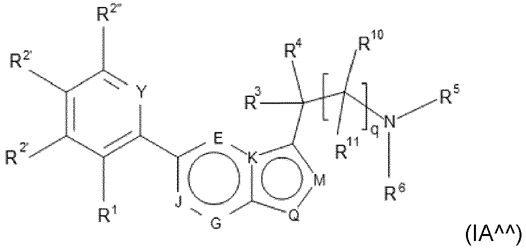 wherein:
      R¹ is a group of formula -X-L-A;
      A is 4-pyrazolyl, said pyrazolyl being optionally substituted with up to 3 substituent groups selected from methyl and -C(O)N(CH₃)₂;
      X is -O- or absent;
      L is -(CH₂)ₘ- or -(CH₂)ₘ-O-;
      m is 2;
      R^{2'} is selected from the group consisting of fluorine or chlorine (preferably fluorine);
      R^{2"} is selected from the group consisting of hydrogen, fluorine or chlorine;
      q is 0;
      R³ is hydrogen or methyl;
      R⁴ is hydrogen or methyl;
      R⁵ is hydrogen or methyl;
      R⁶ is hydrogen or methyl; or
      the R³ group and the R⁵ group and the intervening atoms form a 3 to 7 membered non-aromatic heterocycle composed of the intervening atoms and bonds, (more preferably R⁵ and R⁶ are both methyl);
      E, J, G, K, Q and M are:
         i) E, J and G are each CH, K is carbon, Q is N(R⁸), M is nitrogen; and R⁸ is hydrogen or methyl; or
         ii) E, J, G and M are each CH, and K and Q are each nitrogen;
   with the proviso that A is substituted with no more than one -C(O)N(CH₃)₂ group.

### Synthesis

The NMT inhibitors for use in the present invention can be prepared by any suitable technique known in the art. Particular processes for the preparation of the NMT inhibitors described herein may be found in WO00/37464 (Roche), WO2010/026365 (University of Dundee), WO2013/083991 (Imperial Innovations Limited) and WO2017/001812 (Imperial Innovations Limited).

In the description of the synthetic methods described herein and in any of the references noted above, it is to be understood that all proposed reaction conditions, including choice of solvent, reaction atmosphere, reaction temperature, duration of the experiment and workup procedures, can be selected by a person skilled in the art.

Numerous synthetic routes to the NMT inhibitors described herein can be devised by a person skilled in the art and the exemplified synthetic routes described in the above references do not limit the invention. Many methods exist in the literature for the synthesis of heterocycles, for example: Joule, J. A.; Mills, K., Heterocyclic Chemistry, 2010, 5th Edition, Pub. Wiley.

### EXAMPLES

### NMT INHIBITORS

Throughout the accompanying Examples section, and throughout the specification as a whole, reference to Compounds 1, 2, 3, 4, 5, 6 and/or 7 will be understood to be a reference to the compound(s) shown below:

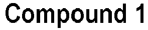

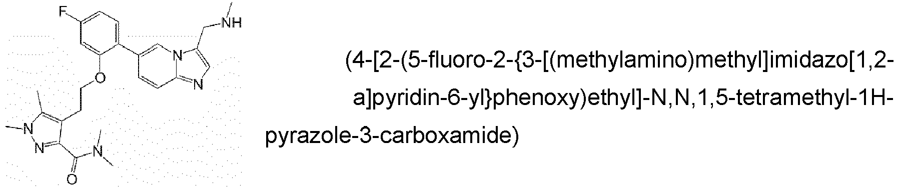

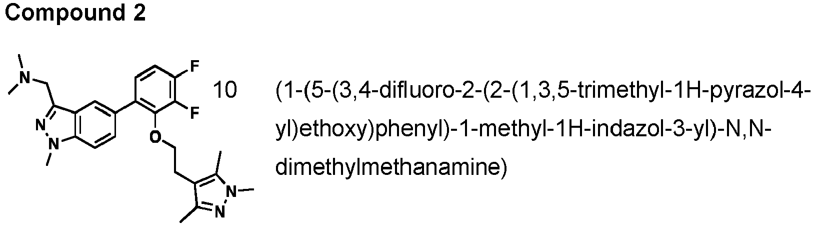

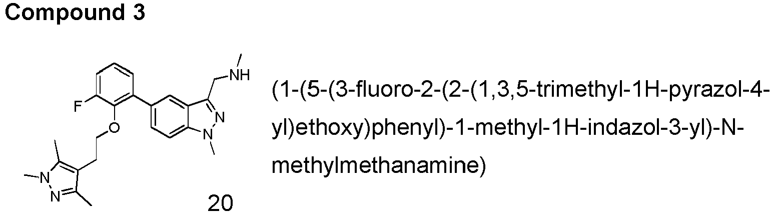

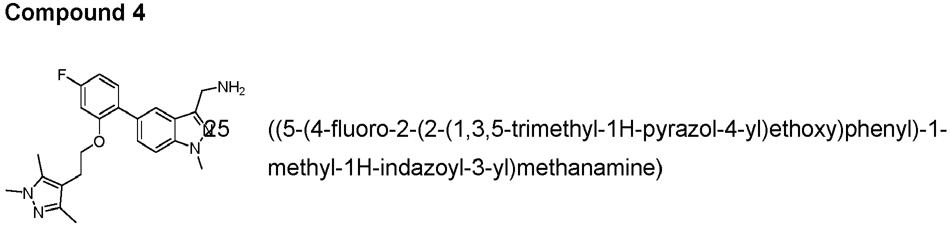

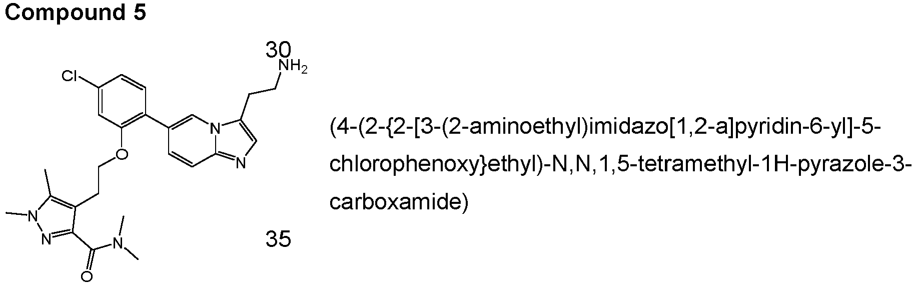

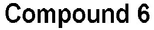

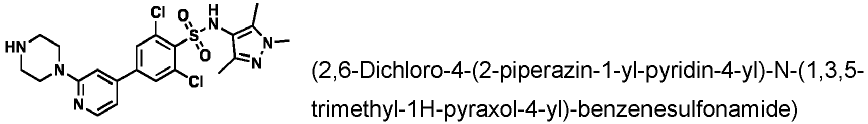

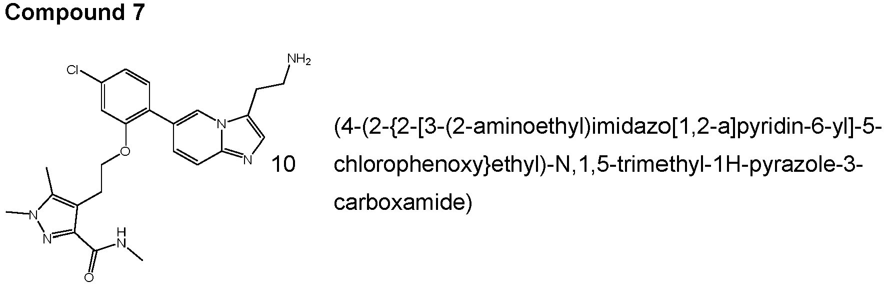

### METHODS

### Synthetic procedures

### General Remarks

Compounds 1, 2, 3 and 4 were prepared using the synthetic procedures described in WO2017/001812. For the avoidance of doubt, Compounds 1, 2, 3 and 4 of the present invention correspond to Examples 77, 49, 35 and 31 of WO2017/001812 respectively.

Compound 6 may be prepared using the synthetic procedure described in WO2010/026365. For the avoidance of doubt, Compound 6 of the present invention corresponds to the compound DDD85646 of WO2010/026365.

Compounds 5 and 7 were prepared as described hereinbelow.

### General Experimental Details

### LC-MS

Compounds requiring purification under basic conditions were purified on an LC-MS system equipped with a YMC Actus Triart C18 5µm (20 x 250mm) column or Gemini NX 5µm C18 (100 x 30mm) column, using a gradient elution of acetonitrile in water containing 20 mM Ammonium bicarbonate (10-45% over 30min then 95% acetonitrile for 2 minutes). *HPLC*

The purity of Compound 5 and 7 (was determined by analytical HPLC using an Eclipse Extend 5µm C18 (150 x 4.6mm) or Shimadzu L Column 2 ODS 5µm C18 (150x4.6mm) column using gradient elution of acetonitrile in water containing 10 mM ammonium acetate over 12 min.

### NMR

¹H NMR and ¹³C spectra were recorded on 400 MHz and 101 MHz respectively instruments at room temperature unless specified otherwise were referenced to residual solvent signals. Data are presented as follows: chemical shift in ppm, integration, multiplicity (br = broad, app = apparent, s = singlet, d = doublet, t = triplet, q = quartet, p = pentet, m = multiplet) and coupling constants in Hz.

### Synthetic Procedures

### Boc Deprotection (Method B)

The Boc protected amine was dissolved in dioxane and treated with a solution of HCl in dioxane (6M, 2 mL). The reaction mixture was stirred at room temperature overnight. All volatiles were removed under reduced pressure and the product triturated with ether redissolved in water and freeze dried.

### Preparation of starting materials

All of the starting materials for making the intermediate and example compound were obtained from commercial sources or using literature methods, except for methyl 4-(2-hydroxyethyl)-1,5-dimethyl-1H-pyrazole-3-carboxylate, which was made as follows:

### Preparation of methyl 4-(2-hydroxyethyl)-1,5-dimethyl-1H-pyrazole-3-carboxylate starting material:

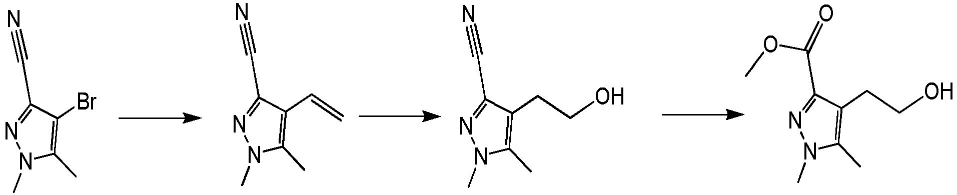

### Step 1

A solution of 4-bromo-1,5-dimethyl-1H-pyrazole-carbonitrile (8.0 g, 40 mmol) in dry DMF (40 mL) was treated with tributylvinylstannane (23.4 mL, 80 mmol). The mixture was purged with argon for 15 minutes before addition of tetrakis(triphenylphosphine) palladium(0) (2.3 g, 2 mmol). The reaction was heated to 110°C overnight, diluted with ethyl acetate and washed with potassium fluoride solution, water and brine, dried over Na₂SO₄, concentrated under reduced pressure. The crude product was purified by flash column chromatography by elution with ethylacetate/hexane (20:80) to provide 4-ethenyl-1,5-dimethyl-1H-pyrazole-3-carbonitrile (4.0 g, 68%). ¹H NMR (400 MHz, CDCl₃) 6.45 (dd, 1H), 5.80 (dd, 1H), 5.34 (dd, 1H), 3.82 (s, 3H), 2.29 (s, 3H).

### Step 2

A solution of 4-ethenyl-1,5-dimethyl-1H-pyrazole-3-carbonitrile (1.2 g, 8.2 mmol) in dioxane (5 mL) was treated with a solution of 9-BBN (0.5M in THF, 32 mL, 16 mmol) under a nitrogen atmosphere. The reaction was heated to 100°C overnight. The mixture was re-cooled to 0°C, and was treated with ethanol (4.8 mL), NaOH solution (6M, 2.4 mL), H₂O₂ (50% solution, 3.6 mL). The reaction mixture was heated at RT for 2hr diluted with DCM/methanol (95:5), dried over sodium sulphate and concentrated under reduced pressure. The crude product purified by flash column chromatography by elution with DCM/methanol (98:2) to provide the compound 4-(2-hydroxyethyl)-1,5-dimethyl-1H-pyrazole-3-carbonitrile (500 mg, 37%). ¹H NMR (400 MHz, CDCl₃) 3.81(s, 3H), 3.78 (q, 2H), 2.74 (t, 2H), 2.55 (s, 3H), 1.86 (t, 1H).

### Step 3

A solution of 4-(2-hydroxyethyl)-1,5-dimethyl-1H-pyrazole-3-carbonitrile (1.0 g, 6.1 mmol) in methanol (12 mL) was treated with a solution of HCl in dioxane (4M, 12 mL). The reaction mixture was stirred at 80°C for 5 hr and evaporated under reduced pressure. The crude product was basified with sat. NaHCO₃ solution and diluted with EtOAc, washed with water, brine, dried over sodium sulfate and evaporated under reduced pressure to give methyl 4-(2-hydroxyethyl)-1,5-dimethyl-1H-pyrazole-3-carboxylate (1.1 g, 92%). ¹H NMR (400 MHz, CDCl₃) 3.90 (s, 3H), 3.84 (s, 3H), 3.77 (q, 2H), 2.93 (t, 2H), 2.23 (s, 3H), 2.07 (t, 1H).

### Preparation of Intermediate 1

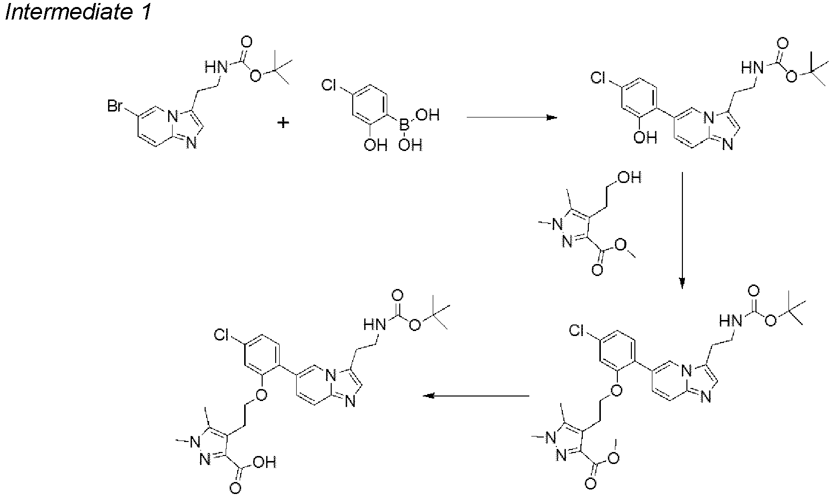

### 4-(2-{2-[3-(2-{(tert-butoxy)carbonyl]amino}ethyl)imidazo[1,2-a]pyridin-6-yl]-5chlorophenoxy}ethyl)-1,5-dimethyl-1H-pyrazole-3-carboxylic acid

### Step 1

A solution of tert-butyl N-(2-{6-bromoimidazo[1,2-a]pyridin-3-yl}ethyl)carbamate (7.0 g, 20.5 mmol) was dissolved in dioxane/water (5:1, 175 mL) and treated with 4-chloro-2-hydroxybenzene boronic acid (8.0 g, 46.3 mmol) and tetrakis(triphenylphosphine) palladium(0) (937 mg, 2.0 mmol), followed by potassium phosphate (13 g, 61.7 mmol). The reaction mixture was purged with argon then heated to 100°C for 3 hr, cooled to room temperature and filtered through a bed of Celite^{™} and washed with ethyl acetate. The ethyl acetate layer taken dried over Na₂SO₄, and evaporated under reduced pressure. The crude product was purified by column chromatography eluting with 3% MeOH in DCM to give *tert-*butyl N-{2-[6-(4-chloro-2-hydroxyphenyl)imidazo[1,2-a]pyridin-3-yl]ethyl}carbamate (7.98g, 97%). ¹H NMR (400 MHz, DMSO-d₆) δ 8.49 (s, 1H), 7.43-7.62 (m, 4H), 6.97-7.01 (m, 3H), 5.76 (s, 1H), 3.27 (t, 2H), 3.05 (t, 2H), 1.34 (s, 9H).

### Step 2

A solution of methyl tert-butyl N-{2-[6-(4-chloro-2-hydroxyphenyl)imidazo[1,2-a]pyridin-3-yl]ethyl}carbamate (5.0 g, 12.9 mmol) in toluene (50 mL) was reacted with methyl 4-(2-hydroxyethyl)-1,5-dimethyl-1H-pyrazole-3-carboxylate (3.07 g, 15.5 mmol) and cyanomethylene tributylphosphorane (6.77 mL, 25.8 mmol) at 100°C for 16 hr. The reaction mixture was then diluted with ethyl acetate, and washed with water and brine, dried over sodium sulphate and concentrated. This crude material was purified by column chromatography by elution with DCM: methanol (95:5) to give methyl 4-(2-{2-[3-(2-{[(*tert-*butoxy)carbonyl]amino}ethyl)imidazo[1,2-a]pyridin-6-yl]-5-chlorophenoxy}ethyl)-1,5-dimethyl-1H-pyrazole-3-carboxylate (3.8g, 52%) as a brown gum. ¹H NMR (400 MHz, DMSO-d₆) δ 8.39 (s, 1H), 7.53 (d, 1H), 7.45 (s, 1H), 7.43 (d, 1H), 7.24-7.27 (m, 2H), 7.11 (dd, 1H), 6.97 (br,t, 1H), 5.75 (s, 1H), 4.14 (t, 2H), 3.71 (s, 3H), 3.68 (s, 3H), 2.99-3.03 (m, 4H), 1.91 (s, 3H), 1.30 (s, 9H).

### Step 3

A solution of methyl 4-(2-{2-[3-(2-{[(tert-butoxy)carbonyl]amino}ethyl)imidazo[1,2-a]pyridin-6-yl]-5-chlorophenoxy}ethyl)-1,5-dimethyl-1H-pyrazole-3-carboxylate (3.0 g, 5.3 mmol) in THF-water (4:1, 50 mL) was treated with methanol (0.1 mL) followed by lithium hydroxide hydrate (444 mg, 10.6 mmol). The resulting mixture was stirred at rt for 16 hr. The reaction mixture was cooled to 0°C and was acidified with saturated citric acid solution and extracted with DCM. The final organic layer was dried over sodium sulphate and concentrated to afford desired product 4-(2-{2-[3-(2-{[(tert-butoxy)carbonyl]amino}ethyl)imidazo[1,2-a]pyridin-6-yl]-5-chlorophenoxy}ethyl)-1,5-dimethyl-1H-pyrazole-3-carboxylic acid (2.7g, 92%) as a brown solid. ¹H NMR (400 MHz, DMSO-d₆) δ 8.42 (s, 1H), 7.56 (d, 1H), 7.44-7.55 (m, 2H), 7.32 (d, 1H), 7.27 (s, 1H), 7.11 (d, 1H), 6.98 (m, 1H), 5.76 (s, 1H), 4.13 (t, 2H), 3.68 (s, 3H), 3.32 (t, 2H), 3.01-3.04 (m, 4H), 1.93 (s, 3H), 1.29 (s, 9H).

### Preparation of Compound 5

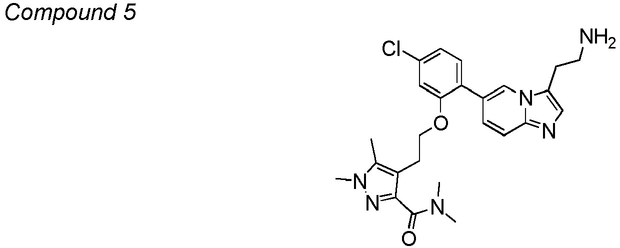

### 4-(2-{2-[3-(2-aminoethyl)imidazo[1,2-a]pyridin-6-yl]-5-chlorophenoxy}ethyl)-N,N,1,5-tetramethyl-1H-pyrazole-3-carboxamide

### Step 1

A solution of 4-(2-{2-[3-({[2-(tert-butoxy)carbonyl]amino}ethyl)imidazo[1,2-a]pyridin-6-yl]-4-chlorophenoxy}ethyl)-1,5-dimethyl-1H-pyrazole-3-carboxylic acid (Intermediate 1, 1.8 g, 3.25 mmol) in THF (20 mL) was added carbonyldimidazole (790 mg, 4.9 mmol) and the reaction mixture was stirred at rt for 3 hr. The mixture was treated with triethylamine (1.4 mL, 9.7 mmol) followed by dimethylamine solution (2M in THF, 3.2 mL, 6.5 mmol).The reaction mixture was stirred at rt for 16 hrs, quenched by addition of saturated sodium bicarbonate solution, extracted with ethyl acetate. The organic extract was dried over sodium sulphate and concentrated. The crude product was purified by prep TLC (3% MeOH/DCM) to give *tert-*butyl*N*-[2-(6-{2-[2-(3-(dimethyl)carbamoyl-1,5-dimethyl-1H-pyrazol-4-yl)ethoxy]-4-chlorophenyl}imidazo[1,2-a]pyridin-3-yl)ethyl]-carbamate as an off-white solid (1.0 g, 53%). ¹H NMR (400 MHz, DMSO-d₆) δ 8.38 (s, 1H), 7.51 (d, 1H), 7.42-7.45 (m, 2H), 7.23-7.26 (m, 2H), 7.11 (dd, 1H), 6.98 (m, 1H), 5.76 (s, 1H), 4.12 (t, 2H), 3.65 (s, 3H), 3.36 (t, 2H), 3.03 (s, 3H), 3.00 (t, 2H), 2.90 (s, 3H), 2.84 (t, 2H), 1.95 (s, 3H), 1.29 (d, 9H).

### Step 2

According to the general method for Boc deprotection (method A) *tert*-butyl *N*-[2-(6-{2-[2-(3-(dimethyl)carbamoyl-1,5-dimethyl-1H-pyrazol-4-yl)ethoxy]-4-chlorophenyl}imidazo[1,2-a]pyridin-3-yl)ethyl]-carbamate (1.40 g, 2.4 mmol) was treated with a solution of HCl in ether (2M, 70 mL). The solution stirred at room temperature for 3 hr and was evaporated under reduced pressure. The crude product dissolved in water and freeze dried to give the title compound as an off white solid (1.23 g, 92%) hplc rt 6.3 min LC-MS MH⁺ 481; ¹H NMR (400 MHz, DMSO-d₆) δ 14.9 (br.s, 1H), 9.01 (s, 1H), 8.36 (br. s, 3H), 8.17 (s, 1H), 8.02 (dd, 2H), 7.55 (d, 1H), 7.32 (d, 1H), 7.17 (d, 1H), 4.14 (t, 2H), 3.70 (s, 3H), 3.48 (t, 2H), 3.19 (t, 2H), 3.07 (s, 3H), 2.91 (s, 3H), 2.86 (t, 2H), 2.07 (s, 3H).

### Preparation of Compound 7

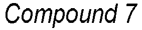

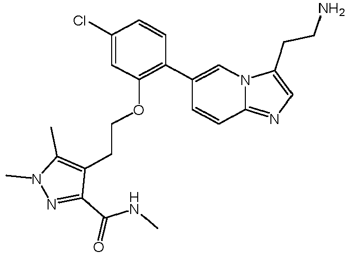

### 4-(2-{2-[3-(2-aminoethyl)imidazo[1,2-a]pyridin-6-yll-5-chlorophenoxylethyl)-N,1,5-trimethyl-1H-pyrazole-3-carboxamide

### Step 1

A solution of 4-(2-{2-[3-(2-{[(tert-butoxy)carbonyl]amino}ethyl)imidazo[1,2-a]pyridin-6-yl]-4-chlorophenoxy}ethyl)-1,5-dimethyl-1H-pyrazole-3-carboxylic acid (Intermediate 1, 64mg, 0.19 mmol) in THF (2 mL) was treated with triethylamine (0.048 mL, 0.35 mmol), methylamine solution (2M in THF, 0.17 mL, 0.35 mmol), hydroxybenztriazole (23.4 mg, 0.17 mmol) and EDCI (33.2 mg, 0.17 mmol).The reaction mixture was stirred at rt for 16 hrs, quenched with saturated NaHCO₃, extracted with ethyl acetate. The combined extracts were washed with water and brine, dried over sodium sulphate and concentrated. The crude product was purified by prep TLC (5% MeOH/DCM) to give tert-butyl N-{2-[6-(4-chloro-2-{2-[1,5-dimethyl-3-(methylcarbamoyl)-1H-pyrazol-4-yl]ethoxy}phenyl)imidazo[1,2-a]pyridin-3-yl]ethyl}carbamate as an off-white solid (30 mg, 46%). ¹H NMR (400 MHz, DMSO-d₆) δ 8.37 (s, 1H), 7.90 (d, 1H), 7.52 (d, 1H), 7.42-7.45 (m, 2H), 7.23-7.27 (m, 2H), 7.09 (d, 1H), 6.98 (t, 1H), 5.75 (s, 1H), 4.16 (t, 2H), 4.02 (q, 1H), 3.66 (s, 3H), 3.31 (t, 2H), 3.01 (m, 4H), 2.68 (s, 3H), 1.90 (s, 3H), 1.30 (d, 9H).

### Step 2

According to the general method for Boc deprotection (method B) tert-butyl N-{2-[6-(4-chloro-2-{2-[1,5-dimethyl-3-(methylcarbamoyl)-1H-pyrazol-4-yl]ethoxy}phenyl)imidazo[1,2-a]pyridin-3-yl]ethyl}carbamate (30 mg, 0.053 mmol) was dissolved in dioxane (2 mL), cooled to 0°C and treated with a solution of HCl in ether (2M, 2 mL). The solution stirred at room temperature for 3 hr and was evaporated under reduced pressure. The crude product dissolved in water and freeze dried to give the title compound as a light brown solid (20 mg, 81%) hplc rt 3.9 min LC-MS MH⁺ 467; ¹H NMR (400 MHz, DMSO-d₆) δ 14.7 (br.s, 1H), 8.98 (s, 1H), 8.19 (br. s, 3H), 8.15 (s, 1H), 8.05 (d, 1H), 8.01 (d, 1H), 7.89 (q, 1H), 7.55 (dd, 1H), 7.37 (d, 1H), 7.19 (dd, 1H), 4.18 (t, 2H), 3.72 (s, 3H), 3.46 (t, 2H), 3.20 (q, 2H), 3.04 (t, 2H), 2.67 (d, 3H), 2.07 (s, 3H).

### Biological data

### Materials

### Cell lines

*P-493-6*

The P-493-6 cell line is a non-transformed B cell line that can be genetically engineered to have either "low" levels of c-Myc, "medium" levels of c-Myc or "high" levels of c-Myc. The terms "low", "medium" and "high" are descriptors commonly used in the art and will be readily understood to mean:
i) "low" levels of c-Myc - a level of MYC expression which represents non-proliferating normal cells (i.e. a level of MYC expression not sufficient to cause the cycling of the cells);
ii) "medium" levels of c-Myc - a level of MYC expression which represents normal proliferating cells; and
iii) ""high" levels of c-Myc - a level of MYC expression which represents enforced/deregulated expression of c-Myc, typically observed in cancereous cells.

The P-493-6 cell lines were sourced from Chi Van Dang, Ludwig Cancer Research. The modified P-493-6 cell lines may be established using methodology well known in the art, see for example Int. J. Cancer, 2000, 87(6), 787-793.

### SKNAS and Shep

The SKNAS and Shep cell lines are both human neuroblastoma cell lines. The SKNAS cell line used herein was sourced from Linda Valentijn, Academic Medical Center, Amsterdam. The Shep cell line used herein was sourced from Michael D. Hogarty, The Children's Hospital of Philadelphia. The SKNAS and Shep cell lines used herein may be prepared using methods known in the art, see, for example, Cancer Res., 2005, 65(8), 3136-3145 and Oncogene, 2008, 27(24), 3424-3434 respectively.

In the experimental protocols described herein the SKNAS and Shep cell lines were genetically engineered to model differences between high grade neuroblastoma (which are usually marked by *MYCN* amplifications and with particular poor clinical prognosis) and low grade neuroblastoma. Accordingly, the SKNAS and/or Shep cell lines "with N-MYC induction" described herein will be understood to be cell lines treated with tamoxifen and represent aggressive, high grade type cancer cells. Conversely, the SKNAS and/or Shep cell lines "without N-MYC induction" described herein will be understood to be cell lines not treated with tamoxifen and represent less aggressive forms of cancer cells (Huang et al., Cold Spring Harb. Perspect. Med., 2013, 3(10), 1-22).

### General Experimental

### Cell Culture:

Shep-ER-N-Myc, SKNAS-ER-N-Myc, and the P-493-6 cell line were cultured in DMEM high glucose (ThermoFisher Scientific, 61965059), supplemented with 10% FBS at 5% CO₂. The BL41 cell line was cultured in RPMI 1640 (ThermoFisher Scientific, 61870044), supplemented with 10% FBS at 5% CO₂. HeLa cells were cultured in DMEM low glucose (Sigma, D6046), supplemented with 10% FBS at 10% CO₂. The Shep-ER-N-Myc was sourced from Linda Valentijn, and firstly reported in Valentijn et. al., Cancer Res, 2005, 65(8), 3136-3145. The SKNAS-ER-N-Myc was sourced from Michael D. Hogarty, and firstly reported in Ushmorov et. al., Oncogene, 2008, 27, 3424-3434. The P-493-6 cell line was sourced from Chi Van Dang, and firstly reported in Pajic et. al., Int J Cancer, 2000, 87(6), 787-793. BL41 and HeLa cell lines were sourced from the Cell Service Standard Technology Platform at the Francis Crick Institute.

### Western Blot:

Shep (200,000/well) and SKNAS (500,000/well) were seeded on a 6 well plate on day 0. On day 1, media was exchanged and media containing 100 nM Tamoxifen (H6278 Sigma) was added to the cells. Ethanol was used as control. 24 and 48 hours later, cells were lysed in cold buffer (PBS, 1% Tryton, 0.1% SDS). Protein concentration was measured with BCA assay (ThermoFisher Scientific, 23250) according to the manufacturer instructions. 20 µg of proteins were loaded on 10% acrylamide gel and transferred onto Nitrocellulose membrane. Membrane was blocked in 1X TBS 0.1 % Tween-20 containing 5% non-fat milk for 1 hour. *N-Myc* antibody (Cell Signalling, 9405) was prepared in 5% BSA 1XTBS 0.1 % Tween-20^{™} and the membrane was left incubating at 4 degrees centigrade overnight with gentle shaking. The following day Glyceraldehyde 3-phosphate dehydrogenase (GAPDH, Cell Signalling, 2118) was added for 1 hour as loading control in 5% BSA 1X TBS 0.1% tween-20. Membranes were washed 3 times in TBS 0.1%. Membranes were developed using secondary anti-rabbit (HRP-DAKO) antibody using an IMAGEQUANT 600 RGB.

### CellTiter Blue:

### General

At the respective end points of the experiments 20 µL/well of the CellTiter Blue Assay (Promega, G8081) were added and the cells were incubated at 37 °C for 3 hours. The fluorescence was measured at 570 nM using an EnVision^{™} plate reader. As a positive control, the highest DMSO concentration was added to the cells (usually 0.4% DMSO); as a negative control, the cells were treated with 10 µg/mL Puromycin and 1 uM Staurosporine (Merck, P7255 and S4400, respectively).

### P-493-6

The experiment was performed in a 96 well plate and in technical quadruplicate. On day 0, 2500 cells/well in 50 µL media were seeded in, for the *MYC* high condition: just media; for the MYC medium condition: 1 uM β-Estradiol (Merck, E8875) and 0.1 µg/mL Doxycycline (Merck, D9891); for the MYC low condition: 0.1 µg/mL Doxycycline. On day 1, 50 µL of media for the respective MYC condition and 2X NMT inhibitor, in the respective concentration, were added to the respective wells. On day 3 (for 48 hours incubation) or day 4 (for 72 hours) of incubation with the NMT inhibitor, the experiment was stopped according to the general experimental procedures for the CellTiter Blue experiments.

### Shep:

The experiment was performed in a 96 well plate and in technical quadruplicate. On day 0, 1,000 cells/well were seeded in 50 µL media. On day 1, 50 µL media containing 200 nM Tamoxifen (Merck, H7904), to induce N-Myc, or ethanol as control were added to the medium for 24 hours. On day 2, 100 µL of medium for the respective N-Myc condition and 2X NMT inhibitor, in the respective concentration, were added to the wells. On day 4 (for 72 hours incubation), the experiment was stopped according to the general experimental procedures for the CellTiter Blue experiments.

### SKNAS:

The experiment was performed in a 48 well plate and in technical triplicate. On day 0, 5,500 cells/well were seeded in 200 µL media. On day 1, the media was removed and 200 µL of media with Tamoxifen, to induce N-Myc, or ethanol as control were added. On day 2, the media was removed and 200 µL of medium for the respective N-Myc condition and 1X NMT inhibitor, in the respective concentration, were added. On day 6 (for 96 hours of incubation), the experiment was stopped according to the general experimental procedures for the CellTiter Blue experiments.

### Flow Cytometry

### General

Single-cell suspensions were stained with the following monoclonal antibody: α-Active Caspase3 (BD Biosciences, C92-605, 550821, PE) and c-Myc (Abcam, Y69, ab190560). To analyse DNA content, we stained with FxCycle Violet (ThermoFisher Scientific, F10347). To assess proliferation, we used the Click-iT EdU Alexa Fluor 488 Flow Cytometry Assay Kit (ThermoFisher Scientific, C10420) according to manufacturer's instructions. Zombie NIR (Biolegend, 423105) was used to exclude dead cells. The cells were fixed in a 4% solution of PFA, permeabilised with Cytofix/Cytoperm (BD Bioscience, 554714) and washed in Perm/Wash Buffer (BD Bioscience, 554723). Samples were acquired on a MACSQuant VYB (Miltenyi Biotec) and analysed using the FlowJo software (v10.4, Tree star).

### P-493-6

The experiment was performed in a 96 U-well plate and in technical triplicate to validate the system and in technical duplicate to study the effect of NMT inhibitors in the different *MYC* states.

For the validation of the system, on day 0, 5,000 cells/well in 100 µL media were seeded in, for the MYC high condition: just media; for the MYC medium condition: 1 uM β-Estradiol (Merck, E8875) and 0.1 µg/mL Doxycycline (Merck, D9891); for the MYC low condition: 0.1 µg/mL Doxycycline. On day 3, the cells were treated according to the general procedures for flow cytometry.

To study the effect of the NMT inhibitors, on day 0, 2,500 cells/well in 50 µL were seeded in the respective media conditions for the different MYC conditions. On day 1 50 µL of media for the respective MYC condition and 2X NMT inhibitor, in the respective concentration, were added to the respective wells. After 6 hours, 24 hours, 48 hours and 72 hours the cells were treated according to the general procedures for flow cytometry. After 24 hours, to the plates for the 48 hours and 72 hours-time points 100 µL of fresh media, for the respective MYC condition and 1x NMT inhibitor for the respective concentration were added to the wells, to a total of 200 µL of media.

### Shep and SKNAS

The experiment was performed in a 24 well plate and in technical duplicate. On day 0, 7,800 cells/well for the Shep and 15,000 cells/well for the SKNAS were seeded in 500 µL media. On day 1, the media was removed and 600 µL of media with 100 nM of Tamoxifen, to induce N-Myc, or ethanol as control. On day 2, the respective concentrations of NMT inhibitor were added. After 6 hours, 24 hours, 48 hours and 72 hours the cells were treated according to the general procedures; however, all the media was collected to not loose apoptotic cells, and Trypsin (ThermoFisher Scientific, 25200056) was used to harvest the cells. The combined cells and media were collected in a tube and spun at 2000 g x 3 minutes, and transferred to a 96 well plate for further staining according to the general procedures for flow cytometry.

### RNA sequencing

Samples were purified with the miRNeasy Mini Kit (Qiagen, 217004) according to manufacturer's instructions. The RNA sequencing was performed at the Advanced Sequencing Unit, The Francis Crick Institute. Samples were prepared with the Nugen cDNA kit and sequenced using Illumina HiSeq system. Gene expression raw data was analysed by the Bioinformatics and Biostatistics Unit, The Francis Crick Institute. The analysis was performed on biological replicates for each condition on biological replicates generating approximately 30 million 100 bp paired-end reads. The RSEM package (v1.3.0) (Li and Dewy, BMC Bioinformatics, 2011) and STAR (v2.5.2a) (Dobin et. al., Bioinformatics, 2013) were used to align reads to the human hg38 transcriptome, taken from Ensembl (v. GRChg38) available at UCSC (http://hqdownload.soe.ucsc.edu/downloads.html). For RSEM, all parameters were run as default using the "-forward-prob 0" option for strand specific protocol. Differential expression analysis was carried out with DESeq2 (v1.12.4) (Love, et. al., Genome Biol, 2014) within R (v3.3.1).

### BL41

The experiment was performed in T-25 flasks in biological quadruplicate. On day 0, 500,000 cells/mL were seeded in 10 mL of media. On day 1, 100 nM of Compound 2 or DMSO as control were added. On day 2 (24 hours of incubation), the RNA was purified with the miRNeasy kit, according to manufacturer's instructions.

### HeLa

The experiment was performed in 10 cm² dishes, in biological quadruplicate. On day 0, 1,000,000 cells/dish were plated in 10 mL of media. On day 1, 100 nM of Compound 2 or DMSO as control were added. On day 2 (24 hours of incubation), the RNA was purified with the miRNeasy kit, according to manufacturer's instructions.

### Gene set enrichment analysis

Gene set enrichment analysis (GSEA) was performed using GSEA (v3.0) (Subramanian et. al., PNAS, 2005, 102(43), 15545-15550). Gene sets were obtained from the Broad Institute Signatures data base (http://software.broadinstitute.org/gsea/msigdb/index.jsp). For the in-house RNAseq data, GSEA was carried out with ranked gene lists, using Wald statistics. All parameters were kept as default, except for the enrichment statistic (classic) and the max size that was changed to 500,000 respectively. For the microarray data, the pre-processed data was obtained from the Sanger Institute (Lorio et. al., Cell, 2016, 166(3), 740-754; https://www.cancerrxgene.org/downloads). All parameters were kept as default, except the max size that was changed to 500,000 respectively. For the RNAseq data and CRISPR data sets from the Broad Institute, the pre-processed data was obtained from the DepMap project (CCLE and GDSC Consortium, Nature, 2015, 528, 84-87 and Robin et. al., Nature Genetics, 2017, 49(12), 1779-1784; https://depmap.org/portal/download/; Public 18Q4 Versions). All parameters in the GSEA were kept to default, except the max size that was changed to 500,000 respectively.

### Pharmacogenomics screens

The sensitivity data, measured as EC50, for cancer cell lines treated with Compound 4, Compound 3, and Compound 6 were provided by the Sanger Institute, using their methods described in Lorio et. al., Cell, 2016, 166(3), 740-754. The sensitivity data, measured as EC50, on Compound 3 and Compound 4 is not yet published; the sensitivity data for Compound 6 is available on: https://www.cancerrxgene.org/translation/Drug/1266. For the microarray data, copy number data, recurrently altered chromosomal segments data, and genomic variants, used to compare the expression of different genes or the presence/absence of structural alterations in the Myc paralogs loci with sensitivity, the pre-processed data was obtained from the Sanger Institute (https://www.cancerrxgene.org/downloads).

### RESULTS

### NMT inhibition

The NMT inhibition activity data for Compounds 1, 2, 3 and 4 is described in WO2017/001812. In particular, the NMT inhibition data is provided in Table 1 of WO2017/001812.

The inhibition activity data for Compound 6 is described in WO2010/026365, more specifically in the Table on page 118 of WO2010/026365.

### COMPOUNDS 5 and 7

### Example (a): HsNMT1 IC₅₀

The IC₅₀ values for human NMT1 (HsNMT1) of Compounds 5 and 7 were measured using a sensitive fluorescence-based assay based on detection of CoA by 7-diethylamino-3-(4-maleimido-phenyl)-4-methylcoumarin, as described in Goncalves, V., et al., Analytical Biochemistry, 2012, 421, 342-344 and Goncalves, V., et al., J. Med. Chem, 2012, 55, 3578.

The HsNMT1 IC₅₀ value for Compound 5 was observed to be 1.5 nM.

The HsNMT1 IC₅₀ value for Compound 7 was observed to be 0.4 nM.

### Example (b) Metabolic Activity Assay (MTS Assay)

Compounds 5 and 7 were also tested for activity in an *in vitro* metabolic activity assay using the human cell line MRC5. Compounds having activity in inhibiting metabolic activity in the assay are expected to be useful as agents for preventing and/or treating cancer, by virtue of being inhibitors of human NMT1 and/or NMT2. The compounds with the highest activity in the assay are expected to be the most potent inhibitors of human NMT1 and/or NMT2.

### Cell Preparation

MRC5 cells (cell type: fibroblast cells) were grown in DMEM media (supplemented with 10% FBS) and were seeded in a 96-well plate, 24 h prior to treatment. Cell suspensions were prepared by adjusting the cell density to the appropriate concentration (as stated in the Table 1 below) and 50 µL of the cell suspension was transferred to wells B-G in columns 2-11 of a 96-well plate.

**Table 1 - Number of cells plated**

| | MRC5 |
|---|---|
| Cell suspension concentration (cells/mL) | 38,000 |
| cells per well | 1,900 |

### Assay procedure

100 µL of growth media (DMEM media) containing 0.2% DMSO was added to wells B-G in columns 2 and 11 as positive controls, and 100 µL of growth media containing Puromycin (3 µg/mL; final concentration in the plate 2 µg/mL) was added to wells B-G in column 3 as a negative control. Seven concentrations of NMT inhibitor stock solution were prepared for Compound 5 (same final percentage of DMSO, dilution factor = 3 starting from 15 µM or 150 µM). 100 µL of inhibitor stock solution was added to wells B-G in columns 4-10 of a 96-well plate (final concentration of Commpound 5 in the plate starting from 10 µM or 100 µM; total volume in each well was 150 µL). The plate was incubated at 37 °C with 5% CO₂ level.

After 72 h, 20 µL MTS reagent (Promega, prepared according to the supplier protocol) was added to each well of the 96-well plate. The plate was incubated at 37 °C for 2 h and the absorbance per well was measured at 490 nm with an EnVision plate reader. The average absorbance value of the negative control (Puromycin-treated cells) was subtracted from each value and the metabolic activity was calculated as a percentage relative to the positive control (DMSO-treated cells). EC₅₀ values were calculated using GraphPad.

The EC₅₀ value for Compound 5 was observed to be 13 nM.

The EC₅₀ value for Compound 7 was observed to be 37 nM.

### Correlations between alterations in MYC and the effectiveness of a NMT inhibitor

### P-493-6 cell lines

### Validation of the P-493-6 cell lines

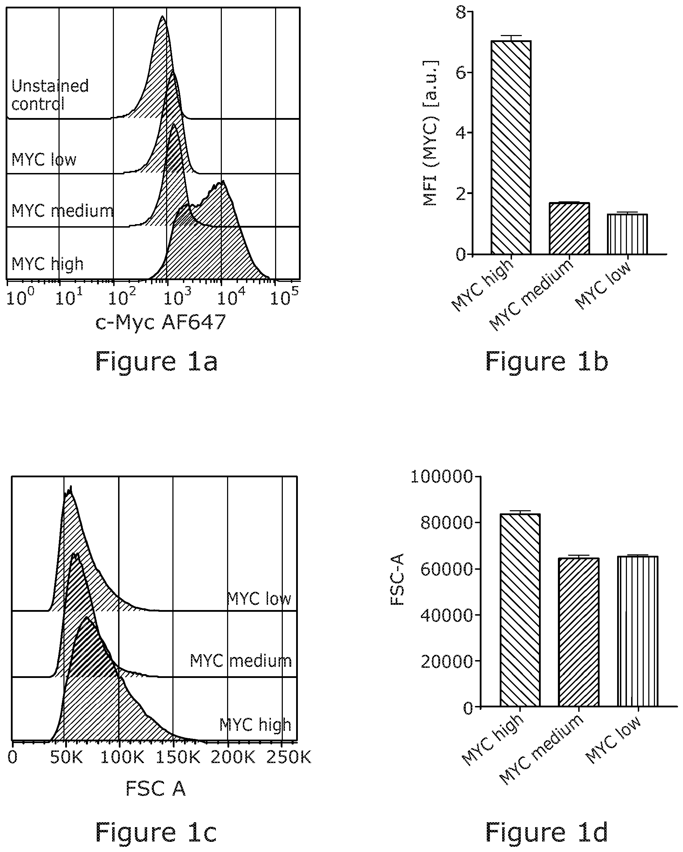

Figures 1, 2 and 3 show the flow cytometry analysis for the expression of *c-MYC* in the P-493-6 cell line. This data shows that the genetically engineered P-493-6 cell lines (immortalised B cell lines with an inducible system) allow for three different *MYC* levels: high, medium and low (as defined above). This makes the P-493-6 cell line a particularly good cell line to use for determining the correlation between the effectiveness of an NMT inhibitor and *MYC* expression.

High levels of *MYC* expression was found to cause increased cell size, which was measured and determined by increased forward scatter.

Figure 1 shows: a) representative flow cytometry analysis for the expression of the c-Myc in the P-493-6 cell line from an unstained control (first row), low levels of c-Myc (second row), medium levels of c-Myc (third row), and high levels of c-Myc (fourth row); b) quantification of the median fluorescence intensities (MFI), normalised against the unstained control of the expression levels of c-Myc (N = 3); c) representative flow cytometry analysis for the forward scatter area (FSC-A) as a surrogate for cell size in the P-493-6 cell line from low levels of c-Myc (first row), medium levels of c-Myc (second row), and high levels of c-Myc (third row); and d) quantification of the FSC-A geometric mean (N = 3).

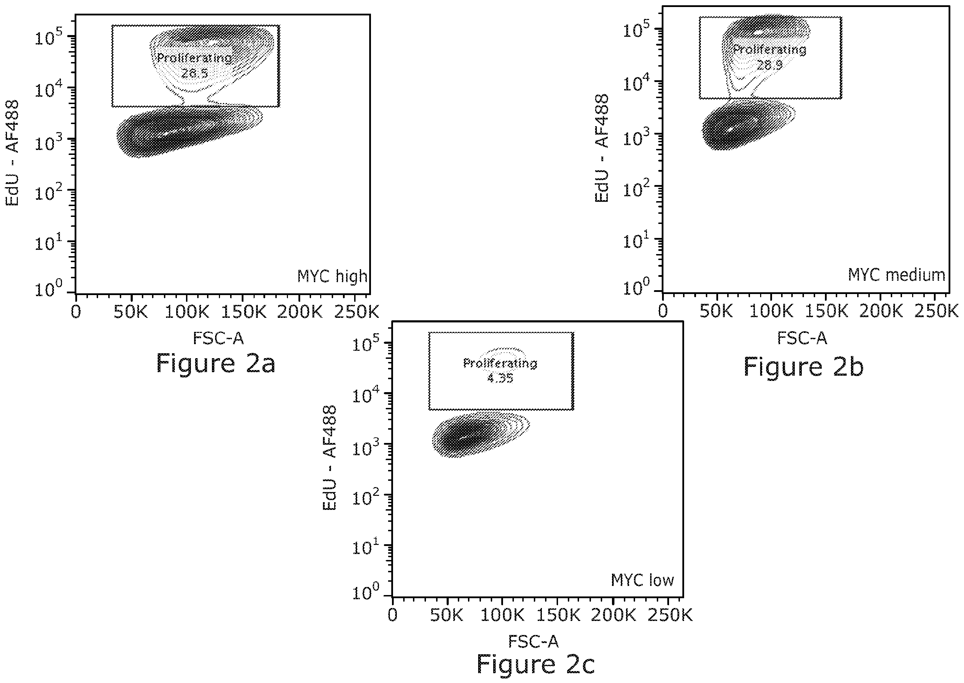

Figure 2 shows: a) representative flow cytometry analysis of EdU incorporation, clicked to an AlexaFluor 488 fluorochrom, in the c-Myc high P-493-6 cell line; b) representative flow cytometry analysis of EdU incorporation, clicked to an AlexaFluor 488 fluorochrom, in the c-Myc medium P-493-6 cell line; and c) representative flow cytometry analysis of EdU incorporation, clicked to an AlexaFluor 488 fluorochrom, in the c-Myc low P-493-6 cell line.

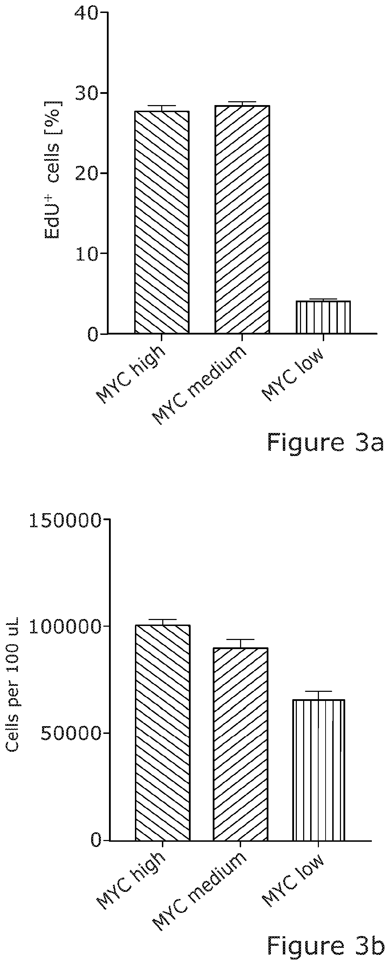

Figure 3 shows: a) quantification of the relative proportion of cells that are EdU positive, hence engaging in DNA synthesis (N = 3); b) quantification of the cell number in 100 uL of cell suspension, analysed via flow cytometry after 72 hours of induction (N = 3).

While there is little difference between DNA synthesis between the P-493-6 cell lines with high, medium or low MYC levels (as measured by EdU incorporation), overall, the cell numbers were found to be higher in the MYC high state P-496-6 cell lines, indicating a potentially faster cycling.

### MYC synthetic lethality data

Figure 4 shows: a) metabolic viability of the P-493-6 cell line treated with Compound 2 at the shown concentrations for 48 hours, with different expression levels of c-Myc induced; b) metabolic viability of the P-493-6 cell line treated with Compound 2 at the shown concentrations for 72 hours, with different expression levels of c-Myc induced.

From Figure 4 it can be seen that at both 48 hours and 72 hours there is a clear *MYC-*dependent increase in cell death in the P-493-6 cell lines. This data suggests that high levels of c-MYC are lethal in combination with an NMT inhibitor, such as Compound 2, and even administering the NMT inhibitor at a concentration as low as 10 nM resulted in measurable effects on cell viability at 72 hours in the Myc high cell lines.

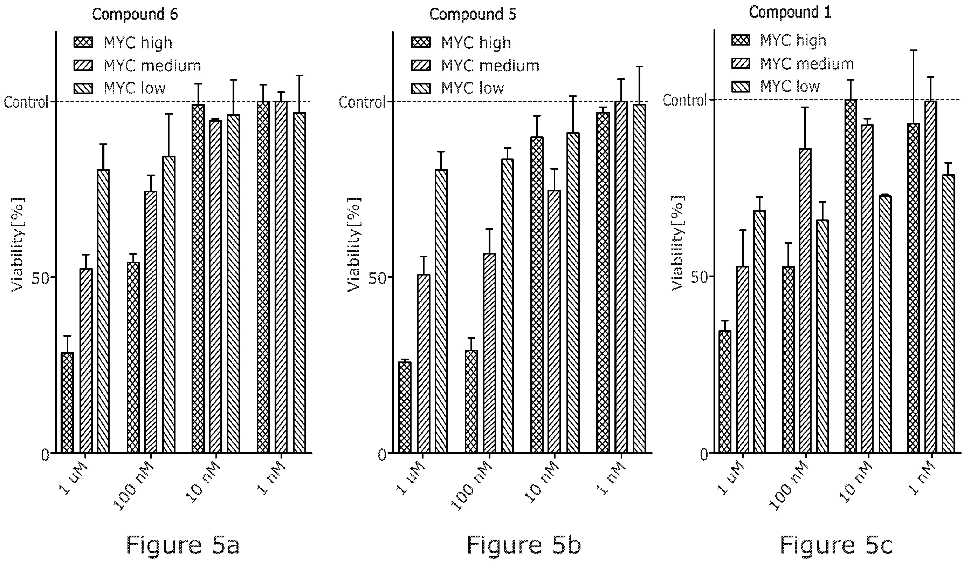

Figure 5 shows: a) metabolic viability of the P-493-6 cell line treated with Compound 6 at the shown concentrations for 72 hours, with different expression levels of c-Myc induced; b) metabolic viability of the P-493-6 cell line treated with Compound 5 at the shown concentrations for 72 hours, with different expression levels of c-Myc induced; and c) metabolic viability of the P-493-6 cell line treated with Compound 1 at the shown concentrations for 72 hours, with different expression levels of c-Myc induced.

Figure 5 shows that the *MYC*-dependent increase in cell death in the P-493-6 cell lines is observed for a range of structurally diverse NMT inhibitor compounds. With the highest levels of cell death being recorded in each case for P-493-6 cell lines with high *MYC* expression.

### Quantification of cell numbers

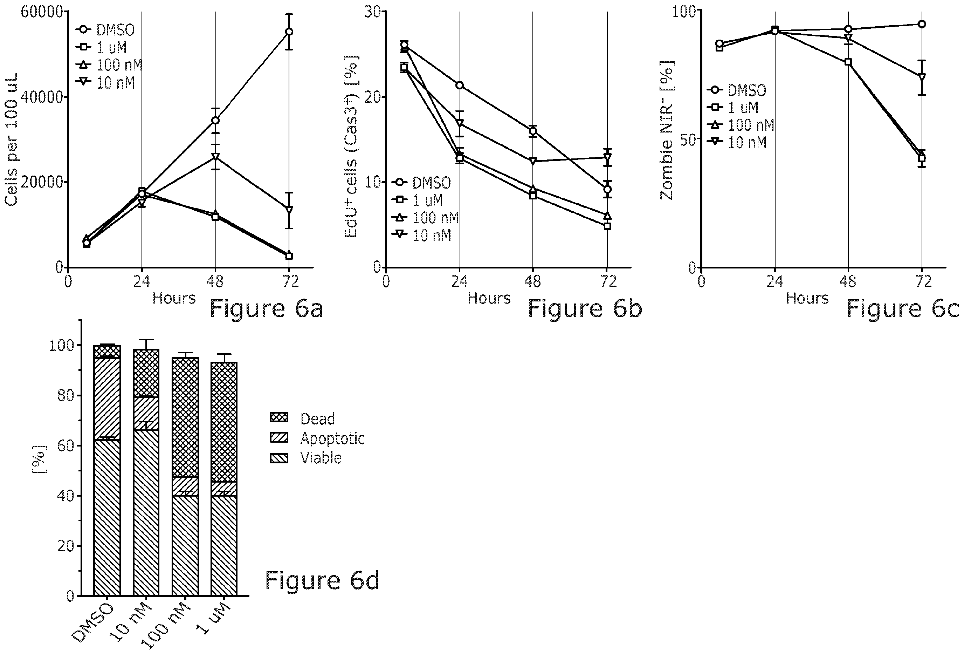

Figure 6 shows: a) quantification of the cell numbers (excluding dead cells) of the P-493-6 cell line with c-MYC high, at the given time points (6, 24, 48 and 72 hours) with the shown concentrations (1 uM, 100 nM, 10 nM and control) of Compound 2 (N = 2); b) quantification of the relative proportion of cells that are EdU positive, hence engaging in DNA synthesis, of the P-493-6 cell line with c-MYC high, at the given time points (6, 24, 48 and 72 hours) with the shown concentrations (1 uM, 100 nM, 10 nM and control) of Compound 2 (N = 2); c) quantification of the relative proportion of cells that are Zombie NIR negative, hence have intact cell membranes, of the P-493-6 cell line with c-MYC high, at the given time points (6, 24, 48 and 72 hours) with the shown concentrations (1 uM, 100 nM, 10 nM and control) of Compound 2 (N = 2); and d) quantification of the relative proportion of cells that are either dead (Zombie NIR positive), undergoing apoptosis (Caspase3 positive) or viable (Zombie NIR negative and Caspase 3 negative) of the P-493-6 cell line with c-Myc high (N = 2).

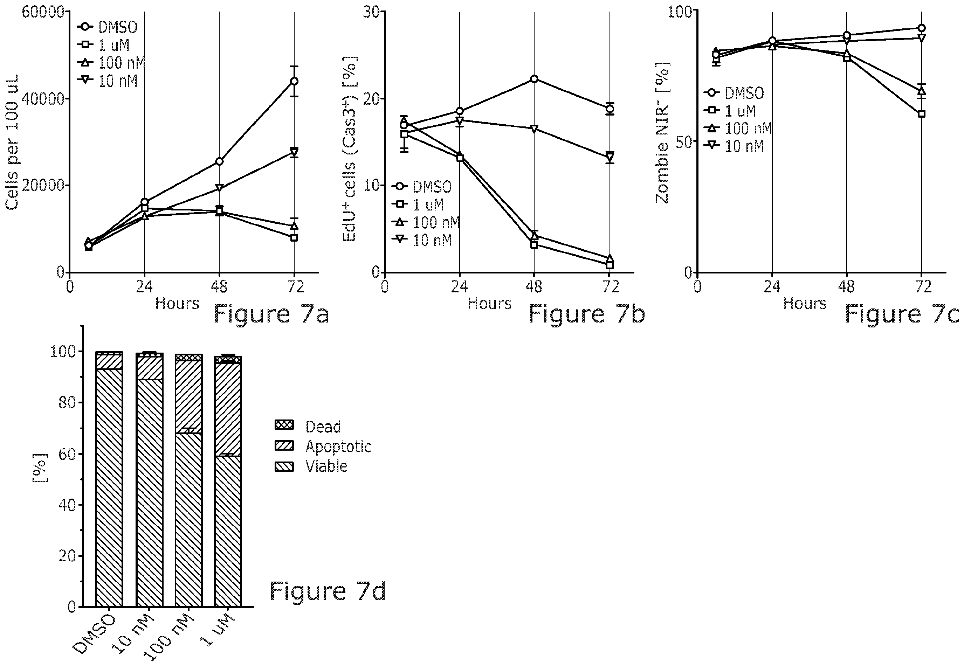

Figure 7 shows: a) quantification of the cell numbers (excluding dead cells) of the P-493-6 cell line with c-MYC medium, at the given time points (6, 24, 48 and 72 hours) with the shown concentrations (1 uM, 100 nM, 10 nM and control) of Compound 2 (N = 2); b) quantification of the relative proportion of cells that are EdU positive, hence engaging in DNA synthesis, of the P-493-6 cell line with c-MYC medium, at the given time points (6, 24, 48 and 72 hours) with the shown concentrations (1 uM, 100 nM, 10 nM and control) of Compound 2 (N = 2); c) quantification of the relative proportion of cells that are Zombie NIR negative, hence have intact cell membranes, of the P-493-6 cell line with c-MYC medium, at the given time points (6, 24, 48 and 72 hours) with the shown concentrations (1 uM, 100 nM, 10 nM and control) of Compound 2 (N = 2); and d) quantification of the relative proportion of cells that are either dead (Zombie NIR positive), undergoing apoptosis (Caspase3 positive) or viable (Zombie NIR negative and Caspase 3 negative) of the P-493-6 cell line with c-Myc medium.

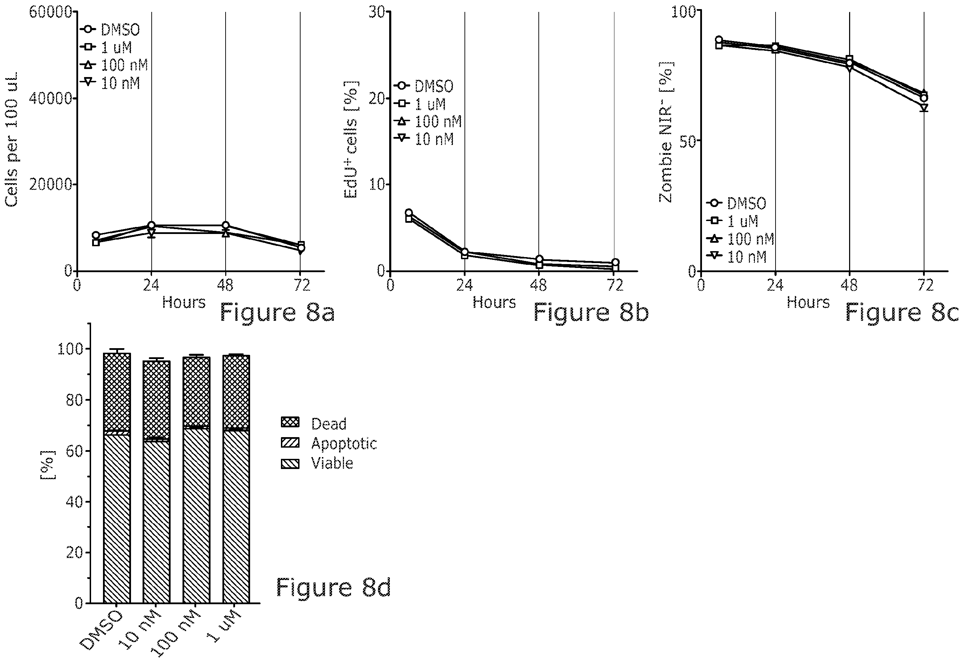

Figure 8 shows: a) quantification of the cell numbers (excluding dead cells) of the P-493-6 cell line with c-MYC low, at the given time points (6, 24, 48 and 72 hours) with the shown concentrations (1 uM, 100 nM, 10 nM and control) of Compound 2 (N = 2); b) quantification of the relative proportion of cells that are EdU positive, hence engaging in DNA synthesis, of the P-493-6 cell line with c-MYC low, at the given time points (6, 24, 48 and 72 hours) with the shown concentrations (1 uM, 100 nM, 10 nM and control) of Compound 2 (N = 2); and c) quantification of the relative proportion of cells that are Zombie NIR negative, hence have intact cell membranes, of the P-493-6 cell line with c-MYC low, at the given time points (6, 24, 48 and 72 hours) with the shown concentrations (1 uM, 100 nM, 10 nM and control) of Compound 2 (N = 2); and d) quantification of the relative proportion of cells that are either dead (Zombie NIR positive), undergoing apoptosis (Caspase3 positive) or viable (Zombie NIR negative and Caspase 3 negative) of the P-493-6 cell line with c-Myc low.

Figures 6, 7 and 8 show that high levels of c-MYC, in combination with an NMT inhibitor, cause rapid depletion of the cells. It can be seen from Figures 6, 7 and 8 that the MYC high cells are being depleted faster than the MYC medium and MYC low cells, with a rapid drop in overall cell viability. Interestingly, DNA synthesis in relative terms is less affected than in the MYC medium case. The MYC low cells are relatively unaffected by the administration of the NMT inhibitor compound.

### Shep ER-N-MYC and SKNAS ER-N-MYC cell lines

### Validation of the Shep ER-N-MYC and SKNAS ER-N-MYC cell lines

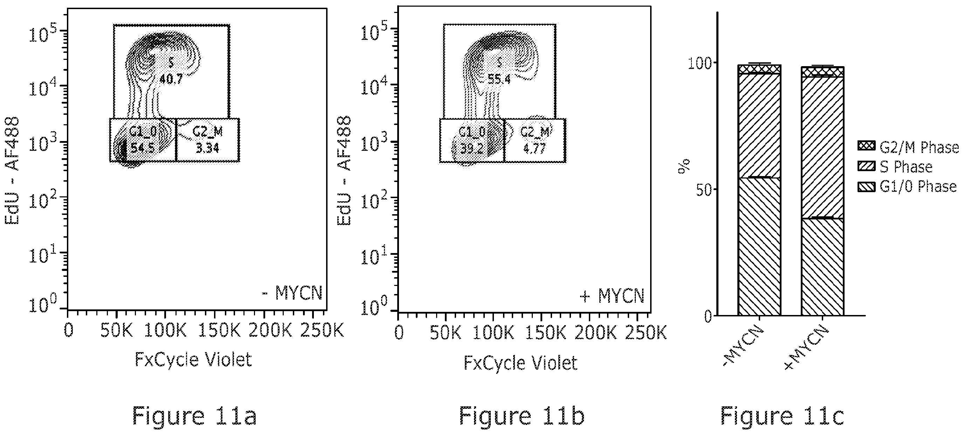

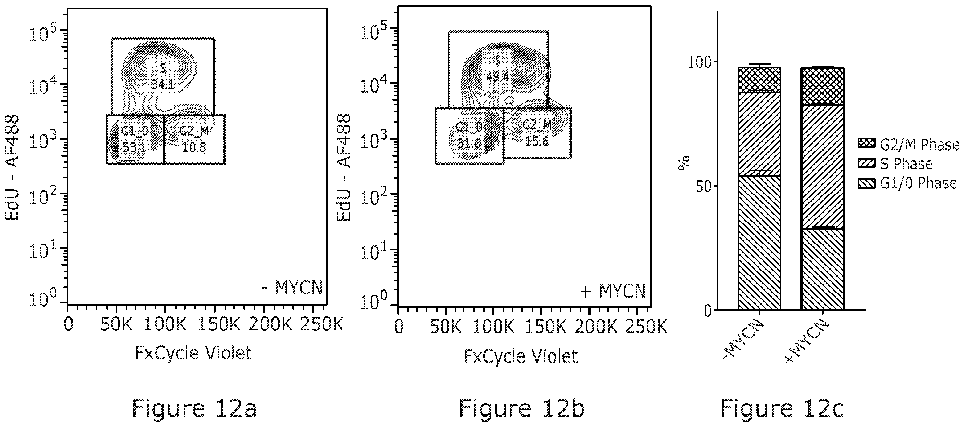

SKNAS and Shep are neuroblastoma cell lines, which usually have little to no *MYCN* expression. Upon treatment with tamoxifen, the cell line induces *MYCN* which increases cycling (seen in the top trace of Figure 11 and 12 measured by increase in S phase) and protein synthesis (seen in Figure 13 and measured by OPP incorporation).

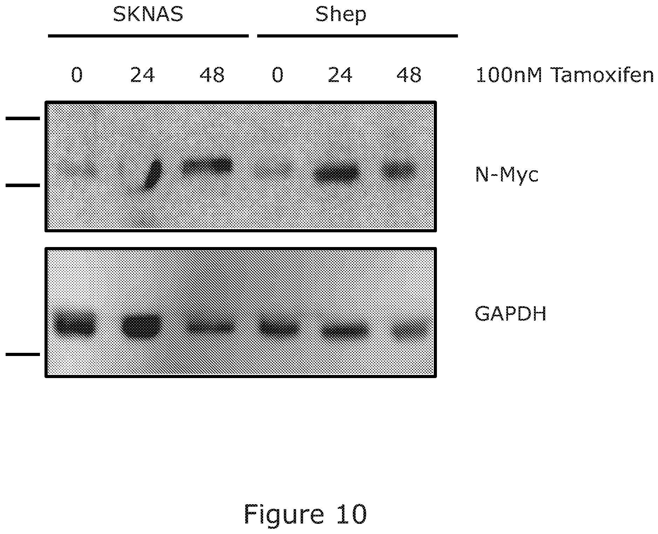

Figure 10 shows representative western blot showing MYCN protein expression following time course treatment with 100 nM of Tamoxifen. GAPDH was used as loading control.

Figure 11 shows: a) representative flow cytometric analysis of the cell cycle profile for the Shep ER-N-MYC cell line, without induction of *MYCN.* Cells in G1/0 phase have low amounts of FxCycle Violet stainings and are EdU negative. Cells in S phase are EdU positive. Cells in G2/M phase have high amounts of FxCycle Violet staining and are EdU negative; b) representative flow cytometric analysis of the cell cycle profile for the Shep ER-N-MYC cell line, with induction of *MYCN.* Cells in G1/0 phase have low amounts of FxCycle Violet stainings and are EdU negative. Cells in S pase are EdU positive. Cells in G2/M phase have high amounts of FxCycle Violet staining and are EdU negative; and c) quantification of the cell cycle profile of the Shep cell, with and without the induction of *MYCN* (N = 3).

Figure 12 shows: a) representative flow cytometric analysis of the cell cycle profile for the SKNAS ER-N-MYC cell line, without induction of *MYCN.* Cells in G1/0 phase have low amounts of FxCycle Violet stainings and are EdU negative. Cells in S pase are EdU positive. Cells in G2/M phase have high amounts of FxCycle Violet staining and are EdU negative; b) representative flow cytometric analysis of the cell cycle profile for the SKNAS ER-N-MYC cell line, with induction of *MYCN.* Cells in G1/0 phase have low amounts of FxCycle Violet stainings and are EdU negative. Cells in S pase are EdU positive. Cells in G2/M phase have high amounts of FxCycle Violet staining and are EdU negative; and c) quantification of the cell cycle profile of the SKNAS ER-N-MYC cell, with and without the induction of *MYCN* (N = 3).

Figure 13 shows: a) representative flow cytometric analysis of the protein synthesis, via incorporation of OPP, of the Shep ER-N-MYC cell line with or without induction of *MYCN;* first row: with induction of MYCN; second row: without induction of *MYCN;* last row: no OPP added; b) quantification of the median fluorescence of OPP, clicked to the AF488 fluorochrome, of the Shep ER-N-MYC cell line with and without induction of N-Myc; c) representative flow cytometric analysis of the protein synthesis, via incorporation of OPP, of the SKNAS ER-N-MYC cell line with or without induction of *MYCN;* first row: with induction of MYCN; second row: without induction of *MYCN;* last row: no OPP added; d.) quantification of the median fluorescence of OPP, clicked to the AF488 fluorochrome, of the SKNAS ER-N-MYC cell line with and without induction of *MYCN.*

### MYC synthetic lethality data

Similar to the case of c-MYC in the P-493-6 cell lines, it can be seen from Figure 14 and 15 that high levels of MYCN induce increased cell death more rapidly at lower concentrations upon treatment with Compound 2 than for low levels of MYCN. Similar results are observed when the Compounds 1 and 6 are administered to the Shep-ER-N-MYC and SKNAS-ER-N-MYC cell lines (see Figures 14 and 15).

Figure 14 shows: a) metabolic viability of the Shep-ER-N-MYC cell line treated with Compound 6 at the shown concentrations for 72 hours, with (grey) or without (black) induction of *MYCN;* b) metabolic viability of the Shep-ER-N-MYC cell line treated with Compound 2 at the shown concentrations for 72 hours, with (grey) or without (black) induction of *MYCN;* c) metabolic viability of the SKNAS-ER-N-MYC cell line treated with Compound 1 at the shown concentrations for 72 hours, with (orange) or without (grey) induction of *MYCN.*

Figure 15 shows the metabolic viability of the SKNAS-ER-N-MYC cell line treated with: a) Compound 6; b) Compound 2; and c) Compound 1 at the shown concentrations for 96 hours, with (grey) or without (black) induction of *MYCN.*

### Quantification of cell numbers

As can be seen from Figures 16 and 18, administration of a NMT inhibitor to a neuroblastoma cell with high levels of MYCN results in rapid depletion of the cells. For the Shep cell line in the high *MYCN* state, apoptosis induction occurs rapidly after 24 hours (up to 60%). In the case of no (or low) MYCN, this only occurs after 48 hours and to a much lesser degree. As similarly observed for c-MYC expression in the P-493-6 cell line, the Shep cells are still trying to cycle at 48 hours with high MYCN. (See Figure 17) Similar results are observed for the SKNAS ER-N-MYC cell line (Figures 18 and 19).

Figure 16 shows: a) quantification of the cell numbers (excluding dead cells) of the Shep-ER-N-MYC cell line without *MYCN* induction, at the given time points (6, 24, 48 and 72 hours) with the shown concentrations (1 uM, 100 nM, 10 nM and control) of Compound 2 (N = 2); b) quantification of the relative proportion of cells that are EdU positive, hence engaging in DNA synthesis, of the Shep-ER-N-MYC cell line without *MYCN* induction, at the given time points (6, 24, 48 and 72 hours) with the shown concentrations (1 uM, 100 nM, 10 nM and control) of Compound 2 (N = 2); c) quantification of the relative proportion of cells that caspase3 positive, hence are undergoing apoptosis, of the Shep-ER-N-MYC cell line without *MYCN* induction, at the given time points (6, 24, 48 and 72 hours) with the shown concentrations (1 uM, 100 nM, 10 nM and control) of Compound 2 (N = 2). d) quantification of the cell numbers (excluding dead cells) of the Shep-ER-N-MYC cell line with *MYCN* induction, at the given time points (6, 24, 48 and 72 hours) with the shown concentrations (1 uM, 100 nM, 10 nM and control) of Compound 2 (N = 2); e) quantification of the relative proportion of cells that are EdU positive, hence engaging in DNA synthesis, of the Shep-ER-N-MYC cell line with *MYCN* induction, at the given time points (6, 24, 48 and 72 hours) with the shown concentrations (1 uM, 100 nM, 10 nM and control) of Compound 2 (N = 2); and f) quantification of the relative proportion of cells that caspase3 positive, hence are undergoing apoptosis, of the Shep-ER-N-MYC cell line with *MYCN* induction, at the given time points (6, 24, 48 and 72 hours) with the shown concentrations (1 uM, 100 nM, 10 nM and control) of Compound 2 (N = 2).

Figure 17 shows: a.) quantification of the cell cycle profile of the Shep-ER-N-Myc cell line without induction of *MYCN,* treated with the shown concentration of Compound 2 (1 uM, 100 nM, 10 nM and control) for 48 hours (N = 2); b.) quantification of the cell cycle profile of the Shep-ER-N-Myc cell line without induction of *MYCN,* treated with the shown concentration of Compound 2 (1 uM, 100 nM, 10 nM and control) for 72 hours (N = 2); c.) quantification of the cell cycle profile of the Shep-ER-N-Myc cell line with induction of *MYCN,* treated with the shown concentration of Compound 2 (1 uM, 100 nM, 10 nM and control) for 48 hours (N = 2); and d.) quantification of the cell cycle profile of the Shep-ER-N-Myc cell line with induction of MYCN, treated with the shown concentration of Compound 2 (1 uM, 100 nM, 10 nM and control) for 72 hours (N = 2).

Figure 18 shows: a) quantification of the cell numbers (excluding dead cells) of the SKNAS-ER-N-MYC cell line without *MYCN* induction, at the given time points (6, 24, 48 and 72 hours) with the shown concentrations (1 uM, 100 nM, 10 nM and control) of Compound 2 (N = 2); b) quantification of the relative proportion of cells that are EdU positive, hence engaging in DNA synthesis, of the SKNAS-ER-N-MYC cell line without *MYCN* induction, at the given time points (6, 24, 48 and 72 hours) with the shown concentrations (1 uM, 100 nM, 10 nM and control) of Compound 2 (N = 2); c) quantification of the relative proportion of cells that caspase3 positive, hence are undergoing apoptosis, of the SKNAS-ER-N-MYC cell line without *MYCN* induction, at the given time points (6, 24, 48 and 72 hours) with the shown concentrations (1 uM, 100 nM, 10 nM and control) of Compound 2 (N = 2). d) quantification of the cell numbers (excluding dead cells) of the SKNAS-ER-N-MYC cell line with *MYCN* induction, at the given time points (6, 24, 48 and 72 hours) with the shown concentrations (1 uM, 100 nM, 10 nM and control) of Compound 2 (N = 2); e) quantification of the relative proportion of cells that are EdU positive, hence engaging in DNA synthesis, of the SKNAS-ER-N-MYC cell line with *MYCN* induction, at the given time points (6, 24, 48 and 72 hours) with the shown concentrations (1 uM, 100 nM, 10 nM and control) of Compound 2 (N = 2); and f) quantification of the relative proportion of cells that caspase3 positive, hence are undergoing apoptosis, of the Shep-ER-N-MYC cell line with *MYCN* induction, at the given time points (6, 24, 48 and 72 hours) with the shown concentrations (1 uM, 100 nM, 10 nM and control) of Compound 2 (N = 2).Figure 19 shows: a) quantification of the cell cycle profile of the Shep-ER-N-Myc cell line with induction of *MYCN,* treated with the shown concentration of Compound 2 (1 uM, 100 nM, 10 nM and control) for 48 hours (left) and 72 hours (right) (N = 2); and b) relative change of the different cell cycle phases, shown as log2-fold changes over the control for 100 nM Compound 2 at 48 hours and 72 hours for the Shep-ER-N-Myc cell line with induction of *MYCN.*

Figure 19 shows: a.) quantification of the cell cycle profile of the SKNAS-ER-N-Myc cell line without induction of *MYCN,* treated with the shown concentration of Compound 2 (1 uM, 100 nM, 10 nM and control) for 48 hours (N = 2); b.) quantification of the cell cycle profile of the SKNAS-ER-N-Myc cell line without induction of *MYCN,* treated with the shown concentration of Compound 2 (1 uM, 100 nM, 10 nM and control) for 72 hours (N = 2); c.) quantification of the cell cycle profile of the SKNAS-ER-N-Myc cell line with induction of *MYCN,* treated with the shown concentration of Compound 2 (1 uM, 100 nM, 10 nM and control) for 48 hours (N = 2); and d.) quantification of the cell cycle profile of the SKNAS-ER-N-Myc cell line with induction of *MYCN,* treated with the shown concentration of Compound 2 (1 uM, 100 nM, 10 nM and control) for 72 hours (N = 2).

As can be seen from Figures 17 and 19, and as was similarly observed for c-MYC, MYCN forces the cells through the G1-S checkpoint increasing the lethality of the NMT inhibitor.

Furthemrore, it was observed that the *MYCN* activated cells do not reduce their cycling even when presented with Compound 2 (see Figures 17 and 19), although there is a slight increase in G2/M accumulation. On the other hand, the cell line without activated *MYCN* was observed to stops cycling at 48 hours.

### Global correlations

The GDSC data shown in Figures 20-22 indicates a correlation between *c-MYC* expression and sensitivity to an NMT inhibitor. A trend was observed indicating that increased *c-MYC* correlates well with higher sensitivity to the three different NMT inhibitors tested in the Sanger cancer line drug screen. Contrary to previous disclosures in the art (see, for example, WO2017/011907A1), Figure 23 shows that there is no correlation between the expression of hsNMT1 or hsNMT2 with the sensitivity to an NMT inhibitor.

Figure 20 shows: a) global comparison of the correlation between sensitivity to the NMT inhibitor Compound 6, measured as EC50, and normalised *c-MYC* expression (the expression data was obtained from Lorio et. al., Cell, 2016, 166(3), 740-754 and a nonparametric Spearman correlation coefficient was used); b) comparison between the top quartile and bottom quartile of cells with the highest (dashed/grey) and lowest expression (black) and their respective responsiveness to the NMT inhibitor Compound 6 (a nonparametric Mann Whitney test was used).

Figure 21 shows: a) global comparison of the correlation between sensitivity to the NMT inhibitor Compound 4, measured as EC50, and normalised *c-MYC* expression (the expression data was obtained from Lorio et. al., Cell, 2016, 166(3), 740-754 and a nonparametric Spearman correlation coefficient was used); and b) comparison between the top quartile and bottom quartile of cells with the highest (dashed/grey) and lowest expression (black) and their respective responsiveness to the NMT inhibitor Compound 4 (a nonparametric Mann Whitney test was used).

Figure 22 shows: a) global comparison of the correlation between sensitivity to the NMT inhibitor Compound 3, measured as EC50, and normalised *c-MYC* expression (the expression data was obtained from Lorio et. al., Cell, 2016, 166(3), 740-754 and a nonparametric Spearman correlation coefficient was used); b) comparison between the top quartile and bottom quartile of cells with the highest (dashed/grey) and lowest expression (black) and their respective responsiveness to the NMT inhibitor Compound 3 (a nonparametric Mann Whitney test was used).

Figure 23 shows: a.) top: global comparison of the correlation between sensitivity to the NMT inhibitor Compound 4, measured as EC50, and normalised *NMT1* expression; bottom: global comparison of the correlation between sensitivity to the NMT inhibitor Compound 4, measured as EC50, and normalised *NMT2* expression (the expression data was obtained from Lorio et. al., Cell, 2016, 166(3), 740-754 and a nonparametric Spearman correlation coefficient was used); b.) top: global comparison of the correlation between sensitivity to the NMT inhibitor Compound 3, measured as EC50, and normalised *NMT1* expression; bottom: global comparison of the correlation between sensitivity to the NMT inhibitor Compound 3, measured as EC50, and normalised *NMT2* expression (the expression data was obtained from Lorio et. al., Cell, 2016, 166(3), 740-754 and a nonparametric Spearman correlation coefficient was used); and c.) top: global comparison of the correlation between sensitivity to the NMT inhibitor Compound 6, measured as EC50, and normalised *NMT1* expression; bottom: global comparison of the correlation between sensitivity to the NMT inhibitor Compound 6, measured as EC50, and normalised *NMT2* expression (the expression data was obtained from Lorio et. al., Cell, 2016, 166(3), 740-754 and a nonparametric Spearman correlation coefficient was used).

### mRNAseq data

Looking at the mRNAseq data sets for BL41 and HeLa (Figures 24 and 25), in particular the case of BL41, it is observed that the *c-MYC* gene sets collapse to a much greater extent than in HeLa. This potentially indicates that the *c-MYC* driven program is particularly disturbed in BL41, a Burkitt's cell line with *c-MYC* translocations. Thus, the mRNAseq in BL41 indicates an increased breakdown of the *MYC* transcriptional program upon administration of a NMT inhibitor compound in a *c-MYC* addicted cancer.

Figure 24 shows the effect of 100 nM Compound 2 for 24 hours on different *MYC* gene sets (obtained from the Broad Institute: http://software.broadinstitute.org/gsea/index.isp), as measured via mRNAseq in BL41, a Burkitt's lymphoma cell line with *c-Myc* chromosomal translocation, with a.) the Hallmark_Myc_Targets_V1, b.) Schuhmacher_Myc_targets_up, and c) Dang_Myc Targets_Up.

Figure 25 shows the effect of 100 nM Compound 2 for 24 hours on different *MYC* gene sets (obtained from the Broad Institute: http://software.broadinstitute.org/asea/index.isp), as measured via mRNAseq in HeLa, a cervical cancer cell line without *c-Myc* chromosomal translocation, with a.) the Hallmark_Myc_Targets_V1, b.) Schuhmacher_Myc_targets_up, and c) Dang_Myc Targets_Up.

### Structural alterations of the MYC/MYCN/MYCL loci

As can be seen in Figure 26, structural alteration of *MYC*/*MYCN*/*MYCL* loci render cells more sensitive to the NMT inhibitor compound. As can be seen in Figure 27, those structural alterations correlate with increased activation of different *MYC* gene sets.

Across the three tested cancer cell line screens, it was observed that cell lines that have structural alterations (that is: copy number gains and/or chromosomal rearrangements and/or mutations in *c-MYC*/*MYCN*/*MYCL)* are more sensitive to an NMT inhibitor than cell lines without such structural alterations. In two of these screens the correlation is statistically significant and in the other screen a strong trend is observed.

Figure 26 shows: a.) the effect of the presence of structural alterations (that is: copy number gains and/or chromosomal rearrangements and/or mutations in *c-MYC*/*MYCN*/*MYCL)* on the sensitivity to the NMT inhibitor Compound 4 (a nonparametric Mann Whitney test was used); b.) the effect of the presence of structural alterations (that is: copy number gains and/or chromosomal rearrangements and/or mutations in *c-MYC*/*MYCN*/*MYCL)* on the sensitivity to the NMT inhibitor Compound 3 (a nonparametric Mann Whitney test was used); and c.) the effect of the presence of structural alterations (that is: copy number gains and/or chromosomal rearrangements and/or mutations in c*-MYC*/*MYCN*/*MYCL*) on the sensitivity to the NMT inhibitor Compound 6 (a nonparametric Mann Whitney test was used).

Figure 27 shows the effect of the presence of structural alterations (that is: copy number gains and/or chromosomal rearrangements and/or mutations in c*-MYC*/*MYCN*/*MYCL*) on different *MYC* gene sets (the gene sets obtained from the Broad Institute: http://software.broadinstitute.org/gsea/index.jsp; the expression data was obtained from Iorio et. al., Cell, 2016), with a.) the Hallmark_Myc_Targets_V1, b.) Dang_Myc Targets_Up, and c) Schuhmacher_Myc_targets_up.

### 'Sensitive to NMTi' gene set and its appliance to MYC driven cancers

As can be seen in Figure 28, the sensitivity to the NMT inhibitor Compound 4 can be pinpointed to certain key biological functions via GSEA: translation, transcription, protein degradation and nuclear trafficking, which are nuclear pore genes.

As can be seen in Figure 29, a leading edge analysis of the top gene sets being enriched in the sensitive cancer cell lines to the NMT inhibitor Compound 4 yields in a *"Sensitive to NMTi'* gene set, that contains 137 genes. This gene set is strongly enriched in the sensitive cancer cell lines to the NMT inhibitors Compound 3, Compound 4 and Compound 6, showing its predictive value. Additionally, as can be seen in Figure 30, the '*Sensitive to NMTi'* gene set is also enriched in cancer cell lines that are more dependent on NMT1 in a gene knock out essentiality screen. Importantly, the gene set *'Sensitive to NMTi'* is also enriched in cells with high *c-Myc* expression and cancer cell lines with structural alterations in the *MYC* loci.

Figure 28 shows: a.) the workflow to identify biological functions that correlate with resistance or sensitivity to the NMT inhibitor Compound 4. I. The cancer cell line screen was divided in the quartiles of the most resistant and the most responsive cell lines; II. The cell lines were matched to the expression data, obtained from Lorio et. al., Cell, 2016, 166(3), 740-754; III. GSEA was performed with the curated gene sets (C2; obtainted from the Broad Institute: http://software.broadinstitute.org/gsea/index.jsp); and b.) the Top 5 gene sets by normalised enrichment score (NES), correlating with sensitivity to the NMT inhibitor Compound 4, were identified as being enriched with genes that are involved in translation, viral infection gene sets (containing genes involved in translation, transcription and protein degradation) and mRNA processing.

Figure 29 shows: a.) the workflow to define a new gene set *'Sensitive to NMTi',* based on the data with the NMT inhibitor Compound 4. I. The top 10 gene sets, correlating with sensitivity to the NMT inhibitor Compound 4 were used for a leading edge analysis; II. The leading edge analysis allows for the identification of driver genes in each gene set (that is: genes that cause strong enrichment of the respective gene set); III. The leading edge analysis yielded in 137 genes that were used to define a new gene set *'Sensitive to NMTi';* IV. The newly defined gene set was applied to the screens with the NMT inhibitors Compound 1 and Compound 3 to validate the gene set; b.) The gene set *'Sensitive to NMTi'* was applied to the cancer cell screen with the NMT inhibitor Compound 4 and shows as expected strong enrichment in the sensitive cell lines; and c.) The gene set *'Sensitive to NMTi'* was applied to the cancer cell line screens with the NMT inhibitors Compound 3 (left) and Compound 6 (right), and shows for both NMT inhibitors strong enrichment in the sensitive cell lines respectively.

Figure 30 shows: a.) the application of the '*Sensitive to NMTi'* gene set to the CRISPR essentiality screening, conducted by the Broad Institute. I. The DepMap CRISPR essentiality scores were obtained from the Broad Institute (Robin, Nat Genetics, 2017, 49(12), 1779-1784; the Q4 2018 data sets are available here: https://depmap.org/portal/download/), and the cancer cell lines were divided into cell lines with high essentiality of NMT1 and low essentiality of NMT1, according to their CERES scores; II. The cell lines were matched to the RNAseq data from the Broad Institute (CCLE and GDSC Consortium, Nature, 2015, 528, 84-87; the Q4 2018 data sets are available here: https://depmap.org/portal/download/ ); III. The gene set '*Sensitive to NMTi'* was applied to the data, showing enrichment in the cells with high essentiality of NMT1; b.) the '*Sensitive to NMTi'* gene set was applied to compare cancer cell lines with high and low expression of *c-MYC* showing strong enrichment in the cells with high *c-MYC* expression; and c.) the *'Sensitive to NMTi'* gene set was applied to compare cancer cell lines with and without structural alterations in the *MYC* loci, showing strong enrichment in the cancer cell lines with structural alterations in the *MYC* loci.

While specific embodiments of the invention have been described herein for the purpose of reference and illustration, various modifications will be apparent to a person skilled in the art without departing from the scope of the invention as defined by the appended claims.

Throughout the specification and the claims which follow, unless the context requires otherwise, the word `comprise', and variations such as `comprises' and 'comprising', will be understood to imply the inclusion of a stated integer, step, group of integers or group of steps but not to the exclusion of any other integer, step, group of integers or group of steps.

## Claims

1. A NMT inhibitor for use in the treatment of diffuse large B-cell lymphoma in a subject who has been identified as benefiting from being administered a NMT inhibitor by a method which comprises administering a therapeutically effective amount of a NMT inhibitor, or a pharmaceutically acceptable salt, solvate or hydrate thereof to the subject, wherein said subject has been identified as benefiting from being administered a NMT inhibitor as determined by a method comprising the steps of:
i) taking a sample of diffuse large B-cell lymphoma cells taken from said subject;
ii) analysing the cells of step i) to check for the presence of one or more structural alterations in the *MYC* locus (e.g. chromosomal rearrangements, copy number gains and/or mutations of the *MYC* oncogene);
iii) determining whether one or more structural alterations (e.g. chromosomal rearrangements, copy number gains and/or mutations) are present in the *MYC* locus of the sample of diffuse large B-cell lymphoma cells when compared to a control; and
iv) determining whether the subject will benefit from being administered a NMT inhibitor in order to treat said diffuse large B-cell lymphoma, wherein if the sample of diffuse large B-cell lymphoma cells contain one or more structural alterations (e.g. chromosomal rearrangements, copy number gains and/or mutations) in the *MYC* locus, then the subject will benefit from being administered a NMT inhibitor, and if the sample of diffuse large B-cell lymphoma cells do not contain one or more structural alterations (e.g. chromosomal rearrangements, copy number gains and/or mutations) in the *MYC* locus, then the subject will not benefit from being administered a NMT inhibitor.

2. A NMT inhibitor for use according to claim 1, wherein the one or more structural alterations are selected from mutations, copy-number gains and/or chromosomal rearrangements.

3. A NMT inhibitor for use according to claim 1 or 2, wherein the one or more structural alterations are mutations.

4. A NMT inhibitor for use in the treatment of diffuse large B-cell lymphoma in a subject who has been identified as benefiting from being administered a NMT inhibitor by a method which comprises administering a therapeutically effective amount of a NMT inhibitor, or a pharmaceutically acceptable salt, solvate or hydrate thereof to the subject, wherein said subject has been identified as benefiting from being administered a NMT inhibitor as determined by a method comprising the steps of:
i) measuring the level of *MYC* expression in a sample of diffuse large B-cell lymphoma cells taken from said subject;
ii) comparing the level of *MYC* expression from step i) with a control;
iii) determining whether the *MYC* expression in the sample of diffuse large B-cell lymphoma cells is increased compared to the control; and
iv) determining whether the subject will benefit from being administered a NMT inhibitor in order to treat said diffuse large B-cell lymphoma, wherein if the *MYC* expression in the sample of diffuse large B-cell lymphoma cells is higher than in the control, then the subject will benefit from being administered a NMT inhibitor, and if the *MYC* expression in the sample of diffuse large B-cell lymphoma cells is not higher than in the control, then the subject will not benefit from being administered a NMT inhibitor.

5. A NMT inhibitor for use according to any one of claims 1 to 4, wherein the NMT inhibitor is a compound of Formula I shown below, or a pharmaceutically acceptable salt, hydrate or solvate thereof: 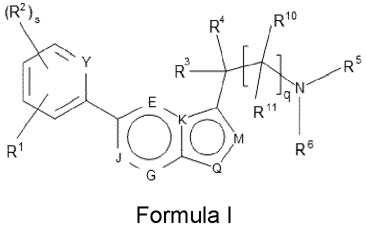 wherein:
Y is selected from the group consisting of -CH-, -C(R²)- and -N-;
R¹ is a group of formula -X-L-A;
wherein:
X is selected from the group consisting of -O-, -N(H)- and -S-, or is absent;
L is selected from the group consisting of -(CHR¹²)ₘ- and -(CHR¹²)ₘO-, or
is absent;
m is 1, 2 or 3; and
A is a 6-10-membered aromatic carbocycle or a 5-10-membered aromatic heterocycle, said aromatic carbocycle or heterocycle being optionally substituted with 1, 2, or 3 substituents each independently selected from the group consisting of -F, -Cl, -Br, -OCH₃, -OCF₃, -CN, -C₁₋₆alkyl optionally substituted by up to 3 halogen, hydroxyl, or -OC₁₋₄alkyl groups, -S(O)₁₋₄alkyl, -S(O)₂C₁₋₄alkyl, -C(O)N(R⁹)₂, -C(O)N(R¹³)C₁₋₄alkylOC₁₋₄alkyl ,-C(O)N(C₁₋₄alkylOC₁₋₄alkyl)₂, -CH₂C(O)N(R⁹)₂, -CH₂C(O)N(R¹³)C₁₋₄alkylOC₁₋₄alkyl , -CH₂C(O)N(C₁₋₄alkylOC₁₋₄alkyl)₂, -S(O)₂NHC₁₋₄alkyl,-S(O)₂N(C₁₋₄alkyl)₂, -NHC₁₋₄alkyl, -N(C₁₋₄alkyl)₂, -NHC(O)C₁₋₄alkyl,-NHC(O)CF₃,-NHS(O)₂C₁₋₄alkyl, CH₂N(R¹³)₂, CH₂N(R¹³)C(O)C₁₋₄alkyl, CH₂N(R¹³)S(O)₂C₁₋₄alkyl, -CH₂S(O)₂C₁₋₄alkyl, and CO₂H;
s is 0, 1, 2, or 3;
each R² is independently selected from the group consisting of -F, -Cl, -Br, -OCH₃, -OCF₃, -CN, -C₁₋₄alkyl optionally substituted by up to 3 halogen or hydroxyl groups, -S(O)C₁₋₄alkyl, -S(O)₂C₁₋₄alkyl, -S(O)₂NHC₁₋₄alkyl, -S(O)₂N(C₁₋₄alkyl)₂, -NHC₁₋₄alkyl, -N(C₁₋₄alkyl)₂, -NHC(O)C₁₋₄alkyl, -NHC(O)CF₃, and -NHS(O)₂C₁₋₄alkyl;
E, J and G are each independently nitrogen or C(R⁷);
K is carbon or nitrogen;
and wherein:
i) when K is carbon, either Q is N(R⁸) and M is nitrogen or C(R⁷), or Q is nitrogen and M is N(R⁸); or
ii) when K is nitrogen, Q is nitrogen or C(R⁷) and M is nitrogen or C(R⁷);
and further wherein at least 2 of E, J, G, K, Q and M are selected from the group consisting of carbon and C(R⁷);
q is 0 or 1;
R³ is hydrogen or methyl; R⁴ is hydrogen or methyl;
R⁵ is hydrogen or C₁₋₆alkyl optionally substituted by up to 3 -F, -Cl, -Br, -OH, - OCH₃, -OCF₃ or -CN groups;
R⁶ is hydrogen or C₁₋₆alkyl optionally substituted by up to 3 -F, -Cl, -Br, -OH, - OCH₃, -OCF₃ or -CN groups;
or the R⁵ and R⁶ groups and the N they are bonded to form a 4 to 7 membered non-aromatic heterocycle, the heterocycle optionally comprising 1 or 2 further heteroatoms selected from N, O and S, optionally substituted by up to 3 -F, -Cl, - Br, -OH, -OCH₃, -OCF₃ or -CN groups;
when present R¹⁰ is hydrogen or methyl;
when present R¹¹ is hydrogen or methyl;
or the R³ group and the R⁵ group and the intervening atoms form a 3 to 7 membered non-aromatic heterocycle composed of the intervening atoms and
bond, or the intervening atoms and -(CHR^{a})ᵣ-;
or the R¹⁰ group and the R⁵ group and the intervening atoms form a 3 to 7 membered non-aromatic heterocycle composed of the intervening atoms and - (CHR^{a})ᵣ-;
r is 1, 2, 3, 4 or 5; R^{a} is hydrogen or methyl;
each R⁷ is independently selected from the group consisting of hydrogen, halogen, C₁₋₄alkoxy, and C₁₋₄alkyl optionally substituted with 1, 2 or 3 halogens; and
R⁸ is selected from the group selected from hydrogen and C₁₋₄alkyl;
each R⁹ is independently selected from the group consisting of hydrogen and C₁₋₄alkyl, or two R⁹ groups and the N they are bonded to form a 4 to 7 membered non-aromatic heterocycle, the heterocycle optionally comprising 1 or 2 further heteroatoms selected from N, O and S;
each R¹² is independently selected from the group consisting of hydrogen, C₁₋₆alkyl optionally substituted by up to 3 -F, -Cl, -Br, I, -OH, -OCH₃, -OCF₃ or -CN groups,
C₁₋₆alkenyl optionally substituted by up to 3 -F, -Cl, -Br, I, -OH, -OCH₃, -OCF₃ or - CN groups, and C₁₋₆alkynyl optionally substituted by up to 3 -F, -Cl, -Br, I, -OH, - OCH₃, -OCF₃ or -CN groups; and each R¹³ is independently selected from the group consisting of hydrogen and C₁₋₄alkyl.

6. A NMT inhibitor for use according to any one of claims 1 to 5, wherein the NMT inhibitor is a compound of Formula (IA^^) shown below, or a pharmaceutically acceptable salt, hydrate or solvate thereof: 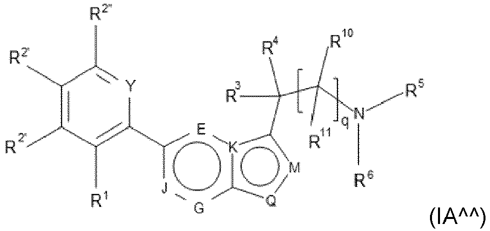 wherein:
R¹ is a group of formula -X-L-A;
A is 4-pyrazolyl, said pyrazolyl being optionally substituted with up to 3 methyl groups;
X is -O- or absent;
L is -(CH₂)ₘ- or -(CH₂)ₘ-O-;
m is 2;
R^{2'} is selected from the group consisting of fluorine, chlorine -CN and
methyl (preferably fluorine);
R^{2"} is selected from the group consisting of hydrogen, fluorine, chlorine, - CN and methyl;
q is 0 or 1;
R³ is hydrogen or methyl;
R⁴ is hydrogen or methyl;
R⁵ is hydrogen or methyl;
R⁶ is hydrogen or methyl; or
the R³ group and the R⁵ group and the intervening atoms form a 3 to 7 membered non-aromatic heterocycle composed of the intervening atoms and bonds, (more preferably R⁵ and R⁶ are both methyl);
R⁷ where present is hydrogen or methyl;
R⁸ where present is hydrogen or methyl;
R⁹ and R¹⁰ where present are hydrogen or methyl; and
E, J, G, K, Q and M are:
i) E, J and G are each C(R⁷), K is carbon, Q is N(R⁸), M is nitrogen; and R⁸ is hydrogen or methyl;
ii) E, J and G are each C(R⁷), and K, Q and M are each nitrogen;
iii) E and G are each C(R⁷), and J, K, Q and M are each nitrogen;
iv) J and G are each C(R⁷), and E, K, Q and M are each nitrogen; or
v) E, J, G and M are each C(R⁷), and K and Q are each nitrogen.

7. A NMT inhibitor for use according to any one of claims 1 to 6, wherein the NMT inhibitor is a compound of Formula (IA^^) shown below, or a pharmaceutically acceptable salt, hydrate or solvate thereof: 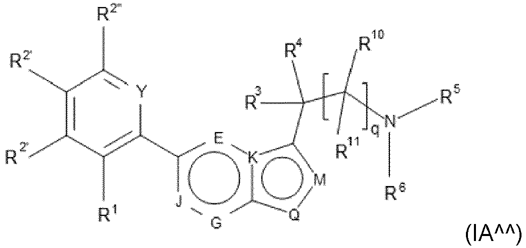 wherein:
R¹ is a group of formula -X-L-A;
A is 4-pyrazolyl, said pyrazolyl being optionally substituted with up to 3 substituent groups selected from methyl and -C(O)N(CH₃)₂;
X is -O- or absent;
L is -(CH₂)ₘ- or -(CH₂)ₘ-O-;
m is 2;
R^{2'} is selected from the group consisting of fluorine or chlorine (preferably fluorine);
R^{2"} is selected from the group consisting of hydrogen, fluorine or chlorine;
q is 0;
R³ is hydrogen or methyl;
R⁴ is hydrogen or methyl;
R⁵ is hydrogen or methyl;
R⁶ is hydrogen or methyl; or
the R³ group and the R⁵ group and the intervening atoms form a 3 to 7 membered non-aromatic heterocycle composed of the intervening atoms and bonds, (more preferably R⁵ and R⁶ are both methyl);
E, J, G, K, Q and M are:
i) E, J and G are each CH, K is carbon, Q is N(R⁸), M is nitrogen; and R⁸ is hydrogen or methyl; or
ii) E, J, G and M are each CH, and K and Q are each nitrogen;
with the proviso that A is substituted with no more than one -C(O)N(CH₃)₂ group.

8. A NMT inhibitor for use according to any one of claims 1 to 7, wherein the NMT inhibitor is a compound selected from: 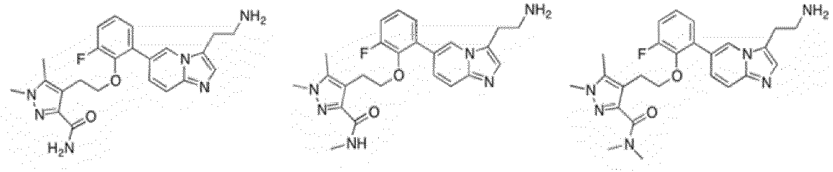 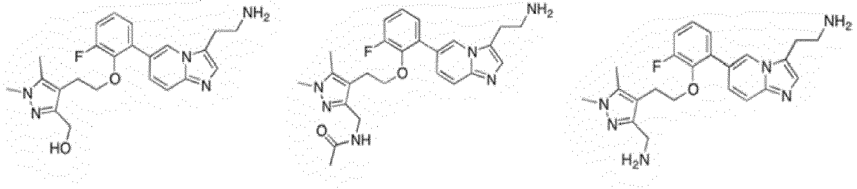 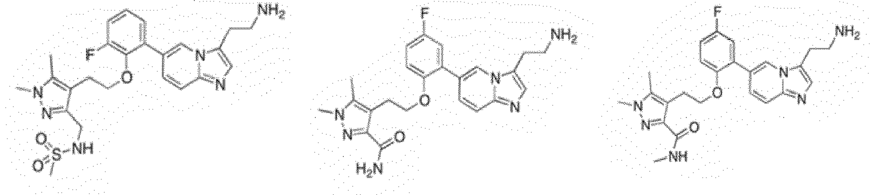 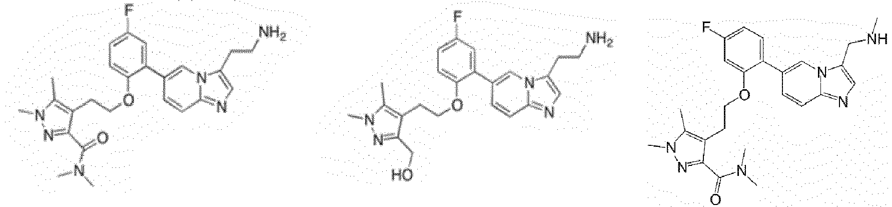 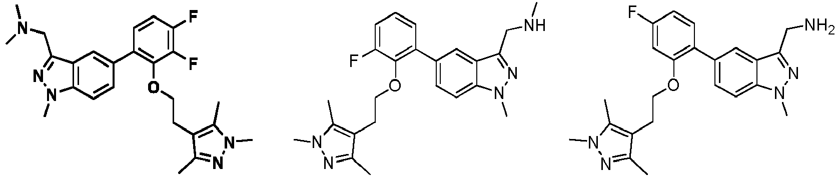 or a pharmaceutically acceptable salt, hydrate or solvate thereof.

9. A NMT inhibitor for use according to any one of claims 1 to 8, wherein the NMT inhibitor is the compound shown below, or a pharmaceutically acceptable salt, hydrate or solvate thereof: 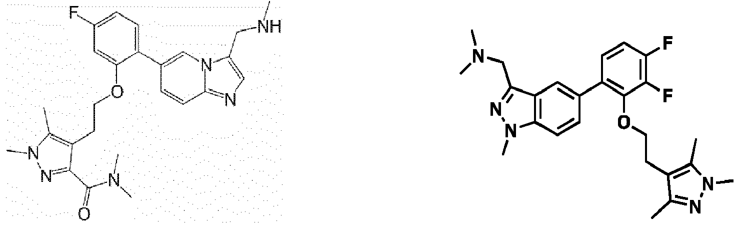 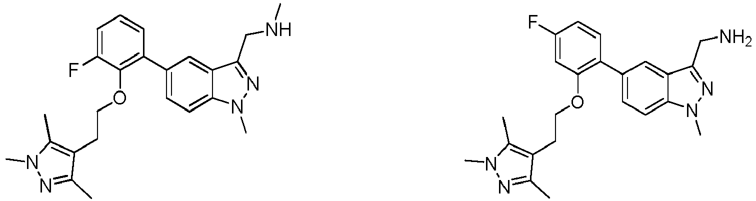 or a pharmaceutically acceptable salt, hydrate or solvate thereof.

10. A NMT inhibitor for use according to any one of claims 1 to 4, wherein the NMT inhibitor is a compound of Formula (Id) shown below, or a pharmaceutically acceptable salt, hydrate or solvate thereof: 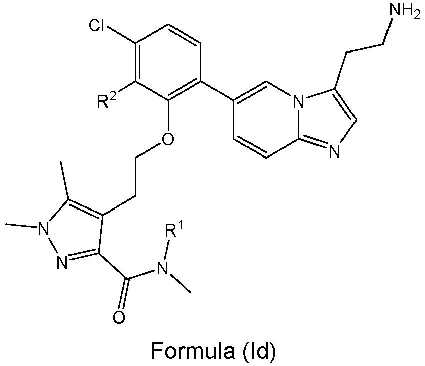 wherein:
R¹ is H or -CH₃; and
R² is H or F.

11. A NMT inhibitor for use according to claim 10, wherein the NMT inhibitor is the compound shown below, or a pharmaceutically acceptable salt, hydrate or solvate thereof:

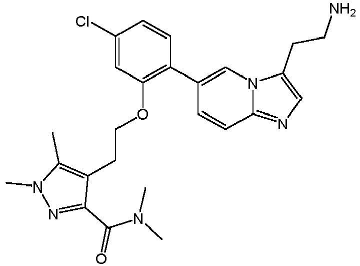

12. A NMT inhibitor for use according to any one of claims 1 to 4, wherein the NMT inhibitor is a compound of Formula (II) or Formula (III) shown below, or a pharmaceutically acceptable salt, hydrate or solvate thereof: 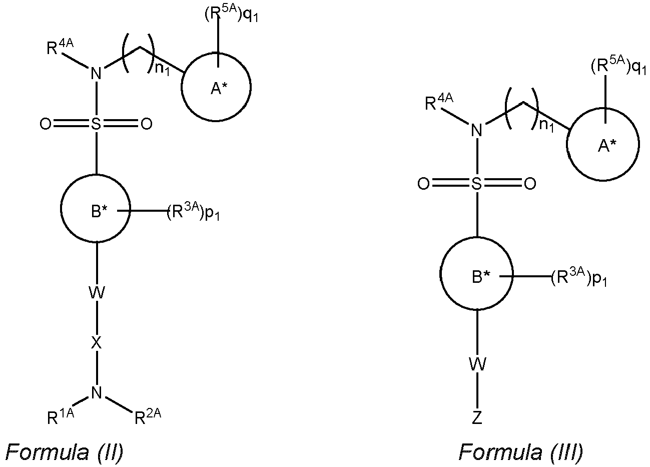 wherein:
n₁ is 0, 1, 2, 3, 4, 5 or 6;
Ring A*, is an optionally substituted nitrogen containing aryl group wherein each substitutable carbon or nitrogen in Ring A* is optionally and independently substituted by one or more R^{5A} and wherein if Ring A* contains an -NH- moiety that nitrogen may be optionally substituted by C₁₋₆alkyl (e.g. methyl); and wherein
R^{4A} and Ring A* together with the atoms to which they are attached may form a cyclic group,
Ring B* is an optionally substituted aryl or heteroaryl group wherein each substitutable carbon or heteroatom in Ring B* is optionally and independently substituted by one or more R^{3A};
W and X, one of which may be absent, are independently selected from R^{11A},
hydrocarbyl (e.g. C₁₋₈ alkyl, alkenyl, alkynyl, or haloalkyl) optionally substituted with R^{11A}, and -(CH₂)ₖ₁-heterocyclyl optionally substituted with R^{12A}; k₁ is 0, 1, 2, 3, 4, 5 or 6;
R^{1A}, R^{2A}, R^{3A}, R^{4A} and R^{5A} are independently selected from hydrogen, R^{12A},
hydrocarbyl (e.g. C₁₋₆alkyl, alkenyl, alkynyl, or haloalkyl) optionally substituted with R^{12A}, and a -(CH₂)_{L1}- heterocyclyl optionally substituted with one or more R^{12A};
wherein R^{1A} and R^{2A} taken together with the atoms to which they are attached may form a heterocycle, optionaly substituted with one or more R^{12A}, wherein R^{1A} and/or
R^{2A} taken together with W or X may form a heterocycle optionally substituted with one or more R^{12A}; and wherein one or more of R^{3A} and R^{5A} taken together with the atoms to which they are attached may form a carbocycle, for example heterocyclyl,
optionally substituted with R^{12A}; L₁ is 0,1, 2, 3, 4, 5 or 6;
wherein:
each R^{11A} and R^{12A} is independently selected from halogen, trifluoromethyl, cyano, thio, nitro, oxo, =NR^{13A}, -OR^{13A}, -SR^{13A}, -C(O)R^{13A}, -C(O)OR^{13A},-OC(O)R^{13A}, -NR^{13A}COR^{14A}, -NR^{13A}CON(R^{13A})₂, -NR^{13A}COR^{14A}, -NR^{13A}CO₂R^{14A}, - S(O)R^{13A}, -S(O)₂R^{13A}, -SON(R^{13A})₂, -NR^{13A}S(O)₂R^{14A}; -CSR^{13A},-N(R^{13A})R^{14A}, -C(O)N(R^{13A})R^{14A}, -SO₂N(R^{13A})R^{14A} and R^{15A};
R^{13A} and R^{14A} are each independently selected from hydrogen or R^{15A};
R^{15A} is selected from hydrocarbyl (e.g. C₁₋₆alkyl, alkenyl, alkynyl, or haloalkyl), carbocyclyl and -(CH₂)ₘ₁-heterocyclyl, and each R^{15A} is optionally and independently substituted with one or more of halogen, cyano, amino, hydroxy, C₁₋₆alkyl or cycloalkyl and C₁₋₆alkoxy;
m₁ is 0, 1, 2, 3, 4, 5 or 6;
p₁ is 0,1, 2, 3 or 4; the values of R^{4A} may be the same or different; and
q₁ is 0, 1, 2, 3 or 4; wherein the values of R^{5A} may be the same or different;
Y and Z, one or both of which may be absent, are independently selected from hydrogen, R^{16A}, hydrocarbyl (e.g.C₁₋₆alkyl, alkenyl, alkynyl.or haloalkyl) optionally substituted with R^{16A}, and -(CH₂)ᵣ₁-heterocyclyl optionally substituted with R^{16A},
wherein each R^{16A} is independently selected from halogen, trifluoromethyl, cyano, thio, nitro, oxo, =NR^{17A}, -OR^{17A}, -SR^{17A}, -C(O)R^{17A}, -C(O)OR^{17A}, -OC(O)R^{17A},-NR^{17A}COR^{18A}, -NR^{17A}CON(R^{18A})₂, -NR^{17A}COR^{18A}, -NR^{17A}CO₂R^{18A}, -S(O)R^{17A},-S(O)₂R^{17A}, - SON(R^{17A})₂, -NR^{17A}S(O)₂R^{18A}; -CSR^{17A}, -N(R^{17A})R^{18A},-C(O)N(R^{17A})R^{18A}, -SO₂N(R^{17A})R^{18A} and R^{19A}; r₁ is 0, 1, 2, 3, 4, 5 or 6;
wherein:
R^{17A} and R^{18A} are each independently selected from hydrogen or R^{19A};
R^{19A} is selected from hydrocarbyl (e.g.C1-6alkyl, alkenyl, alkynyl.or haloalkyl),
carbocyclyl and -(CH₂)ₛ₁-heterocyclyl, and each R^{19A} is optionally and
independently substituted with one or more of halogen, cyano, amino, hydroxy, C₁₋₆alkyl and C₁₋₆alkoxy; and
s₁ is 0, 1, 2, 3, 4, 5 or 6.

13. A NMT inhibitor for use according to claim 12, wherein the NMT inhibitor is a compound of Formula (Ila) shown below, or a pharmaceutically acceptable salt, solvate or hydrate thereof: 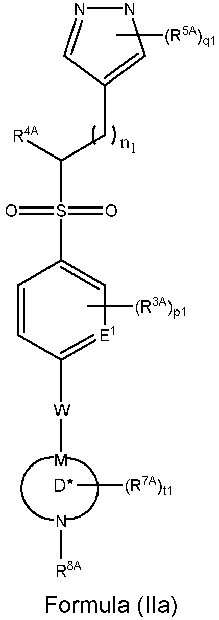 wherein:
n₁ is 0 or 1;
E¹ is C;
W is a (1-4C)hydrocarbyl, an aryl (e.g. phenyl) or heteroaryl group (e.g. pyridinyl);
M is selected from C and N;
R^{3A}, R^{4A} and R^{5A} are independently selected from hydrogen, R^{12A}, and (1-3C)hydrocarbyl optionally substituted with R^{12A};
R^{12A} is independently selected from halogen, trifluoromethyl, cyano, thio, nitro, oxo, -OR^{13A}, -SR^{13A}, -C(O)R^{13A}, -C(O)OR^{13A}, -OC(O)R^{13A},-NR^{13A}COR^{14A} and R^{15A};
R^{13A} and R^{14A} are each independently selected from hydrogen or a (1-4C)hydrocarbyl (e.g. methyl);
Ring D* is an optionally substituted nitrogen containing 6 or 7 membered heterocycle, wherein each substitutable carbon or nitrogen in Ring D* is optionally and independently substituted by one or more R^{7A};
R^{7A} is independently selected from hydrogen, (1-4C)hydrocarbyl, halogen, trifluoromethyl, cyano, thio, nitro or oxo;
R^{8A} is a hydrogen or a (1-4C)hydrocarbyl (e.g. methyl);
p₁ is 0, 1 or 2, wherein the values of R^{4A} may be the same or different;
q₁ is 3, wherein the values of R^{5A} may be the same or different; and
t₁ is 0, 1 or 2, wherein the values of R^{7A} may be the same or different.

14. A NMT inhibitor for use according to claim 12 or 13, wherein the NMT inhibitor is one of the compounds shown below, or a pharmaceutically acceptable salt, hydrate or solvate thereof:

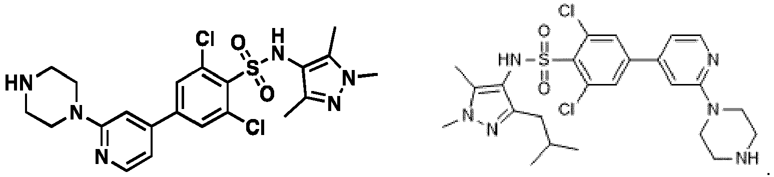

15. A NMT inhibitor for use according to any one of claims 1 to 14 in combination with one or more other therapeutic agents.

## Patentansprüche

1. NMT-Inhibitor zur Verwendung bei der Behandlung von diffusem großzelligem B-Zell-Lymphom bei einem Patienten, bei dem festgestellt wurde, dass er von der Verabreichung eines NMT-Inhibitors durch ein Verfahren profitiert, das das Verabreichen einer therapeutisch wirksamen Menge eines NMT-Inhibitors oder eines pharmazeutisch verträglichen Salzes, Solvats oder Hydrats davon an den Patienten umfasst, wobei festgestellt wurde, dass der Patient von der Verabreichung eines NMT-Inhibitors durch ein Verfahren profitiert, das die Schritte umfasst:
i) Entnehmen einer Probe von diffusen großzelligen B-Zell-Lymphomzellen, die dem Patienten entnommen wurden;
ii) Analysieren der Zellen aus Schritt i), um das Vorhandensein einer oder mehrerer struktureller Veränderungen im MYC-Locus (z. B. chromosomale Umlagerungen, Kopienzahlerhöhungen und/oder Mutationen des MYC-Onkogens) zu überprüfen;
iii) Bestimmen, ob im MYC-Locus der Probe diffuser großzelliger B-Zell-Lymphomzellen im Vergleich zu einer Kontrolle eine oder mehrere strukturelle Veränderungen (z. B. chromosomale Umlagerungen, Kopienzahlerhöhungen und/oder Mutationen) vorhanden sind; und
iv) Bestimmen, ob der Patient von der Verabreichung eines NMT-Inhibitors zur Behandlung des diffusen großzelligen B-Zell-Lymphoms profitieren wird, wobei dann, wenn die Probe der diffusen großzelligen B-Zell-Lymphomzellen eine oder mehrere strukturelle Veränderungen (z. B. chromosomale Umlagerungen, Kopienzahlerhöhungen und/oder Mutationen) im MYC-Locus enthält, der Patient von der Verabreichung eines NMT-Inhibitors profitieren wird, und wenn die Probe diffuser großer B-Zell-Lymphomzellen keine eine oder mehrere strukturelle Veränderungen (z. B. chromosomale Umlagerungen, Kopienzahlerhöhungen und/oder Mutationen) im MYC-Locus enthält, der Patient dann nicht von der Verabreichung eines NMT-Inhibitors profitieren wird.

2. NMT-Inhibitor zur Verwendung nach Anspruch 1, wobei die eine oder die mehreren strukturellen Veränderungen aus Mutationen, Kopienzahlerhöhungen und/oder chromosomalen Umlagerungen ausgewählt sind.

3. NMT-Inhibitor zur Verwendung nach Anspruch 1 oder 2, wobei die eine oder die mehreren strukturellen Veränderungen Mutationen sind.

4. NMT-Inhibitor zur Verwendung bei der Behandlung von diffusem großzelligem B-Zell-Lymphom bei einem Patienten, bei dem festgestellt wurde, dass er von der Verabreichung eines NMT-Inhibitors durch ein Verfahren profitiert, das das Verabreichen einer therapeutisch wirksamen Menge eines NMT-Inhibitors oder eines pharmazeutisch verträglichen Salzes, Solvats oder Hydrats davon an den Patienten umfasst, wobei festgestellt wurde, dass der Patient von der Verabreichung eines NMT-Inhibitors durch ein Verfahren profitiert, das die Schritte umfasst:
i) Messen des *MYC*-Expressionsniveaus in einer Probe diffuser großzelliger B-Zell-Lymphomzellen, die dem Patienten entnommen wurde;
ii) Vergleichen des *MYC-* Expressionsniveaus aus Schritt i) mit einer Kontrolle;
iii) Bestimmen, ob die *MYC-* Expression in der Probe diffuser großzelliger B-Zell-Lymphomzellen im Vergleich zur Kontrolle erhöht ist; und
iv) Bestimmen, ob der Patient von der Verabreichung eines NMT-Inhibitors profitieren wird, um das diffuse großzellige B-Zell-Lymphom zu behandeln, wobei dann, wenn die *MYC-* Expression in der Probe der diffusen großzelligen B-Zell-Lymphomzellen höher ist als in der Kontrolle, der Patient von der Verabreichung eines NMT-Inhibitors profitieren wird, und wenn die *MYC-*Expression in der Probe diffuser großzelliger B-Zell-Lymphomzellen nicht höher ist als in der Kontrolle, der Patient dann nicht von der Verabreichung eines NMT-Inhibitors profitieren wird.

5. NMT-Inhibitor zur Verwendung nach einem der Ansprüche 1 bis 4, wobei der NMT-Inhibitor eine Verbindung der unten gezeigten Formel I oder ein pharmazeutisch verträgliches Salz, Hydrat oder Solvat davon ist: 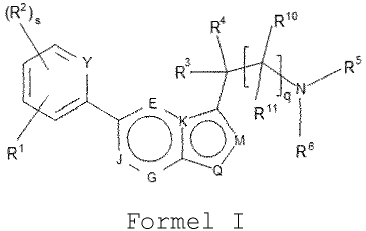 wobei:
Y aus der Gruppe bestehend aus -CH-, -C(R²)- und -N- ausgewählt ist;
R¹ eine Gruppe der Formel -X-L-A ist;
wobei:
X aus der Gruppe bestehend aus -O-, -N(H)- und -S- ausgewählt ist oder fehlt;
L aus der Gruppe bestehend aus - (CHR¹²)ₘ- und - (CHR¹²)ₘO-ausgewählt ist oder fehlt;
m 1, 2 oder 3 ist; und
A ein 6-10-gliedriger aromatischer Carbocyclus oder ein 5-10-gliedriger aromatischer Heterocyclus ist, wobei der aromatische Carbocyclus oder Heterocyclus optional mit 1, 2 oder 3 Substituenten substituiert ist, die jeweils unabhängig voneinander ausgewählt sind aus der Gruppe bestehend aus -F, - Cl, -Br, -OCH₃, -OCF₃, -CN, -C₁₋₆-Alkyl, optional substituiert mit 3 Halogen, Hydroxyl oder -OC₁₋₄-Alkylgruppen, -S(O)C₁₋₄-Alkyl, -S(O)₂C₁₋₄-Alkyl, -C(O)N(R⁹)₂, -C (O)N(R¹³)C₁₋₄-AlkylOC₁₋₄-Alkyl, - C(O)N(C₁₋₄-AlkylOC₁₋₄-Alkyl)₂, -CH₂C(O)N(R⁹)₂, -CH₂C(O)N(R¹³)C₁₋₄-AlkylOC₁₋₄Alkyl , -CH₂C(O)N(C₁₋₄-AlkylOC₁₋₄-Alkyl)₂, -S(O)₂NHC₁₋₄-Alkyl, -S(O)₂N(C₁₋₄-Alkyl)₂, -NHC₁₋₄-Alkyl, -N(C₁₋₄-Alkyl)₂, - NHC(O)C₁₋₄-alkyl, -NHC(O)CF₃, -NHS(O)₂C₁₋₄-Alkyl, CH₂N(R¹³)₂, CH₂N(R¹³)C (O)C₁₋₄-Alkyl, CH₂N(R¹³)S(O)₂C₁₋₄-Alkyl, -CH₂S(O)₂C₁₋₄-Alkyl und CO₂H;
s 0, 1, 2 oder 3 ist;
jedes R² unabhängig voneinander ausgewählt ist aus der Gruppe bestehend aus -F, -Cl, -Br, -OCH₃, -OCF₃, -CN, -C₁₋₄-Alkyl, optional substituiert mit bis zu 3 Halogen- oder Hydroxylgruppen, -S(O)C₁₋₄-Alkyl, -S(O)₂C₁₋₄-Alkyl, -S(O)₂NHC₁₋₄-Alkyl, -S(O)₂N(C₁₋₄-Alkyl)₂, -NHC₁₋₄-Alkyl, -N(C₁₋₄-Alkyl)₂, - NHC(O)C₁₋₄-Alkyl, -NHC(O)CF₃ und -NHS(O)₂C₁₋₄-Alkyl;
E, J und G jeweils unabhängig voneinander Stickstoff oder C(R⁷) sind;
K Kohlenstoff oder Stickstoff ist;
und wobei:
i) wenn K Kohlenstoff ist, entweder Q N(R⁸) ist und M Stickstoff oder C(R⁷) ist, oder Q Stickstoff ist und M N(R⁸) ist; oder
ii) wenn K Stickstoff ist, Q Stickstoff oder C(R⁷) ist und M Stickstoff oder C(R⁷) ist;
und wobei ferner mindestens 2 von E, J, G, K, Q und M aus der Gruppe bestehend aus Kohlenstoff und C(R⁷) ausgewählt sind;
q 0 oder 1 ist;
R³ Wasserstoff oder Methyl ist; R⁴ Wasserstoff oder Methyl ist;
R⁵ Wasserstoff oder C₁₋₆-Alkyl ist, optional substituiert mit bis zu 3 -F-, -Cl-, -Br-, -OH-, -OCH₃, -OCF₃ oder -CN-Gruppen;
R⁶ Wasserstoff oder C₁₋₆-Alkyl ist, optional substituiert mit bis zu 3 -F-, -Cl-, -Br-, -OH-, -OCH₃, -OCF₃ oder -CN-Gruppen;
oder die Gruppen R⁵ und R⁶ und das N, an das sie gebunden sind, einen 4- bis 7-gliedrigen nichtaromatischen Heterocyclus zu bilden, wobei der Heterocyclus optional 1 oder 2 weitere Heteroatome umfasst, die aus N, O und S, optional substituiert mit bis zu 3 - F-, -Cl-, -Br-, -OH-, -OCH₃, -OCF₃ oder -CN-Gruppen, ausgewählt sind;
R¹⁰, wenn vorhanden, Wasserstoff oder Methyl ist;
R¹¹, wenn vorhanden, Wasserstoff oder Methyl ist;
oder die R³-Gruppe und die R⁵-Gruppe und die dazwischen liegenden Atome einen 3- bis 7-gliedrigen nichtaromatischen Heterocyclus bilden, der aus den dazwischen liegenden Atomen und der Bindung oder den dazwischen liegenden Atomen und -(CHR^{a})ᵣ- besteht; oder die R¹⁰-Gruppe und die R⁵-Gruppe und die dazwischen liegenden Atome einen 3- bis 7-gliedrigen nichtaromatischen Heterocyclus bilden, der aus den dazwischen liegenden Atomen und - (CHR^{a})ᵣ- besteht;
r 1, 2, 3, 4 oder 5 ist; R^{a} Wasserstoff oder Methyl ist;
jedes R⁷ unabhängig voneinander aus der Gruppe bestehend aus Wasserstoff, Halogen, C₁₋₄-Alkoxy und C₁₋₄-Alkyl, optional substituiert mit 1, 2 oder 3 Halogenen, ausgewählt ist; und
R⁸ aus der Gruppe ausgewählt aus Wasserstoff und C₁₋₄-Alkyl ausgewählt ist;
jedes R⁹ unabhängig aus der Gruppe ausgewählt ist, die aus Wasserstoff und C₁₋₄-Alkyl besteht, oder zwei R⁹ -Gruppen und das N, an das sie gebunden sind, um einen 4- bis 7-gliedrigen nichtaromatischen Heterocyclus zu bilden, wobei der Heterocyclus optional 1 oder 2 weitere Heteroatome umfasst, die aus N, O und S ausgewählt sind;
jedes R¹² unabhängig voneinander ausgewählt ist aus der Gruppe bestehend aus Wasserstoff, C₁₋₆-Alkyl, optional substituiert mit bis zu 3 -F-, -Cl-, -Br-, I-, -OH-, -OCH₃, -OCF₃ oder -CN-Gruppen, C₁₋₆-Alkenyl, optional substituiert mit bis zu 3 -F, -Cl, -Br, I, -OH, -OCH₃, -OCF₃ oder -CN-Gruppen, und C₁₋₆-Alkinyl, optional substituiert mit bis zu 3 - F-, -Cl-, -Br-, I-, -OH-, -OCH₃, - OCF₃ oder -CN-Gruppen; und jedes R¹³ unabhängig voneinander aus der Gruppe bestehend aus Wasserstoff und C₁₋₄-Alkyl ausgewählt ist.

6. NMT-Inhibitor zur Verwendung nach einem der Ansprüche 1 bis 5, wobei der NMT-Inhibitor eine Verbindung der unten gezeigten Formel (IA^^) oder ein pharmazeutisch verträgliches Salz, Hydrat oder Solvat davon ist: 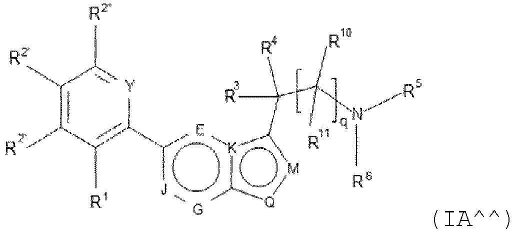 wobei:
R¹ eine Gruppe der Formel -X-L-A ist;
A 4-Pyrazolyl ist, wobei das Pyrazolyl optional mit bis zu 3 Methylgruppen substituiert ist;
X -O- ist oder fehlt;
L-(CH₂)ₘ- oder -(CH₂)ₘ-O- ist;
m 2 ist;
R^{2'} aus der Gruppe bestehend aus Fluor, Chlor-CN und Methyl (vorzugsweise Fluor) ausgewählt ist;
R^{2"} aus der Gruppe bestehend aus Wasserstoff, Fluor, Chlor, -CN und Methyl ausgewählt ist;
q 0 oder 1 ist;
R³ Wasserstoff oder Methyl ist;
R⁴ Wasserstoff oder Methyl ist;
R⁵ Wasserstoff oder Methyl ist;
R⁶ Wasserstoff oder Methyl ist; oder
die R³-Gruppe und die R⁵-Gruppe und die dazwischen liegenden Atome einen 3- bis 7-gliedrigen nichtaromatischen Heterocyclus bilden, der aus den dazwischen liegenden Atomen und Bindungen besteht (bevorzugter sind R⁵ und R⁶ beide Methyl);
R⁷, wenn vorhanden, Wasserstoff oder Methyl ist;
R⁸, wenn vorhanden, Wasserstoff oder Methyl ist;
R⁹ und R¹⁰, wenn vorhanden, Wasserstoff oder Methyl sind; und
E, J, G, K, Q und M sind:
i) E, J und G sind jeweils C(R⁷), K ist Kohlenstoff, Q ist N(R⁸), M ist Stickstoff; und R⁸ ist Wasserstoff oder Methyl;
ii) E, J und G sind jeweils C(R⁷) und K, Q und M sind jeweils Stickstoff;
iii) E und G sind jeweils C(R⁷) und J, K, Q und M sind jeweils Stickstoff;
iv) J und G sind jeweils C(R⁷) und E, K, Q und M sind jeweils Stickstoff; oder
v) E, J, G und M sind jeweils C(R⁷) und K und Q sind jeweils Stickstoff.

7. NMT-Inhibitor zur Verwendung nach einem der Ansprüche 1 bis 6, wobei der NMT-Inhibitor eine Verbindung der unten gezeigten Formel (IA^^) oder ein pharmazeutisch verträgliches Salz, Hydrat oder Solvat davon ist: 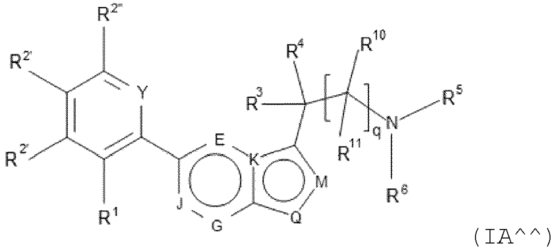 wobei:
R¹ eine Gruppe der Formel -X-L-A ist;
A 4-Pyrazolyl ist, wobei das Pyrazolyl optional mit bis zu 3 Substituentengruppen, ausgewählt aus Methyl und -C(O)N(CH₃)₂, substituiert ist;
X -O- ist oder fehlt;
L-(CH₂)ₘ- oder -(CH₂)ₘ-O- ist;
m 2 ist;
R^{2'} aus der Gruppe bestehend aus Fluor oder Chlor (vorzugsweise Fluor) ausgewählt ist;
R^{2"} aus der Gruppe bestehend aus Wasserstoff, Fluor oder Chlor ausgewählt ist;
q 0 ist;
R³ Wasserstoff oder Methyl ist;
R⁴ Wasserstoff oder Methyl ist;
R⁵ Wasserstoff oder Methyl ist;
R⁶ Wasserstoff oder Methyl ist; oder
die R³-Gruppe und die R⁵-Gruppe und die dazwischen liegenden Atome einen 3- bis 7-gliedrigen nichtaromatischen Heterocyclus bilden, der aus den dazwischen liegenden Atomen und Bindungen besteht (bevorzugter sind R⁵ und R⁶ beide Methyl);
E, J, G, K, Q und M sind:
i) E, J und G sind jeweils CH, K ist Kohlenstoff, Q ist N(R⁸), M ist Stickstoff; und R⁸ ist Wasserstoff oder Methyl; oder
ii) E, J, G und M sind jeweils CH und K und Q sind jeweils Stickstoff;
mit der Maßgabe, dass A mit nicht mehr als einer -C(O)N(CH₃)₂-Gruppe substituiert ist.

8. NMT-Inhibitor zur Verwendung nach einem der Ansprüche 1 bis 7, wobei der NMT-Inhibitor eine Verbindung ist, die ausgewählt ist aus: 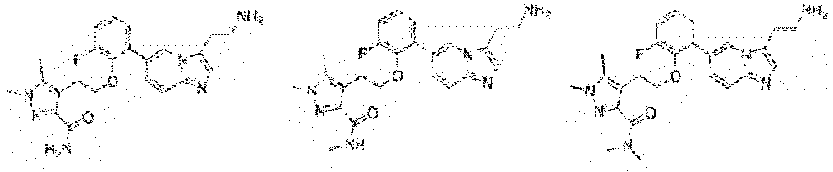 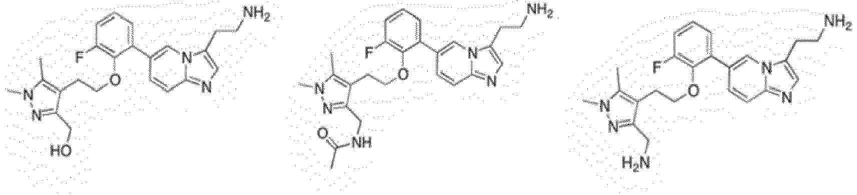 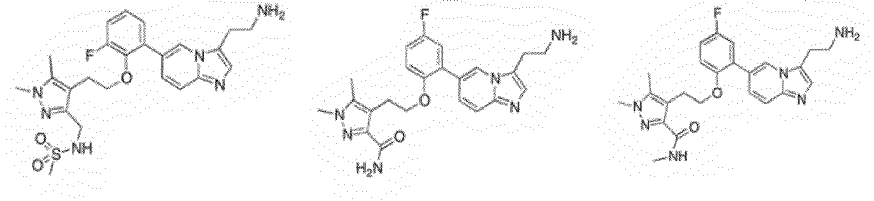 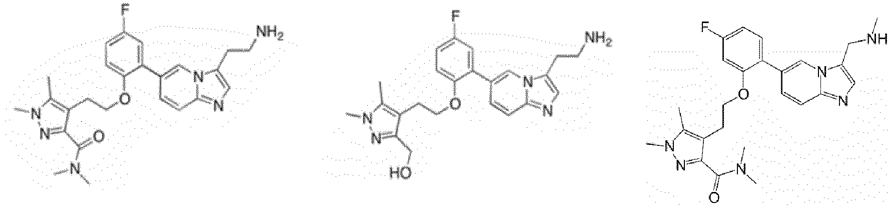 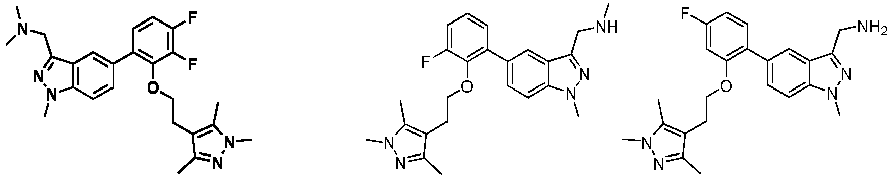 oder ein pharmazeutisch verträgliches Salz, Hydrat oder Solvat davon.

9. NMT-Inhibitor zur Verwendung nach einem der Ansprüche 1 bis 8, wobei der NMT-Inhibitor die unten gezeigte Verbindung oder ein pharmazeutisch verträgliches Salz, Hydrat oder Solvat davon ist: 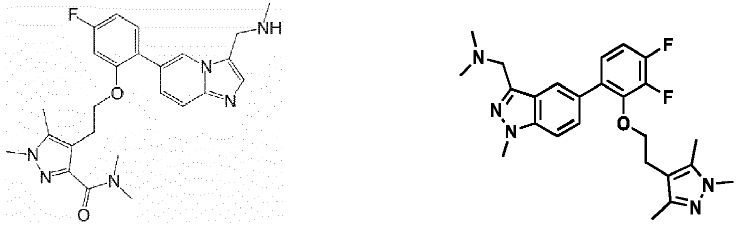 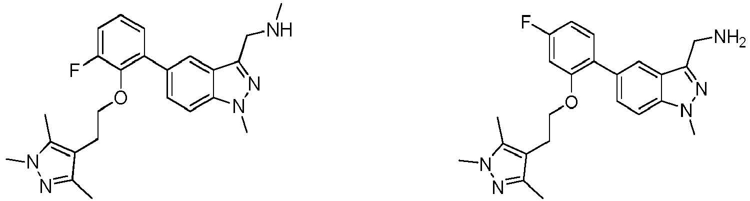 oder ein pharmazeutisch verträgliches Salz, Hydrat oder Solvat davon.

10. NMT-Inhibitor zur Verwendung nach einem der Ansprüche 1 bis 4, wobei der NMT-Inhibitor eine Verbindung der unten gezeigten Formel (Id) oder ein pharmazeutisch verträgliches Salz, Hydrat oder Solvat davon ist: 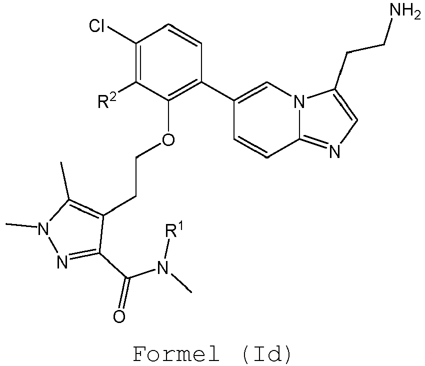 wobei:
R₁ H oder -CH₃ ist; und
R₂ H oder F ist.

11. NMT-Inhibitor zur Verwendung nach Anspruch 10, wobei der NMT-Inhibitor die unten gezeigte Verbindung oder ein pharmazeutisch verträgliches Salz, Hydrat oder Solvat davon ist:

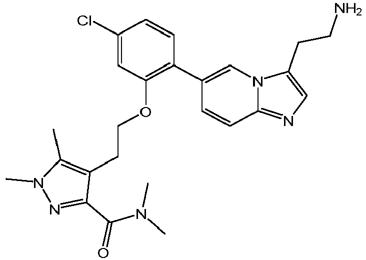

12. NMT-Inhibitor zur Verwendung nach einem der Ansprüche 1 bis 4, wobei der NMT-Inhibitor eine Verbindung der unten gezeigten Formel (II) oder (III) oder ein pharmazeutisch verträgliches Salz, Hydrat oder Solvat davon ist: 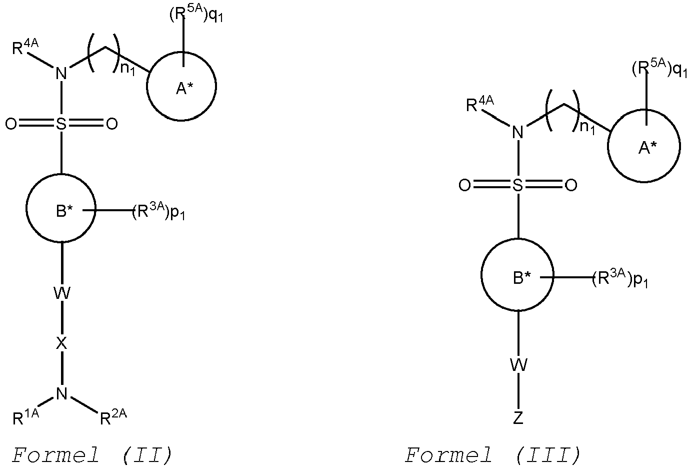 wobei:
n₁ 0, 1, 2, 3, 4, 5 oder 6 ist;
Ring A* eine optional substituierte stickstoffhaltige Arylgruppe ist, wobei jeder substituierbare Kohlenstoff oder Stickstoff in Ring A* optional und unabhängig durch ein oder mehrere R^{5A} substituiert ist und wobei dann, wenn Ring A* eine -NH--Einheit enthält, dieser Stickstoff optional durch C₁₋₆-Alkyl (z. B.
Methyl) substituiert sein kann; und wobei R^{4A} und Ring A* zusammen mit den Atomen, an die sie gebunden sind, eine cyclische Gruppe bilden können,
Ring B* eine optional substituierte Aryl- oder Heteroarylgruppe ist, wobei jedes substituierbare Kohlenstoff- oder Heteroatom in Ring B* optional und unabhängig durch ein oder mehrere R^{3A} substituiert ist;
W und X, von denen eines fehlen kann, unabhängig voneinander ausgewählt sind aus R^{11A}, Hydrocarbyl (z. B. C₁₋₈-Alkyl, Alkenyl, Alkynyl oder Haloalkyl), optional mit R^{11A} substituiert ist, und -(CH₂)ₖ₁-Heterocyclyl optional mit R^{12A} substituiert ist; k₁ 0, 1, 2, 3, 4, 5 oder 6 ist;
R^{1A}, R^{2A}, R^{3A}, R^{4A} und R^{5A} unabhängig voneinander ausgewählt sind aus Wasserstoff, R^{12A}, Hydrocarbyl (z. B. C₁₋₆-Alkyl, Alkenyl, Alkinyl oder Haloalkyl), optional substituiert mit R1^{2A}, und einem - (CH₂)_{L1}-Heterocyclyl, optional substituiert mit einem oder mehreren R^{12A}; wobei R^{1A} und R^{2A} zusammen mit den Atomen, an die sie gebunden sind, einen Heterocyclus bilden können, der optional mit einem oder mehreren R^{12A} substituiert ist, wobei R^{1A} und/oder R^{2A} zusammen mit W oder X einen Heterocyclus bilden können, der optional mit einem oder mehreren R^{12A} substituiert ist; und wobei ein oder mehrere von R^{3A} und R^{5A} zusammen mit den Atomen, an die sie gebunden sind, einen Carbocyclus bilden können, beispielsweise Heterocyclyl, optional substituiert mit R^{12A}; L1 0, 1, 2, 3, 4, 5 oder 6 ist;
wobei:
jedes R^{11A} und R^{12A} unabhängig voneinander ausgewählt ist aus Halogen, Trifluormethyl, Cyano, Thio, Nitro, Oxo, =NR^{13A}, -OR^{13A}, -SR^{13A}, -C(O)R^{13A}, -C(O)OR^{13A}, -OC(O)R^{13A}, -NR^{13A}COR^{14A}, - NR^{13A}CON(R^{13A})₂, -NR^{13A}COR^{14A}, -NR^{13A}CO₂R^{14A}, -S(O)R^{13A}, -S(O)₂R^{13A}, - SON(R^{13A})₂, -NR^{13A}S(O)₂R^{14A}; -CSR^{13A}, -N(R^{13A})R^{14A}, -C(O)N(R^{13A})R^{14A}, - SO₂N(R^{13A})R^{14A} und R^{15A};
R^{13A} und R^{14A} jeweils unabhängig voneinander aus Wasserstoff oder R^{15A} ausgewählt sind;
R^{15A} aus Hydrocarbyl (z. B. C₁₋₆-Alkyl, Alkenyl, Alkinyl oder Haloalkyl), Carbocyclyl und -(CH₂)ₘ₁-Heterocyclyl ausgewählt ist, und jedes R^{15A} optional und unabhängig mit einem oder mehreren von Halogen und Cyano, Amino, Hydroxy, C₁₋₆-Alkyl oder Cycloalkyl und C₁₋₆-Alkoxy substituiert ist;
m₁ 0, 1, 2, 3, 4, 5 oder 6 ist;
p₁ 0,1, 2, 3 oder 4 ist; die Werte von R^{4A} gleich oder unterschiedlich sein können; und
q₁ 0, 1, 2, 3 oder 4 ist; wobei die Werte von R^{5A} gleich oder unterschiedlich sein können;
Y und Z, von denen eines oder beide fehlen können, unabhängig voneinander ausgewählt sind aus Wasserstoff, R^{16A}, Hydrocarbyl (z. B. C₁₋₆-Alkyl, Alkenyl, Alkinyl oder Halogenalkyl), das optional mit R^{16A} substituiert ist, und -(CH₂)ᵣ₁-Heterocyclyl, das optional mit R^{16A} substituiert ist, wobei jedes R^{16A} unabhängig ausgewählt ist aus Halogen, Trifluormethyl, Cyano, Thio, Nitro, Oxo, =NR^{17A}, -OR^{17A}, -SR^{17A}, -C(O)R^{17A}, -C(O)OR^{17A}, -OC(O)R^{17A}, - NR^{17A}COR^{18A}, -NR^{17A}CON(R^{18A})₂, -NR^{17A}COR^{18A}, -NR^{17A}CO₂R^{18A}, -S(O)R^{17A}, - S(O)₂R^{17A}, - SON(R^{17A})₂, -NR^{17A}S(O)₂R^{18A}; -CSR^{17A}, -N(R^{17A})R^{18A}, - C(O)N(R^{17A})R^{18A}, -SO₂N(R^{17A})R^{18A} und R^{19A}; r₁ 0, 1, 2, 3, 4, 5 oder 6 ist;
wobei:
R^{17A} und R^{18A} jeweils unabhängig voneinander aus Wasserstoff oder
R^{19A} ausgewählt sind;
R^{19A} aus Hydrocarbyl (z. B. C₁₋₆-Alkyl, Alkenyl, Alkinyl oder Haloalkyl), Carbocyclyl und -(CH₂)ₛ₁-Heterocyclyl ausgewählt ist, und jedes R^{19A} optional und unabhängig mit einem oder mehreren von Halogen und Cyano, Amino, Hydroxy, C₁₋₆-Alkyl oder Cycloalkyl und C₁₋₆-Alkoxy substituiert ist;
s₁ 0, 1, 2, 3, 4, 5 oder 6 ist.

13. NMT-Inhibitor zur Verwendung nach Anspruch 12, wobei der NMT-Inhibitor eine unten gezeigte Verbindung der Formel (IIa) oder ein pharmazeutisch verträgliches Salz, Hydrat oder Solvat davon ist: 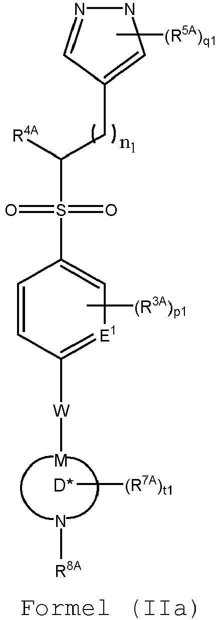 wobei:
n₁ 0 oder 1 ist;
E¹ C ist;
W ein (1-4C)-Kohlenwasserstoff, eine Aryl- (z. B. Phenyl) oder Heteroarylgruppe (z. B. Pyridinyl) ist;
M aus C und N ausgewählt ist;
R^{3A}, R^{4A} und R^{5A} unabhängig voneinander ausgewählt sind aus Wasserstoff, R^{12A} und (1-3C)-Kohlenwasserstoff, optional substituiert mit R^{12A};
R^{12A} unabhängig ausgewählt ist aus Halogen, Trifluormethyl, Cyano, Thio, Nitro, Oxo, -OR^{13A}, -SR^{13A}, -C(O)R^{13A}, -C(O)OR^{13A}, - OC(O)R^{13A}, -NR^{13A}COR^{14A} und R^{15A};
R^{13A} und R^{14A} jeweils unabhängig voneinander aus Wasserstoff oder einem (1-4C)-Kohlenwasserstoff (z. B. Methyl) ausgewählt sind;
Ring D* ein optional substituierter Stickstoff enthaltender 6- oder 7-gliedriger Heterocyclus ist, wobei jeder substituierbare Kohlenstoff oder Stickstoff in Ring D* optional und unabhängig durch ein oder mehrere R^{7A} substituiert ist;
R^{7A} unabhängig ausgewählt ist aus Wasserstoff, (1-4C)-Kohlenwasserstoff, Halogen, Trifluormethyl, Cyano, Thio, Nitro oder Oxo;
R^{8A} ein Wasserstoff oder ein (1-4C)-Kohlenwasserstoff (z. B. Methyl) ist;
p₁ 0, 1 oder 2 ist, wobei die Werte von R^{4A} gleich oder unterschiedlich sein können;
q₁ 3 ist, wobei die Werte von R^{5A} gleich oder unterschiedlich sein können; und
t₁ 0, 1 oder 2 ist, wobei die Werte von R^{7A} gleich oder unterschiedlich sein können.

14. NMT-Inhibitor zur Verwendung nach Anspruch 12 oder 13, wobei der NMT-Inhibitor eine der unten gezeigten Verbindungen oder ein pharmazeutisch verträgliches Salz, Hydrat oder Solvat davon ist:

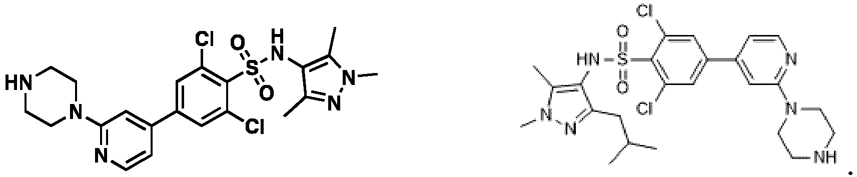

15. NMT-Inhibitor zur Verwendung nach einem der Ansprüche 1 bis 14 in Kombination mit einem oder mehreren anderen therapeutischen Mitteln.

## Revendications

1. Inhibiteur de NMT destiné à être utilisé dans le traitement d'un lymphome diffus à grandes cellules B chez un sujet qui a été identifié comme bénéficiant de l'administration d'un inhibiteur de NMT par un procédé qui comprend l'administration d'une quantité thérapeutiquement efficace d'un inhibiteur de NMT, ou d'un sel, solvate ou hydrate pharmaceutiquement acceptable de celui-ci au sujet, dans lequel ledit sujet ayant été identifié comme bénéficiant de l'administration d'un inhibiteur de NMT tel que déterminé par un procédé comprenant les étapes comprenant :
i) le prélèvement d'un échantillon de cellules de lymphome diffus à grandes cellules B prélevées auprès dudit sujet ;
ii) l'analyse des cellules de l'étape i) pour vérifier la présence d'une ou plusieurs altérations structurelles dans le locus *MYC* (par exemple des réarrangements chromosomiques, des gains en nombre de copies et/ou des mutations de l'oncogène *MYC) ;*
iii) le fait de déterminer si une ou plusieurs altérations structurelles (par exemple des réarrangements chromosomiques, des gains en nombre de copies et/ou des mutations) sont présentes dans le locus *MYC* de l'échantillon de cellules de lymphome diffus à grandes cellules B par rapport à un témoin ; et
iv) le fait de déterminer si le sujet bénéficiera de l'administration d'un inhibiteur de NMT afin de traiter ledit lymphome diffus à grandes cellules B, dans lequel si l'échantillon de cellules de lymphome diffus à grandes cellules B contient une ou plusieurs altérations structurelles (par exemple des réarrangements chromosomiques, des gains en nombre de copies et/ou mutations) dans le locus *MYC,* alors le sujet bénéficiera de l'administration d'un inhibiteur de NMT, et si l'échantillon de cellules de lymphome diffus à grandes cellules B ne contient pas une ou plusieurs altérations structurelles (par exemple, des réarrangements chromosomiques, des gains en nombre de copies et/ou mutations) dans le locus *MYC,* alors le sujet ne bénéficiera pas de l'administration d'un inhibiteur de NMT.

2. Inhibiteur de NMT destiné à être utilisé selon la revendication 1, dans lequel l'une ou les plusieurs altérations structurelles sont choisies parmi des mutations, des gains en nombre de copies et/ou des réarrangements chromosomiques.

3. Inhibiteur de NMT destiné à être utilisé selon la revendication 1 ou 2, dans lequel l'une ou les plusieurs altérations structurelles sont des mutations.

4. Inhibiteur de NMT destiné à être utilisé dans le traitement d'un lymphome diffus à grandes cellules B chez un sujet qui a été identifié comme bénéficiant de l'administration d'un inhibiteur de NMT par un procédé qui comprend l'administration d'une quantité thérapeutiquement efficace d'un inhibiteur de NMT, ou d'un sel, solvate ou hydrate pharmaceutiquement acceptable de celui-ci au sujet, dans lequel ledit sujet ayant été identifié comme bénéficiant de l'administration d'un inhibiteur de NMT tel que déterminé par un procédé comprenant les étapes comprenant :
i) la mesure du niveau d'expression de *MYC* dans un échantillon de cellules de lymphome diffus à grandes cellules B prélevées auprès dudit sujet ;
ii) la comparaison du niveau d'expression de *MYC* de l'étape i) avec un témoin ;
iii) le fait de déterminer si l'expression de *MYC* dans l'échantillon de cellules de lymphome diffus à grandes cellules B est augmentée par rapport au témoin ; et
iv) le fait de déterminer si le sujet bénéficiera de l'administration d'un inhibiteur de NMT afin de traiter ledit lymphome diffus à grandes cellules B, dans lequel si l'expression de *MYC* dans l'échantillon de cellules de lymphome diffus à grandes cellules B est plus élevée que dans le témoin, alors le sujet bénéficiera de l'administration d'un inhibiteur de NMT, et si l'expression de *MYC* dans l'échantillon de cellules de lymphome diffus à grandes cellules B n'est pas plus élevée que dans le témoin, alors le sujet ne bénéficiera pas de l'administration d'un inhibiteur de NMT.

5. Inhibiteur de NMT destiné à être utilisé selon l'une quelconque des revendications 1 à 4, dans lequel l'inhibiteur de NMT est un composé de formule I représenté ci-dessous, ou un sel, hydrate ou solvate pharmaceutiquement acceptable de celui-ci : 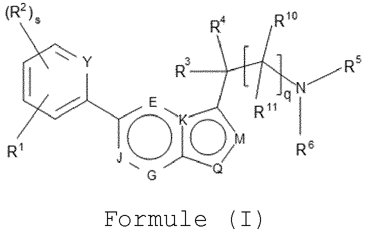 dans laquelle :
Y est choisi dans le groupe constitué de -CH-, -C(R²)- et -N- ;
R¹ est un groupe de formule -X-L-A ;
dans laquelle :
X est choisi dans le groupe constitué de -O-, -N(H)- et -S-, ou est absent ;
L est choisi dans le groupe constitué de - (CHR¹²)ₘ- et - (CHR¹²)ₘO-, ou est absent ;
m vaut 1, 2 ou 3 ; et
A est un carbocycle aromatique à 6 à 10 chaînons ou un hétérocycle aromatique à 5 à 10 chaînons, ledit carbocycle ou hétérocycle aromatique étant éventuellement substitué par 1, 2 ou 3 substituants chacun indépendamment choisi dans le groupe constitué de -F, -Cl, -Br, -OCH₃, -OCF₃, -CN, -alkyle en C₁₋₆ éventuellement substitué par jusqu'à 3 groupes halogène, hydroxyle ou -Oalkyle en C₁₋₄, -S(O)alkyle en C₁₋₄, -S(O)₂alkyle en C₁₋₄, -C(O)N(R⁹)₂, -C(O)N(R¹³)(alkyl en C₁₋₄)Oalkyle en C₁₋₄, - C(O)N((alkyl en C₁₋₄)O(alkyle en C₁₋₄))₂, -CH₂C(O)N(R⁹)₂, - CH₂C(O)N(R¹³)(alkyl en C₁₋₄)Oalkyle en C₁₋₄, - CH₂C(O)N((alkyl en C₁₋₄)O(alkyle en C₁₋₄))₂, -S(O)₂NHalkyle en C₁₋₄, -S(O)₂N(alkyle en C₁₋₄)₂, -NHalkyle en C₁₋₄, -N(alkyle en C₁₋₄)₂, -NHC (O)alkyle en C₁₋₄, -NHC (O)CF₃, -NHS (O)₂alkyle en C₁₋₄, CH₂N(R¹³)₂, CH₂N(R¹³)C(O) alkyle en C₁₋₄, CH₂N(R¹³)S(O)₂alkyle en C₁₋₄,-CH₂S(O)₂alkyle en C₁₋₄ et CO₂H ;
s vaut 0, 1, 2 ou 3 ;
chaque R² est choisi indépendamment dans le groupe constitué de -F, -Cl, -Br, -OCH₃, -OCF₃, -CN, -alkyle en C₁₋₄ éventuellement substitué par jusqu'à 3 groupes halogène ou hydroxyle, - S (O) alkyle en C₁₋₄, -S(O)₂alkyle en C₁₋₄, -S(O)₂NHalkyle en C₁₋₄, - S(O)₂N(alkyle en C₁₋₄)₂, - NHalkyle en C₁₋₄, -N (alkyle en C₁₋₄)₂, - NHC (O) alkyle en C₁₋₄, -NHC(O)CF₃ et -NHS (O)₂alkyle en C₁₋₄ ;
E, J et G sont chacun indépendamment azote ou C(R⁷) ;
K est carbone ou azote ;
et dans laquelle :
i) lorsque K est un carbone, soit Q est N(R⁸) et M est un azote ou C(R⁷), ou Q est un azote et M est N(R⁸) ; ou
ii) lorsque K est un azote, Q est un azote ou C(R⁷) et M est un azote ou C(R⁷) ;
et en outre dans laquelle au moins 2 parmi E, J, G, K, Q et M sont choisis dans le groupe constitué de carbone et C(R⁷) ;
q est 0 ou 1 ;
R³ est hydrogène ou méthyle ; R⁴ est hydrogène ou méthyle ;
R⁵ est hydrogène ou alkyle en C₁₋₆ éventuellement substitué par jusqu'à 3 groupes -F, -Cl, -Br, -OH, -OCH₃, -OCF₃ ou -CN ;
R⁶ est hydrogène ou alkyle en C₁₋₆ éventuellement substitué par jusqu'à 3 groupes -F, -Cl, -Br, -OH, -OCH₃, -OCF₃ ou -CN ;
ou les groupes R⁵ et R⁶ et le N auxquels ils sont liés forment un hétérocycle non aromatique à 4 à 7 chaînons, l'hétérocycle comprenant éventuellement 1 ou 2 autres hétéroatomes choisis parmi N, O et S, éventuellement substitués par jusqu'à 3 groupes -F, -Cl, -Br, -OH, -OCH₃, -OCF₃ ou -CN ;
lorsqu'il est présent, R¹⁰ est hydrogène ou méthyle ;
lorsqu'il est présent, R¹¹ est hydrogène ou méthyle ;
ou le groupe R³ et le groupe R⁵ et les atomes intervenants forment un hétérocycle non aromatique à 3 à 7 chaînons composé des atomes et de la liaison intervenants, ou des atomes intervenants et - (CHR^{a})ᵣ- ;
ou le groupe R¹⁰ et le groupe R⁵ et les atomes intervenants forment un hétérocycle non aromatique à 3 à 7 chaînons composé des atomes intervenants et -(CHR^{a})ᵣ- ;
r vaut 1, 2, 3, 4 ou 5 ; R^{a} est hydrogène ou méthyle ;
chaque R⁷ est indépendamment sélectionné dans le groupe constitué de hydrogène, halogène, alcoxy en C₁₋₄ et alkyle en C₁₋₄ éventuellement substitué par 1, 2 ou 3 halogènes ; et
R⁸ est choisi dans le groupe choisi parmi hydrogène et alkyle en C1-4 ;
chaque R⁹ est choisi indépendamment dans le groupe constitué de hydrogène et alkyle en C₁₋₄, ou deux groupes R⁹ et le N auquel ils sont liés forment un hétérocycle non aromatique à 4 à 7 chaînons, l'hétérocycle comprenant éventuellement 1 ou 2 autres hétéroatomes choisis parmi N, O et S ;
chaque R¹² est choisi indépendamment dans le groupe constitué de hydrogène, alkyle en C₁₋₆ éventuellement substitué par jusqu'à 3 groupes -F, -Cl, -Br, I, -OH, -OCH₃, -OCF₃ ou -CN, alcényle en C₁₋₆ éventuellement substitué par jusqu'à 3 groupes -F, -Cl, - Br, I, -OH, -OCH₃, -OCF₃ ou -CN, et alcynyle en C₁₋₆ éventuellement substitué par jusqu'à 3 groupes -F, -Cl, -Br, I, -OH, -OCH₃, - OCF₃ ou -CN ; et chaque R¹³ est choisi indépendamment dans le groupe constitué de hydrogène et alkyle en C₁₋₄.

6. Inhibiteur de NMT destiné à être utilisé selon l'une quelconque des revendications 1 à 5, dans lequel l'inhibiteur de NMT est un composé de formule (IA^^) représenté ci-dessous, ou un sel, hydrate ou solvate pharmaceutiquement acceptable de celui-ci : 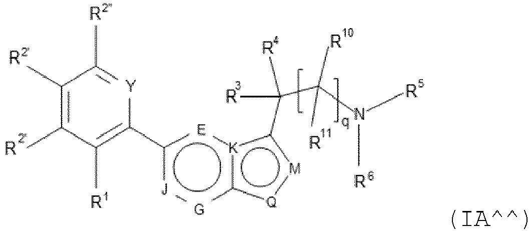 dans laquelle :
R¹ est un groupe de formule -X-L-A ;
A est 4-pyrazolyle, ledit pyrazolyle étant éventuellement substitué par jusqu'à 3 groupes méthyle ;
X est -O- ou absent ;
L est -(CH₂)ₘ- ou -(CH₂)ₘ-O- ;
m vaut 2 ;
R^{2'} est choisi dans le groupe constitué de fluor, chlore -CN et
méthyle (de préférence fluor) ;
R^{2"} est choisi dans le groupe constitué de hydrogène, fluor, chlore, -CN et méthyle ;
q est 0 ou 1 ;
R³ est hydrogène ou méthyle ;
R⁴ est hydrogène ou méthyle ;
R⁵ est hydrogène ou méthyle ;
R⁶ est hydrogène ou méthyle ; ou
le groupe R³ et le groupe R⁵ et les atomes intervenants forment un hétérocycle non aromatique à 3 à 7 chaînons composé des atomes et des liaisons intervenants (plus préférablement R⁵ et R⁶ sont tous deux méthyle) ;
R⁷ lorsqu'il est présent est hydrogène ou méthyle ;
R⁸ lorsqu'il est présent, est hydrogène ou méthyle ;
R⁹ et R¹⁰ lorsqu'ils sont présents sont hydrogène ou méthyle ; et
E, J, G, K, Q et M sont :
i) E, J et G sont chacun C(R⁷), K est carbone, Q est N(R⁸), M est azote ; et R⁸ est hydrogène ou méthyle ;
ii) E, J et G sont chacun C(R⁷), et K, Q et M sont chacun azote ;
iii) E et G sont chacun C(R⁷), et J, K, Q et M sont chacun azote ;
iv) J et G sont chacun C(R⁷), et E, K, Q et M sont chacun azote ; ou
v) E, J, G et M sont chacun C(R⁷), et K et Q sont chacun azote.

7. Inhibiteur de NMT destiné à être utilisé selon l'une quelconque des revendications 1 à 6, dans lequel l'inhibiteur de NMT est un composé de formule (IA^^) représenté ci-dessous, ou un sel, hydrate ou solvate pharmaceutiquement acceptable de celui-ci : 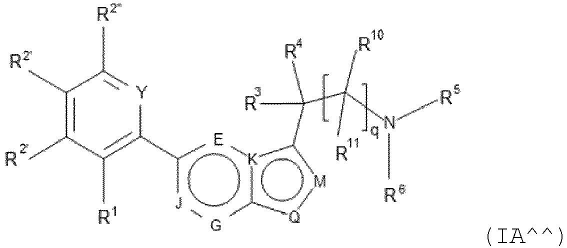 dans laquelle :
R¹ est un groupe de formule -X-L-A ;
A est 4-pyrazolyle, ledit pyrazolyle étant éventuellement substitué par jusqu'à 3 groupes substituants choisis parmi méthyle et -C(O)N(CH₃)₂ ;
X est -O- ou absent ;
L est -(CH₂)ₘ- ou -(CH₂)ₘ-O- ;
m vaut 2 ;
R^{2'} est choisi dans le groupe constitué de fluor, chlore (de préférence fluor) ;
R^{2"} est choisi dans le groupe constitué de hydrogène, fluor ou chlore ;
q vaut 0 ;
R³ est hydrogène ou méthyle ;
R⁴ est hydrogène ou méthyle ;
R⁵ est hydrogène ou méthyle ;
R⁶ est hydrogène ou méthyle ; ou
le groupe R³ et le groupe R⁵ et les atomes intervenants forment un hétérocycle non aromatique à 3 à 7 chaînons composé des atomes et des liaisons intervenants (plus préférablement R⁵ et R⁶ sont tous deux méthyle) ;
E, J, G, K, Q et M sont :
i) E, J et G sont chacun CH, K est carbone, Q est N(R⁸), M est azote ; et R⁸ est hydrogène ou méthyle ; ou
ii) E, J, G et M sont chacun CH, et K et Q sont chacun azote ;
à condition que A ne soit pas substitué par plus d'un groupe - C(O)N(CH3)₂.

8. Inhibiteur de NMT destiné à être utilisé selon l'une quelconque des revendications 1 à 7, dans lequel l'inhibiteur de NMT est un composé choisi parmi : 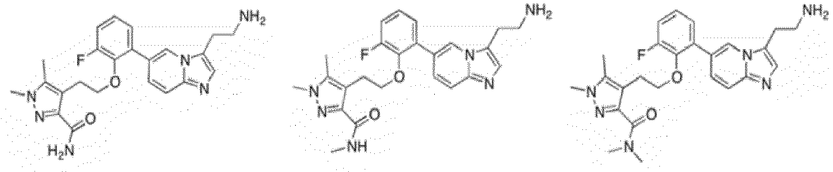 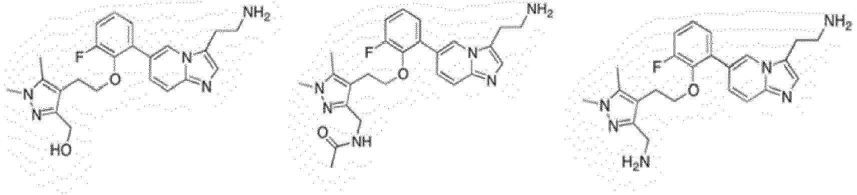 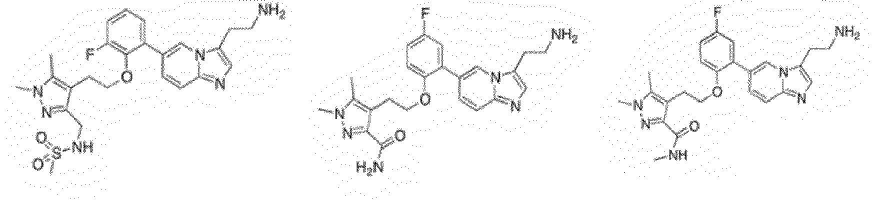 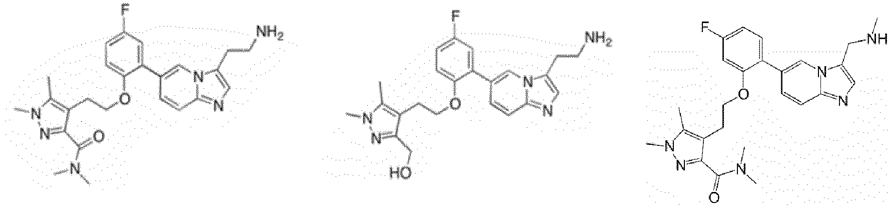 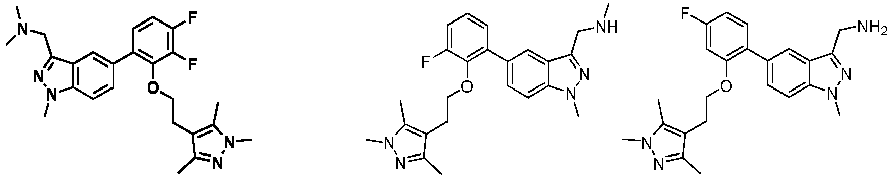 ou un sel, hydrate ou solvate pharmaceutiquement acceptable de celui-ci.

9. Inhibiteur de NMT destiné à être utilisé selon l'une quelconque des revendications 1 à 8, dans lequel l'inhibiteur de NMT est le composé représenté ci-dessous, ou un sel, hydrate ou solvate pharmaceutiquement acceptable de celui-ci : 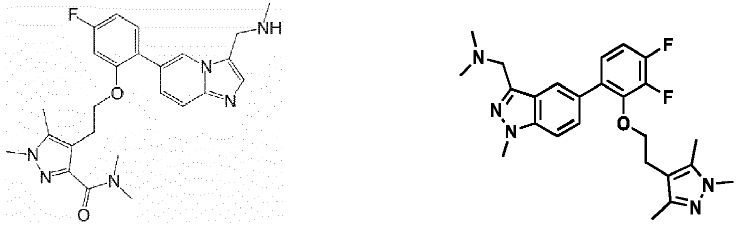 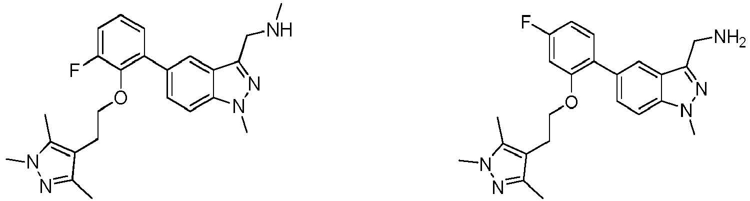 ou un sel, hydrate ou solvate pharmaceutiquement acceptable de celui-ci.

10. Inhibiteur de NMT destiné à être utilisé selon l'une quelconque des revendications 1 à 4, dans lequel l'inhibiteur de NMT est un composé de formule (Id) représenté ci-dessous, ou un sel, hydrate ou solvate pharmaceutiquement acceptable de celui-ci : 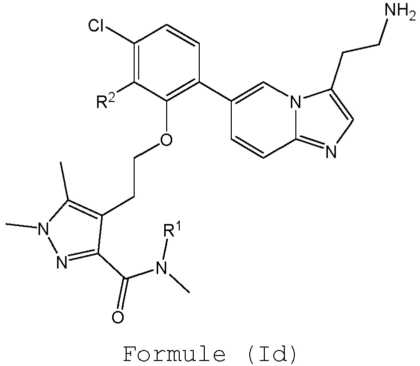 dans laquelle :
R¹ est H ou -CH₃ ; et
R² est H ou F.

11. Inhibiteur de NMT destiné à être utilisé selon la revendication 10, dans lequel l'inhibiteur de NMT est le composé représenté ci-dessous, ou un sel, hydrate ou solvate pharmaceutiquement acceptable de celui-ci :

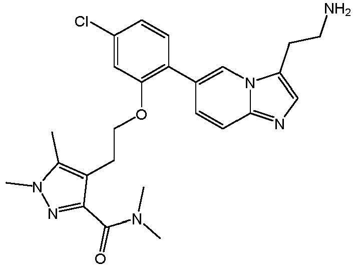

12. Inhibiteur de NMT destiné à être utilisé selon l'une quelconque des revendications 1 à 4, dans lequel l'inhibiteur de NMT est un composé de formule (II) ou de formule (III) représenté ci-dessous, ou un sel, hydrate ou solvate pharmaceutiquement acceptable de celui-ci : 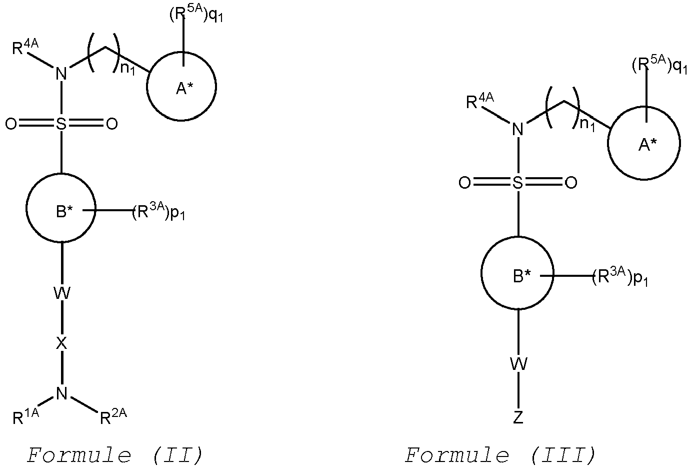 dans lesquelles :
n₁ est 0, 1, 2, 3, 4, 5 ou 6 ;
le Cycle A* est un groupe aryle contenant de l'azote éventuellement substitué dans lequel chaque carbone ou azote substituable dans le Cycle A* est éventuellement et indépendamment substitué par un ou plusieurs R^{5A} et dans lequel si le Cycle A* contient un groupement -NH-, cet azote peut être éventuellement substitué par alkyle en C₁₋₆ (par exemple méthyle) ; et dans lequelles R^{4A} et le Cycle A* conjointement avec les atomes auxquels ils sont attachés peuvent former un groupe cyclique,
le Cycle B* est un groupe aryle ou hétéroaryle éventuellement substitué dans lequel chaque carbone ou hétéroatome substituable dans le Cycle B* est éventuellement et indépendamment substitué par un ou plusieurs R^{3A} ;
W et X, dont l'un peut être absent, sont choisis indépendamment parmi R^{11A}, hydrocarbyle (par exemple alkyle, alcényle, alcynyle ou halogénoalkyle en C₁₋₈) éventuellement substitué par R^{11A}, et - (CH₂)ₖ₁-hétérocyclyle éventuellement substitué par R^{12A} ; k₁ vaut 0, 1, 2, 3, 4, 5 ou 6 ;
R^{1A}, R^{2A}, R^{3A}, R^{4A} et R^{5A} sont choisis indépendamment parmi hydrogène, R^{12A}, hydrocarbyle (par exemple alkyle, alcényle, alcynyle ou halogénoalkyle en C₁₋₆) éventuellement substitué par R^{12A}, et un - (CH₂)_{L1}-hétérocyclyle éventuellement substitué par un ou plusieurs R^{12A} ; dans lesquelles R^{1A} et R^{2A} pris ensemble avec les atomes auxquels ils sont attachés peuvent former un hétérocycle, éventuellement substitué par un ou plusieurs R^{12A}, dans lesquelles R^{1A} et/ou R^{2A} pris ensemble avec W ou X peuvent former un hétérocycle éventuellement substitué avec un ou plusieurs R^{12A} ; et dans lesquelles un ou plusieurs parmi R^{3A} et R^{5A} pris ensemble avec les atomes auxquels ils sont attachés peuvent former un carbocycle, par exemple hétérocyclyle, éventuellement substitué par R^{12A} ; L₁ vaut 0, 1, 2, 3, 4, 5 ou 6 ;
dans lesquelles :
chaque R^{11A} et R^{12A} est indépendamment choisi parmi halogène, trifluorométhyle, cyano, thio, nitro, oxo, =NR^{13A}, -OR^{13A}, -SR^{13A}, -C(O)R^{13A}, -C(O)OR^{13A}, -OC(O)R^{13A}, -NR^{13A}COR^{14A}, -NR^{13A}CON(R^{13A})₂, -NR^{13A}COR^{14A}, -NR^{13A}CO₂R^{14A}, -S(O)R^{13A}, -S(O)₂R^{13A}, -SON(R^{13A})₂, - NR^{13A}S(O)₂R^{14A}; -CSR^{13A}, -N(R^{13A})R^{14A}, -C(O)N(R^{13A})R^{14A}, -SO₂N(R^{13A})R^{14A} et R^{15A} ;
R^{13A} et R^{14A} sont chacun indépendamment choisis parmi hydrogène ou R^{15A} ;
R^{15A} est choisi parmi hydrocarbyle (par exemple alkyle, alcényle, alcynyle ou halogénoalkyle en C₁₋₆), carbocyclyle et - (CH₂)ₘ₁-hétérocyclyle, et chaque R^{15A} est éventuellement et indépendamment substitué par un ou plusieurs halogène, cyano , amino, hydroxy, alkyle ou cycloalkyle en C₁₋₆ et alcoxy en C₁₋₆ ;
m₁ est 0, 1, 2, 3, 4, 5 ou 6 ;
p₁ vaut 0, 1, 2, 3 ou 4 ; les valeurs de R^{4A} peuvent être identiques ou différentes ; et
q₁ vaut 0, 1, 2, 3 ou 4 ; dans lesquelles les valeurs de R^{5A} peuvent être identiques ou différentes ;
Y et Z, dont l'un ou les deux peuvent être absents, sont choisis indépendamment parmi hydrogène, R^{16A}, hydrocarbyle (par exemple alkyle, alcényle, alcynyle ou halogénoalkyle en C₁₋₆) éventuellement substitué par R^{16A}, et - (CH₂)ᵣ₁-hétérocyclyle éventuellement substitué par R^{16A}, dans lesquelles chaque R^{16A} est choisi indépendamment parmi halogène, trifluorométhyle, cyano, thio, nitro, oxo, =NR^{17A}, -OR^{17A}, -SR^{17A}, -C(O)R^{17A}, -C(O)OR^{17A}, - OC(O)R^{17A}, -NR^{17A}COR^{18A}, -NR^{17A}CON(R^{18A})₂, -NR^{17A}COR^{18A}, -NR^{17A}CO₂R^{18A}, - S(O)R^{17A}, -S(O)₂R^{17A}, -SON(R^{17A})₂, -NR^{17A}S(O)₂R^{18A}; -CSR^{17A}, - N(R^{17A})R^{18A}, -C(O)N(R^{17A})R^{18A}, -SO₂N(R^{17A})R^{18A} et R^{19A} ; r₁ vaut 0, 1, 2, 3, 4, 5 ou 6 ;
dans lesquelles :
R^{17A} et R^{18A} sont chacun indépendamment choisis parmi hydrogène ou R^{19A} ;
R^{19A} est choisi parmi hydrocarbyle (par exemple alkyle, alcényle, alcynyle ou halogénoalkyle en C₁₋₆), carbocyclyle et - (CH₂)ₛ₁-hétérocyclyle, et chaque R^{19A} est éventuellement et indépendamment substitué par un ou plusieurs halogène, cyano, amino, hydroxy, alkyle ou cycloalkyle en C₁₋₆ et alcoxy en C₁₋₆ ; s₁ vaut 0, 1, 2, 3, 4, 5 ou 6.

13. Inhibiteur de NMT destiné à être utilisé selon la revendication 12, dans lequel l'inhibiteur de NMT est un composé (IIa) représenté ci-dessous, ou un sel, hydrate ou solvate pharmaceutiquement acceptable de celui-ci : 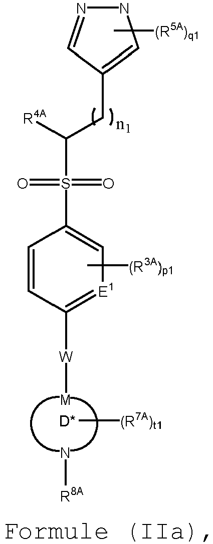 dans laquelle :
n₁ est 0 ou 1 ;
E¹ est C ;
W est un hydrocarbyle (en C1-4), un groupe aryle (par exemple phényle) ou hétéroaryle (par exemple pyridinyle) ;
M est choisi parmi C et N ;
R^{3A}, R^{4A} et R^{5A} sont choisis indépendamment parmi hydrogène, R^{12A} et hydrocarbyle (en C1-3) éventuellement substitué par R^{12A} ;
R^{12A} est choisi indépendamment parmi halogène, trifluorométhyle, cyano, thio, nitro, oxo, -OR^{13A}, -SR^{13A}, -C(O)R^{13A}, -C(O)OR^{13A}, - OC(O)R^{13A}, -NR^{13A}COR^{14A} et R^{15A} ;
R^{13A} et R^{14A} sont choisis chacun indépendamment parmi hydrogène ou hydrocarbyle (en C1-4) (par exemple méthyle) ;
le Cycle D* est un hétérocycle à 6 ou 7 chaînons contenant de l'azote éventuellement substitué, dans lequel chaque carbone ou azote substituable dans le Cycle D* est éventuellement et indépendamment substitué par un ou plusieurs R^{7A} ;
R^{7A} est choisi indépendamment parmi hydrogène, hydrocarbyle (en C1-4), halogène, trifluorométhyle, cyano, thio, nitro ou oxo ;
R^{8A} est hydrogène ou un hydrocarbyle (en C1-4) (par exemple méthyle) ;
p₁ est 0, 1 ou 2, dans laquelle les valeurs de R^{4A} peuvent être identiques ou différentes ;
q₁ vaut 3, dans laquelle les valeurs de R^{5A} peuvent être identiques ou différentes ; et
t₁ vaut 0, 1 ou 2; dans laquelle les valeurs de R^{7A} peuvent être identiques ou différentes.

14. Inhibiteur de NMT destiné à être utilisé selon la revendication 12 ou 13, dans lequel l'inhibiteur de NMT est l'un des composés représentés ci-dessous, ou un sel, hydrate ou solvate pharmaceutiquement acceptable de celui-ci :

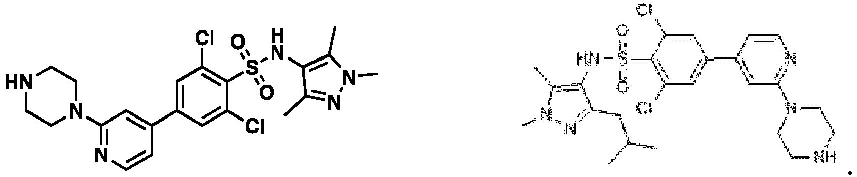

15. Inhibiteur de NMT destiné à être utilisé selon l'une quelconque des revendications 1 à 14 en combinaison avec un ou plusieurs autres agents thérapeutiques.
